# EUROPÄISCHE PATENTSCHRIFT

(11) **EP 2 049 531 B1**
(45) Veröffentlichungstag und Bekanntmachung des Hinweises auf die Patenterteilung: **24.09.2014**
(21) Anmeldenummer: 07786018.7
(22) Anmeldetag: 12.07.2007
(51) Int. Cl.: C07D 413/10, C07D 413/14, C07D 417/10, C07D 417/14, A61K 31/41

(54) **2-(HETEROCYCLYLBENZYL)-PYRIDAZINONDERIVATE**
2-(HETEROCYCLYLBENZYL)PYRIDAZINONE DERIVATIVES
DÉRIVÉS DE 2-(HÉTÉROCYCLYLBENZYL)-PYRIDAZINONE

(30) Priorität: 10.08.2006 DE 102006037478
(43) Veröffentlichungstag der Anmeldung: 22.04.2009
(73) Patentinhaber: Merck Patent GmbH, 64293 Darmstadt (DE)
(72) Erfinder: DORSCH, Dieter, 64372 Ober-Ramstadt (DE); SCHADT, Oliver, 63517 Rodenbach (DE); BLAUKAT, Andree, 69198 Schriesheim (DE); STIEBER, Frank, 69121 Heidelberg (DE)
(86) Internationale Anmeldenummer: PCT/EP2007/006186
(87) Internationale Veröffentlichungsnummer: WO 2008/017361

(56) Entgegenhaltungen:
- EP-A- 1 043 317
- WO-A-03/037349
- WO-A-2005/042520

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der Tyrosinkinasen und/oder Serin/Threonin-Kinasen eine Rolle spielt, ferner pharmazeutische Zusammensetzungen, die diese Verbindungen enthalten, sowie die Verwendung der Verbindungen zur Behandlung kinasebedingter Krankheiten.

Die vorliegende Erfindung betrifft insbesondere Verbindungen und die Verwendung von Verbindungen, bei denen die Hemmung, Regulierung und/oder Modulation der Signaltransduktion von Met-Kinase eine Rolle spielt.

Einer der Hauptmechanismen, durch den die Zellregulation bewirkt wird, ist durch die Transduktion der extrazellulären Signale über die Membran, die wiederum biochemische Wege in der Zelle modulieren. Protein-Phosphorylierung stellt einen Ablauf dar, über den intrazelluläre Signale von Molekül zu Molekül propagiert werden, was schließlich in einer Zellantwort resultiert. Diese Signaltransduktionskaskaden sind hoch reguliert und überlappen häufig, wie aus dem Vorliegen vieler Proteinkinasen wie auch Phosphatasen hervorgeht. Phosphorylierung von Proteinen tritt vorwiegend bei Serin-, Threonin- oder Tyrosinresten auf, und Proteinkinasen wurden deshalb nach ihrer Spezifität des Phosporylierungsortes, d. h. der Serin-/ Threonin-Kinasen und Tyrosin-Kinasen klassifiziert. Da Phosphorylierung ein derartig weit verbreiteter Prozess in Zellen ist und da Zellphänotypen größtenteils von der Aktivität dieser Wege beeinflusst werden, wird zur Zeit angenommen, dass eine Anzahl von Krankheitszuständen und/oder Erkrankungen auf entweder abweichende Aktivierung oder funktionelle Mutationen in den molekularen Komponenten von Kinasekaskaden zurückzuführen sind. Folglich wurde der Charakterisierung dieser Proteine und Verbindungen, die zur Modulation ihrer Aktivität fähig sind, erhebliche Aufmerksamkeit geschenkt (Übersichtsartikel siehe: Weinstein-Oppenheimer et al. Pharma. &. Therap., 2000, 88, 229-279).

Die Rolle der Rezeptortyrosinkinase Met bei der menschlichen Onkogenese, sowie die Möglichkeit der Inhibierung der HGF(hepatocycte growth factor)-abhängigen Met-Aktivierung wird von S. Berthou et al. in Oncogene, Vol. 23, Nr. 31, Seiten 5387-5393 (2004) beschrieben. Der dort beschriebene Inhibitor SU11274, eine Pyrrol-Indolin-Verbindung, ist potentiell zur Krebsbekämpfung geeignet.
Ein anderer Met-Kinase-Inhibitor zur Krebstherapie ist von J.G. Christensen et al. in Cancer Res. 2003, 63(21), 7345-55 beschrieben. Von einem weiterem Tyrosinkinase-Inhibitor zur Krebsbekämpfung berichten H. Hov et al. in Clinical Cancer Research Vol. 10, 6686-6694 (2004). Die Verbindung PHA-665752, ein Indolderivat, ist gegen den HGF-Rezeptor c-Met gerichtet. Weiter wird dort berichtet, daß HGF und Met erheblich zum malignen Prozess verschiedener Krebsformen, wie z.B. multipler Myeloma, betragen.

Die Synthese von kleinen Verbindungen, die die Signaltransduktion der Tyrosinkinasen und/oder Serin/Threonin-Kinasen, insbesondere der Met-Kinase spezifisch hemmen, regulieren und/oder modulieren, ist daher wünschenswert und ein Ziel der vorliegenden Erfindung.

Es wurde gefunden, daß die erfindungsgemäßen Verbindungen und ihre Salze bei guter Verträglichkeit sehr wertvolle pharmakologische Eigenschaften besitzen.

Im einzelnen betrifft die vorliegende Erfindung Verbindungen der Formel I, die die Signaltransduktion der Met-Kinase hemmen, regulieren und/oder modulieren, Zusammensetzungen, die diese Verbindungen enthalten, sowie Verfahren zu ihrer Verwendung zur Behandlung von Met-Kinasebedingten Krankheiten und Leiden wie Angiogenese, Krebs, Tumorentstehung, -wachstum und -verbreitung, Arteriosklerose, Augenerkrankungen, wie altersbedingte Makula-Degeneration, choroidale Neovaskularisierung und diabetische Retinopathie, Entzündungserkrankungen, Arthritis, Thrombose, Fibrose, Glomerulonephritis, Neurodegeneration, Psoriasis, Restenose, Wundheilung, Transplantatabstossung, metabolische und Erkrankungen des Immunsystems, auch Autoimmunerkrankungen, Zirrhose, Diabetes und Erkrankungen der Blutgefässe, dabei auch Instabilität und Durchlässigkeit (Permeabilität) und dergleichen bei Säugetieren.

Feste Tumore, insbesondere schnell wachsende Tumore, können mit Met-Kinasehemmern behandelt werden. Zu diesen festen Tumoren zählen die Monozytenleukämie, Hirn-, Urogenital-, Lymphsystem-, Magen-, Kehlkopf- und Lungenkarzinom, darunter Lungenadenokarzinom und kleinzelliges Lungenkarzinom.

Die vorliegende Erfindung richtet sich auf Verfahren zur Regulation, Modulation oder Hemmung der Met-Kinase zur Vorbeugung und/oder Behandlung von Erkrankungen im Zusammenhang mit unregulierter oder gestörter Met-Kinase-Aktivität. Insbesondere lassen sich die Verbindungen der Formel I auch bei der Behandlung gewisser Krebsformen einsetzen. Weiterhin können die Verbindungen der Formel I verwendet werden, um bei gewissen existierenden Krebschemotherapien additive oder synergistische Effekte bereitzustellen, und/oder können dazu verwendet werden, um die Wirksamkeit gewisser existierender Krebschemotherapien und -bestrahlungen wiederherzustellen.

Weiterhin können die Verbindungen der Formel I zur Isolierung und zur Untersuchung der Aktivität oder Expression von Met-Kinase verwendet werden. Außerdem eigenen sie sich insbesondere zur Verwendung in diagnostischen Verfahren zu Erkrankungen im Zusammenhang mit unregulierter oder gestörter Met-Kinase-Aktivität.

Es kann gezeigt werden, dass die erfindungsgemäßen Verbindungen in einem Xenotransplantat-Tumor-Modell eine in vivo antiproliferative Wirkung aufweisen. Die erfindungsgemäßen Verbindungen werden an einen Patienten mit einer hyperproliferativen Erkrankung verabreicht, z. B. zur Inhibition des Tumorwachstums, zur Verminderung der mit einer lymphoproliferativen Erkrankung einhergehenden Entzündung, zur Inhibition der Transplantatabstoßung oder neurologischer Schädigung aufgrund von Gewebereparatur usw. Die vorliegenden Verbindungen sind nützlich für prophylaktische oder therapeutische Zwecke. Wie hierin verwendet, wird der Begriff "Behandeln" als Bezugnahme sowohl auf die Verhinderung von Krankheiten als auch die Behandlung vorbestehender Leiden verwendet. Die Verhinderung von Proliferation wird durch Verabreichung der erfindungsgemäßen Verbindungen vor Entwicklung der evidenten Krankheit, z. B. zur Verhinderung des Tumorwachstums, Verhinderung metastatischen Wachstums, der Herabsetzung von mit kardiovaskulärer Chirurgie einhergehenden Restenosen usw. erreicht. Als Alternative werden die Verbindungen zur Behandlung andauernder Krankheiten durch Stabilisation oder Verbesserung der klinischen Symptome des Patienten verwendet.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).
Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J.).

Es gibt viele mit einer Deregulation der Zellproliferation und des Zelltods (Apoptose) einhergehende Erkrankungen. Die Leiden von Interesse schließen die folgenden Leiden ein, sind aber nicht darauf beschränkt. Die erfindungsgemäßen Verbindungen sind nützlich bei der Behandlung einer Reihe verschiedener Leiden, bei denen Proliferation und/oder Migration glatter Muskelzellen und/oder Entzündungszellen in die Intimaschicht eines Gefäßes vorliegt, resultierend in eingeschränkter Durchblutung dieses Gefäßes, z. B. bei neointimalen okklusiven Läsionen. Zu okklusiven Transplantat-Gefäßerkrankungen von Interesse zählen Atherosklerose, koronare Gefäßerkrankung nach Transplantation, Venentransplantatstenose, peri-anastomotische Prothesenrestenose, Restenose nach Angioplastie oder Stent-Platzierung und dergleichen.

### STAND DER TECHNIK

Dihydropyridazinone zur Krebsbekämpfung sind in WO 03/037349 A1 beschrieben.
Andere Pyridazine zur Behandlung von Krankheiten des Immunsystems, ischämischer und entzündlicher Erkrankungen kennt man aus EP 1 043 317 A1 und EP 1 061 077 A1.
In EP 0 738 716 A2 und EP 0 711 759 B1 sind andere Dihydropyridazinone und Pyridazinone als Fungizide und Insektizide beschrieben. Andere Pyridazinone sind als cardiotonische Agenzien in US 4,397,854 beschrieben.
In JP 57-95964 sind andere Pyridazinone offenbart.

### ZUSAMMENFASSUNG DER ERFINDUNG

Die Erfindung betrifft Verbindungen der Formel I 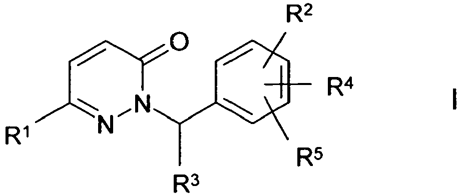 worin
- R¹: Ar oder Het,
- R²: einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, NO₂, CN, COOR³, CON(R³)₂, NR³COA, NR³SO₂A, SO₂N(R³)₂, S(O)ₘA, Het¹, -(C(R³)₂]ₙN(R³)₂, -[C(R³)₂]ₙHet¹, O[C(R³)₂]ₙN(R³)₂, O[C(R³)₂]ₙHet¹, S[C(R³)₂]ₙN(R³)₂, S[C(R₃)₂]ₙHet¹, -NR³[C(R³)₂]ₙN(R³)₂, -NR³[C(R³)₂]ₙHet¹, -NR³Het¹, NHCON(R³)₂, NHCONH[C(R³)₂]ₙN(R³)₂, NHCONH[C(R³)₂]ₙHet¹, CON(R³)₂, ONR³[C(R³)₂]ₙN(R³)₂, CONR³[C(R³)₂]ₙHet¹, COHet¹, COA und/oder =O (Carbonylsauerstoff) substituiert sein kann,
- R³: H oder A,
- R⁴, R⁵: jeweils unabhängig voneinander H, Hal, A, OR³, CN, COOR³, CON(R³)₂, NR³COA, NR³SO₂A, SO₂N(R³)₂ oder S(O)ₘA,
- A: unverzweigtes oder verzweigtes Alkyl mit 1-10 C-Atomen,
worin 1-7 H-Atome durch F, Cl und/oder Br ersetzt sein können,
und/oder worin eine oder zwei CH₂-Gruppen durch O, S, SO, SO₂ und/oder CH=CH-Gruppen ersetzt sein können, oder
cyclisches Alkyl mit 3-7 C-Atomen,
- Ar: unsubstituiertes oder ein-, zwei- oder dreifach durch Hal, A, OR³, N(R³)₂, SR³, NO₂, CN, COOR³, CON(R³)₂, NR³COA, NR³SO₂A, SO₂N(R³)₂, S(O)ₘA, CO-Het¹, Het¹, O[C(R³)₂]ₙN(R³)₂, O[C(R³)₂]ₙHet¹, NHCOOA, NHCON(R³)₂,
NHCOO[C(R³)₂]ₙN(R³)₂, NHCOO[C(R³⁾₂]ₙHet¹, NHCONH[C(R³)₂]ₙN(R³)₂, NHCONH[C(R³)₂]ₙHet¹, OCONH[C(R³)₂]ₙN(R3)₂, OCONH[C(R³)₂]ₙHet¹ und/oder COA substituiertes Phenyl, Naphthyl oder Biphenyl,
- Het: einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR³, N(R³)₂, SR³, NO₂, CN, COOR³, CON(R³)₂, NR³COA, NR³SO₂A, SO₂N(R³)₂, S(O)ₘA, CO-Het¹, Het¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹, CO-Het¹, CHO, COA, =S, =NH, =NA und/oder =O (Carbonylsauerstoff) substituiert sein kann,
- Het¹: einen einkernigen gesättigten oder aromatischen Heterocyclus mit 1 bis 2 N und/oder O-Atomen, der ein- oder zweifach durch A, OA, OH, Hal, (CH₂)ₙN(R³)₂, (CH₂)ₙOR³, (CH₂)ₙHet² und/oder =O (Carbonylsauerstoff) substituiert sein kann,
- Het²: Pyrrolidino, Piperidino oder Morpholino,
- Hal: F, Cl, Br oder I,
- m: 0, 1 oder 2,
- n: 1, 2, 3 oder 4
bedeuten,
sowie ihre pharmazeutisch verwendbaren Solvate, Salze, Tautomere und Stereoisomere, einschließlich deren Mischungen in allen Verhältnissen,

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.
Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.
Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der Verbindungen der Formel I, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I nach den Ansprüchen 1-11 sowie ihrer pharmazeutisch verwendbaren Salze, Solvate, Tautomeren und Stereoisomeren, dadurch gekennzeichnet, daß man
a) eine Verbindung der Formel II 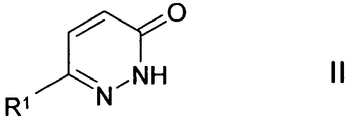 worin R¹ die in Anspruch 1 angegebene Bedeutung hat,
   mit einer Verbindung der Formel III 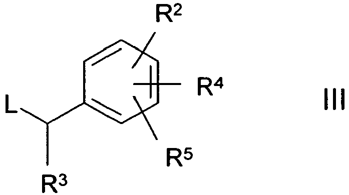 worin R², R³, R⁴ und R⁵ die in Anspruch 1 angegebenen Bedeutungen haben und
   L Cl, Br, I oder eine freie oder reaktionsfähig funktionell abgewandelte OH-Gruppe bedeutet,
      umsetzt,
   oder
b) einen Rest R² in einen anderen Rest R² umwandelt, indem man
   i) eine Aminogruppe acyliert oder alkyliert,
   ii) ein Oxy-amidinderivat zu einem Oxadiazolderivat cyclisiert,
   iii) ein Amidderivat zu einem Oxazolderivat cyclisiert,
   iv) ein Alkylesterderivat mit einem N-Hydroxyamidinderivat zu einem Oxadiazolderivat umsetzt,
   v) ein N-(Aminothiocarbonyl)-hydrazidderivat zu einem Oxadiazolderivat cyclisiert,
   vi) eine SH-Gruppe alkyliert,
   vii) eine Cyangruppe mit einem Azidderivat zu einem Tetrazolderivat umsetzt
   oder
c) daß man sie aus einem ihrer funktionellen Derivate durch Behandeln mit einem solvolysierenden oder hydrogenolysierenden Mittel in Freiheit setzt,
und/oder
eine Base oder Säure der Formel I in eines ihrer Salze umwandelt.

Vor- und nachstehend haben die Reste R¹, R², R³, R⁴ und R⁵ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
Cyclisches Alkyl (Cycloalkyl) bedeutet vorzugsweise Cyclopropyl, Cyclobutyl, Cylopentyl, Cyclohexyl oder Cycloheptyl.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl, o-, m- oder p-Aminophenyl, o-, m- oder p-(N-Methylamino)-phenyl, o-, m- oder p-(N-Methylaminocarbonyl)-phenyl, o-, m- oder p-Acetamidophenyl, o-, m- oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p- (N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m- oder p-Bromphenyl, o-, m- oder p- Chlorphenyl, o-, m- oder p-(Methyl-sulfonamido)-phenyl, o-, m- oder p-(Methylsulfonyl)-phenyl, o-, m- oder p-Methylsulfanylphenyl, o-, m- oder p-Cyanphenyl, o-, m- oder p-Carboxyphenyl, o-, m- oder p-Methoxycarbonylphenyl, o-, m- oder p-Formylphenyl, o-, m- oder p-Acetylphenyl, o-, m- oder p-Aminosulfonylphenyl, o-, m- oder p-(Morpholin-4-ylcarbonyl)-phenyl, o-, m- oder p-(Morpholin-4-ylcarbonyl)-phenyl, o-, m- oder p-(3-Oxo-morpholin-4-yl)-phenyl, o-, m- oder p-(Piperidinyl-carbonyl)-phenyl, o-, m- oder p-[2-(Morpholin-4-yl)ethoxy]-phenyl, o-, m- oder p-[3-(N,N-Diethylamino)propoxy]-phenyl, o-, m- oder p-[3-(3-Diethylaminopropyl)-ureido]-phenyl, o-, m- oder p-(3-Diethylamino-propoxy-carbonylamino)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitro-phenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethyl-aminophenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

In einer weiteren Ausführungsform bedeutet Ar vorzugsweise unsubstituiertes oder ein-, zwei- oder dreifach durch Hal, CN und/oder S(O)ₘA substituiertes Phenyl.

Het bedeutet, ungeachtet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, Indazolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo[1,4]oxazinyl, weiter bevorzugt 1,3-Benzodioxol-5-yl, 1,4-Benzodioxan-6-yl, 2,1,3-Benzothiadiazol-4- oder -5-yl, 2,1,3-Benzoxadiazol-5-yl oder Dibenzofuranyl.
Die heterocyclischen Reste können auch teilweise oder vollständig hydriert sein.
Ungeachtet weiterer Substitutionen können Het und Het² also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder-3-thienyl, 2,3-Dihydro-1-, -2-, -3-, -4- oder -5-pyrrolyl, 2,5-Dihydro-1-, -2-, - 3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder -4-pyranyl, 1,4-Dioxanyl, 1,3-Dioxan-2-, -4- oder -5-yl, Hexahydro-1-, -3- oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder -8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydro-benzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl, 2,3-Dihydro-2-oxo-furanyl, 3,4-Dihydro-2-oxo-1H-chinazolinyl, 2,3-Dihydro-benzoxazolyl, 2-Oxo-2,3-dihydro-benzoxazolyl, 2,3-Dihydro-benzimidazolyl, 1,3-Dihydroindol, 2-Oxo-1,3-dihydro-indol oder 2-Oxo-2,3-dihydro-benzimidazolyl.

In einer weiteren Ausführungsform bedeutet Het vorzugsweise einen ein- oder zweikernigen aromatischen Heterocyclus mit 1 bis 3 N-, O- und/oder S-Atomen, der unsubstituiert oder ein-, zwei- oder dreifach durch Hal substituiert sein kann.

Het bedeutet besonders bevorzugt Thiazolyl, Thienyl, Pyridyl, Benzo[1,2,5]thiadiazolyl oder Benzo[1,3]dioxolyl.

Het¹ bedeutet vorzugsweise einen einkernigen gesättigten oder aromatischen Heterocyclus mit 1 bis 2 N und/oder O-Atomen, der ein- oder zweifach durch A substituiert sein kann. Het¹ bedeutet besonders bevorzugt Piperidin-1-yl, Pyrrolidin-1-yl, Morpholin-4-yl, Piperazin-1-yl, 1,3-Oxazolidin-3-yl, Imidazolidinyl, Oxazolyl, Thiazolyl, [1,4]Diazepanyl, Thienyl, Furanyl oder Pyridyl, wobei die Reste auch ein- oder zweifach durch A, (CH²)ₙHet², (CH₂)ₙN(R³)₂ und/oder (CH₂)ₙOR³ substituiert sein können.

R¹ bedeutet vorzugsweise 4-Fluorphenyl, 3,5-Difluorphenyl, 3,4-Difluorphenyl, 3,4,5-Trifluorphenyl, 2-Chlorphenyl, 3-Chlorphenyl, 3-Cyanphenyl, 4-Cyanphenyl, 4-Methylsulfonylphenyl, Pyridyl, Benzo[1,3]dioxolyl oder Benzo[1,2,5]thiadiazolyl.

R² bedeutet vorzugsweise einen ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann.
Hierbei bedeutet ungesättigter oder aromatischer 5-gliedriger Heterocyclus mit 1 bis 4 N-, O- und/oder S-Atomen z.B. 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, Dihydro-oxazolyl, 3-, 4- oder 5-Isoxazolyl, 2-, 4- oder 5-Thiazolyl, Dihydrothiazolyl, 3-, 4- oder 5-Isothiazolyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder-5-yl, 1,3,4-Thiadiazol-2-oder-5-yl, 1,2,4-Thiadiazol-3-oder-5-yl, 1,2,3-Thiadiazol-4- oder -5-yl.

R² bedeutet weiterhin vorzugsweise einen gesättigten 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann.

R² bedeutet besonders bevorzugt einen Heterocyclus ausgewählt aus der Gruppe [1,2,4]Oxadiazolyl, [1,3,4]Oxadiazolyl, Oxazolyl, Dihydro-oxazolyl, Pyrazolyl, Imidazolyl, Triazolyl, Tetrazolyl, Pyrrolyl, Furanyl, Thienyl, Thiazolyl, Dihydrothiazolyl, Thiadiazolyl, Oxazolidinyl, Pyrrolidinyl, Piperidinyl, der ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann.

R³ bedeutet vorzugsweise H, Methyl, Ethyl, Propyl, Isopropyl, Butyl oder tert.-Butyl.
R⁴ und R⁵ bedeuten vorzugsweise H.

Hal bedeutet vorzugsweise F, Cl oder Br, aber auch I, besonders bevorzugt F oder Cl.

Dess-Martin-Periodinan ist ein käufliches Oxidationsmittel folgender Struktur:

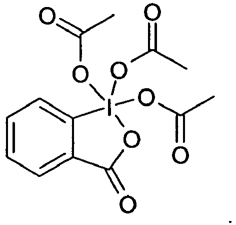

Für die gesamte Erfindung gilt, daß sämtliche Reste, die mehrfach auftreten, gleich oder verschieden sein können, d.h. unabhängig voneinander sind.
Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Im ausgedrückt werden, die der Formel I entsprechen und worin die nicht näher bezeichneten Reste die bei der Formel I angegebene Bedeutung haben, worin jedoch
in la
   - A: unverzweigtes oder verzweigtes Alkyl mit 1-10 C-Atomen,
   worin 1-7 H-Atome durch F und/oder Cl ersetzt sein können,
   bedeutet;
in Ib
   - Ar: unsubstituiertes oder ein-, zwei- oder dreifach durch Hal, CN und/oder S(O)ₘA substituiertes Phenyl,
   bedeutet;
in Ic
   - Het: einen ein- oder zweikernigen aromatischen Heterocyclus mit 1 bis 3 N-, O- und/oder S-Atomen, der unsubstituiert oder ein-, zwei- oder dreifach durch Hal substituiert sein kann,
   bedeutet;
in Id
   - Het: Thiazolyl, Thienyl, Pyridyl, Benzo[1,2,5]thiadiazolyl oder Benzo[1,3]dioxolyl,
   bedeutet;
in le
   - R²: einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann,
   bedeutet;
in If
   - R²: einen Heterocyclus ausgewählt aus der Gruppe [1,2,4]Oxadiazolyl, [1,3,4]Oxadiazolyl, Oxazolyl, Dihydro-oxazolyl, Pyrazolyl, Imidazolyl, Triazolyl, Tetrazolyl, Pyrrolyl, Furanyl, Thienyl, Thiazolyl, Dihydrothiazolyl,Thiadiazolyl, Oxazolidinyl, Pyrrolidinyl, Piperidinyl, der ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann,
   bedeutet;
in Ig
   - R⁴, R⁵: H bedeutet;
in Ih
   - R³: H, Methyl, Ethyl, Propyl, Isopropyl, Butyl oder tert.-Butyl
   bedeutet;
in li
   - R¹: 4-Fluorphenyl, 3,5-Difluorphenyl, 3,4-Difluorphenyl, 3,4,5-Trifluorphenyl, 2-Chlorphenyl, 3-Chlorphenyl, 3-Cyanphenyl, 4-Cyanphenyl, 4-Methylsulfonylphenyl, Pyridyl, Benzo[1,3]dioxolyl oder Benzo[1,2,5]thiadiazolyl
   bedeutet;
in Ij
   - Het¹: einen einkernigen gesättigten oder aromatischen Heterocyclus mit 1 bis 2 N und/oder O-Atomen, der ein- oder zweifach durch A, N(R³)₂ und/oder (CH₂)ₙOR₃ substituiert sein kann,
   bedeutet;
in lk
   - Het¹: Piperidin-1-yl, Pyrrolidin-1-yl, Morpholin-4-yl, Piperazin-1-yl, 1,3-Oxazolidin-3-yl, Imidazolidinyl, Oxazolyl, Thiazolyl, [1,4]Diazepanyl, Thienyl, Furanyl oder Pyridyl, wobei die Reste auch ein- oder zweifach durch A, (CH₂)ₙHet², (CH₂)ₙN(R³)₂ und/oder (CH₂)ₙOR³ substituiert sein können,
   bedeutet;
in II
   - R¹: Ar oder Het,
   - R²: einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³ Het¹, COHet¹ und/oder =O (Carbonylsauerstoff) substituiert sein kann,
   - R³: H oder A,
   - R⁴, R⁵: H,
   - A: unverzweigtes oder verzweigtes Alkyl mit 1-10 C-Atomen,
   worin 1-7 H-Atome durch F und/oder Cl ersetzt sein können,
   - Ar: unsubstituiertes oder ein-, zwei- oder dreifach durch Hal, CN und/oder S(O)ₘA substituiertes Phenyl,
   - Het: einen ein- oder zweikernigen aromatischen Heterocyclus mit 1 bis 3 N-, O- und/oder S-Atomen, der unsubstituiert oder ein-, zwei- oder dreifach durch Hal substituiert sein kann,
   - Het¹: einen einkernigen gesättigten oder aromatischen Heterocyclus mit 1 bis 2 N und/oder O-Atomen, der ein- oder zweifach durch A, (CH₂)ₙHet², (CH₂)ₙN(R³)₂ und/oder (CH₂)ₙOR³ substituiert sein kann,
   - Het²: Pyrrolidino, Piperidino oder Morpholino,
   - Hal: F, Cl, Br oder I,
   - m: 0, 1 oder 2,
   - n: 1, 2, 3 oder 4
   bedeuten;
in Im
   - R¹: Ar oder Het,
   - R²: einen Heterocyclus ausgewählt aus der Gruppe [1,2,4]Oxadiazolyi, [1,3,4]Oxadiazolyl, Oxazolyl, Dihydro-oxazolyl, Pyrazolyl, Imidazolyl, Triazolyl, Tetrazolyl, Pyrrolyl, Furanyl, Thienyl, Thiazolyl, Dihydrothiazolyl, Thiadiazolyl, Oxazolidinyl, der ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann,
   - R³: H, Methyl, Ethyl, Propyl, Isopropyl, Butyl oder tert.-Butyl,
   - R⁴, R⁵: H,
   - A: unverzweigtes oder verzweigtes Alkyl mit 1-10 C-Atomen,
   worin 1-7 H-Atome durch F und/oder Cl ersetzt sein können,
   - Ar: unsubstituiertes oder ein-, zwei- oder dreifach durch Hal, CN und/oder S(O)ₘA substituiertes Phenyl,
   - Het: Thiazolyl, Thienyl, Pyridyl, Benzo[1,2,5]thiadiazolyl oder Benzo[1,3]dioxolyl,
   - Het¹: Piperidin-1-yl, Pyrrolidin-1-yl, Morpholin-4-yl, Piperazin-1-yl, 1,3-Oxazolidin-3-yl, Imidazolidinyl, Oxazolyl, Thiazolyl, [1,4]Diazepanyl, Thienyl, Furanyl oder Pyridyl, wobei die Reste auch ein- oder zweifach durch A, (CH₂)ₙHet², (CH₂)ₙN(R³)₂ und/oder (CH₂)ₙOR³ substituiert sein können,
   - Het²: Pyrrolidino, Piperidino oder Morpholino,
   - Hal: F, Cl, Br oder I,
   - m: 0, 1 oder 2,
   - n: 1, 2, 3 oder 4
   bedeuten;
sowie ihre pharmazeutisch verwendbaren Salze, Solvate, Tautomere und ₌ Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II und III sind in der Regel bekannt. Sind sie neu, so können sie aber nach an sich bekannten Methoden hergestellt werden.
Die verwendeten Pyridazinone der Formel II werden, wenn nicht käuflich erhältlich, in der Regel nach W. J. Coates, A. McKillop, Synthesis, 1993, 334-342 hergestellt.

Verbindungen der Formel I können vorzugsweise erhalten werden, indem man eine Verbindung der Formel II mit einer Verbindung der Formel III umsetzt.
In den Verbindungen der Formel III bedeutet L vorzugsweise Cl, Br, I oder eine freie oder eine reaktionsfähig abgewandelte OH-Gruppe wie z.B. ein aktivierter Ester, ein Imidazolid oder Alkylsulfonyloxy mit 1-6 C-Atomen (bevorzugt Methylsulfonyloxy oder Trifluormethylsulfonyloxy) oder Arylsulfonyloxy mit 6-10 C-Atomen (bevorzugt Phenyl- oder p-Tolylsulfonyloxy).

Die Umsetzung erfolgt in der Regel in Gegenwart eines säurebindenden Mittels vorzugsweise einer organischen Base wie DIPEA, Triethylamin, Dimethylanilin, Pyridin oder Chinolin.
Auch der Zusatz eines Alkali- oder Erdalkalimetall-hydroxids, -carbonats oder -bicarbonats oder eines anderen Salzes einer schwachen Säure der Alkali- oder Erdalkalimetalle, vorzugsweise des Kaliums, Natriums, Calciums oder Cäsiums kann günstig sein.
Die Reaktionszeit liegt je nach den angewendeten Bedingungen zwischen einigen Minuten und 14 Tagen, die Reaktionstemperatur zwischen etwa -30° und 140°, normalerweise zwischen -10° und 90°, insbesondere zwischen etwa 0° und etwa 70°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, 1-Methyl-pyrrolidinon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt ist Acetonitril, Dichlormethan, NMP und/oder DMF.

Es ist weiterhin möglich, eine Verbindung der Formel I in eine andere Verbindung der Formel I umzuwandeln, indem man einen Rest R² in einen anderen Rest R² umwandelt.
Z.B. kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60° und +30°.
Weiterhin kann man ein Oxy-amidinderivat zu einem Oxadiazolderivat cyclisieren, vorzugsweise in THF mit dem Burgess-Reagenz bei Temperaturen zwoschen 60° und 80°.
Man kann auch ein Amidderivat, vorzugsweise mit AuCl₃ in THF, zu einem Oxazolderivat cyclisieren.

Weiterhin kann man ein Alkylesterderivat mit einem N-Hydroxyamidinderivat zu einem Oxadiazolderivat umsetzen, wobei vorzugsweise DMF als Lösungsmittel verwendet wird.
Ferner kann man ein N-(Aminothiocarbonyl)-hydrazidderivat zu einem Oxadiazolderivat cyclisieren, vorzugsweise mit Hg(OAc)₂ in Methanol. Weiterhin kann man eine SH-Gruppe alkylieren, zweckmäßig in einem inerten Lösungsmittel wie Dichlormethan, DMF oder THF und / oder in Gegenwart einer Base wie Caesiumcarbonat, Triethylamin oder Pyridin bei Temperaturen zwischen -60° und +30°.
Man kann auch eine Cyangruppe mit einem Azidderivat zu einem Tetrazolderivat umsetzen, wobei die Umsetzung vorzugsweise mit NH₄Cl, LiCl in DMF bei 80° - 120° durchgeführt wird

Es ist ferner möglich, eine Verbindung der Formel I in eine andere Verbindung der Formel I umzuwandeln, indem man einen Rest R² in einen anderen Rest R² umwandelt, z.B. indem man Nitrogruppen (beispielsweise durch Hydrierung an Raney-Nickel oder Pd-Kohle in einem inerten Lösungsmittel wie Methanol oder Ethanol) zu Aminogruppen reduziert.

Die Verbindungen der Formeln I können ferner erhalten werden, indem man sie aus ihren funktionellen Derivaten durch Solvolyse, insbesondere Hydrolyse, oder durch Hydrogenolyse in Freiheit setzt.

Bevorzugte Ausgangsstoffe für die Solvolyse bzw. Hydrogenolyse sind solche, die anstelle einer oder mehrerer freier Amino- und/oder Hydroxygruppen entsprechende geschützte Amino- und/oder Hydroxygruppen enthalten, vorzugsweise solche, die anstelle eines H-Atoms, das mit einem N-Atom verbunden ist, eine Aminoschutzgruppe tragen, z. B. solche, die der Formel I entsprechen, aber anstelle einer NH₂-Gruppe eine NHR'-Gruppe (worin R' eine Aminoschutzgruppe bedeutet, z. B. BOC oder CBZ) enthalten.

Ferner sind Ausgangsstoffe bevorzugt, die anstelle des H-Atoms einer Hydroxygruppe eine Hydroxyschutzgruppe tragen, z. B. solche, die der Formel I entsprechen, aber anstelle einer Hydroxyphenylgruppe eine R"O-phenylgruppe enthalten (worin R" eine Hydroxyschutzgruppe bedeutet).

Es können auch mehrere - gleiche oder verschiedene - geschützte Amino- und/oder Hydroxygruppen im Molekül des Ausgangsstoffes vorhanden sein. Falls die vorhandenen Schutzgruppen voneinander verschieden sind, können sie in vielen Fällen selektiv abgespalten werden.

Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte oder substituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie POA; Alkoxycarbonyl wie Methoxycarbonyl, Ethoxycarbonyl, 2,2,2-Trichlorethoxycarbonyl, BOC, 2-lodethoxycarbonyl; Aralkyloxycarbonyl wie CBZ ("Carbobenzoxy"), 4-Methoxybenzyloxycarbonyl, FMOC; Arylsulfonyl wie Mtr, Pbf oder Pmc. Bevorzugte Aminoschutzgruppen sind BOC und Mtr, ferner CBZ, Fmoc, Benzyl und Acetyl.

Der Ausdruck "Hydroxyschutzgruppe" ist ebenfalls allgemein bekannt und bezieht sich auf Gruppen, die geeignet sind, eine Hydroxygruppe vor chemischen Umsetzungen zu schützen, die aber leicht entfernbar sind, nachdem die gewünschte chemische Reaktion an anderen Stellen des Moleküls durchgeführt worden ist. Typisch für solche Gruppen sind die oben genannten unsubstituierten oder substituierten Aryl-, Aralkyl- oder Acylgruppen, ferner auch Alkylgruppen. Die Natur und Größe der Hydroxyschutzgruppen ist nicht kritisch, da sie nach der gewünschten chemischen Reaktion oder Reaktionsfolge wieder entfernt werden; bevorzugt sind Gruppen mit 1-20, insbesondere 1-10 C-Atomen. Beispiele für Hydroxyschutzgruppen sind u.a. tert.-Butoxycarbonyl, Benzyl, p-Nitrobenzoyl, p-Toluolsulfonyl, tert.-Butyl und Acetyl, wobei Benzyl und tert.-Butyl besonders bevorzugt sind. Die COOH-Gruppen in Asparaginsäure und Glutaminsäure werden bevorzugt in Form ihrer tert.-Butylester geschützt (z. B. Asp(OBut)).

Das In-Freiheit-Setzen der Verbindungen der Formel I aus ihren funktionellen Derivaten gelingt - je nach der benutzten Schutzgruppe - z. B. mit starken Säuren, zweckmäßig mit TFA oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie DMF, halogenierte Kohlenwasserstoffe wie Dichlormethan, ferner auch Alkohole wie Methanol, Ethanol oder Isopropanol, sowie Wasser. Ferner kommen Gemische der vorgenannten Lösungsmittel in Frage. TFA wird vorzugsweise im Überschuß ohne Zusatz eines weiteren Lösungsmittels verwendet, Perchlorsäure in Form eines Gemisches aus Essigsäure und 70 %iger Perchlorsäure im Verhältnis 9:1. Die Reaktionstemperaturen für die Spaltung liegen zweckmäßig zwischen etwa 0 und etwa 50°, vorzugsweise arbeitet man zwischen 15 und 30° (Raumtemperatur).

Die Gruppen BOC, OBut, Pbf, Pmc und Mtr können z. B. bevorzugt mit TFA in Dichlormethan oder mit etwa 3 bis 5n HCl in Dioxan bei 15-30° abgespalten werden, die FMOC-Gruppe mit einer etwa 5- bis 50 %igen Lösung von Dimethylamin, Diethylamin oder Piperidin in DMF bei 15-30°. Die Tritylgruppe wird zum Schutz der Aminosäuren Histidin, Asparagin, Glutamin und Cystein eingesetzt. Die Abspaltung erfolgt, je nach gewünschtem Endprodukt, mit TFA / 10% Thiophenol, wobei die Tritylgruppe von allen genannten Aminosäuren abgespalten wird, bei Einsatz von TFA / Anisol oder TFA / Thioanisol wird nur die Tritylgruppe von His, Asn und Gln abgespalten, wogegen sie an der Cys-Seitenkette verbleibt. Die Pbf (Pentamethylbenzofuranyl)-gruppe wird zum Schutz von Arg eingesetzt. Die Abspaltung erfolgt z.B. mit TFA in Dichlormethan.

Hydrogenolytisch entfernbare Schutzgruppen (z. B. CBZ oder Benzyl) können z. B. durch Behandeln mit Wasserstoff in Gegenwart eines Katalysators (z. B. eines Edelmetallkatalysators wie Palladium, zweckmäßig auf einem Träger wie Kohle) abgespalten werden. Als Lösungsmittel eignen sich dabei die oben angegebenen, insbesondere z. B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt. Eine Hydrogenolyse der CBZ-Gruppe gelingt z. B. gut an 5 bis 10 %igem Pd/C in Methanol oder mit Ammomiumformiat (anstelle von Wasserstoff) an Pd/C in Methanol/DMF bei 20-30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I werden größtenteils konventionell hergestellt. Sofern die Verbindung der Formel I eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der Verbindungen der Formel I zählen ebenfalls dazu. Bei bestimmten Verbindungen der Formel I lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxy-ethansulfonat, lodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Besonders bevorzugt sind Hydrochlorid, Dihydrochlorid, Hydrobromid, Maleat, Mesylat, Phosphat, Sulfat und Succinat.

Die Säureadditionssalze basischer Verbindungen der Formel I werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine Verbindung der Formel I und/oder ihre pharmazeutisch verwendbaren Salze, Solvate und Stereoisomere, einschließlich deren Mischungen in allen Verhältnissen, sowie gegebenenfalls Träger- und/oder Hilfsstoffe. Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel. An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I und/oder ihre pharmazeutisch verwendbaren Salze, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer Verbindung der Formel I und/oder ihrer pharmazeutisch verwendbaren Salze, Solvate und Stereoisomere, einschließlich deren Mischungen in allen Verhältnissen, und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die vorliegenden Verbindungen eignen sich als pharmazeutische Wirkstoffe für Säugetiere, insbesondere für den Menschen, bei der Behandlung von tyrosinkinasebedingten Krankheiten. Zu diesen Krankheiten zählen die Proliferation von Tumorzellen, die pathologische Gefäßneubildung (oder Angiogenese), die das Wachstum fester Tumoren fördert, die Gefäßneubildung im Auge (diabetische Retinopathie, altersbedingte Makula-Degeneration und dergleichen) sowie Entzündung (Schuppenflechte, rheumatoide Arthritis und dergleichen).

Die vorliegende Erfindung umfasst die Verwendung der Verbindungen der Formel I und/oder ihre physiologisch unbedenklichen Salze und Solvate zur Herstellung eines Arzneimittels zur Behandlung oder Vorbeugung von Krebs. Bevorzugte Karzinome für die Behandlung stammen aus der Gruppe Hirnkarzinom, Urogenitaltraktkarzinom, Karzinom des lymphatischen Systems, Magenkarzinom, Kehlkopfkarzinom und Lungenkarzinom. Eine weitere Gruppe bevorzugter Krebsformen sind Monozytenleukämie, Lungenadenokarzinom, kleinzellige Lungenkarzinome, Bauchspeicheldrüsenkrebs, Glioblastome und Brustkarzinom.
Ebensfalls umfasst ist die Verwendung der erfindungsgemäßen Verbindungen nach Anspruch 1 und/oder ihre physiologisch unbedenklichen Salze und Solvate zur Herstellung eines Arzneimittels zur Behandlung oder Vorbeugung einer Krankheit, an der Angiogenese beteiligt ist.
Eine derartige Krankheit, an der Angiogenese beteiligt ist, ist eine Augenkrankheit, wie Retina-Vaskularisierung, diabetische Retinopathie, altersbedingte Makula-Degeneration und dergleichen.
Die Verwendung von Verbindungen der Formel I und/oder ihre physiologisch unbedenklichen Salze und Solvate zur Herstellung eines Arzneimittels zur Behandlung oder Vorbeugung von Entzündungskrankheiten, fällt ebenfalls unter den Umfang der vorliegenden Erfindung. Zu solchen Entzündungskrankheiten zählen zum Beispiel rheumatoide Arthritis, Schuppenflechte, Kontaktdermatitis, Spät-Typ der Überempfindlichkeitsreaktion und dergleichen.
Ebenfalls umfasst ist die Verwendung der Verbindungen der Formel I und/oder ihre physiologisch unbedenklichen Salze und Solvate zur Herstellung eines Arzneimittels zur Behandlung oder Vorbeugung einer tyrosinkinasebedingten Krankheit bzw. eines tyrosinkinasebedingten Leidens bei einem Säugetier, wobei man diesem Verfahren einem kranken Säugetier, das einer derartigen Behandlung bedarf, eine therapeutisch wirksame Menge einer erfindungsgemäßen Verbindung verabreicht. Die therapeutische Menge hängt von der jeweiligen Krankheit ab und kann vom Fachmann ohne allen großen Aufwand bestimmt werden.
Die vorliegende Erfindung umfasst auch die Verwendung Verbindungen der Formel I und/oder ihre physiologisch unbedenklichen Salze und Solvate zur Herstellung eines Arzneimittels zur Behandlung oder Vorbeugung von Retina-Vaskularisierung.
Verfahren zur Behandlung oder Vorbeugung von Augenkrankheiten wie diabetischer Retinopathie und altersbedingter Makula-Degeneration sind ebenfalls ein Bestandteil der Erfindung. Die Verwendung zur Behandlung oder Vorbeugung von Entzündungskrankheiten wie rheumatoider Arthritis, Schuppenflechte, Kontaktdermatitis und Spät-Typen der Überempfindlichkeitsreaktion, sowie die Behandlung oder Vorbeugung von Knochen-Pathologien aus der Gruppe Osteosarkom, Osteoarthritis und Rachitis, fällt ebenfalls unter den Umfang der vorliegenden Erfindung.
Der Ausdruck "tyrosinkinasebedingte Krankheiten oder Leiden" bezieht sich auf pathologische Zustände, die von der Aktivität einer oder mehrerer Tyrosinkinasen abhängig sind. Die Tyrosinkinasen sind entweder direkt oder indirekt an den Signaltransduktionswegen verschiedener Zellaktivitäten, darunter Proliferation, Adhäsion und Migration sowie Differenzierung beteiligt. Zu den Krankheiten, die mit Tyrosinkinaseaktivität assoziiert sind, zählen die Proliferation von Tumorzellen, die pathologische Gefäßneubildung, die das Wachstum fester Tumore fördert, Gefäßneubildung im Auge (diabetische Retinopathie, altersbedingte Makula-Degeneration und dergleichen) sowie Entzündung (Schuppenflechte, rheumatoide Arthritis und dergleichen).

Die Verbindungen der Formel I können an Patienten zur Behandlung von Krebs, insbesondere schnell wachsenden Tumoren, verabreicht werden.

Gegenstand der Erfindung ist somit die Verwendung von Verbindungen der Formel I, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation der Signaltransduktion von Kinasen eine Rolle spielt.

Bevorzugt ist hierbei die Met-Kinase.

Bevorzugt ist die Verwendung von Verbindungen der Formel I, sowie ihrer pharmazeutisch verwendbaren Salze, Solvate und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1 beeinflußt werden.

Besonders bevorzugt ist die Verwendung zur Herstellung eines Arzneimittels zur Behandlung von Krankheiten, die durch Inhibierung von Met-Kinase durch die Verbindungen nach Anspruch 1 beeinflußt werden. Insbesondere bevorzugt ist die Verwendung zur Behandlung einer Krankheit, wobei die Krankheit ein fester Tumor ist.

Der feste Tumor ist vorzugsweise ausgewählt aus der Gruppe der Tumoren der Lunge, des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens und/oder des Kehlkopfs.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die offenbarten Verbindungen der Formel I können in Verbindung mit anderen Therapeutika, einschließlich Antikrebsmitteln, verabreicht werden. Wie hier verwendet, betrifft der Begriff "Antikrebsmittel" jedes Mittel, das einem Patienten mit Krebs zum Zweck der Behandlung des Krebses verabreicht wird.

Die hier definierte Antikrebsbehandlung kann als alleinige Therapie angewendet werden oder zusätzlich zu der erfindungsgemäßen Verbindung herkömmliche Operation oder Strahlungstherapie oder Chemotherapie umfassen. Eine derartige Chemotherapie kann eine oder mehrere der folgenden Kategorien von Antitumormitteln umfassen:
(i) antiproliferative/antineoplastische/DNA schädigende Mittel und Kombinationen davon, wie in der medizinischen Onkologie verwendet, wie Alkylierungsmittel (zum Beispiel Cisplatin, Carboplatin, Cyclophosphamid, Nitrogen Mustard, Melphalan, Chlorambucil, Busulphan und Nitrosoharnstoffe); Antimetaboliten (z.B. Antifolate, wie Fluorpyrimidine, wie 5-Fluoruracil und Tegafur, Raltitrexed, Methotrexat, Cytosinarabinosid, Hydroxyharnstoff und Gemcitabin); Antitumor-Antibiotika (z.B. Anthracycline, wie Adriamycin, Bleomycin, Doxorubicin, Daunomycin, Epirubicin, Idarubicin, Mitomycin-C, Dactinomycin und Mithramycin); antimitotische Mittel (zum Beispiel Vinca-Alkaloide, wie Vincristin, Vinblastin, Vindesin und Vinorelbin, und Taxoide, wie Taxol und Taxoter); Topoisomerase-Inhibitoren (zum Beispiel Epipodophyllotoxine, wie Etoposid und Teniposid, Amsacrin, Topotecan, Irinotecan und Camptothecin) und zelldifferenzierende Mittel (zum Beispiel all-trans-Retinsäure, 13-cis-Retinsäure und Fenretinid);
(ii) zytostatische Mittel, wie Anti-Östrogene (z.B. Tamoxifen, Toremifen, Raloxifen, Droloxifen und lodoxyfen), den Östrogenrezeptor nach unten regulierende Mittel (zum Beispiel Fulvestrant), Anti-Androgene (z.B. Bicalutamid, Flutamid, Nilutamid und Cyproteronacetat), LHRH-Antagonisten oder LHRH-Agonisten (zum Beispiel Goserelin, Leuprorelin und Buserelin), Progesterone (zum Beispiel Megestrolacetat), Aromatase-Inhibitoren (zum Beispiel Anastrozol, Letrozol, Vorazol und Exemestan) und Inhibitoren der 5α-Reduktase, wie Finasterid;
(iii) Mittel, die die Invasion von Krebszellen hemmen (zum Beispiel Metalloproteinase-Inhibitoren, wie Marimastat und Inhibitoren der Urokinase-Plasminogenaktivator-Rezeptor-Funktion);
(iv) Inhibitoren der Wachstumsfaktor-Funktion, zum Beispiel umfassen solche Inhibitoren Wachstumsfaktor-Antikörper, Wachstumsfaktor-Rezeptor-Antikörper (zum Beispiel den Anti-erbb2-Antikörper Trastuzumab [Herceptin™] und den Anti-erbb1-Antikörper Cetuximab [C225]), Farnesyltransferase-Inhibitoren, Tyrosinkinase-Inhibitoren und Serin / Threonin-Kinase-Inhibitoren, zum Beispiel Inhibitoren der epidermalen Wachstumsfaktor-Familie (zum Beispiel Inhibitoren der Tyrosinkinasen der EGFR-Familie, wie N-(3-Chlor-4-fluorphenyl)-7-methoxy-6-(3-morpholinopropoxy)-chinazolin-4-amin (Gefitinib, AZD1839), N-(3-Ethinylphenyl)-6,7-bis(2-methoxyethoxy)chinazolin-4-amin (Erlotinib, OSI-774) und 6-Acrylamido-N-(3-chlor-4-fluorphenyl)-7-(3-morpholinopropoxy)chinazolin-4-amin (Cl 1033)), zum Beispiel Inhibitoren der von Plättchen abstammenden Wachstumsfaktor-Familie und zum Beispiel Inhibitoren der Hepatozytenwachstumsfaktor-Familie;
(v) antiangiogene Mittel, wie solche, die die Wirkungen des vaskulären endothelialen Wachstumsfaktors hemmen (zum Beispiel der Antikörper gegen den vaskulären Endothelzell-Wachstumsfaktor Bevacizumab [Avastin™], Verbindungen, wie die in den veröffentlichten internationalen Patentanmeldungen WO 97/22596, WO 97/30035, WO 97/32856 und WO 98/13354 offenbarten) und Verbindungen, die durch andere Mechanismen wirken (zum Beispiel Linomid, Inhibitoren der Integrin-ανβ3-Funktion und Angiostatin);
(vi) gefäßschädigende Mittel, wie Combretastatin A4 und in den internationalen Patentanmeldungen WO 99/02166, WO 00/40529, WO 00/41669, WO 01/92224, WO 02/04434 und WO 02/08213 offenbarte Verbindungen;
(vii) Antisense-Therapien, zum Beispiel diejenigen, die gegen die vorstehend aufgelisteten Ziele gerichtet sind, wie ISIS 2503, ein anti-Ras-Antisense;
(viii) Genetherapieansätze, einschließlich beispielsweise Ansätze zum Ersetzen von veränderten Genen, wie verändertem p53 oder verändertem BRCA1 oder BRCA2, GDEPT- (gene-directed enzyme pro-drug-Therapie-) Ansätze, die diejenigen, die Cytosindesaminase, Thymidinkinase oder ein bakterielles Nitroreduktase-Enzym verwenden, sowie Ansätze zur Erhöhung der Patiententoleranz gegenüber Chemotherapie oder Strahlungstherapie, wie Multi-Drug-Resistence-Gen-Therapie; und
(ix) Immuntherapieansätze, einschließlich beispielsweise Ex-vivo- und In-vivo-Ansätzen zur Erhöhung der Immunogenität von Patiententumorzellen, wie Transfektion mit Cytokinen, wie Interleukin 2, Interleukin 4 oder Granulozyten-Makrophagen-Kolonie-stimulierendem Faktor, Ansätze zur Verringerung der T-Zell-Anergie, Ansätze unter Verwendung transfizierter Immunzellen, wie mit Cytokin transfizierter dendritischer Zellen, Ansätze unter Verwendung mit Cytokin transfizierter Tumorzelllinien und Ansätze unter Verwendung anti-idiotypischer Antikörper.

Bevorzugt aber nicht ausschliesslich werden die Arzneimittel der nachstehenden Tabelle 1 mit den Verbindungen der Formel I kombiniert.

| Tabelle 1. | | |
|---|---|---|
| Alkylierungsmittel | Cyclophosphamid | Lomustin |
| | Busulfan | Procarbazin |
| | Ifosfamid | Altretamin |
| | Melphalan | Estramustinphosphat |
| | Hexamethylmelamin | Mechlorethamin |
| | Thiotepa | Streptozocin |
| | Chlorambucil | Temozolomid |
| | Dacarbazin | Semustin |
| | Carmustin | |
| | | |
| Platinmittel | Cisplatin | Carboplatin |
| | Oxaliplatin | ZD-0473 (AnorMED) |
| | Spiroplatin | Lobaplatin (Aetema) |
| | Carboxyphthalatoplatinum | Satraplatin (Johnson Matthey) |
| | Tetraplatin | |
| | Ormiplatin | BBR-3464 (Hoffrnann-La Roche) |
| | Iproplatin | |
| | | SM-11355 (Sumitomo) |
| | | AP-5280 (Access) |
| | | |
| Antimetabolite | Azacytidin | Tomudex |
| | Gemcitabin | Trimetrexate |
| | Capecitabin | Deoxycoformycin |
| | 5-Fluoruracil | Fludarabin |
| | Floxuridin | Pentostatin |
| | 2-Chlordesoxyadenosin | Raltitrexed |
| | 6-Mercaptopurin | Hydroxyharnstoff |
| | 6-Thioguanin | Decitabin (SuperGen) |
| | Cytarabin | Clofarabin (Bioenvision) |
| | 2-Fluordesoxycytidin | Irofulven (MGI Pharrna) |
| | Methotrexat | DMDC (Hoffmann-La Roche) |
| | Idatrexate | |
| | | Ethinylcytidin (Taiho ) |
| | | |
| TopoisomeraseInhibitoren | Amsacrin | Rubitecan (SuperGen) |
| | Epirubicin | Exatecanmesylat (Daiichi) |
| | Etoposid | Quinamed (ChemGenex) |
| | Teniposid oder | Gimatecan (Sigma- Tau) |
| | Mitoxantron | Diflomotecan (Beaufour-Ipsen) |
| | Irinotecan (CPT-11) | |
| | 7-Ethyl-10-hydroxycamptothecin | TAS-103 (Taiho) |
| | | Elsamitrucin (Spectrum) |
| | Topotecan | J-107088 (Merck & Co) |
| | Dexrazoxanet (TopoTarget) | BNP-1350 (BioNumerik) |
| | | CKD-602 (Chong Kun Dang) |
| | Pixantron (Novuspharrna) | |
| | Rebeccamycin-Analogon (Exelixis) | KW-2170 (Kyowa Hakko) |
| | BBR-3576 (Novuspharrna) | |
| | | |
| Antitumor-Antibiotika | Dactinomycin (Actinomycin D) | Amonafid |
| | | Azonafid |
| | Doxorubicin (Adriamycin) | Anthrapyrazol |
| | Deoxyrubicin | Oxantrazol |
| | Valrubicin | Losoxantron |
| | Daunorubicin (Daunomycin) | Bleomycinsulfat (Blenoxan) |
| | Epirubicin | Bleomycinsäure |
| | Therarubicin | Bleomycin A |
| | Idarubicin | Bleomycin B |
| | Rubidazon | Mitomycin C |
| | Plicamycinp | MEN-10755 (Menarini) |
| | Porfiromycin | GPX-100 (Gem Pharmaceuticals) |
| | Cyanomorpholino-doxorubicin | |
| | Mitoxantron (Novantron) | |
| | | |
| Antimitotische Mittel | Paclitaxel | SB 408075 (GlaxoSmithKline) |
| | Docetaxel | |
| | Colchicin | E7010 (Abbott) |
| | Vinblastin | PG-TXL (Cell Therapeutics) |
| | Vincristin | |
| | Vinorelbin | IDN 5109 (Bayer) |
| | Vindesin | A 105972 (Abbott) |
| | Dolastatin 10 (NCl) | A 204197 (Abbott) |
| | Rhizoxin (Fujisawa) | LU 223651 (BASF) |
| | Mivobulin (Warner-Lambert) | D 24851 (ASTA Medica) |
| | | ER-86526 (Eisai) |
| | Cemadotin (BASF) | Combretastatin A4 (BMS) |
| | RPR 109881A (Aventis) | Isohomohalichondrin-B (PharmaMar) |
| | TXD 258 (Aventis) | |
| | Epothilon B (Novartis) | ZD 6126 (AstraZeneca) |
| | T 900607 (Tularik) | PEG-Paclitaxel (Enzon) |
| | T 138067 (Tularik) | AZ10992 (Asahi) |
| | Cryptophycin 52 (Eli Lilly) | !DN-5109 (Indena) |
| | Vinflunin (Fabre) | AVLB (Prescient |
| | Auristatin PE (Teikoku Hormone) | NeuroPharma) |
| | | Azaepothilon B (BMS) |
| | BMS 247550 (BMS) | BNP- 7787 (BioNumerik) |
| | BMS 184476 (BMS) | CA-4-Prodrug (OXiGENE) |
| | BMS 188797 (BMS) | Dolastatin-10 (NrH) |
| | Taxoprexin (Protarga) | CA-4 (OXiGENE) |
| | | |
| AromataseInhibitoren | Aminoglutethimid | Exemestan |
| | Letrozol | Atamestan (BioMedicines) |
| | Anastrazol | YM-511 (Yamanouchi) |
| | Formestan | |
| | | |
| Thymidylatsynthase-Inhibitoren | Pemetrexed (Eli Lilly) | Nolatrexed (Eximias) |
| | ZD-9331 (BTG) | CoFactor™ (BioKeys) |
| DNA-Antagonisten | Trabectedin (PharmaMar) | Mafosfamid (Baxter International) |
| | Glufosfamid (Baxter International) | |
| | | Apaziquon (Spectrum Pharmaceuticals) |
| | Albumin + 32P (Isotope Solutions) | |
| | | O6-Benzylguanin (Paligent) |
| | Thymectacin (NewBiotics) | |
| | Edotreotid (Novartis) | |
| | | |
| Farnesyltrans-ferase-Inhibitoren | Arglabin (NuOncology Labs) | Tipifarnib (Johnson & Johnson) |
| | lonafarnib (Schering-Plough) | Perillylalkohol (DOR BioPharma) |
| | BAY-43-9006 (Bayer) | |
| | | |
| Pumpen-Inhibitoren | CBT-1 (CBA Pharma) | Zosuquidar-Trihydrochlorid (Eli Lilly) |
| | Tariquidar (Xenova) | |
| | MS-209 (Scherinq AG) | Biricodar-Dicitrat (Vertex) |
| | | |
| Histonacetyltrans-ferase-Inhibitoren | Tacedinalin (Pfizer) | Pivaloyloxymethylbutyrat (Titan) |
| | SAHA (Aton Pharma) | |
| | MS-275 (Schering AG) | Depsipeptid (Fujisawa) |
| | | |
| Metalloproteinase-Inhibitoren Ribonucleosidred uktase-Inhibitoren | Neovastat (Aeterna Laboratories) | CMT -3 (CollaGenex) |
| | | BMS-275291 (Celltech) |
| | Marimastat (British Biotech) | Tezacitabin (Aventis) |
| | | Didox (Molecules for Health) |
| | Galliummaltolat (Titan) | |
| | Triapin (Vion) | |
| | | |
| TNF-alpha-Agonisten / Antagonisten | Virulizin (Lorus Therapeutics) | Revimid (Celgene) |
| | CDC-394 (Celgene) | |
| | | |
| Endothelin-A-Rezeptor-Antaqonisten | Atrasentan (Abbot) | YM-598 (Yamanouchi) |
| | ZD-4054 (AstraZeneca) | |
| | | |
| Retinsäurerezeptor-Agonisten | Fenretinid (Johnson & Johnson) | Alitretinoin (Ligand) |
| | LGD-1550 (Ligand) | |
| | | |
| Immunmodulatoren | Interferon | Dexosom-Therapie (Anosys) |
| | Oncophage (Antigenics) | |
| | GMK (Progenics) | Pentrix (Australian Cancer Technology) |
| | Adenokarzinom-Impfstoff (Biomira) | |
| | | JSF-154 (Tragen) |
| | CTP-37 (AVI BioPharma) | Krebsimpfstoff (Intercell) |
| | JRX-2 (Immuno-Rx) | Norelin (Biostar) |
| | PEP-005 (Peplin Biotech) | BLP-25 (Biomira) |
| | Synchrovax-Impfstoffe (CTL Immuno) | MGV (Progenics) |
| | | !3-Alethin (Dovetail) |
| | Melanom-Impfstoff (CTL Immuno) | CLL-Thera (Vasogen) |
| | p21-RAS-Impfstoff (GemVax) | |
| | | |
| Hormonelle und antihormonelle Mittel | Östrogene | Prednison |
| | konjugierte Östrogene | Methylprednisolon |
| | Ethinylöstradiol | Prednisolon |
| | Chlortrianisen | Aminoglutethimid |
| | Idenestrol | Leuprolid |
| | Hydroxyprogesteron- | Goserelin |
| | caproat | Leuporelin |
| | Medroxyprogesteron | Bicalutamid |
| | Testosteron | Flutamid |
| | Testosteronpropionat | Octreotid |
| | Fluoxymesteron | Nilutamid |
| | Methyltestosteron | Mitotan |
| | Diethylstilbestrol | P-04 (Novogen) |
| | Megestrol | 2-Methoxyöstradiol (EntreMed) |
| | Tamoxifen | |
| | Toremofin | Arzoxifen (Eli Lilly) |
| | Dexamethason | |
| | | |
| Photodynamische Mittel | Talaporfin (Light Sciences | ) Pd-Bacteriopheophorbid (Yeda) |
| | Theralux (Theratechnologies) | |
| | | Lutetium-Texaphyrin (Pharmacyclics) |
| | Motexafin-Gadolinium (Pharmacyclics) | |
| | | Hypericin |
| | | |
| Tyrosinkinase-Inhibitoren | Imatinib (Novartis) | Kahalid F (PharmaMar) |
| | Leflunomid (Sugen/Pharmacia) | CEP- 701 (Cephalon) |
| | | CEP-751 (Cephalon) |
| | ZDI839 (AstraZeneca) | MLN518 (Millenium) |
| | Erlotinib (Oncogene Science) | PKC412 (Novartis) |
| | | Phenoxodiol O |
| | Canertjnib (Pfizer) | Trastuzumab (Genentech) |
| | Squalamin (Genaera) | C225 (ImClone) |
| | SU5416 (Pharmacia) | rhu-Mab (Genentech) |
| | SU6668 (Pharmacia) | MDX-H210 (Medarex) |
| | ZD4190 (AstraZeneca) | 2C4 (Genentech) |
| | ZD6474 (AstraZeneca) | MDX-447 (Medarex) |
| | Vatalanib (Novartis) | ABX-EGF (Abgenix) |
| | PKI166 (Novartis) | IMC-1C11 (ImClone) |
| | GW2016 (GlaxoSmithKline) | |
| | EKB-509 (Wyeth) | |
| | EKB-569 (Wyeth) | |
| Verschiedene Mittel | SR-27897 (CCK-A-Inhibitor, Sanofi-Synthelabo) | BCX-1777 (PNP-Inhibitor, BioCryst) |
| | | Ranpirnase (Ribonuclease-Stimulans, Alfacell) |
| | Tocladesin (cyclisches-AMP-Agonist, Ribapharm) | |
| | Alvocidib (CDK-Inhibitor, Aventis) | Galarubicin (RNA-Synthese-Inhibitor, Dong-A) |
| | CV-247 (COX-2-lnhibitor, Ivy Medical) | |
| | | Tirapazamin (Reduktionsmittel, SRI International) |
| | P54 (COX-2-Inhibitor, Phytopharm) | |
| | CapCell™ (CYP450-Stimulans, Bavarian Nordic) | N-Acetylcystein (Reduktionsmittel, Zambon) |
| | GCS-IOO (gal3-Antagonist, GlycoGenesys) | R-Flurbiprofen (NF-kappaB-Inhibitor, Encore) |
| | | 3CPA (NF-kappaB-Inhibitor, Active Biotech) |
| | G17DT-Immunogen (Gastrin-Inhibitor, Aphton) | |
| | | Seocalcitol (Vitamin-D-Rezeptor-Agonist, Leo) |
| | Efaproxiral (Oxygenator, Allos Therapeutics) | |
| | | 131-I-TM-601 (DNA-Antagonist, TransMolecular) |
| | PI-88 (Heparanase-Inhibitor, Progen) | |
| | Tesmilifen (Histamin-Antagonist, YM BioSciences) | Eflornithin (ODC-Inhibitor, ILEX Oncology) |
| | | Minodronsäure (Osteoclasten-Inhibitor, Yamanouchi) |
| | Histamin (Histamin-H2-Rezeptor- Agonist, Maxim) | |
| | Tiazofurin (IMPDH-Inhibitor, Ribapharm) | Indisulam (p53-Stimulans, Eisai) |
| | Cilengitid (Integrin-Antagonist, Merck KGaA) | Aplidin (PPT-Inhibitor, PharmaMar) |
| | SR-31747 (IL-1-Antagonist, Sanofi-Synthelabo) | Rituximab (CD20-Antikörper, Genentech) |
| | | Gemtuzumab (CD33-Antikörper, Wyeth Ayerst) |
| | CCI-779 (mTOR-Kinase-Inhibitor, Wyeth) | |
| | | PG2 (Hämatopoese-Verstärker, Pharmagenesis) |
| | Exisulind (PDE-V-Inhibitor, Cell Pathways) | |
| | CP-461 (PDE-V-Inhibitor, Cell Pathways) | Immunol™ (Triclosan-Oralspülung, Endo) |
| | AG-2037 (GART-Inhibitor, Pfizer) | Triacetyluridin (Uridin-Prodrug, Wellstat) |
| | WX-UK1 (Plasminogenaktivator-Inhibitor, Wilex) | SN-4071 (Sarkom-Mittel, Signature BioScience) |
| | | TransMID-107™ (Immunotoxin, KS Biomedix) |
| | PBI-1402 (PMN-Stimulans, ProMetic LifeSciences) | |
| | Bortezomib (Proteasom-Inhibitor, Millennium) | PCK-3145 (Apoptose-Förderer, Procyon) |
| | SRL-172 (T-Zell-Stimulans, SR Pharma) | Doranidazol (Apoptose-Förderer, Pola) |
| | TLK-286 (Glutathion-S-Transferase-Inhibitor, Telik) | CHS-828 (cytotoxisches Mittel, Leo) |
| | | trans-Retinsäure (Differentiator, NIH) |
| | PT-100 (Wachstumsfaktor-Agonist, Point Therapeutics) | |
| | | MX6 (Apoptose-Förderer, MAXIA) |
| | Midostaurin (PKC-Inhibitor, Novartis) | Apomin (Apoptose-Förderer, ILEX Oncology) |
| | Bryostatin-1 (PKC-Stimulans, GPC Biotech) | Urocidin (Apoptose-Förderer, Bioniche) |
| | CDA-II (Apoptose-Förderer, Everlife) | Ro-31-7453 (Apoptose-Förderer, La Roche) |
| | SDX-101 (Apoptose-Förderer, Salmedix) | Brostallicin (Apoptose-Förderer, Pharmacia) |
| | Ceflatonin (Apoptose-Förderer, ChemGenex) | |
| | | |

Eine derartige gemeinsame Behandlung kann mithilfe gleichzeitiger, aufeinander folgender oder getrennter Dosierung der einzelnen Komponenten der Behandlung erzielt werden. Solche Kombinationsprodukte setzen die erfindungsgemäßen Verbindungen ein.

### ASSAYS

Die in den Beispielen beschriebenen Verbindungen der Formel I wurden in den unten beschriebenen Assays geprüft, und es wurde gefunden, dass sie eine kinasehemmende Wirkung aufweisen. Weitere Assays sind aus der Literatur bekannt und könnten vom Fachmann leicht durchgeführt werden (siehe z.B. Dhanabal et al., Cancer Res. 59:189-197; Xin et al., J. Biol. Chem. 274:9116-9121; Sheu et al., Anticancer Res. 18:4435-4441; Ausprunk et al., Dev. Biol. 38:237-248; Gimbrone et al., J. Natl. Cancer Inst. 52:413-427; Nicosia et al., In Vitro 18:538- 549).

### Messung der Met Kinase Aktivität

Die Met Kinase wird laut Herstellerangaben (Met, active, Upstate, Katalog-Nr. 14-526) zum Zweck der Proteinproduktion in Insektenzellen (Sf21; S. frugiperda) und der anschließenden affinitätschromatographischen Aufreinigung als "N-terminal 6His-tagged" rekombinantes humanes Protein in einem Baculovirus-Expressionsvektor exprimiert.

Zur Messung der Kinase-Aktivität kann auf verschiedene zur Verfügung stehender Meßsysteme zurückgegriffen werden. Beim Scintillation-Proximity- (Sorg et al., J. of. Biomolecular Screening, 2002, 7, 11-19), dem FlashPlate-Verfahren oder dem Filterbindungstest wird die radioaktive Phosphorylierung eines Proteins oder Peptids als Substrat mit radioaktiv markiertem ATP (³²P-ATP, ³³P-ATP) gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-Antikörper bindet nur das phosphorylierte Substrat. Diese Bindung ist mit einem zweiten Peroxidase-konjugierten Antikörper durch Chemilumineszenz nachweisbar (Ross et al., 2002, Biochem. J.).

### Flashplate-Verfahren (Met Kinase):

Als Testplatten dienen 96-well Flashplate^{R} Mikrotiterplatten der Firma Perkin Elmer (Kat.-Nr. SMP200). In die Assay Platte werden die Komponenten der unten beschriebenen Kinasereaktion pipettiert.
Die Met Kinase und das Substrat poly Ala-Glu-Lys-Tyr, (pAGLT, 6:2:5:1). werden mit radioaktiv markiertem ³³P-ATP in An- und Abwesenheit von Testsubstanzen in einem Gesamtvolumen von 100 µl bei Raumtemperatur 3 Std. inkubiert. Die Reaktion wird mit 150 µl einer 60mM EDTA-Lösung abgestoppt. Nach Inkubation für weitere 30 min bei Raumtemperatur werden die Überstände abgesaugt und die Wells dreimal mit je 200 µl 0,9% NaCl-Lösung gewaschen. Die Messung der gebundenen Radioaktivität erfolgt mittels eines Szintillationsmessgerätes (Topcount NXT, Fa. Perkin-Elmer).
Als Vollwert wird die Inhibitor-freie Kinasereaktion verwendet. Dieser sollte ca. im Bereich von 6000-9000 cpm liegen. Als pharmakologischer Nullwert wird Staurosporin in einer Endkonzentration von 0,1 mM verwendet. Eine Bestimmung der Hemmwerte (IC50) erfolgt unter Verwendung des Programms RS1_MTS ().

Kinase-Reaktionsbedingungen pro well:
30 µl Assaypuffer
10 µl zu testende Substanz in Assaypuffer mit 10 % DMSO
10 µl ATP (Endkonzentration 1 µM kalt, 0,35 µCi ³³P-ATP)
50 µl Gemisch Met Kinase/Substrat in Assaypuffer;
   (10 ng Enzym/well, 50 ng pAGLT/well)

Verwendete Lösungen:
- Assay-Puffer:
   50 mM HEPES
   3 mM Magnesiumchlorid
   3 µM Natrium orthovanadat
   3 mM Mangan (II) chlorid
   1 mM Dithiothreitol (DTT)
   pH= 7,5 (einzustellen mit Natriumhydroxid)
- Stopp-Lösung:
   60 mM Titriplex III (EDTA)
- ³³P-ATP: Perkin-Elmer;
- Met Kinase: Upstate, Kat.-Nr. 14-526, Stock 1 µg/10 µl; spez. Aktivität 954 U/mg;
- Poly-Ala-Glu-Lys-Tyr, 6:2:5:1: Sigma Kat.-Nr. P1152

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-Ionisation) M⁺ |
| | FAB (Fast Atom Bombardment) (M+H)⁺ |
| | ESI (Electrospray lonization) (M+H)⁺ |

APCI-MS (atmospheric pressure chemical ionization - mass spectrometry) (M+H)⁺.

### Retentionszeit Rₜ [min] : Bestimmung erfolgt mit HPLC

Säule: Chromolith SpeedROD, 50 x 4.6 mm² (Best.Nr. 1.51450.0001) von Merck
Gradient: 5.0 min, t = 0 min, A:B = 95:5, t = 4.4 min: A:B = 25:75,
t = 4.5 min bis t = 5.0 min: A:B = 0:100
Fluß: 3.00 ml/min
Laufmittel A: Wasser + 0,1% TFA (Trifluoressigsäure),
Laufmittel B: Acetonitril + 0,08% TFA
Wellenlänge: 220 nm

Die verwendeten Pyridazinone wurden, wenn nicht käuflich erhältlich, in der Regel nach W. J. Coates, A. McKillop, Synthesis, 1993, 334-342 hergestellt.

### Beispiel 1

Die Herstellung von 6-(3,5-Difluor-phenyl)-2-[3-(5-methyl-[1,2,4]oxadiazol-3-yl)-benzyl]-2*H*-pyridazin-3-on ("A1") erfolgt analog nachstehendem

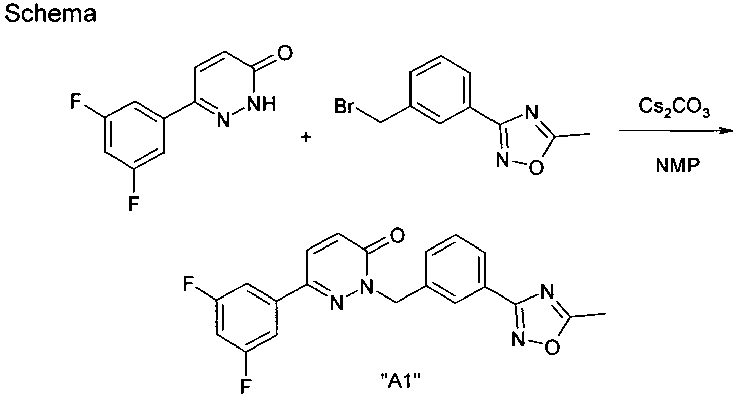

Eine Lösung von 104 mg (0.50 mmol) 6-(3,5-Difluorphenyl)-2H-pyridazin-3-on und 127 mg (0.50 mmol) 3-(3-Brommethyl-phenyl)-5-methyl-[1,2,4]oxadiazol (hergestellt nach W. W. K. R. Mederski et al, Tetrahedron 55, 1999, 12757-12770) in 1 ml 1-Methylpyrrolidinon (NMP) wird mit 163 mg (0.50 mmol) Caesiumcarbonat versetzt und die entstandene Suspension 18 Stunden bei Raumtemperatur gerührt. Das Reaktionsgemisch wird mit Wasser versetzt, der entstandene Niederschlag abfiltriert, mit Wasser gewaschen und getrocknet: 6-(3,5-Difluorphenyl)-2-[3-(5-methyl-[1,2,4]oxadiazol-3-yl)-benzyl]-2*H*-pyridazin-3-on ("A1") als leicht gelbliche Kristalle; ESI 381.

Analog erhält man die nachstehenden Verbindungen

| Nr. | Name und/oder Struktur | ESI |
|---|---|---|
| "A2" | 2-[3-(5-Methyl-[1,2,4]oxadiazol-3-yl)-benzyl]-6-(3,4,5-trifluor-phenyl)-2*H*-pyridazin-3-on | 399 |
| | ¹H-NMR (d₆-DMSO): δ [ppm] = 2.66 (s, 3H), 5.43 (s, 2H), 7.15 (d, J = 9.5 Hz, 1H), 7.57 (m, 2H), 7.99 (m, 3H), 8.05 (bs, 1H), 8.14 (d, J = 9.5 Hz, 1H) | |
| "A3" | 6-(3,5-Difluor-phenyl)-2-[4-(5-methyl-[1,2,4]oxadiazol-3-yl)-benzyl]-2*H*-pyridazin-3-on | 381 |
| "A4" | 6-(4-Fluor-phenyl)-2-[3-(5-methyl-[1,2,4]oxadiazol-3-yl)-benzyl]-2*H*-pyridazin-3-on | 363 |
| "A5" | 2-[3-(5-Methyl-[1,2,4]oxadiazol-3-yl)-benzyl]-6-pyridin-3-yl-2*H*-pyridazin-3-on | 346 |
| | 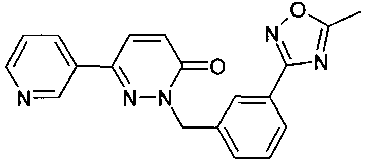 | |
| "A6" | 6-(3-Chlor-phenyl)-2-[3-(5-methyl-[1,2,4]oxadiazol-3-yl)-benzyl]-2*H*-pyridazin-3-on | 379 |
| "A7" | 6-(3-Cyan-phenyl)-2-[3-(5-methyl-[1,2,4]oxadiazol-3-yl)-benzyl]-2*H*-pyridazin-3-on | 370 |
| "A8" | 6-(4-Cyan-phenyl)-2-[3-(5-methyl-[1,2,4]oxadiazol-3-yl)-benzyl]-2*H*-pyridazin-3-on | 370 |
| "A9" | 6-(4-Methylsulfonyl-phenyl)-2-[3-(5-methyl-[1,2,4]oxadiazol-3-yl)-benzyl]-2*H*-pyridazin-3-on | 423 |
| "A35" | 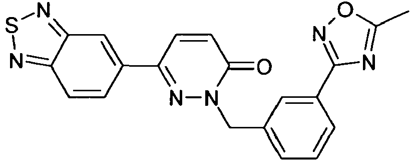 | |
| "A39" | 6-(3,4-Difluorphenyl)-2-[3-(5-methyl-[1,2,4]oxadiazol-3-yl)-benzyl]-2*H*-pyridazin-3-on | 381 |

### Beispiel 2

Die Herstellung von 6-(3,5-Difluor-phenyl)-2-[3-(5-dimethylaminomethyl-[1,2,4]oxadiazol-3-yl)-benzyl]-2*H*-pyridazin-3-on ("A10") erfolgt analog nachstehendem Schema 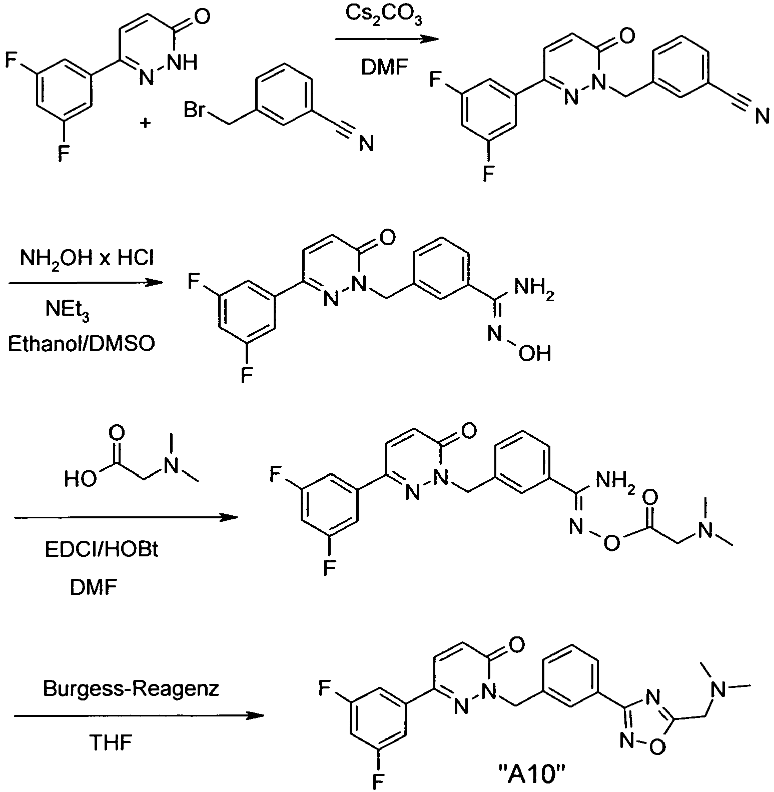
2.1 Eine Lösung von 10.4 g (50.0 mmol) 6-(3-Fluorphenyl)-2*H-*pyridazin-3-on und 9.80 g (50. mmol) 3-(Brommethyl)-benzonitril in 100 ml DMF wird mit 16.3 g (50.0 mmol) Caesiumcarbonat versetzt und die entstandene Suspension 18 Stunden bei Raumtemperatur gerührt. Das Reaktionsgemisch wird auf Wasser gegossen, der entstandene Niederschlag abgesaugt, mit Wasser gewaschen und getrocknet: 3-[3-(3,5-Difluorphenyl)-6-oxo-6*H*-pyridazin-1-ylmethyl]-benzonitril als farblose Kristalle; ESI 324.
2.2 Eine Suspension von 4.85 g (15.0 mmol) 3-[3-(3,5-Difluorphenyl)-6-oxo-6*H*-pyridazin-1-ylmethyl]-benzonitril in einem Gemisch von 10 ml Ethanol und 3 ml DMSO werden mit 5.21 g (75.0 mmol) Hydroxylammoniumchlorid und 7.59 g (75.0 mmol) Triethylamin versetzt und 70 Stunden bei Raumtemperatur gerührt. Das Reaktionsgemisch wird auf Wasser gegossen, der entstandene Niederschlag abgesaugt, mit Wasser gewaschen und getrocknet: 3-[3-(3,5-Difluorphenyl)-6-oxo-6*H-*pyridazin-1-ylmethyl]-*N*-hydroxy-benzamidin als farblose Kristalle; ESI 357.
2.3 Eine Lösung von 56.7 mg (0.505 mmol) Dimethylaminoessigsäure in 2 ml DMF wird mit 125 mg (0.65 mmol) N-(3-Dimethylaminopropyl)-N'-ethylcarbodiimid-hydrochlorid (EDCI) und 76.8 mg (0.50 mmol) 1-Hydroxybenzotriazol-hydrat (HOBt) versetzt und die Reaktionslösung 15 Minuten bei Raumtemperatur gerührt. Dann werden 178 mg (0.50 mmol) 3-[3-(3,5-Difluorphenyl)-6-oxo-6*H*-pyridazin-1-ylmethyl]-N-hydroxy-benzamidin zugegeben und das Reaktionsgemisch 3 Stunden bei Raumtemperatur gerührt. Das Reaktionsgemisch wird mit Wasser versetzt, wobei sich ein allmählich kristallisierender Niederschlag abscheidet: 3-[3-(3,5-Difluorphenyl)-6-oxo-6*H*-pyridazin-1-ylmethyl]-N-(dimethylaminoacetyloxy)-benzamidin als gelbliche Kristalle; ESI 442.
2.4 Eine Lösung von 150 mg (0.34 mmol) 3-[3-(3,5-Difluorphenyl)-6-oxo-6*H*-pyridazin-1-ylmethyl]-N-(dimethylaminoacetyloxy)-benzamidin und 105 mg (1.3 mmol) (Methoxycarbonylsulfamoyl)-triethylammonium-betain (Burgess-Reagenz) in 1 ml THF wird 18 Stunden bei 80° C gerührt. Das Reaktionsgemisch wird zwischen gesättigter Natriumhydrogencarbonat-lösung und Dichlormethan verteilt. Die organische Phase wird über Natriumsulfat getrocknet und eingedampft. Der kristalline Rückstand wird mit tert.-Butylmethylether digeriert, abgesaugt und der Rückstand im Vakuum getrocknet. Dieses Material wird in 4 ml einer 0.1 M Lösung von Chlorwasserstoff in 2-Propanol unter Erwärmen gelöst und mit tert.-Butyl-methylether versetzt. Der entstandene Niederschlag wird abgesaugt, mit tert.Butylmethylether gewaschen und im Vakuum getrocknet: 6-(3,5-Difluorphenyl)-2-[3-(5-dimethylaminomethyl-[1,2,4]oxadiazol-3-yl)-benzyl]-2*H*-pyridazin-3-on Hydrochlorid ("A10") als farblose Kristalle; ESI 424.

Analog erhält man die Verbindungen 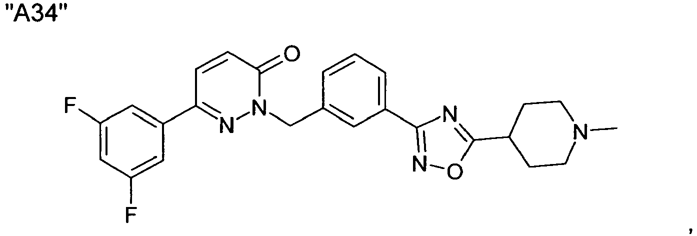 Hydrochlorid, ESI 464 und
(über die BOC-geschützte Verbindung)
"A41" 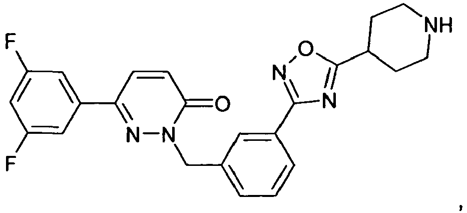 Hydrochlorid, ESI 450.

### Beispiel 3

Die Herstellung von 6-(3,5-Difluorphenyl)-2-[3-(5-ethylamino-[1,2,4]oxadiazol-3-yl)-benzyl]-2*H*-pyridazin-3-on ("A11") und von 6-(3,5-Difluorphenyl)-2-{3-[5-(3-dimethylamino-propylamino)-[1,2,4]oxadiazol-3-yl]-benzyl}-2*H*-pyridazin-3-on ("A12") erfolgt analog nachstehendem

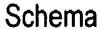

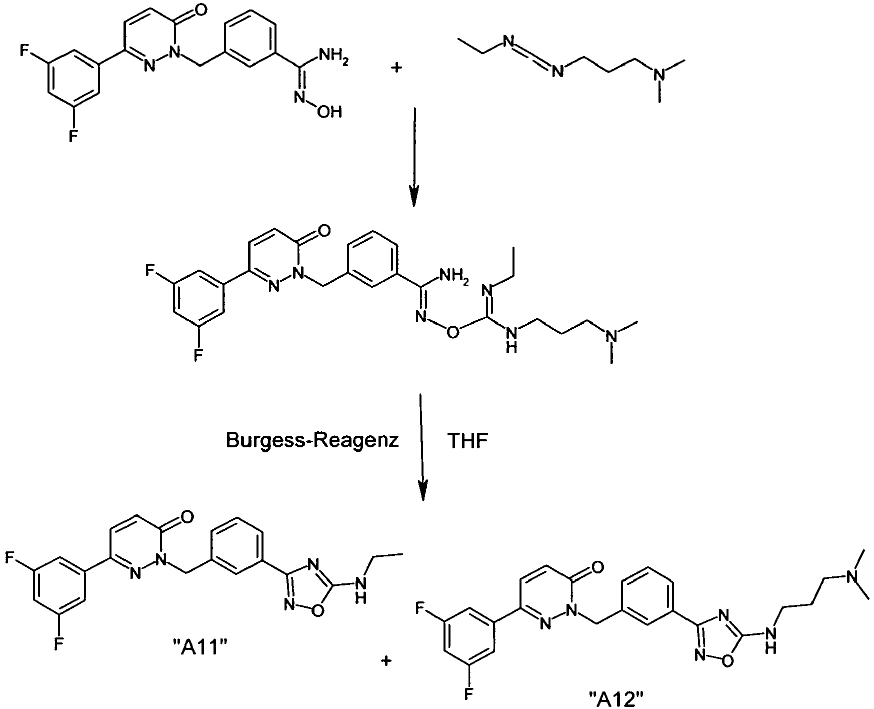

Eine Lösung von 178 mg (0.50 mmol) 3-[3-(3,5-Difluorphenyl)-6-oxo-6*H-*pyridazin-1-ylmethyl]-N-hydroxy-benzamidin in 2 ml DMF wird mit 125 mg (0.65 mmol) N-(3-Dimethylaminopropyl)-N'-ethylcarbodiimid-hydrochlorid (EDCI) versetzt und 18 Stunden bei Raumtemperatur gerührt. Das Reaktionsgemisch wird zwischen gesättigter Natriumhydrogencarbonatlösung und Dichlormethan verteilt. Die organische Phase wird über Natriumsulfat getrocknet und eingedampft. Man erhält das Addukt von EDCI an das Hydroxyamidinderivat als gelbes Öl (ESI 512). Dieses Material wird ohne weitere Reinigung in 2 ml THF gelöst, mit 119 mg (0.50 mmol) (Methoxycarbonylsulfamoyl)-triethylammonium-betain (Burgess-Reagenz) versetzt und drei Stunden bei 60° C gerührt. Das Reaktionsgemisch wird eingedampft und der Rückstand durch präparative HPLC getrennt. Man erhält 6-(3,5-Difluorphenyl)-2-[3-(5-ethylamino-[1,2,4]oxadiazol-3-yl)-benzyl]-2*H*-pyridazin-3-on als farblose Kristalle (ESI 410) und 6-(3,5-Difluorphenyl)-2-{3-[5-(3-dimethylamino-propylamino)-[1,2,4]oxadiazol-3-yl]-benzyl}-2*H*-pyridazin-3-on (ESI 467). Letzteres wird durch Lösen in 1 N Salzsäure und Lyophilisieren in das Hydrochlorid überführt.

"A11": ¹H-NMR (d₆-DMSO): δ [ppm] = 1.17 (t, J = 7 Hz, 3H), 3.33 (quintett, J = 7 Hz, 2H), 5.43 (s, 2H), 7.14 (d, J = 9.5 Hz, 1 H), 7.36 (tt, J₁ = 9 Hz, J₂ = 2 Hz, 1 H), 7.49 (t, J = 7.5 Hz, 1 H), 7.54 (d, J = 7.5 Hz, 1 H), 7.66 (m, 2H), 7.82 (d, J = 7.5 Hz, 1 H), 7.95 (bs, 1 H), 8.15 (d, J = 9.5 Hz, 1 H), 8.42 (t, J = 5 Hz, 1H).

"A12": ¹H-NMR (d₆-DMSO): δ [ppm] = 1.97 (m, 2H), 2.76 (d, J = 4 Hz, 6H), 3.12 (m, 2H), 3.39 (q, J = 6.5 Hz, 2H), 5.42 (s, 2H), 7.14 (d, J = 9.5 Hz, 1H), 7.36 (tt, J₁ = 9 Hz, J₂ = 2 Hz, 1H), 7.50 (t, J = 7.5 Hz, 1H), 7.55 (d, J = 7.5 Hz, 1 H), 7.65 (m, 2H), 7.83 (d, J = 7.5 Hz, 1 H), 7.94 (bs, 1 H), 8.16 (d, J = 9.5 Hz, 1 H), 8.56 (t, J = 5 Hz, 1 H), 9.96 (bs, 1H).

### Beispiel 4

Alternativ kann "A12" analog nachstehendem Schema hergestellt werden: 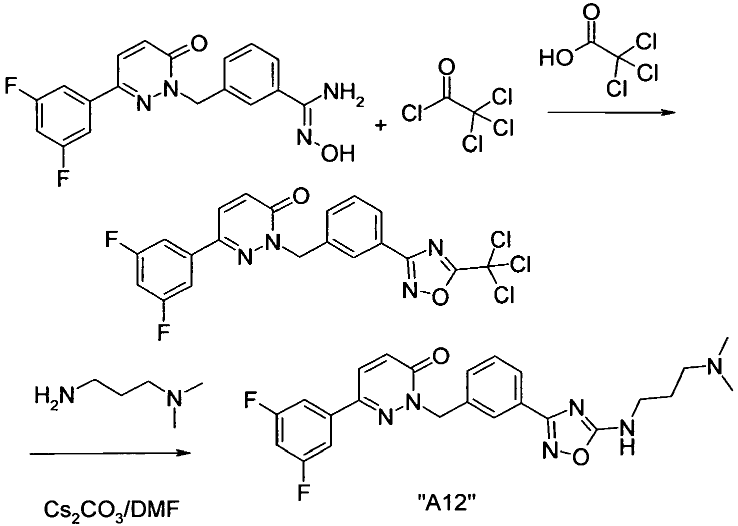
4.1 Eine Suspension von 1.42 g (4.0 mmol) 3-[3-(3,5-Difluorphenyl)-6-oxo-6*H*-pyridazin-1-ylmethyl]-N-hydroxy-benzamidin in 2.6 g Trichloressigsäure wird unter Rühren auf 85° C erhitzt. Man gibt tropfenweise 2.23 g (12.0 mmol) Trichloracetylchlorid zu und rührt über 18 Stunden bei 94° C. Man lässt abkühlen und verteilt das Reaktionsgemisch zwischen Wasser und Ethylacetat. Die wässrige Phase wird noch zweimal mit Ethylacetat extrahiert. Die vereinigten organischen Phasen werden mit gesättigter Natriumhydrogencarbonatlösung gewaschen, über Natriumsulfat getrocknet und eingedampft. Der Rückstand, ein langsam kristallisierendes Öl, wird mit Wasser verrührt und abgesaugt und im Vakuum getrocknet: 6-(3,5-Difluorphenyl)-2-[3-(5-trichlormethyl-[1,2,4]oxadiazol-3-yl)-benzyl]-2*H-*pyridazin-3-on als farblose Kristalle; ESI 483.
4.2 Eine Lösung von 967 mg (2.00 mmol) 6-(3,5-Difluorphenyl)-2-[3-(5-trichlormethyl-[1,2,4]oxadiazol-3-yl)-benzyl]-2*H*-pyridazin-3-on in 4 ml DMF wird mit 652 mg (2.00 mmol) Caesiumcarbonat und 245 mg N,N-Dimethyl-trimethylendiamin versetzt und über Nacht bei Raumtemperatur gerührt. Das Reaktionsgemisch wird mit Wasser versetzt. Der entstandene Niederschlag wird abgesaugt, im Vakuum getrocknet und an einer Kieselgelsäule mit Dichlormethan/Methanol als Laufmittel chromatographiert. Die Produkt enthaltenden Fraktionen werden eingeengt und der Rückstand mit 0.1 N Chlorwasserstoffsäure in 2-Propanol ins Hydrochlorid überführt: 6-(3,5-Difluorphenyl)-2-{3-[5-(3-dimethylamino-propylamino)-[1,2,4]oxadiazol-3-yl]-benzyl}-2*H*-pyridazin-3-on Hydrochlorid als farblose Kristalle; ESI 467.

### Analog erhält man die Verbindungen

6-(3,5-Difluor-phenyl)-2-{3-[5-(2-dimethylamino-ethylamino)-[1,2,4]oxadiazol-3-yl]-benzyl}-2*H*-pyridazin-3-on ("A27"), ESI 453;

(über die BOC-geschützte Verbindung)
6-(3,5-Difluor-phenyl)-2-{3-[5-(piperidin-4-ylamino)-[1,2,4]oxadiazol-3-yl]-benzyl}-2*H*-pyridazin-3-on ("A42") 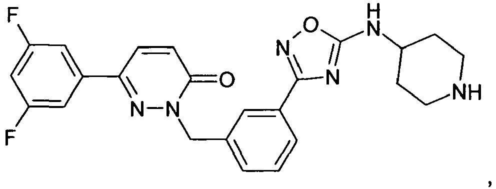 Hydrochlorid, ESI 465;

2-{3-[5-(1-Methyl-piperidin-4-ylamino)-[1,2,4]oxadiazol-3-yl]-benzyl}-6-(3,4,5-trifluor-phenyl)-2*H*-pyridazin-3-on ("A43") 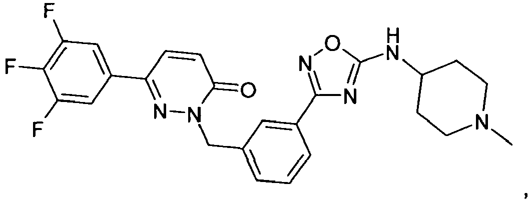 Hydrochlorid, ESI 497.

### Beispiel 5

Die Herstellung von 6-(3,5-Difluor-phenyl)-2-[3-(5-methyl-oxazol-2-yl)-benzyl]-2*H*-pyridazin-3-on ("A13"), ESI 380, erfolgt analog nachstehendem

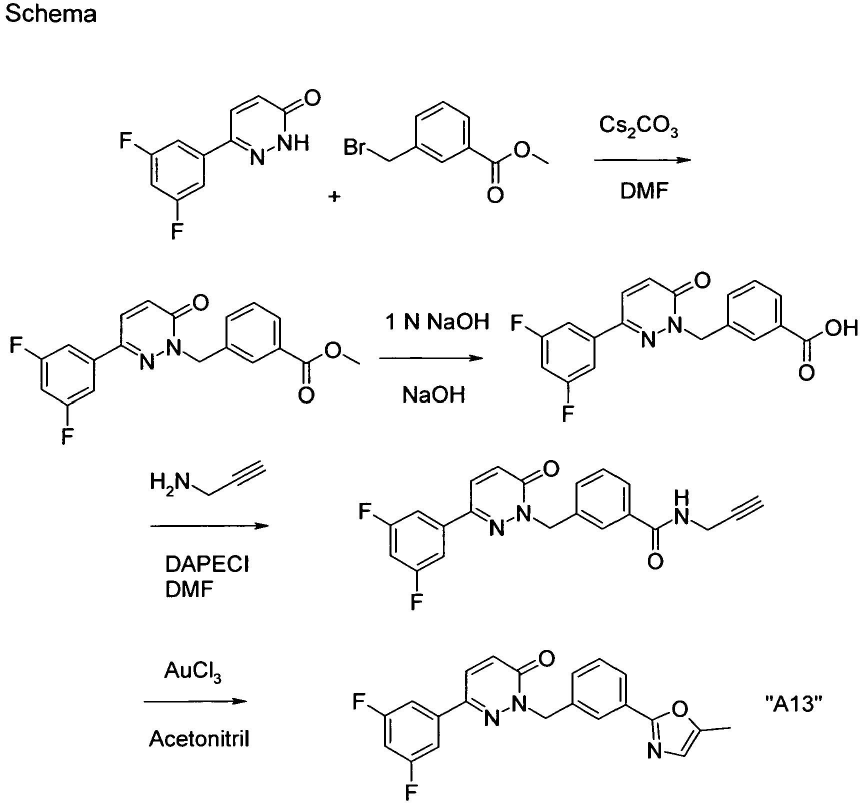

Die letzte Stufe wird durchgeführt nach A. S. K. Hashmi et al., Org. Lett. 2004, Vol. 6, 4391-4394.

Analog werden die nachstehenden Verbindungen erhalten

| Nr. | Name und/oder Struktur | ESI |
|---|---|---|
| "A32" | 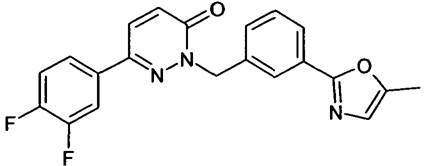 | 380 |
| "A33" | 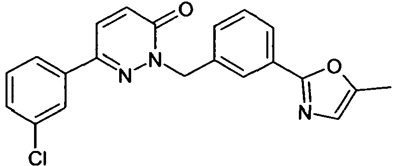 | 378 |
| ¹H-NMR (d₆-DMSO): δ [ppm] = 2.38 (d, J = 1Hz, 3H), 5.42 (s, 2H), 6.98 (q, J = 1Hz, 1H), 7.13 (d, J = 10 Hz, 1H), 7.51 (m, 4H), 7.86 (m, 2H), 7.95 (s, 1H), 7.97 (s, 1H), 8.13 (d, J = 10 Hz, 1H) | | |
| "A44" | 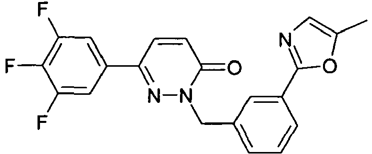 | 398 |

### Beispiel 6

Die Herstellung von 6-(3,5-Difluor-phenyl)-2-[3-(3-methyl-[1,2,4]oxadiazol-5-yl)-benzyl]-2*H*-pyridazin-3-on ("A14"), erfolgt analog nachstehendem

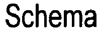

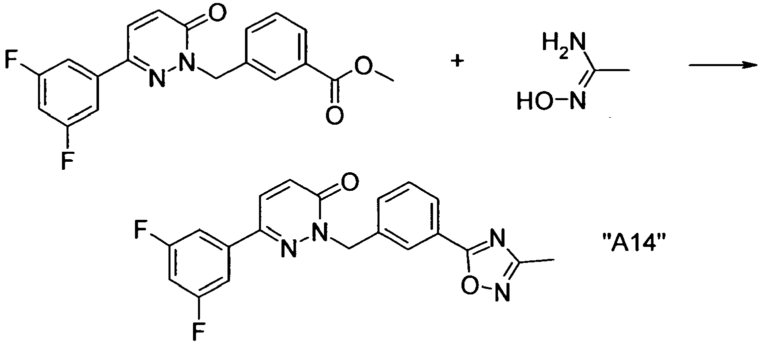

Eine Suspension von 222 mg (3.00 mmol) N-Hydroxyacetamidin in 6 ml DMF wird mit 326 mg (1.00 mmol) Caesiumcarbonat versetzt und 1 Stunde gerührt. Dann werden 356 mg (1.00 mmol) 3-[3-(3,5-Difluorphenyl)-6-oxo-6*H*-pyridazin-1-ylmethyl]-benzoesäuremethylester zugegeben und 18 Stunden bei Raumtemperatur gerührt. Das Reaktionsgemisch wird zwischen Wasser und Dichlormethan verteilt. Die organische Phase wird über Natriumsulfat getrocknet, eingedampft und mit präparativer HPLC gereinigt: 6-(3,5-Difluorphenyl)-2-[3-(3-methyl-[1,2,4]oxadiazol-5-yl)-benzyl]-2*H*-pyridazin-3-on ("A14") als farbloser Feststoff; ESI 381.

### Beispiel 7

Die Herstellung von 6-(3,5-Difluorphenyl)-2-[3-(5-oxo-4,5-dihydro-[1,3,4]oxadiazol-2-yl)-benzyl]-2*H*-pyridazin-3-on ("A15") erfolgt analog nachstehendem Schema: 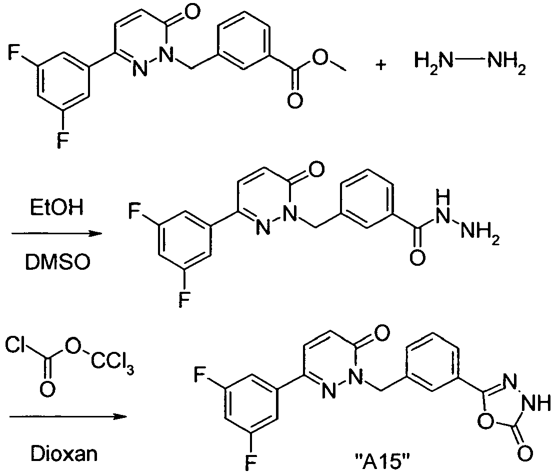
7.1 Eine Suspension von 1.07 g (3.0 mmol) 3-[3-(3,5-Difluorphenyl)-6-oxo-6*H*-pyridazin-1-ylmethyl]-benzoesäuremethylester in einem Gemisch von 10 ml Ethanol und 4 ml Dimethylsulfoxid wird mit 1.50 g (10 mmol) Hydrazinhydrat versetzt und 40 Stunden bei 70° C gerührt. Das Reaktionsgemisch wird mit Wasser versetzt. Der entstandene Niederschlag wird abgesaugt, mit Wasser gewaschen und im Vakuum getrocknet. Das Rohprodukt wird aus 2-Propanol umkristallisiert: 3-[3-(3,5-Difluorphenyl)-6-oxo-6*H*-pyridazin-1-ylmethyl]-benzoesäurehydrazid als farblose Kristalle; ESI 357.
7.2 Eine Suspension von 178 mg (0.50 mmol) 3-[3-(3,5-Difluorphenyl)-6-oxo-6*H*-pyridazin-1-ylmethyl]-benzoesäurehydrazid in 1 ml Dioxan wird mit einer Lösung von 131 mg (0.65 mmol) Trichlormethylformiat in 3 ml Dioxan versetzt und 4 Stunden zum Sieden erhitzt. Man lässt das Reaktionsgemisch abkühlen und versetzt es mit Wasser. Der entstandene Niederschlag wird abgesaugt, mit Wasser gewaschen und getrocknet. Der Rückstand wird an einer Kieselgelsäule mit Dichlormethan/Methanol chromatographiert. Man erhält 6-(3,5-Difluorphenyl)-2-[3-(5-oxo-4,5-dihydro-[1,3,4]oxadiazol-2-yl)-benzyl]-2*H*-pyridazin-3-on ("A15") als farblose Kristalle; ESI 383;

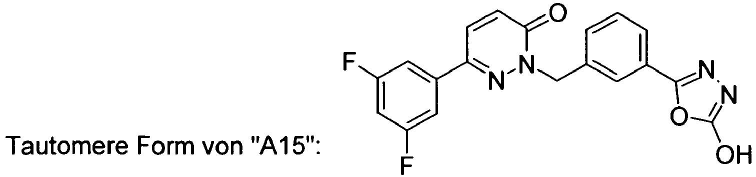

### Beispiel 8

Die Herstellung von 6-(3,5-Difluorphenyl)-2-{3-[5-(2-morpholin-4-yl-ethylamino)-[1,3,4]oxadiazol-2-yl]-benzyl}-2*H*-pyridazin-3-on ("A16") erfolgt analog nachstehendem Schema 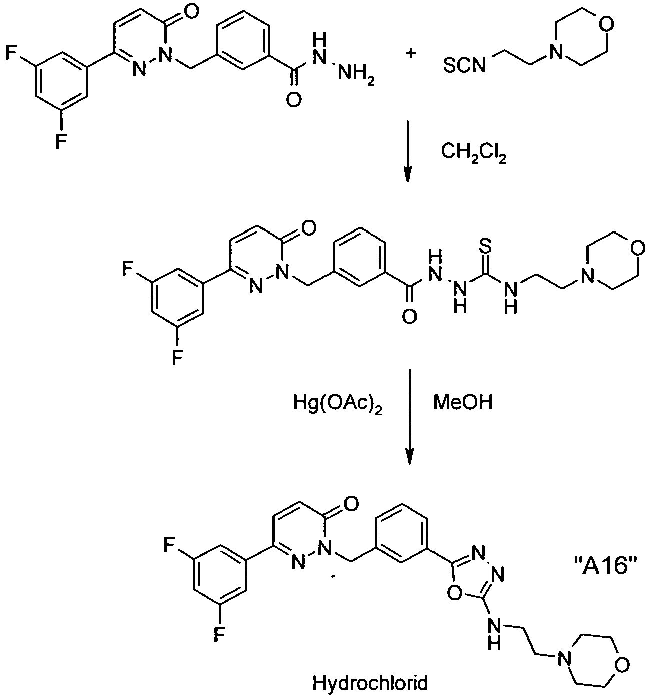
8.1 Eine Suspension von 287 mg (0.80 mmol) 3-[3-(3,5-Difluorphenyl)-6-oxo-6*H*-pyridazin-1-ylmethyl]-benzoesäurehydrazid in 3 ml Dichlormethan wird mit 142 mg (0.80 mmol) 4-(2-Isothiocyanato-ethyl)-morpholin versetzt und 24 Stunden bei 60° C gerührt. Das Reaktionsgemisch wird eingedampft: 3-[3-(3,5-Difluorphenyl)-6-oxo-6*H*-pyridazin-1-ylmethyl]-benzoesäure-N'-(4-morpholin-4-yl-ethyl-aminothiocarbonyl)-hydrazid als gelbliches Öl; ESI 529.
8.2 Eine Lösung von 260 mg (0.49 mmol) 3-[3-(3,5-Difluorphenyl)-6-oxo-6H-pyridazin-1-ylmethyl]-benzoesäure-N'-(4-morpholin-4-yl-ethyl-aminothiocarbonyl)-hydrazid in 2 ml Methanol wird mit 172 mg (0.54 mmol) Quecksilber(II)-acetat versetzt und das Reaktionsgemisch 1.5 Stunden bei 80° C gerührt. Das Reaktionsgemisch wird über Kieselgur filtriert und das Filtrat eingedampft. Der Rückstand wird an einer Kieselgelsäule mit Dichlormethan/Methanol als Laufmittel chromatographiert. Das Produkt wird mit 5 ml 0.1 N Chlorwasserstoffsäure in 2-Propanol ins Hydrochlorid überführt: 6-(3,5-Difluorphenyl)-2-{3-[5-(2-morpholin-4-yl-ethylamino)-[1,3,4]oxadiazol-2-yl]-benzyl}-2*H*-pyridazin-3-on Hydrochlorid als leicht gelbliche Kristalle; ESI 495.

### Beispiel 9

Die Herstellung von 6-(3,5-Difluorphenyl)-2-[3-(5-oxo-4,5-dihydro-[1,2,4]oxadiazol-3-yl)-benzyl]-2*H*-pyridazin-3-on ("A17") erfolgt analog nachstehendem Schema

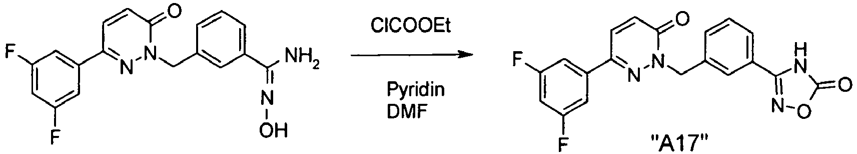

Eine Lösung von 3.56 g (10.0 mmol) 3-[3-(3,5-Difluorphenyl)-6-oxo-6*H-*pyridazin-1-ylmethyl]-N-hydroxy-benzamidin in 20 ml DMF wird mit 2.37 g (30.0 mmol) Pyridin und 1.19 g (11.0 mmol) Ethylchlorformiat versetzt und 18 Stunden bei 80° C und 24 Stunden bei 100° C gerührt. Das Reaktionsgemisch wird mit 60 ml 1 N Salzsäure und Wasser versetzt. Der entstandene Niederschlag wird abgesaugt, mit Wasser gewaschen und im Vakuum getrocknet. Das Rohprodukt wir aus Isopropanol umkristallisiert: 6-(3,5-Difluorphenyl)-2-[3-(5-oxo-4,5-dihydro-[1,2,4]oxadiazol-3-yl)-benzyl]-2*H*-pyridazin-3-on ("A17") als farblose Kristalle; ESI 383.

### Beispiel 10

Die Herstellung von 6-(3,5-Difluorphenyl)-2-[3-(5-thioxo-4,5-dihydro-[1,2,4]oxadiazol-3-yl)-benzyl]-2*H*-pyridazin-3-on ("A18") und von 6-(3,5-Difluorphenyl)-2-{3-[5-(3-dimethylamino-propylsulfanyl)-[1,2,4]oxadiazol-3-yl]-benzyl}-2*H*-pyridazin-3-on ("A19") erfolgt analog nachstehendem 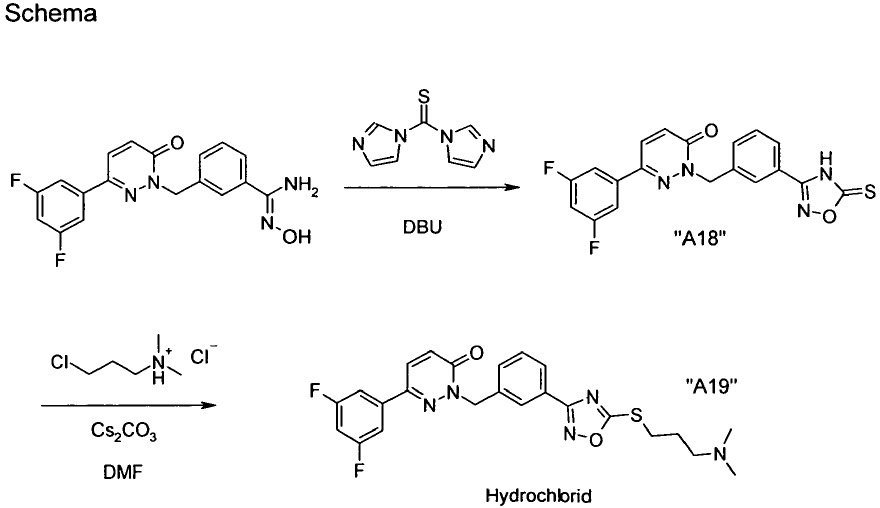
10.1 Eine Suspension von 356 mg (1.00 mmol) 3-[3-(3,5-Difluorphenyl)-6-oxo-6*H*-pyridazin-1-ylmethyl]-N-hydroxybenzamidin in 8 ml Acetonitril wird mit 274 mg (1.49 mmol) 1,1-Thiocarbonyldiimidazol und 609 mg (4.00 mmol) 1,8-Diazabicyclo[5.4.0]undec-7-en versetzt und 18 Stunden bei Raumtemperatur gerührt. Das Reaktionsgemisch wird mit 16 ml Wasser versetzt, mit 2 N Salzsäure neutralisiert, der entstandene Niederschlag abgesaugt und im Vakuum getrocknet. Das Rohprodukt wird aus 2-Propanol umkristallisiert: 6-(3,5-Difluorphenyl)-2-[3-(5-thioxo-4,5-dihydro-[1,2,4]oxadiazol-3-yl)-benzyl]-2*H*-pyridazin-3-on ("A18") als farblose Kristalle; ESI 399.
10.2 Eine Lösung von 140 mg (0.35 mmol) 6-(3,5-Difluorphenyl)-2-[3-(5-thioxo-4,5-dihydro-[1,2,4]oxadiazol-3-yl)-benzyl]-2*H*-pyridazin-3-on in 1 ml DMF wird mit 172 mg (0.42 mmol) Caesiumcarbonat und 67 mg (0.42 mmol) (3-Chlorpropyl)-dimethyl-ammonium-chlorid versetzt und die entstandene Suspension 5 Tage bei Raumtemperatur gerührt. Das Reaktionsgemisch wird mit Wasser versetzt. Der entstandene Niederschlag wird abfiltriert und an einer Kieselgelsäule mit Dichlormethan / Methanol als Laufmittel chromatographiert. Das Produkt wird mit 5 ml 0.1 N Chlorwasserstoffsäure in 2-Propanol ins Hydrochlorid überführt: 6-(3,5-Difluorphenyl)-2-{3-[5-(3-dimethylamino-propylsulfanyl)-[1,2,4]oxadiazol-3-yl]-benzyl}-2*H*-pyridazin-3-on Hydrochlorid ("A19") als farblose Kristalle; ESI 484.

### Beispiel 11

Die Herstellung von 6-(3,5-Difluorphenyl)-2-{3-[5-(4-methyl-piperazin-1-yl)-[1,2,4]oxadiazol-3-yl]-benzyl}-2*H*-pyridazin-3-on ("A20") und 6-(3,5-Difluorphenyl)-2-{3-[5-(4-methylpiperazin-1-carbonyl)-[1,2,4]oxadiazol-3-yl]-benzyl}-2*H*-pyridazin-3-on ("A21") erfolgt analog nachstehendem

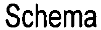

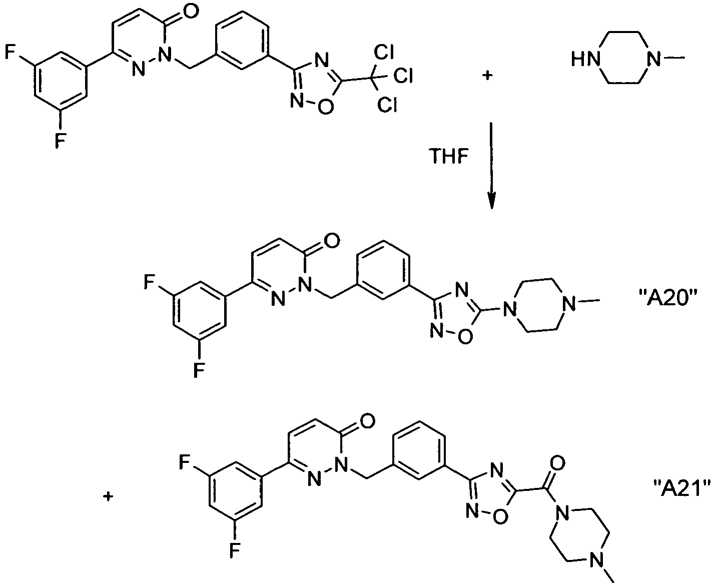

Eine Lösung von 242 mg (0.50 mmol) 6-(3,5-Difluorphenyl)-2-[3-(5-trichlormethyl-[1,2,4]oxadiazol-3-yl)-benzyl]-2*H*-pyridazin-3-on in 1 ml THF wird mit 100 mg (1.00 mmol) 1-Methylpiperazin versetzt und 18 Stunden bei Raumtemperatur gerührt. Das Reaktionsgemisch wird eingedampft und durch präparative HPLC getrennt. Man erhält 6-(3,5-Difluorphenyl)-2-{3-[5-(4-methyl-piperazin-1-yl)-[1,2,4]oxadiazol-3-yl]-benzyl}-2*H*-pyridazin-3-on ("A20") Formiat als farbloses Öl (ESI 465) und 6-(3,5-Difluorphenyl)-2-{3-[5-(4-methylpiperazin-1-carbonyl)-[1,2,4]oxadiazol-3-yl]-benzyl}-2*H*-pyridazin-3-on ("A21") Formiat als farblosen Feststoff (ESI 493);
¹H-NMR (d₆-DMSO): δ [ppm] = 2.22 (s, 3H), 2.35 (m, 2H), 2.43 (m, 2H), 3.69 (m, 4H), 5.47 (s, 2H), 7.16 (d, J = 9.5 Hz, 1H), 7.37 (tt, J₁ = 9 Hz, J₂ = 2 Hz, 1 H), 7.61 (t, J = 7.5 Hz, 1 H), 7.67 (m, 3H), 7.99 (d, J = 7.5 Hz, 1 H), 8.08 (bs, 1 H), 8.15 (s, 1 H, HCOO⁻), 8.17 (d, J = 9.5 Hz, 1 H), 10.05 (bs, 1H).

Analog werden die nachstehenden Verbindungen erhalten

| Nr. | Name und/oder Struktur | ESI |
|---|---|---|
| "A22" | 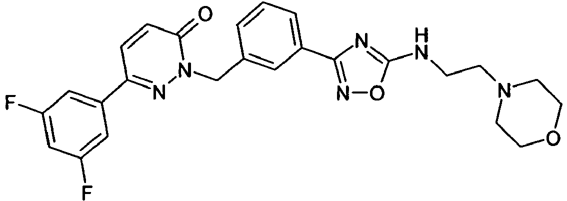 | 495 |
| | Hydrochlorid | |
| "A23" | 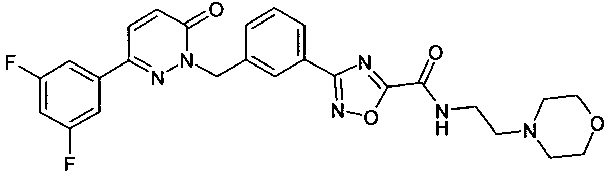 | 523 |
| "A28" | 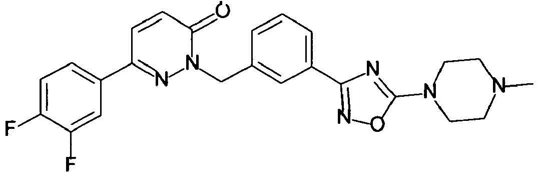 | |
| "A29" | 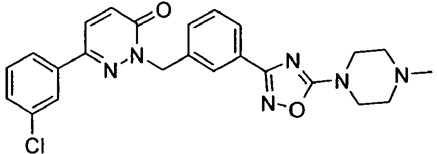 | |

### Beispiel 12

Eine alternative Herstellung von 6-(3,5-Difluorphenyl)-2-{3-[5-(4-methylpiperazin-1-yl)-[1,2,4]oxadiazol-3-yl]-benzyl}-2*H*-pyridazin-3-on ("A20") erfolgt analog nachstehendem Schema 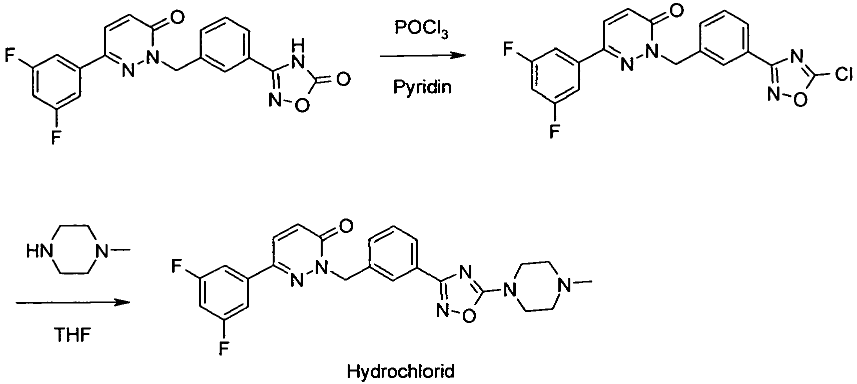
12.1 Eine Suspension von 765 mg (2.00 mmol) 6-(3,5-Difluorphenyl)-2-[3-(5-oxo-4,5-dihydro-[1,2,4]oxadiazol-3-yl)-benzyl]-2*H*-pyridazin-3-on in 4 ml Dichlormethan wird mit 316 mg (4.00 mmol) Pyridin und 613 mg (4.00 mmol) Phosphorylchlorid versetzt und die Reaktionslösung über Nacht bei Raumtemperatur gerührt. Das Reaktionsgemisch wird auf Eis gegeben und mit Dichlormethan extrahiert. Die organische Phase wird über Natriumsulfat getrocknet und eingedampft: 2-[3-(5-Chlor-[1,2,4]oxadiazol-3-yl)-benzyl]-6-(3,5-difluorphenyl)-2*H*-pyridazin-3-on als braunes Öl (ESI 401), das ohne weitere Reinigung in die folgende Reaktion eingesetzt wird.
12.2 Eine Lösung von 450 mg (ca. 0.7 mmol) rohem 2-[3-(5-Chlor-[1,2,4]oxadiazol-3-yl)-benzyl]-6-(3,5-difluorphenyl)-2*H*-pyridazin-3-on in 3 ml THF wird mit 140 mg (1.40 mmol) 1-Methylpiperazin versetzt und über Nacht bei Raumtemperatur gerührt. Das Reaktionsgemisch wird zwischen Dichlormethan und gesättigter Natriumhydrogencarbonat-lösung verteilt. Die organische Phase wird über Natriumsulfat getrocknet eingedampft und der Rückstand an einer Kieselgelsäule mit Dichlormethan / Methanol als Laufmittel chromatographiert. Das Produkt wird mit 12 ml 0.1 N Chlorwasserstoffsäure in 2-Propanol ins Hydrochlorid überführt: 6-(3,5-Difluorphenyl)-2-{3-[5-(4-methyl-piperazin-1-yl)-[1,2,4]oxadiazol-3-yl]-benzyl}-2*H*-pyridazin-3-on Hydrochlorid ("A20a") als gelbliche Kristalle; ESI 465; ¹H-NMR (d₆-DMSO): δ [ppm] = 2.81 (s, 3H), 3.22 (b, 2H), 3.49 (b, 2H), 3.62 (b, 2H), 4.18 (b, 2H), 5.42 (s, 2H), 7.13 (d, J = 9.5 Hz, 1H), 7.36 (tt, J₁ = 9 Hz, J₂ = 2 Hz, 1 H), 7.52 (t, J = 7.5 Hz, 1 H), 7.57 (d, J = 7.5 Hz, 1H), 7.65 (m, 2H), 7.85 (d, J = 7.5 Hz, 1H), 7.99 (bs, 1H), 8.15 (d, J = 9.5 Hz, 1 H), 10.79 (bs, 1H).

Analog werden die nachstehenden Verbindungen erhalten

| Nr. | Name und/oder Struktur | ESI |
|---|---|---|
| "A28" | 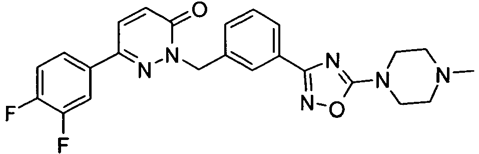 | 465 |
| "A29" | 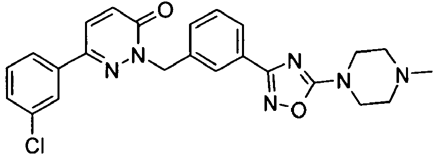 | 463 |
| | Hydrochlorid | |
| "A30" | 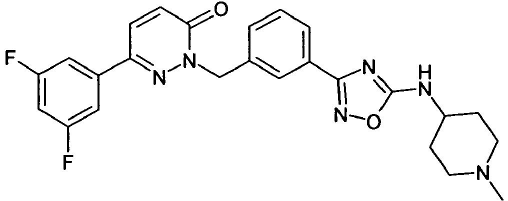 | 479 |
| | Hydrochlorid | |
| "A31" | 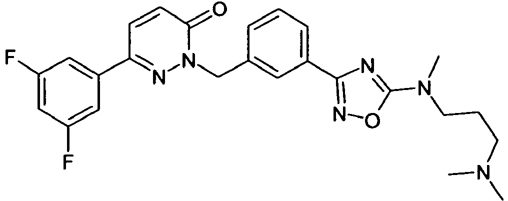 | 481 |
| | Hydrochlorid | |
| "A45" | 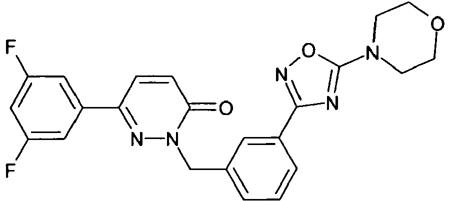 | 452 |
| "A46" | 6-(3,5-Difluor-phenyl)-2-(3-{5-[methyl-(1-methyl-piperidin-4-yl)-amino]-[1,2,4]oxadiazol-3-yl}-benzyl)-2*H*-pyridazin-3-on | 493 |
| | 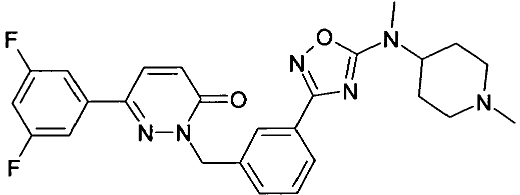 | |
| | Hydrochlorid | |
| ¹H-NMR (d₆-DMSO): δ [ppm] = 1.95 (m, 2H), 2.20 (m, 2H), 2.75 (s, 3H), 3.04 (s, 3H), 3.16 (m, 2H), 3.49 (m, 2H), 4.24 (m, 1H), 5.42 (s, 2H), 7.14 (d, J = 10 Hz, 1H), 7.36 (tt, J₁ = 9.5 Hz, J₂ = 2 Hz, 1H), 7.50 (t, J = 7.5 Hz, 1H), 7.54 (d, J = 7.5 Hz, 1H), 7.65 (m, 2H), 7.84 (d, J = 7.5 Hz, 1H), 7.97 (s, 1H), 8.16 (d, J = 10 Hz, 1H), 10.3 (bs, 1H) | | |
| "A47" | 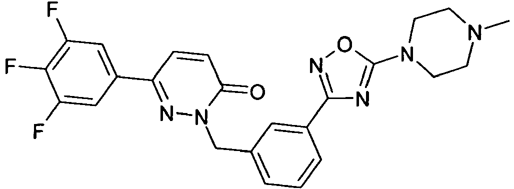 | 483 |
| | Hydrochlorid | |
| "A48" | 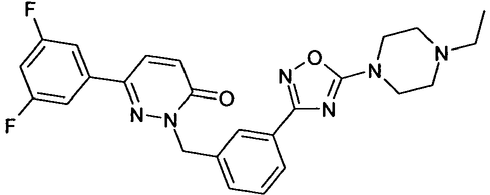 | 479 |
| | Hydrochlorid | |
| ¹H-NMR (d₆-DMSO): δ [ppm] = 1.27 (t, J = 7 Hz, 3H), 3.17 (m, 4H), 3.56 (m, 2H), 3.67 (m, 2H), 4.20 (m, 2H), 5.42 (s, 2H), 7.14 (d, J = 10 Hz, 1H), 7.36 (tt, J₁ = 9.5 Hz, J₂ = 2 Hz, 1H), 7.52 (t, J = 7.5 Hz, 1H), 7.57 (d, J = 7.5 Hz, 1H), 7.66 (m, 2H), 7.85 (d, J = 7.5 Hz, 1H), 7.99 (s, 1H), 8.15 (d, J = 10 Hz, 1H), 10.75 (bs, 1H) | | |
| "A49" | 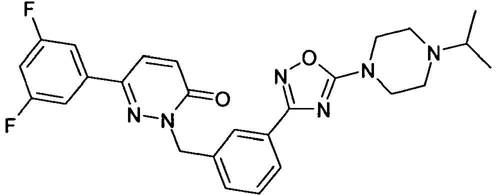 | 493 |
| | Hydrochlorid | |
| "A50" | 6-(3,5-Difluor-phenyl)-2-(3-{5-[4-(2-methoxy-ethyl)-piperazin-1-yl]-[1,2,4]oxadiazol-3-yl}-benzyl)-2*H-*pyridazin-3-on | 509 |
| | 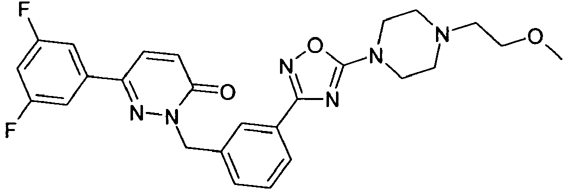 | |
| | Hydrochlorid | |
| ¹H-NMR (d₆-DMSO): δ [ppm] = 3.33 (m, 2H), 3.37 (s, 3H), 3.42 (t, J = 4.5 Hz, 2H), 3.65 (m, 4H), 3.73 (t, J = 4.5 Hz, 2H), 4.18 (m, 2H), 5.42 (s, 2H), 7.14 (d, J = 10 Hz, 1H), 7.36 (tt, J₁ = 9.5 Hz, J₂ = 2 Hz, 1H), 7.52 (t, J = 7.5 Hz, 1H), 7.57 (d, J = 7.5 Hz, 1H), 7.65 (m, 2H), 7.85 (d, J = 7.5 Hz, 1H), 7.99 (s, 1H), 8.15 (d, J = 10 Hz, 1H), 10.4 (bs, 1H) | | |
| "A51" | 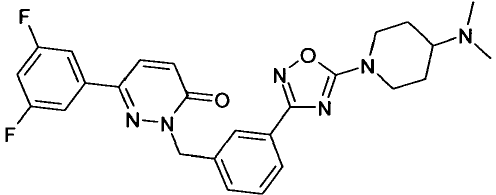 | 493 |
| | Hydrochlorid | |
| "A52" | 6-(3,5-Difluor-phenyl)-2-(3-{5-[4-(2-dimethylamino-ethyl)-piperazin-1-yl]-[1,2,4]oxadiazol-3-yl}-benzyl)-2*H*-pyridazin-3-on | 522 |
| | 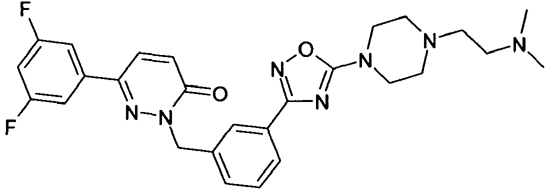 | |
| | Dihydrochlorid | |
| "A53" | 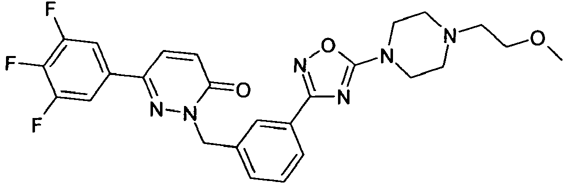 | 527 |
| | Hydrochlorid | |
| "A123" | 6-(3,5-Difluorphenyl)-2-(3-{5-[4-(2-ethoxyethyl)-piperazin-1-yl]-[1,2,4]oxadiazol-3-yl}-benzyl)-2*H-*pyridazin-3-on | 523 |
| | 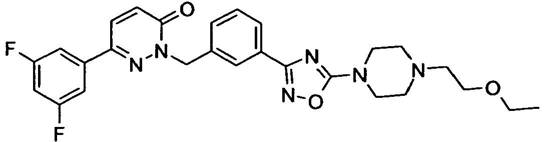 | |
| | Hydrochlorid | |
| "A124" | 6-(3,5-Difluorphenyl)-2-(3-{5-[4-(3-methoxypropyl)-piperazin-1-yl]-[1,2,4]oxadiazol-3-yl}-benzyl)-2H-pyridazin-3-on | 523 |
| | 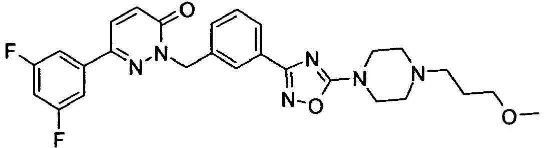 | |
| | Hydrochlorid | |
| ¹H-NMR (DMSO-d₆): δ [ppm] = 2.03 (m, 2H), 3.23 (m, 2H), 3.28 (m, 2H), 3.32 (s, 3H), 3.47 (t, J = 6Hz, 2H), 3.65 (m, 2H), 3.72 (m, 2H), 4.24 (m, 2H), 5.48 (s, 2H), 7.20 (d, J = 10 Hz, 1H), 7.42 (tt, J₁ = 9.5 Hz, J₂ = 2 Hz, 1H), 7.58 (t, J = 7.5 Hz, 1H), 7.63 (d, J = 7.5 Hz, 1H), 7.72 (m, 2H), 7.91 (d, J = 7.5 Hz, 1H), 8.05 (s, 1H), 8.21 (d, J = 10 Hz, 1H), 10.7 (bs, 1H) | | |
| "A125" | 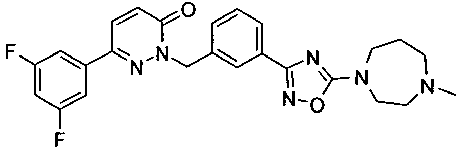 | |
| "A126" | 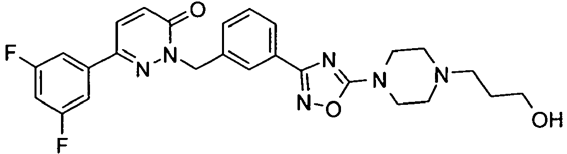 | |
| "A127" | 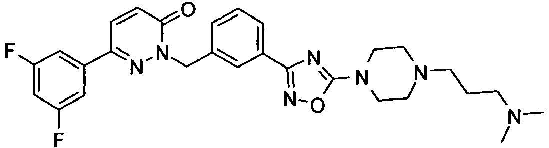 | |
| "A128" | 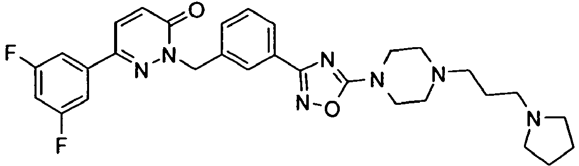 | |
| "A129" | 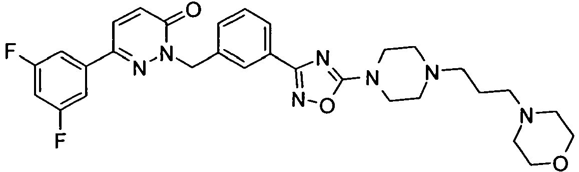 | |
| | | |

### Beispiel 13

Die Herstellung von 6-(3,5-Difluor-phenyl)-2-[3-(5-piperazin-1-yl-[1,2,4]oxadiazol-3-yl)-benzyl]-2*H*-pyridazin-3-on ("A24"), ESI 451, erfolgt analog nachstehendem Schema

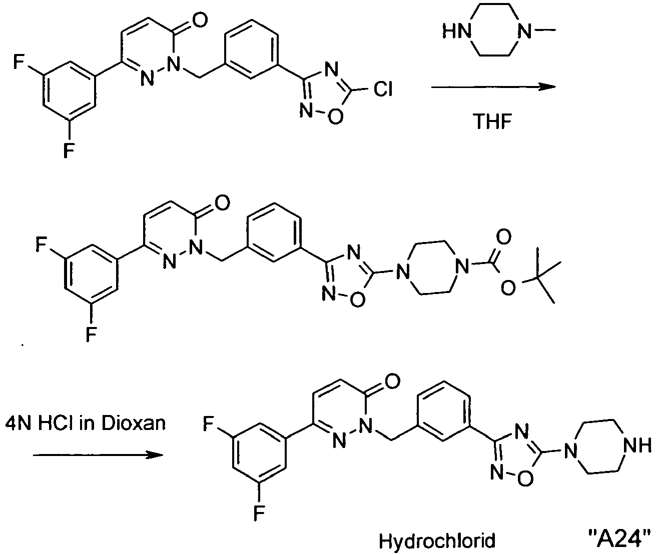

Analog erhält man die Verbindungen 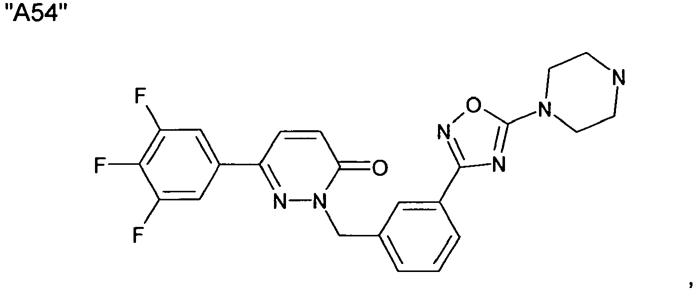 Hydrochlorid, ESI 469;

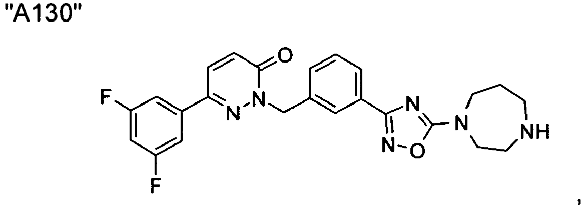

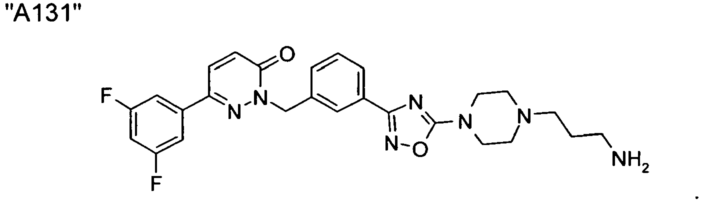

### Beispiel 14

Die Herstellung von 6-(3,5-Difluorphenyl)-2-{3-[4-(3-dimethylamino-propyl)-5-oxo-4,5-dihydro-[1,2,4]oxadiazol-3-yl]-benzyl}-2H-pyridazin-3-on ("A25") erfolgt analog nachstehendem Schema

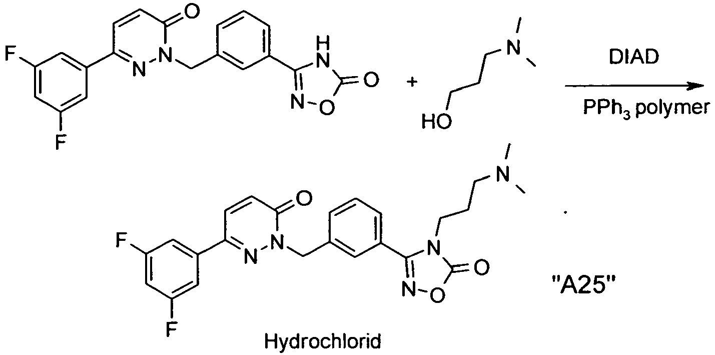

Eine Suspension von 382 mg (1.00 mmol) 6-(3,5-Difluor-phenyl)-2-[3-(5-oxo-4,5-dihydro-[1,2,4]oxadiazol-3-yl)-benzyl]-2H-pyridazin-3-on in 10 ml THF wird mit 0.234 ml (2.00 mmol) 3-(Dimethylamino)-1-propanol und 1.0 g polymergebundenem Triphenylphosphin versetzt. Zu dieser Suspension werden 0.393 ml (2.00 mmol) Diisopropylazodicarboxylat langsam zugetropft und das Reaktionsgemisch 18 Stunden bei Raumtemperatur gerührt. Das Reaktionsgemisch wird filtriert, das Filtrat eingedampft und der Rückstand durch präparative HPLC gereinigt. Das Produkt wird in 25%iger wässriger Salzsäure gelöst, im Vakuum eingedampft und lyophilisiert: 6-(3,5-Difluorphenyl)-2-{3-[4-(3-dimethylamino-propyl)-5-oxo-4,5-dihydro-[1,2,4]oxadiazol-3-yl]-benzyl}-2*H*-pyridazin-3-on Hydrochlorid ("A25") als farbloses Pulver; ESI 468.

### Analog erhält man die Verbindung "A55"

6-(3,5-Difluor-phenyl)-2-[3-(5-oxo-4-piperidin-4-yl-4,5-dihydro-[1,2,4]oxadiazol-3-yl)-benzyl]-2*H*-pyridazin-3-on 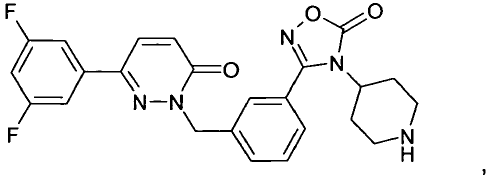 Hydrochlorid, ESI 466.

### Beispiel 15

Die Herstellung von 6-(3,5-Difluorphenyl)-2-[3-(1*H*-tetrazol-5-yl)-benzyl]-2*H*-pyridazin-3-on ("A26") erfolgt analog nachstehendem Schema

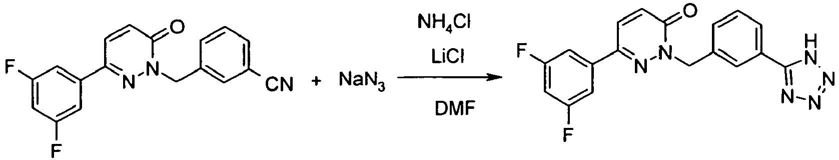

Eine Lösung von 162 mg (0.50 mmol) 3-[3-(3,5-Difluorphenyl)-6-oxo-6*H-*pyridazin-1-ylmethyl]-benzonitril in 1 ml DMF wird mit 81.3 mg (2.5 mmol) Natriumazid, 26.7 mg (0.50 mmol) Ammoniumchlorid und 21.2 mg (0.50 mmol) Lithiumchlorid versetzt und 18 Stunden bei 100° C gerührt. Das Reaktionsgemisch wird mit 1 N HCl versetzt. Der entstandene Niederschlag wird abgesaugt, mit Wasser gewaschen und im Vakuum getrocknet: 6-(3,5-Difluorphenyl)-2-[3-(1*H*-tetrazol-5-yl)-benzyl]-2*H*-pyridazin-3-on ("A26") als farblose Kristalle; ESI 367.

### Beispiel 16

Die Herstellung von 6-(3,5-Difluorphenyl)-2-[3-(5-methyl-1H-imidazol-2-yl)-benzyl]-2*H*-pyridazin-3-on ("A36") erfolgt analog nachstehendem

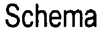

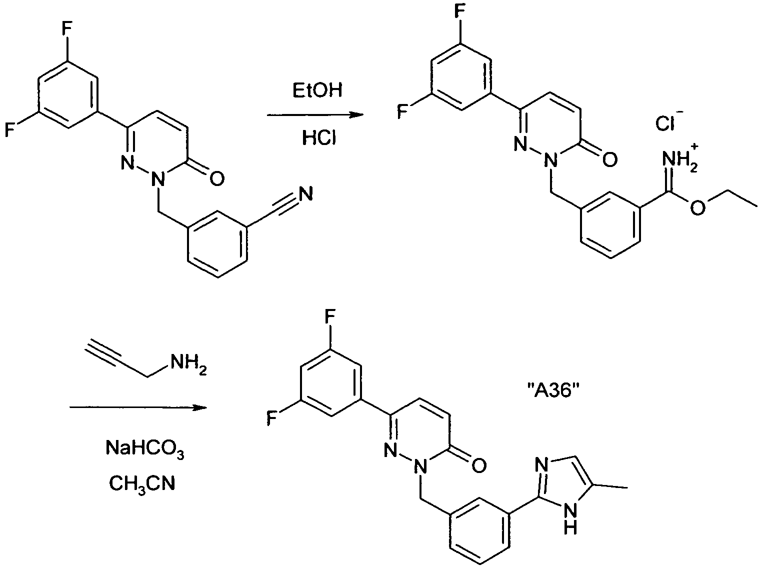

Die letzte Stufe wird analog C. M. Tice, Tetrahedron 57, 2001, S. 2689, durchgeführt.

### Alternativ kann "A36" wie folgt hergestellt werden

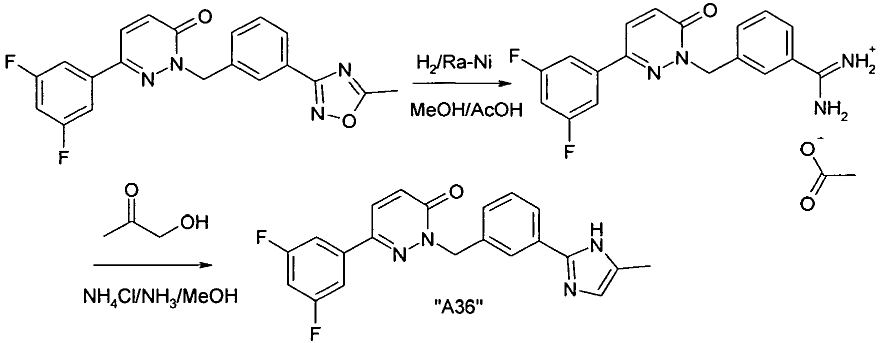

1. Eine Lösung von 6.34 g (16.7 mmol) 6-(3,5-Difluorphenyl)-2-[3-(5-methyl-[1,2,4]oxadiazol-3-yl)-benzyl]-2H-pyridazin-3-on (Herstellung siehe Synthesebeispiel1) in 70 ml Methanol wird mit 2 ml Essigsäure, 2 ml Wasser und 9 g Raney-Nickel versetzt und 21 Stunden bei Raumtemperatur und Normaldruck hydriert. Das Reaktionsgemisch wird filtriert und das Filtrat eingedampft. Der Rückstand wird in tert.-Butylmethylether aufgenommen, erhitzt und abkühlen lassen. Der Niederschlag wird abgesaugt, mit tert.-Butylmethylether gewaschen und im Vakuum getrocknet: 3-[3-(3,5-Difluorphenyl)-6-oxo-6H-pyridazin-1-ylmethyl]-benzamidinium-Acetat als farblose Kristalle; ESI 341.
2. Eine Suspension von 401 mg (1.00 mmol) 3-[3-(3,5-Difluorphenyl)-6-oxo-6H-pyridazin-1-ylmethyl]-benzamidinium-Acetat in einem Gemisch von 5 ml 32%iger wässriger Ammoniaklösung und 15 ml 10%iger Lösung von Ammoniak in Methanol wird mit 221 mg (4.12 mmol) Ammoniumchlorid und 138 µl Hydroxyaceton versetzt und 3 Tage bei 80° C gerührt. Das Reaktionsgemisch wird im Vakuum eingeengt, mit Ethylacetat versetzt und filtriert. Die organische Phase wird abgetrennt, über Natriumsulfat getrocknet und eingedampft. Der Rückstand wird an einer Kieselgelsäule mit Dichlormethan/Methanol chromatographiert. Die Produkt enthaltenden Fraktionen werden vereinigt, eingedampft, in 1,1 Äquivalenten 0.1 N HCl in 2-Propanol gelöst und eingedampft. Der Rückstand wird mit tert.-Butylmethylether verrührt: "A36", Hydrochlorid, als gelbliche Kristalle; ESI 379.

Analog erhält man die Verbindung 6-(3,5-Difluor-phenyl)-2-[3-(5-hydroxymethyl-1*H*-imidazol-2-yl)-benzyl]-2*H*-pyridazin-3-on ("A56"), ESI 395,

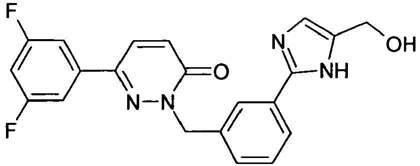

### Beispiel 17

Die Herstellung von 6-(3,5-Difluorphenyl)-2-{3-[5-(4-methyl-piperazin-1-yl)-oxazol-2-yl]-benzyl}-2*H*-pyridazin-3-on ("A37") erfolgt analog nachstehendem Schema

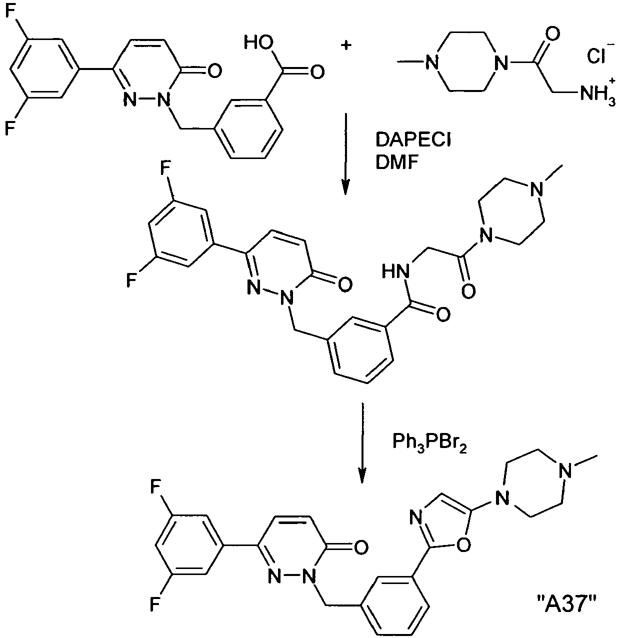

Die Cyclisierung erfolgt nach R. Mazurkiewicz, Synthesis 1992, S. 941.

### Beispiel 18

Die Herstellung von 6-(3,5-Difluorphenyl)-2-[3-(1-piperidin-4-yl-1*H-*pyrazol-4-yl)-benzyl]-2*H*-pyridazin-3-on ("A38") erfolgt analog nachstehendem Schema, wobei zwei alternative Synthesewege möglich sind:

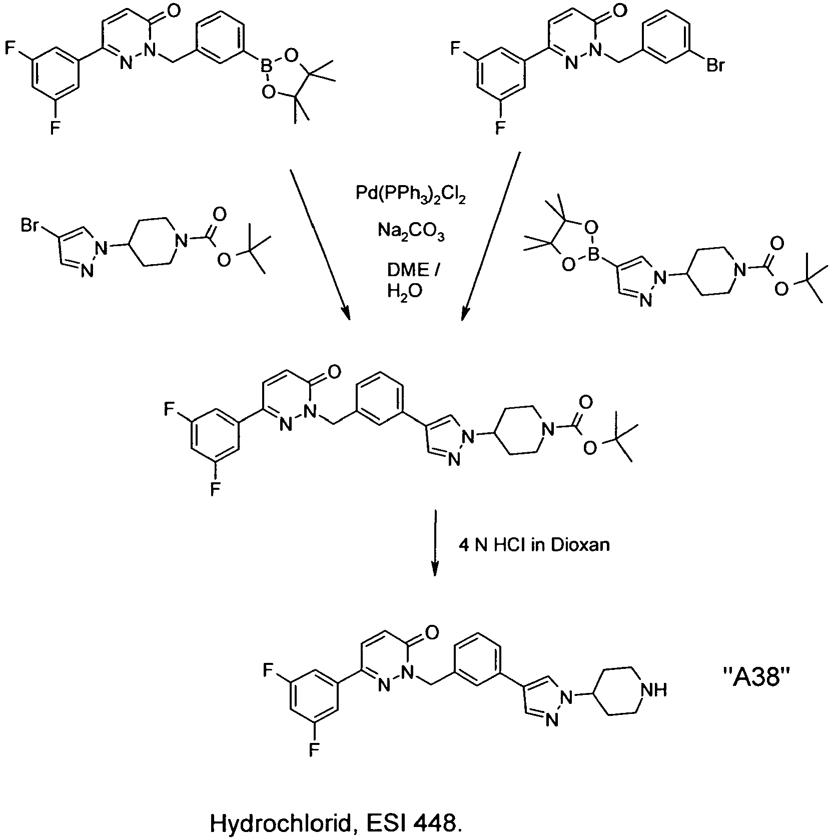

### Beispiel 19

Die Herstellung von 2-[3-(5-Amino-[1,2,4]oxadiazol-3-yl)-benzyl]-6-(3,4,5-trifluor-phenyl)-2*H*-pyridazin-3-on ("A57") und von 2-(3-{5-[Methyl-(1-methyl-piperidin-4-yl)-amino]-[1,2,4]oxadiazol-3-yl}-benzyl)-6-(3,4,5-trifluor-phenyl)-2*H*-pyridazin-3-on ("A58") erfolgt analog nachstehendem Schema: 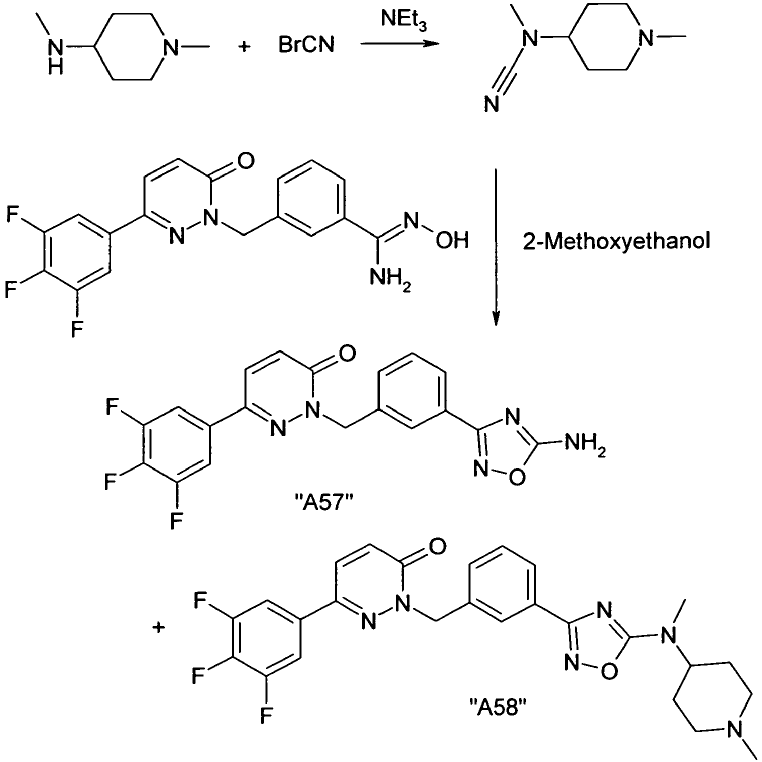
19.1 Eine auf 0° gehaltene Lösung von 6.41 g (50.0 mmol) Methyl-(1-methylpiperidin-4-yl)-amin und 5.06 g (50.0 mmol) Triethylamin in 150 ml THF wird unter Rühren portionsweise mit 5.46 g (50.0 mmol) Bromcyan versetzt. Das Reaktionsgemisch wird 4 Stunden bei Raumtemperatur gerührt. Der entstandene Niederschlag wird abgesaugt und mit tert.-Butylmethylether gewaschen. Das Filtrat wird eingedampft: Methyl-(1-methylpiperidin-4-yl)-cyanamid als rötliches Öl; ESI 154.
19.2 Eine Suspension von 376 mg (1.00 mmol) N-Hydroxy-3-[6-oxo-3-(3,4,5-trifluorphenyl)-6*H*-pyridazin-1-ylmethyl]-benzamidin (Herstellung nach Beispiel 2) in 2 ml 2-Methoxyethanol wird mit 185 mg (1.2 mmol) Methyl-(1-methylpiperidin-4-yl)-cyanamid versetzt und 3 Tage bei einer Temperatur von 120° C gerührt. Man lässt abkühlen, saugt das Reaktionsgemisch ab und wäscht den Rückstand mit Dichlormethan. Der Rückstand wird im Vakuum getrocknet: 2-[3-(5-Amino-[1,2,4]-oxadiazol-3-yl)-benzyl]-6-(3,4,5-trifluorphenyl)-2*H*-pyridazin-3-on ("A57") als rötliche Kristalle; ESI 400.
   Das Filtrat wird mit Wasser versetzt. Die organische Phase wird abgetrennt, zweimal mit Wasser gewaschen, über Natriumsulfat getrocknet und eingedampft. Der Rückstand wird an einer Kieselgelsäule mit Dichlormethan/Methanol als Laufmittel chromatographiert. Die Produkt enthaltenden Fraktionen werden eingedampft und mit 0.1 N HCl in 2-Propanol ins Hydrochlorid überführt. Das Hydrochlorid wird in Wasser gelöst, filtriert und das Filtrat lyophilisiert: 2-(3-{5-[Methyl-(1-methylpiperidin-4-yl)-amino]-[1,2,4]oxadiazol-3-yl}-benzyl)-6-(3,4,5-trifluorphenyl)-2*H*-pyridazin-3-on Hydrochlorid ("A58") als leicht gelbliches Lyophilisat; ESI 511.

### Analog werden die nachstehenden Verbindungen erhalten

| Nr. | Name und/oder Struktur | ESI |
|---|---|---|
| "A59" | 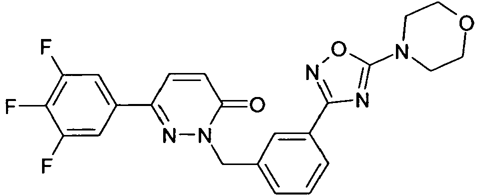 | 470 |
| "A60" | 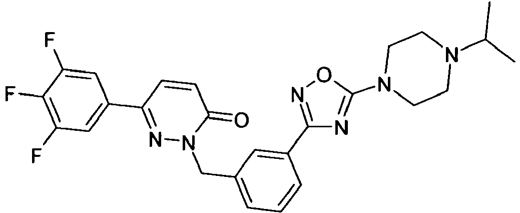 | 511 |
| | Hydrochlorid | |
| "A61" | 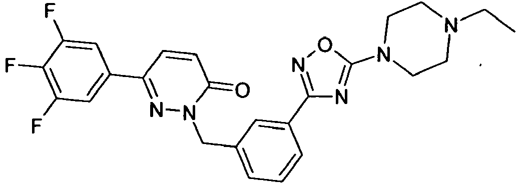 | 497 |
| | Hydrochlorid | |
| "A62" | 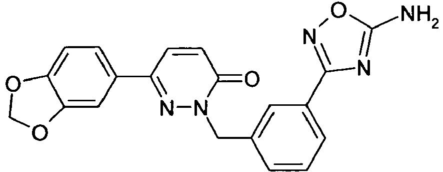 | 390 |
| "A63" | 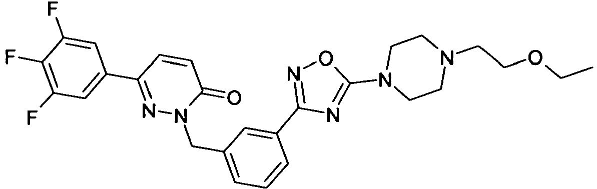 | 541 |
| | Hydrochlorid | |
| "A64" | 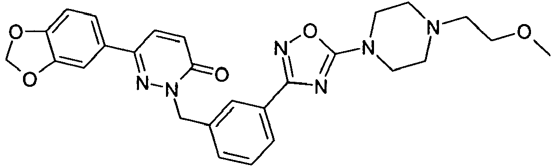 | 517 |
| | Hydrochlorid | |
| "A65" | 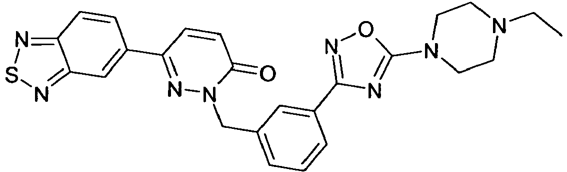 | 501 |
| "A66" | 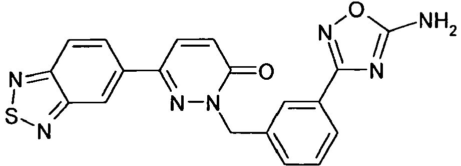 | 404 |
| "A67" | 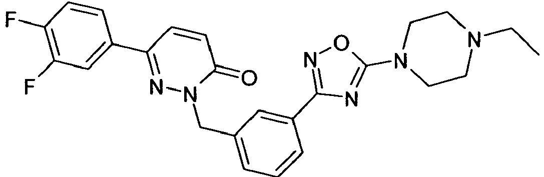 | 479 |
| | Hydrochlorid | |
| "A68" | 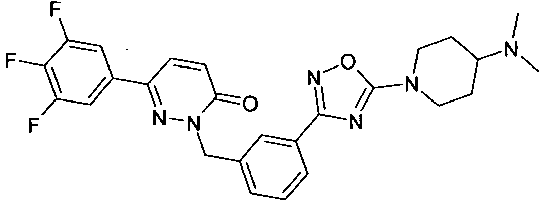 | 511 |
| | Hydrochlorid | |
| "A69" | 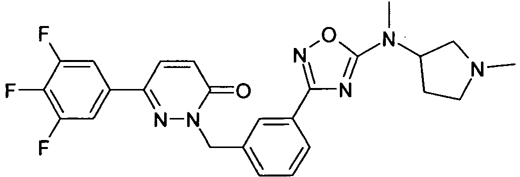 | 497 |
| | Hydrochlorid | |
| "A70" | 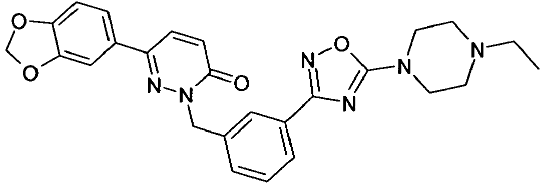 | 487 |
| | Formiat | |
| "A71" | 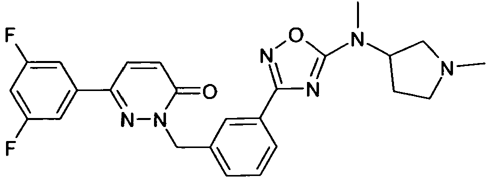 | 479 |
| | Hydrochlorid | |
| "A71a" | 2-(3-{5-[(1-Ethyl-piperidin-4-yl)-methyl-amino]-[1,2,4]oxadiazol-3-yl}-benzyl)-6-(3,4,5-trifluor-phenyl)-2*H*-pyridazin-3-on | 525 |
| | 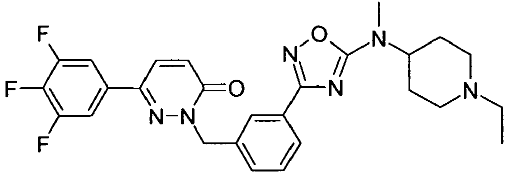 | |
| | Hydrochlorid | |
| "A71 b" | 6-(3,5-Difluor-phenyl)-2-(3-{5-[(1-ethyl-piperidin-4-yl)-methyl-amino]-[1,2,4]oxadiazol-3-yl}-benzyl)-2*H-*pyridazin-3-on | 507 |
| | 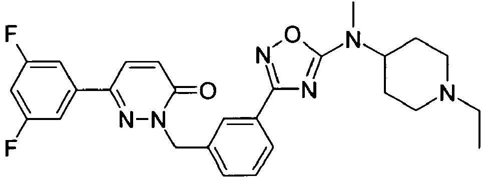 | |
| | Hydrochlorid | |
| "A71c" | 6-(3,5-Difluor-phenyl)-2-(3-{5-[methyl-(1-methyl-piperidin-4-ylmethyl)-amino]-[1,2,4]oxadiazol-3-yl}-benzyl)-2*H*-pyridazin-3-on | |
| | 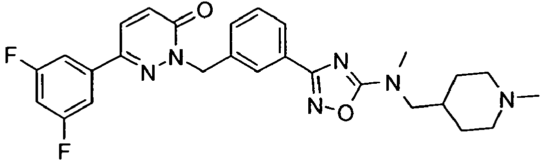 | |

### Beispiel 20

Die Herstellung von 2-[3-(5-Amino-[1,2,4]oxadiazol-3-yl)-benzyl]-6-(3,5-difluor-phenyl)-2*H*-pyridazin-3-on ("A72"), 6-(3,5-Difluor-phenyl)-2-{3-[5-(methyl-piperidin-4-yl-amino)-[1,2,4]oxadiazol-3-yl]-benzyl}-2*H*-pyridazin-3-on ("A73") und von 6-(3,5-Difluor-phenyl)-2-(3-{5-[methyl-(1-methyl-piperidin-4-yl)-amino]-[1,2,4]oxadiazol-3-yl}-benzyl)-2*H*-pyridazin-3-on ("A46"; alternative Herstellung) erfolgt analog nachstehendem Schema: 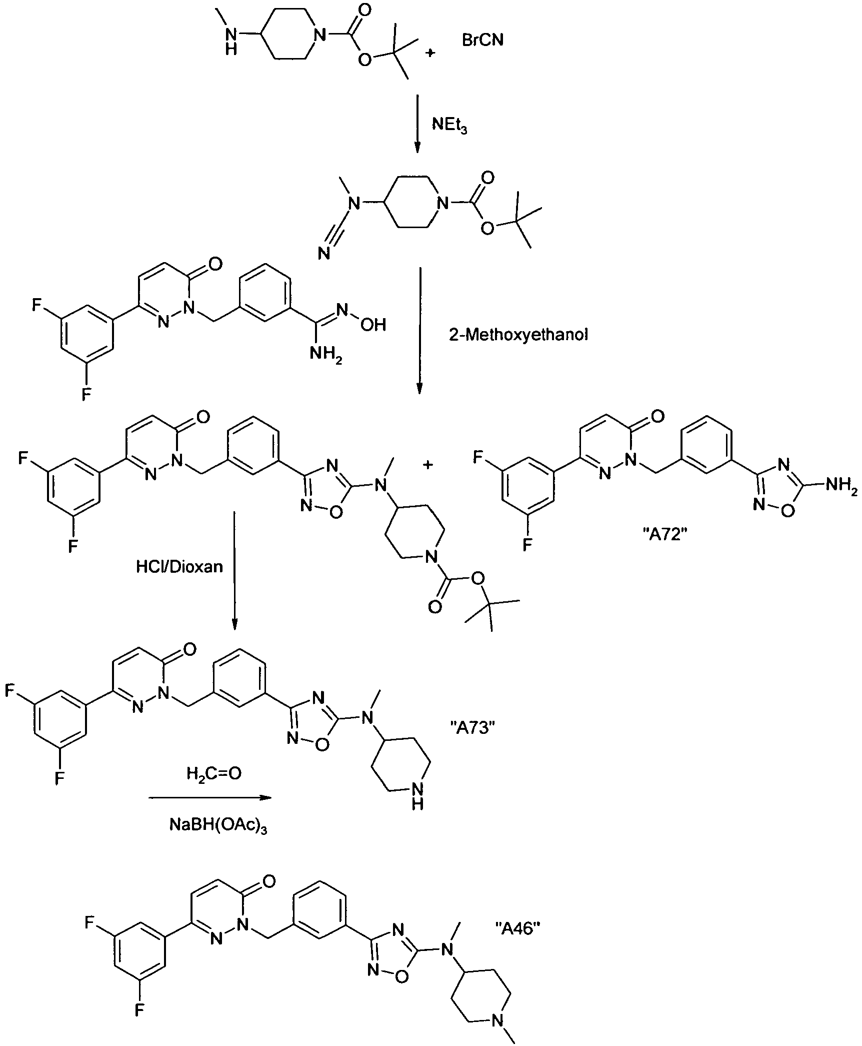
20.1 Eine auf 0° gehaltene Lösung von 17.1 g (80.0 mmol) 1-Boc-4-methylaminopiperidin und 11.1 ml (80.0 mmol) Triethylamin in 160 ml THF wird unter Rühren portionsweise mit 8.74 g (80.0 mmol) Bromcyan versetzt. Das Reaktionsgemisch wird 4 Stunden bei Raumtemperatur gerührt. Der entstandene Niederschlag wird abgesaugt und mit tert.-Butylmethylether gewaschen. Das Filtrat wird eingedampft. Der Rückstand, ein langsam kristallisierendes Öl, wird mit Petrolether aufgekocht und abgekühlt. Der Niederschlag wird abgesaugt, mit Petrolether gewaschen und im Vakuum getrocknet: 4-(Cyan-methyl-amino)-piperidin-1-carbonsäure-tert.-butylester als farblose Kristalle; ESI 240.

Eine Suspension von 5.35 g (15.0 mmol) N-Hydroxy-3-[6-oxo-3-(3,5-difluorphenyl)-6*H*-pyridazin-1-ylmethyl]-benzamidin (Herstellung siehe Beispiel 2) in 30 ml 2-Methoxyethanol wird mit 4.31 g (18.0 mmol) 4-(Cyan-methyl-amino)-piperidin-1-carbonsäure-tert.-butylester versetzt und 3 Tage bei einer Temperatur von 120° C gerührt. Man lässt abkühlen, saugt das Reaktionsgemisch ab und wäscht den Rückstand mit Dichlormethan. Der Rückstand wird im Vakuum getrocknet: 2-[3-(5-Amino-[1,2,4]oxadiazol-3-yl)-benzyl]-6-(3,5-difluorphenyl)-2*H*-pyridazin-3-on ("A72") als rötliche Kristalle; ESI 382.
Das Filtrat wird mit Dichlormethan und 1 N HCl versetzt. Die organische Phase wird abgetrennt, mit 1 N NaOH und mit Wasser gewaschen, über Natriumsulfat getrocknet und eingedampft. Der Rückstand wird an einer Kieselgelsäule mit tert.-Butylmethylether als Laufmittel chromatographiert: 4-[(3-{3-[3-(3,5-Difluorphenyl)-6-oxo-6*H*-pyridazin-1-ylmethyl]-phenyl}-[1,2,4]oxadiazol-5-yl)-methyl-amino]-piperidin-1-carbonsäure-tert.-butylester als bräunliches Öl; ESI 579.

Eine Lösung von 1.25 g (2.17 mmol) 4-[(3-{3-[3-(3,5-Difluorphenyl)-6-oxo-6H-pyridazin-1-ylmethyl]-phenyl}-[1,2,4]oxadiazol-5-yl)-methyl-amino]-piperidin-1-carbonsäure-tert.-butylester in 2 ml Dioxan wird mit 2 ml 4 N HCl in Dioxan versetzt und 3 Stunden bei Raumtemperatur belassen. Das Reaktionsgemisch wird mit Wasser und Ethylacetat versetzt. Die organische Phase wird abgetrennt. Die wässrige Phase wird mit 1 N NaOH auf einen pH-Wert von 14 gebracht und zweimal mit Ethylacetat extrahiert. Die organische Phase wird über Natriumsulfat getrocknet und eingedampft: 6-(3,5-Difluorphenyl)-2-{3-[5-(methyl-piperidin-4-yl-amino)-[1,2,4]oxadiazol-3-yl]-benzyl}-2H-pyridazin-3-on ("A73") als leicht gelbliche Kristalle; ESI 479.

Eine Suspension von 450 mg (0.93 mmol) "A73" in 1.8 ml 37%iger wässriger Formaldehydlösung wird mit 848 mg (4.0 mmol) Natriumtriacetoxyborhydrid versetzt und 2 Tage bei Raumtemperatur gerührt. Das Reaktionsgemisch wird nacheinander mit gesättigter wässriger Natriumhydrogencarbonat-Lösung und 15%iger wässriger Natronlauge versetzt und mit Ethylacetat extrahiert. Die organische Phase wird über Natriumsulfat getrocknet und eingedampft. Der Rückstand wird an einer Kieselgelsäule mit Dichlormethan/Methanol als Laufmittel chromatographiert: 6-(3,5-Difluorphenyl)-2-(3-{5-[methyl-(1-methyl-piperidin-4-yl)-amino]-[1,2,4]oxadiazol-3-yl}-benzyl)-2*H*-pyridazin-3-on ("A46") als farblose Kristalle; ESI 493.

### Analog erhält man "A74", Hydrochlorid, ESI 537,

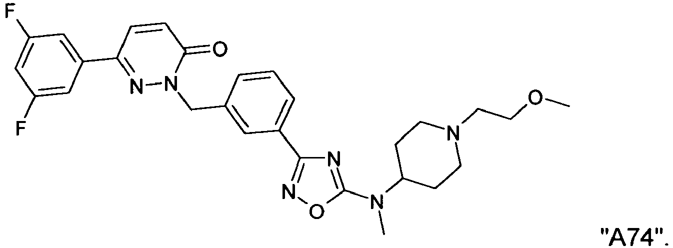

### Beispiel 21

Die Herstellung von 2-{3-[5-(4-Methyl-piperazin-1-ylmethyl)-[1,2,4]oxadiazol-3-yl]-benzyl}-6-(3,4,5-trifluor-phenyl)-2*H*-pyridazin-3-on ("A75") erfolgt analog nachstehendem Schema: 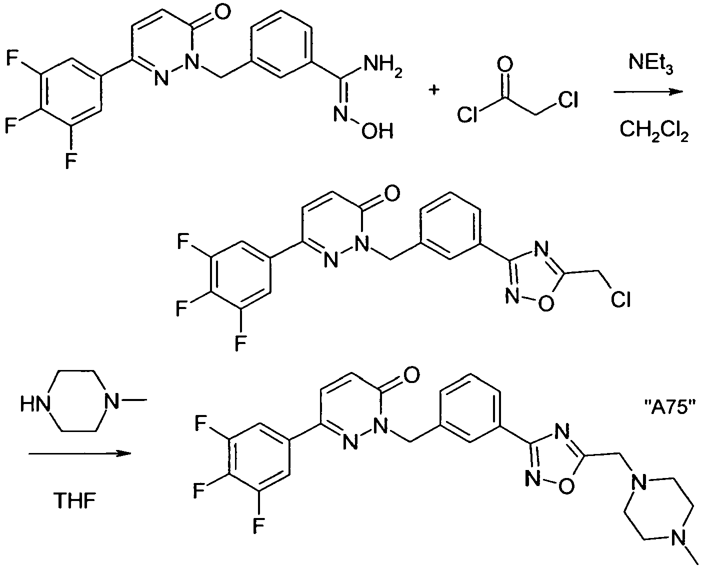
21.1 Zu einer Suspension von 1.13 g (3.00 mmol) N-Hydroxy-3-[6-oxo-3-(3,4,5-trifluorphenyl)-6*H*-pyridazin-1-ylmethyl]-benzamidin und 1.05 ml (7.54 mmol) Triethylamin in 10 ml Dichlormethan wird unter Rühren eine Lösung von 712 mg (6.30 mmol) Chloracetylchlorid in 1 ml Dichlormethan zugetropft. Das Reaktionsgemisch wird 5 Tage bei Raumtemperatur gerührt. Es wird mit Dichlormethan verdünnt, mit 1 N NaOH, 1 N HCl und Wasser gewaschen. Die organische Phase wird über Natriumsulfat getrocknet und eingedampft. Der Rückstand wird an einer Kieselgelsäule mit Petrolether/Ethylacetat als Laufmittel chromatographiert: 2-[3-(5-Chlormethyl-[1,2,4]oxadiazol-3-yl)-benzyl]-6-(3,4,5-trifluorphenyl)-2*H*-pyridazin-3-on als gelblicher Feststoff; ESI 433.
21.2 Eine Lösung von 216 mg (0.50 mmol) 2-[3-(5-Chlormethyl-[1,2,4]oxadiazol-3-yl)-benzyl]-6-(3,4,5-trifluorphenyl)-2*H*-pyridazin-3-on in 2 ml THF wird mit 100 mg (1.00 mmol) 1-Methylpiperazin versetzt und 18 Stunden bei Raumtemperatur gerührt. Das Reaktionsgemisch wird zwischen Dichlormethan und Wasser verteilt. Die organische Phase wird über Natriumsulfat getrocknet und eingedampft. Der Rückstand wird in tert.-Butylmethylether aufgekocht, abgekühlt und abgesaugt. Der Rückstand wird mit tert.-Butylmethylether gewaschen, im Vakuum getrocknet und in 10 ml 0.1 N Salzsäure in 2-Propanol gelöst. Nach kurzer Zeit fällt ein Niederschlag aus. Dieser wird abgesaugt, mit tert.-Butylmethylether gewaschen und im Vakuum getrocknet: 2-{3-[5-(4-Methylpiperazin-1-ylmethyl)-[1,2,4]oxadiazol-3-yl]-benzyl}-6-(3,4,5-trifluorphenyl)-2*H*-pyridazin-3-on ("A75") Hydrochlorid als farblose Kristalle; ESI 497.

### Beispiel 22

Die Herstellung von 2-[3-(5-Trifluormethyl-[1,2,4]oxadiazol-3-yl)-benzyl]-6-(3,4,5-trifluor-phenyl)-2*H*-pyridazin-3-on ("A76"), ESI 453, erfolgt aus 2-[3-(5-Amino-[1,2,4]oxadiazol-3-yl)-benzyl]-6-(3,4,5-trifluorphenyl)-2*H-*pyridazin-3-on und Trifluoressigsäureanhydrid nach S. Kitamura et al., Chem. Pharm. Bull. 49(3), 268-277 (2001).

### Beispiel 23

Die Herstellung von 2-(3-[1,2,4]Oxadiazol-3-yl-benzyl)-6-(3,4,5-trifluorphenyl)-2*H*-pyridazin-3-on ("A77"), ESI 385, erfolgt aus 2-[3-(5-Amino-[1,2,4]oxadiazol-3-yl)-benzyl]-6-(3,4,5-trifluorphenyl)-2*H*-pyridazin-3-on und Triethylorthoformiat nach C. Ainsworth et al., J. Med. Chem. 10, 208(1967).

### Beispiel 24

Die Herstellung von 6-(3,5-Difluoro-phenyl)-2-[3-((S)-5-hydroxymethyl-2-oxo-oxazolidin-3-yl)-benzyl]-2H-pyridazin-3-on ("A78") erfolgt analog nachstehendem Schema 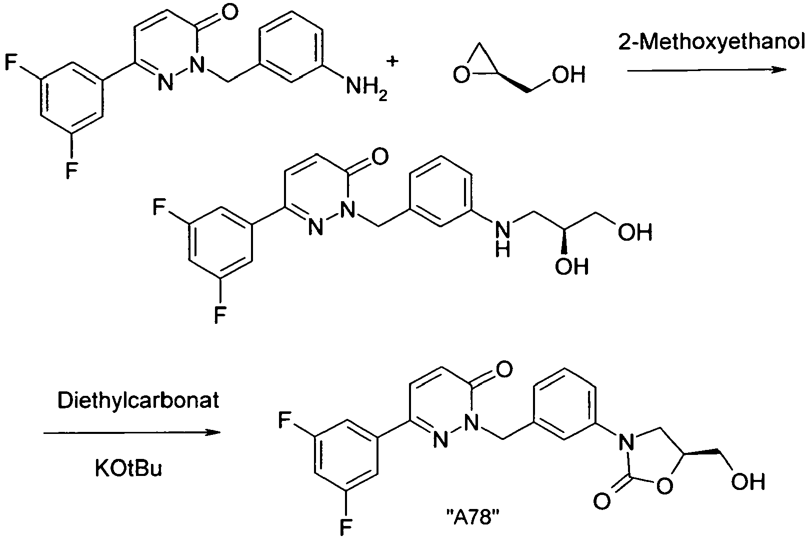
24.1 Eine Lösung von 627 mg (2.00 mmol) 2-(3-Aminobenzyl)-6-(3,5-difluorphenyl)-2*H*-pyridazin-3-on (hergestellt durch Umsetzung von 6-(3,5-Difluorphenyl)-2H-pyridazin-3-on mit 3-Nitrobenzylbromid ähnlich wie in Beispiel 1 mit anschließender Hydrierung mit Raney-Nickel als Katalysator) in 4 ml 2-Methoxyethanol wird mit 0.138 ml (2.00 mmol) (R)-Glycidol versetzt und 18 Stunden auf 120° C erhitzt. Das Reaktionsgemisch wird mit Wasser versetzt und mit Ethylacetat extrahiert. Die organische Phase wird über Natriumsulfat getrocknet und eingedampft. Der Rückstand wird an einer Kieselgelsäule mit Dichlormethan / Methanol als Laufmittel chromatographiert: 6-(3,5-Difluorphenyl)-2-[3-((S)-2,3-dihydroxy-propylamino)-benzyl]-2*H*-pyridazin-3-on als farbloses Öl; ESI 388.

Eine Lösung von 394 mg (1.02 mmol) 6-(3,5-Difluorphenyl)-2-[3-((S)-2,3-dihydroxy-propylamino)-benzyl]-2*H*-pyridazin-3-on in 3 ml Diethylcarbonat wird mit 7.0 mg (0.06 mmol) Kalium-tert.-butanolat versetzt und 3 Stunden bei 110° C gerührt. Das Reaktionsgemisch wird mit Wasser versetzt und mit Dichlormethan extrahiert. Die organische Phase wird über Natriumsulfat getrocknet und eingedampft. Der Rückstand wird an einer Kieselgelsäule mit Petrolether/Ethylacetat als Laufmittel chromatographiert: 6-(3,5-Difluorphenyl)-2-[3-((S)-5-hydroxymethyl-2-oxo-oxazolidin-3-yl)-benzyl]-2H-pyridazin-3-on ("A78") als gelbliche Kristalle; ESI 414.

Analog erhält man die Verbindung 6-(3,5-Difluor-phenyl)-2-[3-((R)-5-hydroxymethyl-2-oxo-oxazolidin-3-yl)-benzyl]-2*H*-pyridazin-3-on ("A79"), ESI 414.

### Beispiel 25

### Die Herstellung von 6-(3,5-Difluor-phenyl)-2-[3-(2-oxo-oxazolidin-3-yl)-benzyl]-2H-pyridazin-3-on ("A80") erfolgt analog nachstehendem

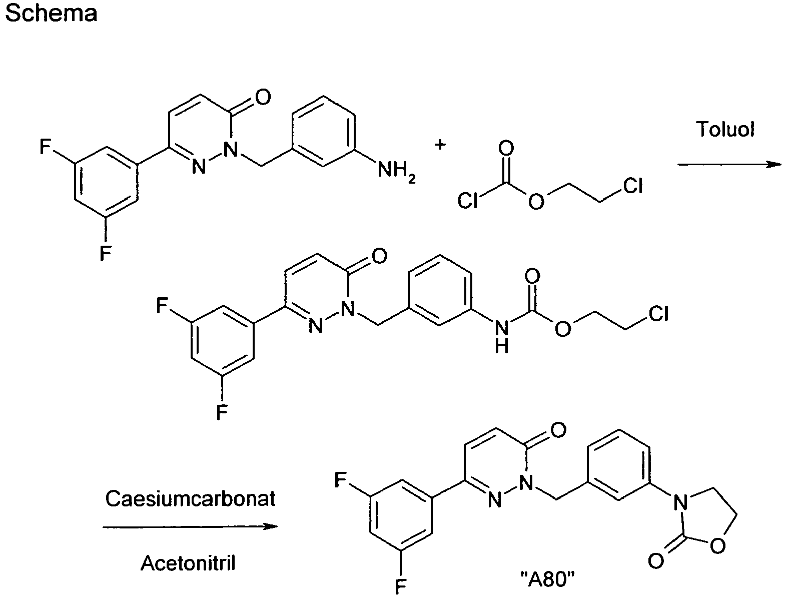

25.1 Eine Suspension von 313 mg (1.00 mmol) 2-(3-Aminobenzyl)-6-(3,5-difluorphenyl)-2*H*-pyridazin-3-on in 4 ml Toluol wird mit 0.155 ml (1.50 mmol) 2-Chlorethyl-chlorformiat versetzt und 18 Stunden auf 110° erhitzt. Man lässt abkühlen, saugt den entstandenen Niederschlag ab und wäscht ihn mit tert.-Butylmethylether: Der Rückstand wird an einer Kieselgelsäule mit Dichlormethan/Methanol als Laufmittel chromatographiert: {3-[3-(3,5-Difluorphenyl)-6-oxo-6*H*-pyridazin-1-ylmethyl]-phenyl}-carbaminsäure-2-chlorethylester als farblose Kristalle; ESI 420.

Eine Lösung von 373 mg (0.89 mmol) {3-[3-(3,5-Difluorphenyl)-6-oxo-6*H-*pyridazin-1-ylmethyl]-phenyl}-carbaminsäure-2-chlorethylester in 4 ml Acetonitril wird mit 579 mg (1.78 mmol) Caesiumcarbonat versetzt und die resultierende Suspension 18 Stunden bei Raumtemperatur gerührt. Das Reaktionsgemisch wird filtriert und der Rückstand mit Acetonitril gewaschen. Das Filtrat wird eingedampft: 6-(3,5-Difluorphenyl)-2-[3-(2-oxo-oxazolidin-3-yl)-benzyl]-2*H*-pyridazin-3-on ("A80") als farblose Kristalle; ESI 384.

### Beispiel 26

Die Herstellung von 6-(3,5-Difluor-phenyl)-2-[3-(2-methyl-thiazol-4-yl)-benzyl]-2*H*-pyridazin-3-on ("A81") erfolgt analog nachstehendem Schema

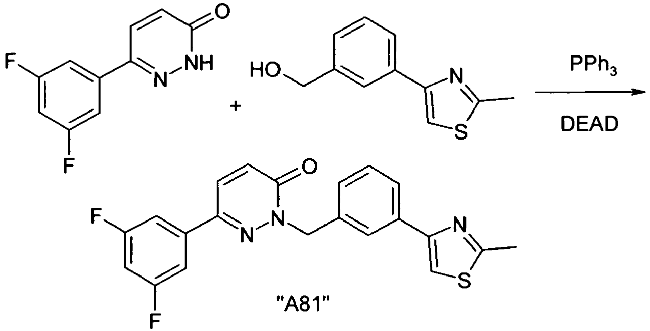

Eine Lösung von 200 mg (0.96 mmol) 6-(3,5-Difluorphenyl)-2*H-*pyridazin-3-on, 296 mg (1.44 mmol) [3-(2-Methylthiazol-4-yl)-phenyl]-methanol und 378 mg (1.44 mmol) Triphenylphosphin in 25 ml THF wird unter Eiskühlung tropfenweise mit 227 µl (1.44 mmol) Diethylazodicarboxylat versetzt. Das Reaktionsgemisch wird 18 Stunden bei Raumtemperatur gerührt, eingedampft und der Rückstand mit präparativer HPLC chromatographiert: 6-(3,5-Difluorphenyl)-2-[3-(2-methyl-thiazol-4-yl)-benzyl]-2*H*-pyridazin-3-on ("A81") als farblose Kristalle; ESI 396.

### Analog erhält man die Verbindungen

6-(3-Cyanphenyl)-2-[3-(2-methyl-thiazol-4-yl)-benzyl]-2*H*-pyridazin-3-on ("A82"), ESI 385;
6-(3,5-Difluor-phenyl)-2-[3-(1*H*-imidazol-2-yl)-benzyl]-2*H*-pyridazin-3-on ("A83"), ESI 365 (der verwendete [3-(1*H*-Imidazol-2-yl)-phenyl]-methanol wird hergestellt durch Lithiumalanat-Reduktion von 3-(1*H-*Imidazol-2-yl)-benzoesäuremethylester, der wiederum in Analogie zu T. Mano et al., Bioorg. Med. Chem. 11(18), 3879 (2003) hergestellt wird).

### Beispiel 27

Die Herstellung von 6-(3,5-Difluor-phenyl)-2-[3-(5-methyl-thiazol-2-yl)-benzyl]-2*H*-pyridazin-3-on ("A84") und von 6-(3,5-Difluor-phenyl)-2-[3-(5-methyl-4,5-dihydro-oxazol-2-yl)-benzyl]-2*H*-pyridazin-3-on ("A85") erfolgt analog nachstehendem Schema

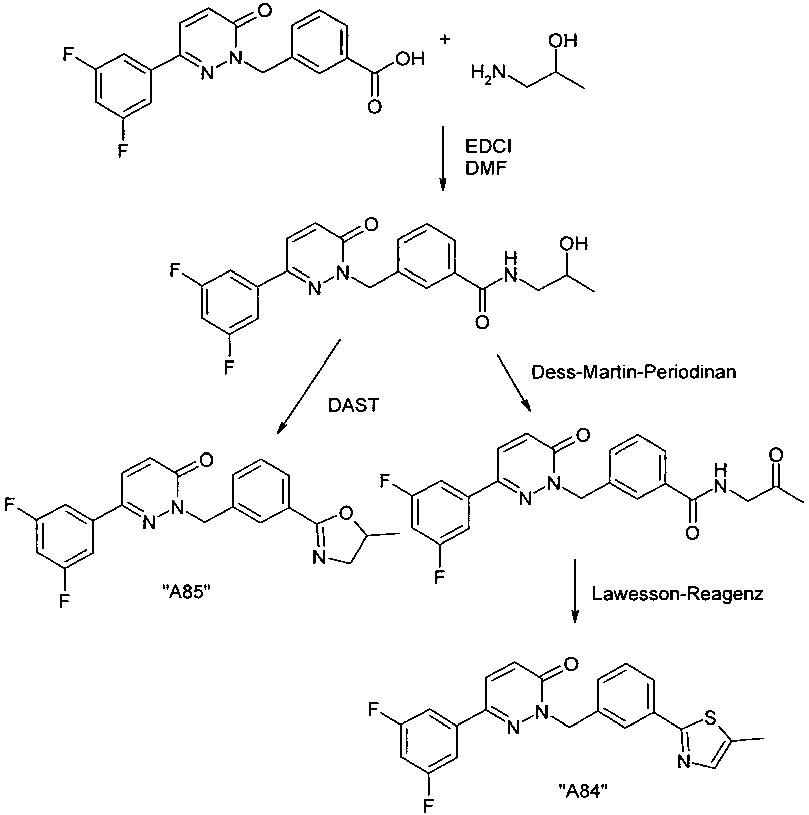

Eine Lösung von 685 mg (2.00 mmol) 3-[3-(3,5-Difluorphenyl)-6-oxo-6*H-*pyridazin-1-ylmethyl]-benzoesäure und 156 µl (2.00 mmol) 1-Amino-propan-2-ol in 4 ml DMF wird mit 498 mg (2.60 mmol) N-(3-Dimethylaminopropyl)-N'-ethylcarbodiimid-hydrochlorid versetzt und 18 Stunden bei Raumtemperatur gerührt. Das Reaktionsgemisch wird auf 1 N HCl gegeben, der entstandene Niederschlag abgesaugt und mit Wasser gewaschen. Der Rückstand wird im Vakuum getrocknet: 3-[3-(3,5-Difluorphenyl)-6-oxo-6*H*-pyridazin-1-ylmethyl]-N-(2-hydroxy-propyl)-benzamid als farblose Kristalle; ESI 400.

Eine Lösung von 126 mg (0.32 mmol) 3-[3-(3,5-Difluorphenyl)-6-oxo-6*H-*pyridazin-1-ylmethyl]-N-(2-hydroxy-propyl)-benzamid in 3 ml Dichlormethan wird auf -78° C gekühlt und unter Rühren 46 µl (0.35 mmol) Diethylaminoschwefeltrifluorid zugegeben. Das Reaktionsgemisch wird 1 Stunde bei -78° C gerührt. Dann werden 34.8 mg (0.25 mmol) Kaliumcarbonat zugegeben, das Reaktionsgemisch wird auf Raumtemperatur erwärmt und 18 Stunden bei Raumtemperatur gerührt. Das Reaktionsgemisch wird mit gesättigter Natriumhydrogencarbonatlösung und Dichlormethan versetzt. Die organische Phase wird abgetrennt, über Natriumsulfat getrocknet und eingedampft. Der Rückstand wird an einer Kieselgelsäule mit Dichlormethan/Methanol als Laufmittel chromatographiert: 6-(3,5-Difluorphenyl)-2-[3-(5-methyl-4,5-dihydro-oxazol-2-yl)-benzyl]-2*H*-pyridazin-3-on ("A85") als farbloser Feststoff; ESI 382.

Eine Lösung von 280 mg (0.70 mmol) 3-[3-(3,5-Difluorphenyl)-6-oxo-6*H-*pyridazin-1-ylmethyl]-N-(2-hydroxy-propyl)-benzamid in 5 ml Dichlormethan wird mit 4.0 g einer 15%igen Lösung von Dess-Martin-Periodinan in Dichlormethan versetzt und 1 Stunde bei Raumtemperatur gerührt. Das Reaktionsgemisch wird mit 5 ml Wasser, 5 ml gesättigter Natriumhydrogencarbonat-Lösung und 15 ml einer 10%igen wässrigen Natriumthiosulfat-Lösung versetzt. Nach 2 Stunden Rühren bei Raumtemperatur wird der entstandene Niederschlag abgesaugt. Die organische Phase des Filtrats wird abgetrennt, über Natriumsulfat getrocknet und eingedampft: 3-[3-(3,5-Difluorphenyl)-6-oxo-6*H*-pyridazin-1-ylmethyl]-N-(2-oxo-propyl)-benzamid als farblose Kristalle; ESI 398.

Eine Suspension von 119 mg (0.30 mmol) 3-[3-(3,5-Difluorphenyl)-6-oxo-6*H*-pyridazin-1-ylmethyl]-N-(2-oxo-propyl)-benzamid und 60.7 mg (0.15 mmol) 2,4-Bis(4-methoxyphenyl)-2,4-dithioxo-1,3,2,4-dithiaphosphetan (Lawesson-Reagenz) in 3 ml Toluol wird 18 Stunden auf 110° erhitzt. Man lässt abkühlen und gibt gesättigte Natriumhydrogencarbonat-Lösung und Dichlormethan zu. Die organische Phase wird abgetrennt, über Natriumsulfat getrocknet und eingedampft. Der Rückstand wird an einer Kieselgelsäule mit Dichlormethan/Methanol als Laufmittel chromatographiert: 6-(3,5-Difluorphenyl)-2-[3-(5-methyl-thiazol-2-yl)-benzyl]-2*H*-pyridazin-3-on ("A84") als gelblicher Feststoff; ESI 396.

Analog erhält man die Verbindung 6-(3-Chlor-phenyl)-2-[3-(5-methyl-thiazol-2-yl)-benzyl]-2*H*-pyridazin-3-on ("A86"), ESI 394.

### Beispiel 28

Die Herstellung von 6-(3,5-Difluor-phenyl)-2-[3-(5-piperidin-4-yl-oxazol-2-yl)-benzyl]-2*H*-pyridazin-3-on ("A87") und von 6-(3,5-Difluor-phenyl)-2-{3-[5-(1-methyl-piperidin-4-yl)-oxazol-2-yl]-benzyl}-2*H*-pyridazin-3-on ("A88") erfolgt analog nachstehendem Schema 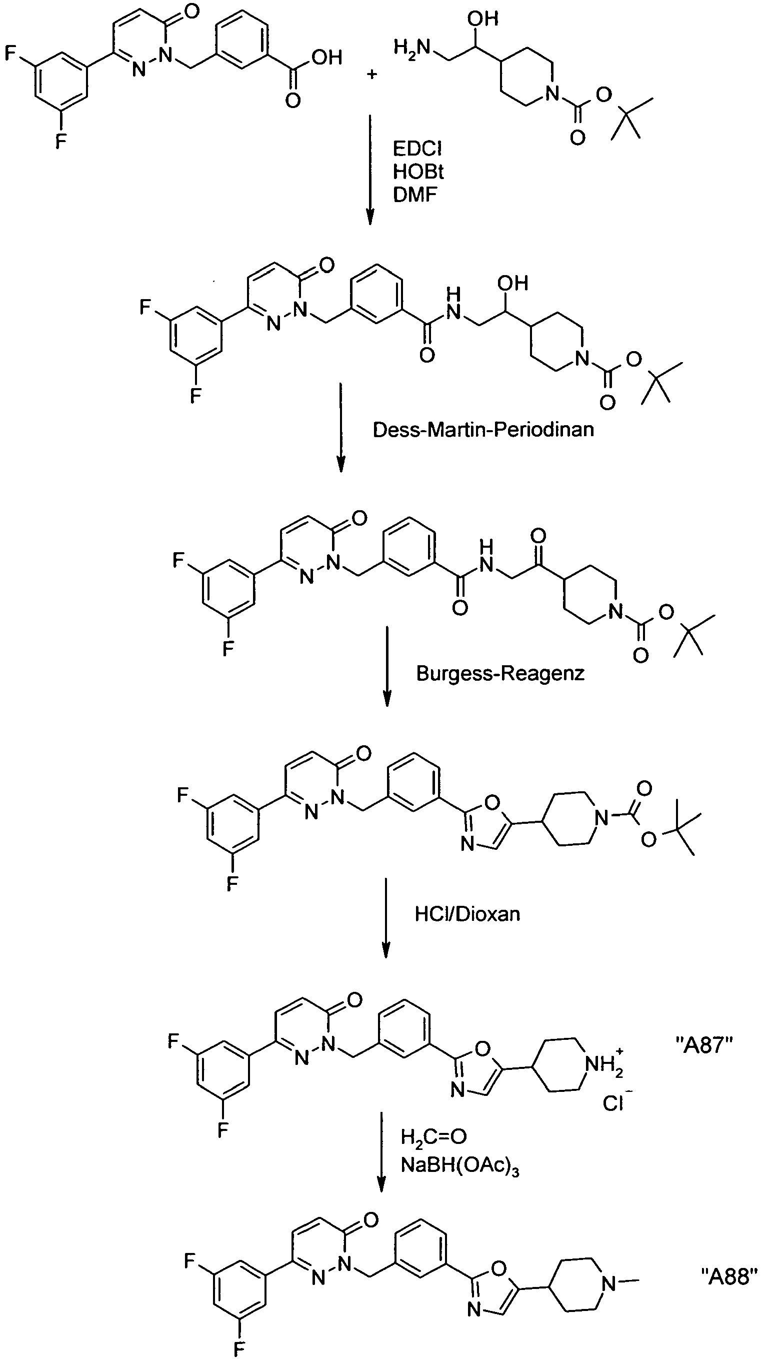
28.1 Eine Lösung von 1.08 g (3.16 mmol) 3-[3-(3,5-Difluorphenyl)-6-oxo-6*H*-pyridazin-1-ylmethyl]-benzoesäure und 771 mg (3.16 mmol) 4-(2-Amino-1-hydroxy-ethyl)-piperidin-1-carbonsäure-tert.-butylester (hergestellt nach WO00/59502 oder WO2006/019768) in 20 ml DMF wird mit 484 mg (3.16 mmol) 1-Hydroxybenzotriazol-Hydrat und 786 mg (4.10 mmol) N-(3-Dimethylaminopropyl)-N'-ethylcarbodiimid-hydrochlorid versetzt und 18 Stunden bei Raumtemperatur gerührt. Das Reaktionsgemisch wird mit Wasser versetzt. Der entstandene Niederschlag wird abgesaugt, mit Wasser gewaschen und im Vakuum getrocknet: 4-(2-{3-[3-(3,5-Difluorphenyl)-6-oxo-6H-pyridazin-1-ylmethyl]-benzoylamino}-1-hydroxyethyl)-piperidin-1-carbonsäure-tert.-butylester als farbloser Feststoff; ESI 569.
28.2 Eine Lösung von 1.14 g (2.00 mmol) 4-(2-{3-[3-(3,5-Difluor-phenyl)-6-oxo-6*H*-pyridazin-1-ylmethyl]-benzoylamino}-1-hydroxy-ethyl)-piperidin-1-carbonsäure-tert.-butylester in 15 ml Dichlormethan wird mit 16.7 g einer 15%igen Lösung von Dess-Martin-Periodinan in Dichlormethan versetzt und eine Stunde bei Raumtemperatur gerührt. Das Reaktionsgemisch wird mit 15 ml Wasser, 15 ml gesättigter Natriumhydrogencarbonat-Lösung und 15 ml einer 10%igen wässrigen Natriumthiosulfat-Lösung versetzt. Nach 2 Stunden Rühren bei Raumtemperatur wird der entstandene Niederschlag abgesaugt. Die organische Phase des Filtrats wird abgetrennt, über Natriumsulfat getrocknet und eingedampft: 4-(2-{3-[3-(3,5-Difluorphenyl)-6-oxo-6*H*-pyridazin-1-ylmethyl]-benzoylamino}-acetyl)-piperidin-1-carbonsäure-tert.-butylester; ESI 567.
28.3 Eine Lösung von 453 mg (0.8 mmol) 4-(2-{3-[3-(3,5-Difluor-phenyl)-6-oxo-6*H*-pyridazin-1-ylmethyl]-benzoylamino}-acetyl)-piperidin-1-carbonsäure-tert.-butylester und 381 mg (1.60 mmol) (Methoxycarbonyl-sulfamoyl)-triethylammonium-betain (Burgess-Reagenz) in 5 ml THF wird 18 Stunden auf 60° C erhitzt. Das Reaktionsgemisch wird zwischen Dichlormethan und gesättigter wässriger Natriumhydrogencarbonat-Lösung verteilt. Die organische Phase wird über Natriumsulfat getrocknet, eingedampft und der Rückstand an einer Kieselgelsäule mit Dichlormethan/Methanol als Laufmittel chromatographiert: 4-(2-{3-[3-(3,5-Difluorphenyl)-6-oxo-6*H*-pyridazin-1-ylmethyl]-phenyl}-oxazol-5-yl)-piperidin-1-carbonsäure-tert.-butylester als farblose Kristalle; ESI 549.
28.4 237 mg (0.43 mmol) 4-(2-{3-[3-(3,5-Difluorphenyl)-6-oxo-6*H-*pyridazin-1-ylmethyl]-phenyl}-oxazol-5-yl)-piperidin-1-carbonsäure-tert.-butylester werden in 1.5 ml 4 N HCl in Dioxan gelöst und 18 Stunden bei Raumtemperatur belassen. Der entstandene Niederschlag wird abgesaugt und mit Dioxan und tert.-Butylmethylether gewaschen und im Vakuum getrocknet: 6-(3,5-Difluorphenyl)-2-[3-(5-piperidin-4-yl-oxazol-2-yl)-benzyl]-2*H*-pyridazin-3-on ("A87") Hydrochlorid als farblose Kristalle; ESI 449; ¹H-NMR (d₆-DMSO): δ [ppm] = 1.84 (m, 2H), 2.16 (m, 2H), 3.03 (m, 2H), 3.16 (m, 1H), 3.32 (m, 2H), 5.43 (s, 2H), 7.12 (s, 1H), 7.15 (d, J = 10 Hz, 1 H), 7.36 (tt, J₁ = 9.5 Hz, J₂ = 2 Hz, 1 H), 7.52 (m, 2H), 7.66 (m, 2H), 7.88 (m, 1 H), 7.99 (s, 1 H), 8.16 (d, J = 10 Hz, 1 H), 8.72 (m, 1 H), 9.00 (m, 1H).
28.5 Eine Lösung von 80.3 mg (0.17 mmol) 6-(3,5-Difluorphenyl)-2-[3-(5-piperidin-4-yl-oxazol-2-yl)-benzyl]-2*H*-pyridazin-3-on Hydrochlorid in 1.5 ml 37%iger wässriger Formaldehydlösung wird mit 148 mg (0.66 mmol) Natriumtriacetoxyborhydrid versetzt und 18 Stunden bei Raumtemperatur gerührt. Das Reaktionsgemisch wird mit gesättigter Natriumhydrogencarbonat-Lösung und 15%iger wässriger Natronlauge versetzt und mit Ethylacetat extrahiert. Die organische Phase wird über Natriumsulfat getrocknet und eingedampft. Der Rückstand wird in tert.-Butylmethylether aufgekocht, abkühlen lassen, abgesaugt und im Vakuum getrocknet: 6-(3,5-Difluorphenyl)-2-{3-[5-(1-methylpiperidin-4-yl)-oxazol-2-yl]-benzyl}-2*H*-pyridazin-3-on ("A88") als farblose Kristalle; ESI 463;

¹H-NMR (d₆-DMSO): δ [ppm] = 1.63 (m, 2H), 1.94 (m, 2H), 1.99 (m, 2H), 2.18 (s, 3H), 2.72 (m, 1H), 2.78 (m, 2H), 5.42 (s, 2H), 7.00 (d, J = 1Hz, 1H), 7.14 (d, J = 10 Hz, 1H), 7.35 (tt, J₁ = 9.5 Hz, J₂ = 2 Hz, 1 H), 7.50 (m, 2H), 7.66 (m, 2H), 7.86 (m, 1H), 7.99 (s, 1H), 8.15 (d, J = 10 Hz, 1H).

### Analog werden die nachstehenden Verbindungen hergestellt

2-[3-(5-Piperidin-4-yl-oxazol-2-yl)-benzyl]-6-(3,4,5-trifluor-phenyl)-2*H*-pyridazin-3-on ("A89"), ESI 467,

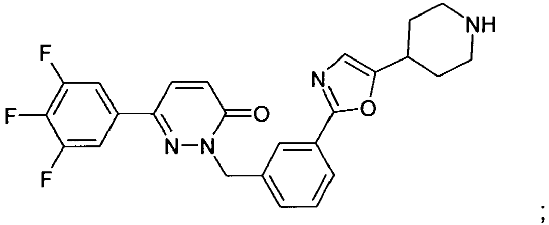

2-[3-(5-Piperidin-4-yl-oxazol-2-yl)-benzyl]-6-(3-chlor-phenyl)-2*H-*pyridazin-3-on ("A90"),

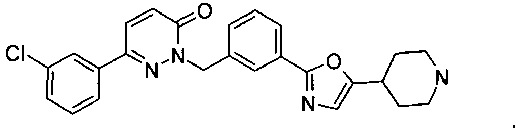

### Beispiel 29

Die Herstellung von 6-(3,5-Difluor-phenyl)-2-[3-(5-piperidin-4-yl-thiazol-2-yl)-benzyl]-2*H*-pyridazin-3-on ("A91") und von 6-(3,5-Difluor-phenyl)-2-{3-[5-(1-methyl-piperidin-4-yl)-thiazol-2-yl]-benzyl}-2*H*-pyridazin-3-on ("A92") erfolgt analog nachstehendem Schema 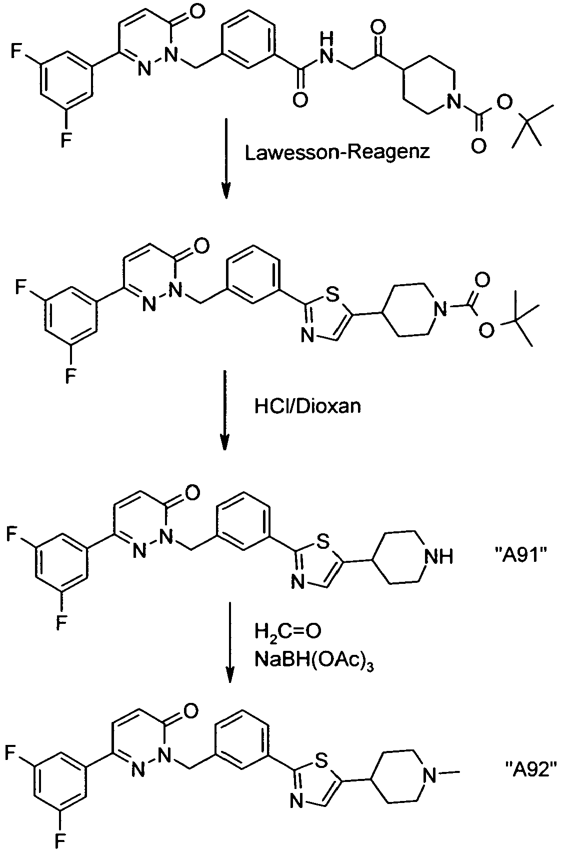
29.1 Eine Suspension von 1.90 g (3.36 mmol) 4-(2-{3-[3-(3,5-Difluorphenyl)-6-oxo-6*H*-pyridazin-1-ylmethyl]-benzoylamino}-acetyl)-piperidin-1-carbonsäure-tert.-butylester und 681 mg (1.68 mmol) 2,4-Bis(4-methoxyphenyl)-2,4-dithioxo-1,3,2,4-dithiaphosphetan (Lawesson-Reagenz) in 6 ml Toluol wird 18 Stunden auf 110° C erhitzt. Man lässt abkühlen und gibt gesättigte Natriumhydrogencarbonat-Lösung und Dichlormethan zu. Die organische Phase wird abgetrennt, über Natriumsulfat getrocknet und eingedampft. Der Rückstand wird an einer Kieselgelsäule mit Dichlormethan/Methanol als Laufmittel chromatographiert: 4-(2-{3-[3-(3,5-Difluorphenyl)-6-oxo-6*H*-pyridazin-1-ylmethyl]-phenyl}-thiazol-5-yl)-piperidin-1-carbonsäure-tert.-butylester als braungelbes Öl; ESI 565.
29.2 1.13 g (0.43 mmol) 4-(2-{3-[3-(3,5-Difluorphenyl)-6-oxo-6*H-*pyridazin-1-ylmethyl]-phenyl}-thiazol-5-yl)-piperidin-1-carbonsäure-tert.-butylester werden in 12 ml 4 N HCl in Dioxan gelöst und 18 Stunden bei Raumtemperatur belassen. Das Reaktionsgemisch wird mit Wasser und Ethylacetat versetzt. Die organische Phase wird abgetrennt. Die wässrige Phase wird mit 15%iger wässriger Natronlauge alkalisiert und mit Ethylacetat extrahiert. Die organische Phase wird über Natriumsulfat getrocknet und eingedampft. Der Rückstand wird an einer Kieselgelsäule mit Dichlormethan/Methanol chromatographiert: 6-(3,5-Difluorphenyl)-2-[3-(5-piperidin-4-yl-thiazol-2-yl)-benzyl]-2*H*-pyridazin-3-on ("A91") als gelbliche Kristalle; ESI 465;
   ¹H-NMR (d₆-DMSO): δ [ppm] = 1.54 (m, 2H), 1.94 (m, 2H), 2.65 (m, 2H), 3.04 (m, 3H), 5.43 (s, 2H), 7.15 (d, J = 10 Hz, 1H), 7.36 (tt, J1 = 9.5 Hz, J2 = 2 Hz, 1H), 7.47 (m, 2H), 7.66 (m, 3H), 7.80 (d, J = 7.5 Hz, 1H), 7.96 (s, 1 H), 8.16 (d, J = 10 Hz, 1H).

Analog zu Beispiel 28.5 wird 6-(3,5-Difluorphenyl)-2-[3-(5-piperidin-4-yl-thiazol-2-yl)-benzyl]-2*H*-pyridazin-3-on mit Formaldehyd und Natriumtriacetoxyborhydrid zu 6-(3,5-Difluorphenyl)-2-{3-[5-(1-methylpiperidin-4-yl)-thiazol-2-yl]-benzyl}-2*H*-pyridazin-3-on ("A92"), ESI 479, umgesetzt.

### Analog werden nachstehende Verbindungen erhalten

2-[3-(5-Piperidin-4-yl-thiazol-2-yl)-benzyl]-6-(3,4,5-trifluor-phenyl)-2*H*-pyridazin-3-on ("A93") Formiat, ESI 483;
6-(3-Chlor-phenyl)-2-[3-(5-piperidin-4-yl-thiazol-2-yl)-benzyl]-2H-pyridazin-3-on ("A94") Formiat, ESI 464;
6-(3,5-Difluorphenyl)-2-[3-(5-piperidin-3-yl-thiazol-2-yl)-benzyl]-2*H*-pyridazin-3-on ("A96") Hydrochlorid, ESI 465;
2-[3-(5-Azetidin-3-yl-thiazol-2-yl)-benzyl]-6-(3,5-difluor-phenyl)-2H-pyridazin-3-on ("A97") 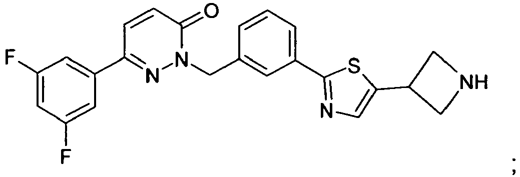 6-(3,5-Difluorphenyl)-2-[3-(5-piperidin-4-ylmethyl-thiazol-2-yl)-benzyl]-2H-pyridazin-3-on ("A98"), Trifluoracetat, ESI 479;
6-(3,5-Difluor-phenyl)-2-(3-{5-[1-(2-methoxy-ethyl)-piperidin-4-yl]-thiazol-2-yl}-benzyl)-2*H*-pyridazin-3-on ("A99"), Hydrochlorid, ESI 523, 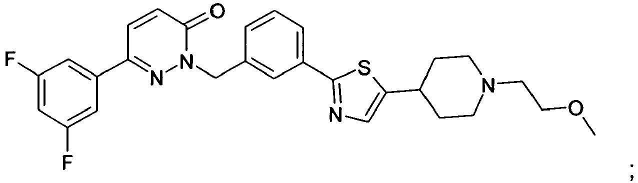 6-(3,5-Difluor-phenyl)-2-{3-[5-(1-methyl-piperidin-4-ylmethyl)-thiazol-2-yl]-benzyl}-2*H*-pyridazin-3-on ("A99a");
6-(3,5-Difluorphenyl)-2-[3-(5-pyrrolidin-2-yl-thiazol-2-yl)-benzyl]-2*H*-pyridazin-3-on ("A100").

### Beispiel 30

Die Herstellung von 6-(3-Chlor-phenyl)-2-{3-[5-(4-methyl-piperazin-1-ylmethyl)-thiazol-2-yl]-benzyl}-2*H*-pyridazin-3-on ("A95") erfolgt analog nachstehendem Schema 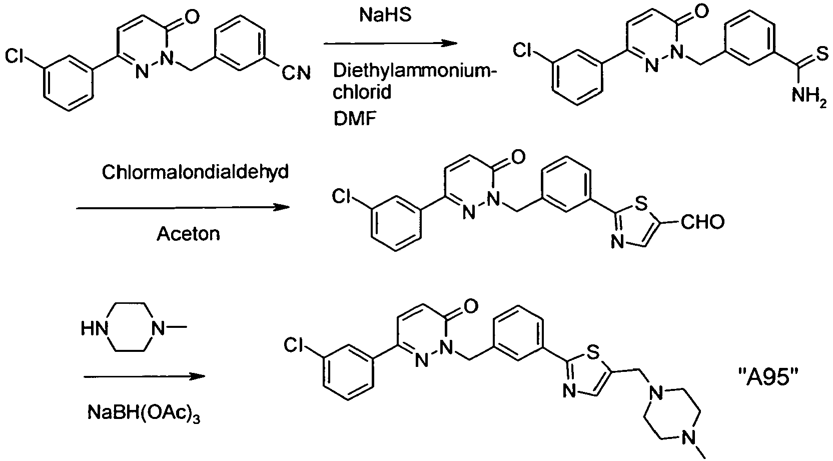
30.1 Eine Lösung von 4.56 g (13.9 mmol) 3-[3-(3-Chlorphenyl)-6-oxo-6*H*-pyridazin-1-ylmethyl]-benzonitril in 10 ml DMF wird mit 3.44 g (41.7 mmol) Natriumhydrogensulfid-hydrat und 4.57 g Diethylammoniumchlorid versetzt und die resultierende Suspension unter Stickstoff 18 Stunden bei 55° C gerührt. Nach Abkühlen auf Raumtemperatur werden 30 ml Wasser zugegeben. Der entstandene Niederschlag wird abgesaugt, mit Wasser gewaschen und im Vakuum getrocknet: 3-[3-(3-Chlorphenyl)-6-oxo-6*H-*pyridazin-1-ylmethyl]-thiobenzamid als gelbe Kristalle; ESI 356.
30.2 Eine Suspension von 3.97 g (10.6 mmol) 3-[3-(3-Chlorphenyl)-6-oxo-6*H*-pyridazin-1-ylmethyl]-thiobenzamid und 1.55 g (13.8 mmol) Chlormalondialdehyd in 22 ml Aceton wird 5 Stunden unter Rühren zum Sieden erhitzt. Das Reaktionsgemisch wird eingedampft und der Rückstand im Vakuum getrocknet: 2-{3-[3-(3-Chlorphenyl)-6-oxo-6*H-*pyridazin-1-ylmethyl]-phenyl}-thiazol-5-carbaldehyd als braunes Glas, das ohne weitere Reinigung für Folgereaktionen eingesetzt wird; ESI 408.
30.3 Eine Lösung von 527 mg (ca. 1.0 mmol) rohem 2-{3-[3-(3-Chlorphenyl)-6-oxo-6*H*-pyridazin-1-ylmethyl]-phenyl}-thiazol-5-carbaldehyd und 128 mg (1.3 mmol) 1-Methylpiperazin in 4 ml Dichlormethan wird mit 60 mg (1.0 mmol) Essigsäure und 320 mg (1.51 mmol) Natriumtriacetoxyborhydrid versetzt und 18 Stunden bei Raumtemperatur gerührt. Das Reaktionsgemisch wird mit 1 N NaOH versetzt und die organische Phase abgetrennt. Die organische Phase wird über Natriumsulfat getrocknet und eingedampft. Der Rückstand wird an einer Kieselgelsäule mit Dichlormethan/Methanol als Laufmittel chromatographiert: 6-(3-Chlorphenyl)-2-{3-[5-(4-methyl-piperazin-1-ylmethyl)-thiazol-2-yl]-benzyl}-2*H*-pyridazin-3-on ("A95") als ockergelbe Kristalle; ESI 493.

### Analog werden die nachstehenden Verbindungen hergestellt

6-(3,5-Difluor-phenyl)-2-{3-[5-(4-methyl-piperazin-1-ylmethyl)-thiazol-2-yl]-benzyl}-2*H*-pyridazin-3-on ("A101"), ESI 494;
6-(3,5-Difluor-phenyl)-2-[3-(5-morpholin-4-ylmethyl-thiazol-2-yl)-benzyl]-2*H*-pyridazin-3-on ("A102"), ESI 481.

### Beispiel 31

Die Herstellung von 6-(3,5-Difluor-phenyl)-2-[3-(4-methyl-thiazol-2-yl)-benzyl]-2*H*-pyridazin-3-on ("A103") erfolgt analog nachstehendem

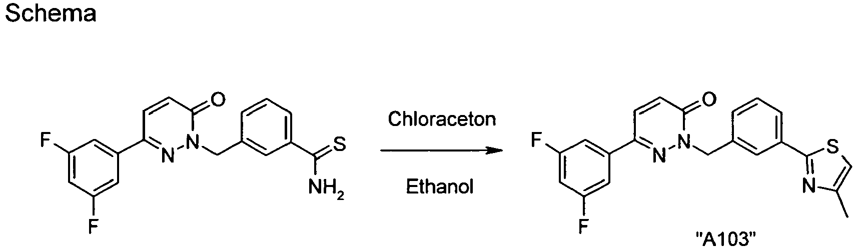

Eine Lösung von 179 g (0.50 mmol) 3-[3-(3,5-Difluorphenyl)-6-oxo-6*H-*pyridazin-1-ylmethyl]-thiobenzamid in 3 ml Ethanol wird mit 60 µl Chloraceton versetzt und 5 Stunden zum Sieden erhitzt. Das Reaktionsgemisch wird eingedampft und der Rückstand an einer Kieselgelsäule mit Dichlormethan/Methanol chromatographiert: 6-(3,5-Difluorphenyl)-2-[3-(4-methyl-thiazol-2-yl)-benzyl]-2*H*-pyridazin-3-on ("A103") als gelblicher Feststoff; ESI 396.

### Analog erhält man die Verbindung

6-(3,5-Difluorphenyl)-2-(3-thiazol-2-yl-benzyl)-2*H*-pyridazin-3-on ("A104"), ESI 382.

### Beispiel 32

Die Herstellung von 6-(3,5-Difluor-phenyl)-2-{3-[1-(2-piperidin-1-yl-ethyl)-1*H*-[1,2,3]triazol-4-yl]-benzyl}-2H-pyridazin-3-on ("A105") erfolgt analog nachstehendem Schema

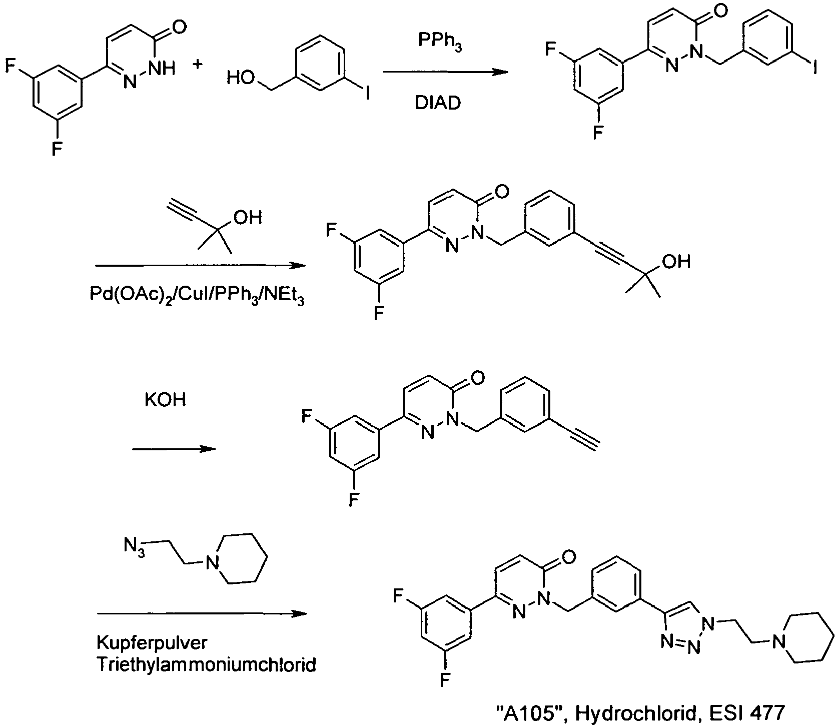

Analog erhält man die Verbindung 6-(3,5-Difluor-phenyl)-2-{3-[1-(2-morpholin-4-yl-ethyl)-1*H*-[1,2,3]triazol-4-yl]-benzyl}-2*H*-pyridazin-3-on ("A106"), ESI 479.

### Beispiel 33

Die Herstellung von 6-(3,5-Difluor-phenyl)-2-{3-[5-(4-methyl-piperazin-1-carbonyl)-isoxazol-3-yl]-benzyl}-2*H*-pyridazin-3-on ("A107") erfolgt analog nachstehendem Schema

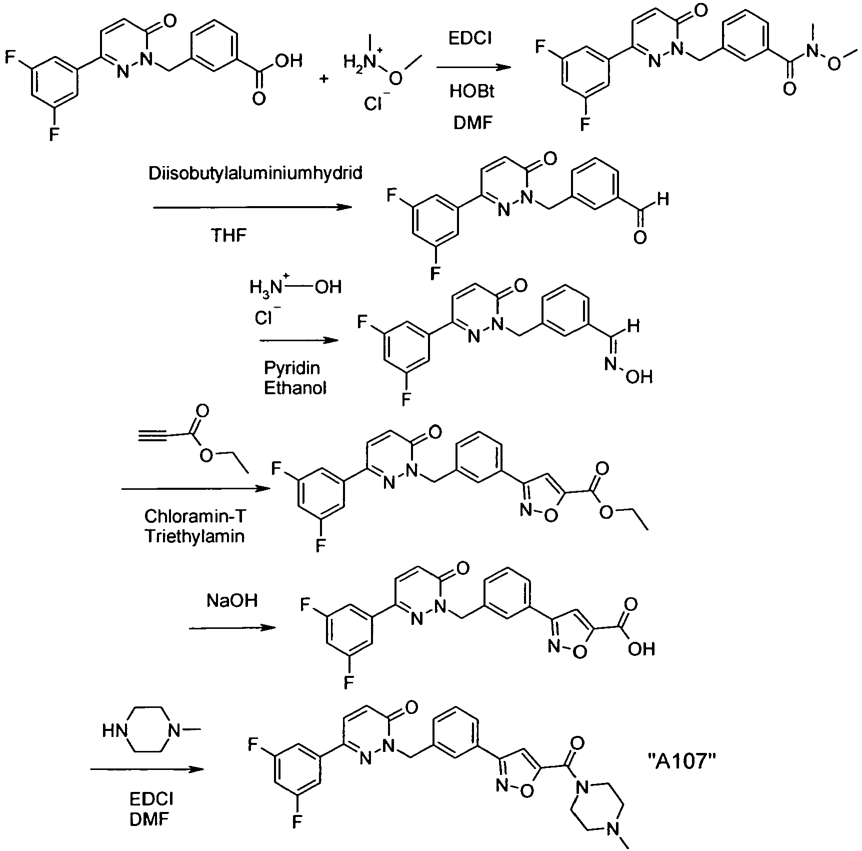

### Beispiel 34

Die Herstellung von 6-(3,5-Difluor-phenyl)-2-[3-(5-oxo-4,5-dihydro-[1,2,4]thiadiazol-3-yl)-benzyl]-2*H*-pyridazin-3-on ("A108"), ESI 399, erfolgt analog nachstehendem Schema

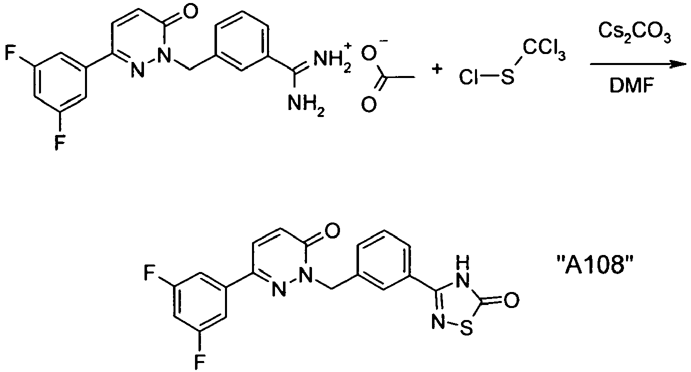

### Beispiel 35

Die Herstellung von 6-(3,5-Difluor-phenyl)-2-{3-[5-(4-methyl-piperazin-1-yl)-[1,2,4]thiadiazol-3-yl]-benzyl}-*2*H-pyridazin-3-on ("A109") erfolgt analog nachstehendem Schema

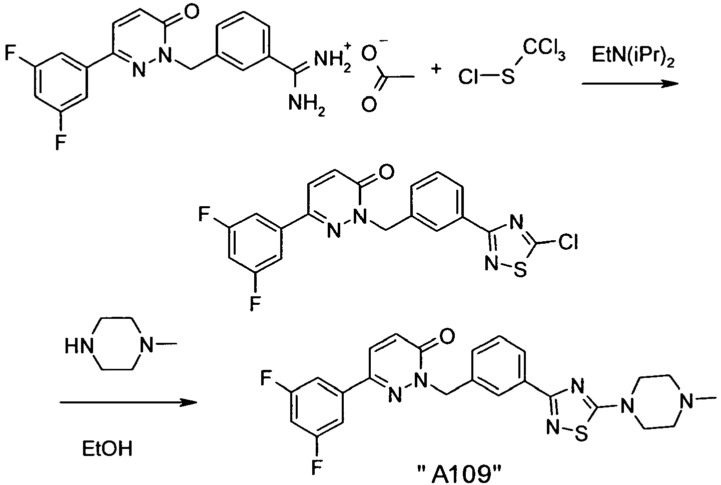

Analog erhält man die Verbindung 6-(3,5-Difluor-phenyl)-2-(3-{5-[4-(2-methoxy-ethyl)-piperazin-1-yl]-[1,2,4]thiadiazol-3-yl}-benzyl)-2*H-*pyridazin-3-on ("A109a"), Hydrochlorid, ESI 525

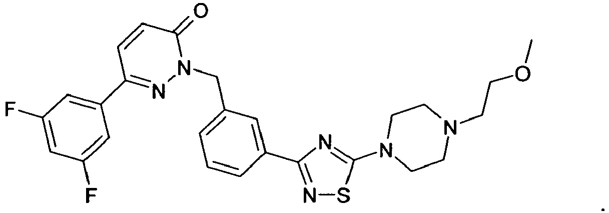

### Beispiel 36

Die Herstellung von 6-(3,5-Difluor-phenyl)-2-[3-(5-morpholin-4-ylmethyl-oxazol-2-yl)-benzyl]-2*H*-pyridazin-3-on ("A110"), ESI 465, erfolgt analog nachstehendem Schema

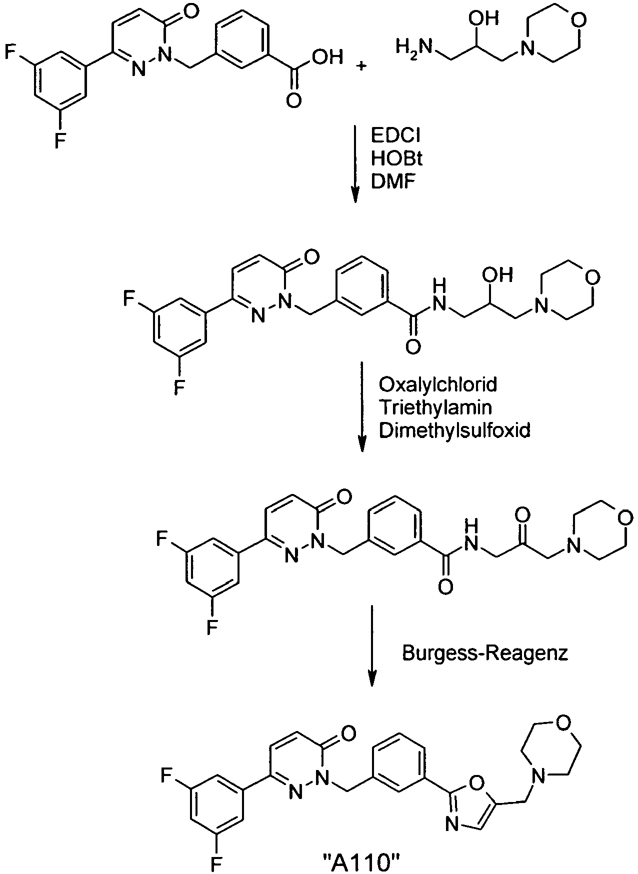

### Beispiel 37

Die Herstellung von 6-(3,5-Difluorphenyl)-2-(3-{5-[4-(2-hydroxyethyl)-piperazin-1-yl]-[1,2,4]oxadiazol-3-yl}-benzyl)-2H-pyridazin-3-on ("A111") erfolgt analog nachstehendem Schema: 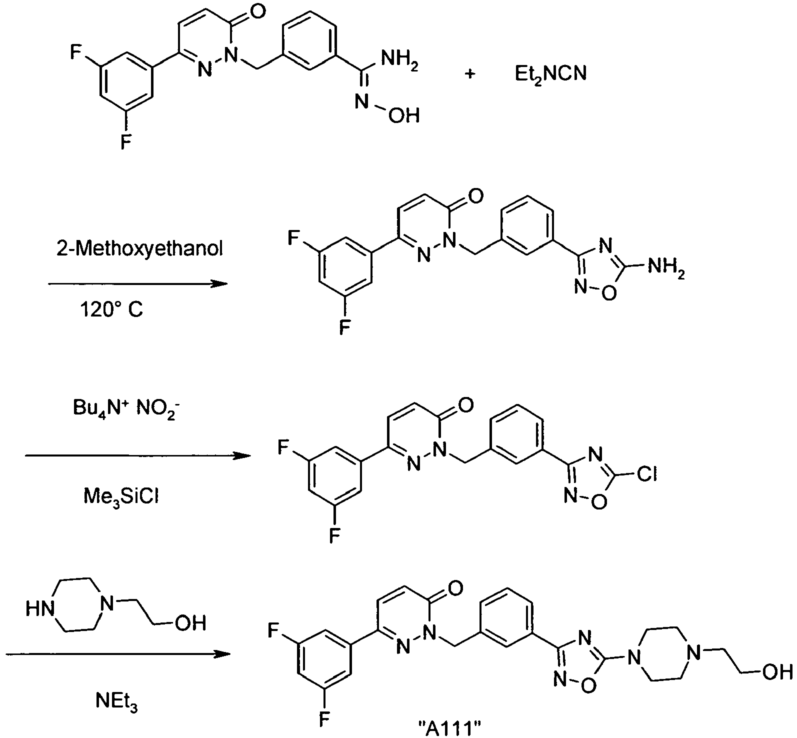
37.1 Eine Suspension von 5.35 g (15.0 mmol) N-Hydroxy-3-[6-oxo-3-(3,5-difluorphenyl)-6*H*-pyridazin-1-ylmethyl]-benzamidin in 30 ml 2-Methoxyethanol wird mit 1.77 g (18.0 mmol) Diethylcyanamid versetzt und 18 Stunden bei einer Temperatur von 120° gerührt. Man lässt abkühlen, saugt das Reaktionsgemisch ab und wäscht den Rückstand mit Dichlormethan. Der Rückstand wird im Vakuum getrocknet: 2-[3-(5-Amino-[1,2,4]oxadiazol-3-yl)-benzyl]-6-(3,5-difluorphenyl)-2*H*-pyridazin-3-on als rötliche Kristalle; ESI 382.
37.2 Eine Suspension von 381 mg (1.00 mmol) 2-[3-(5-Amino-[1,2,4]oxadiazol-3-yl)-benzyl]-6-(3,5-difluorphenyl)-2*H*-pyridazin-3-on in 5 ml Dichlormethan wird mit 1.14 ml (9.00 mmol) Trimethylchlorsilan versetzt. Zu dieser Suspension wird unter Rühren bei Raumtemperatur eine Lösung von 865 mg (3.00 mmol) Tetrabutylammoniumnitrit in 3 ml Dichlormethan langsam zugetropft. Nach 16 Stunden Rühren bei Raumtemperatur wird das Reaktionsgemisch filtriert. Das Filtrat wird zwischen verdünnter Natriumhydrogencarbonat-Lösung und Dichlormethan verteilt. Die organische Phase wird über Natriumsulfat getrocknet und eingedampft. Man erhält rohes 2-[3-(5-Chlor-[1,2,4]-oxadiazol-3-yl)-benzyl]-6-(3,5-difluorphenyl)-2*H*-pyridazin-3-on als gelbliches Öl, das ohne weitere Reinigung für die nächste Reaktion eingesetzt wird; ESI 401.
37.3 Eine Lösung von 260 mg (ca. 0.32 mmol) rohem 2-[3-(5-Chlor-[1,2,4]oxadiazol-3-yl)-benzyl]-6-(3,5-difluorphenyl)-2*H*-pyridazin-3-on in 1 ml THF wird mit 63.3 mg (0.49 mmol) Hydroxyethylpiperazin und 39 mg (0.40 mmol) Triethylamin versetzt und 2 Stunden bei Raumtemperatur gerührt. Das Reaktionsgemisch wird mit gesättigter Natriumchlorid-Lösung und Ethylacetat versetzt. Die wässrige Phase wird abgetrennt und die organische Phase zweimal mit Wasser gewaschen, über Natriumsulfat getrocknet und eingedampft. Der Rückstand wird durch präparative HPLC gereinigt. Die Produkt enthaltenden Fraktionen werden mit 0.3 ml 1 N HCl versetzt und lyophilisiert: 6-(3,5-Difluorphenyl)-2-(3-{5-[4-(2-hydroxyethyl)piperazin-1-yl]-[1,2,4]oxadiazol-3-yl}-benzyl)-2*H*-pyridazin-3-on Hydrochlorid als farblose Kristalle; ESI 496.

Analog werden die nachstehenden Verbindungen erhalten

| Nr. | Name und/oder Struktur | ESI |
|---|---|---|
| "A112" | 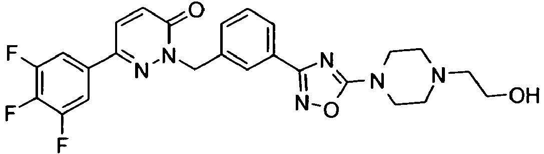 | |
| "A113" | 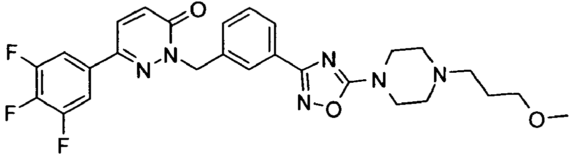 | |
| "A114" | 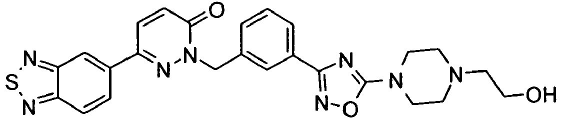 | |
| "A115" | 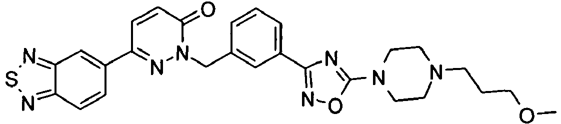 | |
| "A116" | 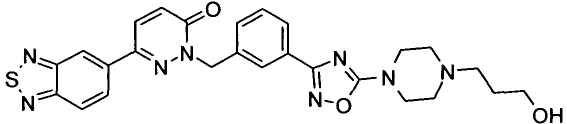 | |

### Beispiel 38

Die Herstellung von 4-(3-{3-[3-(3,5-Difluorphenyl)-6-oxo-6H-pyridazin-1-ylmethyl]-phenyl}-[1,2,4]oxadiazol-5-yl)-morpholin-3-on ("A117") erfolgt analog nachstehendem Schema: 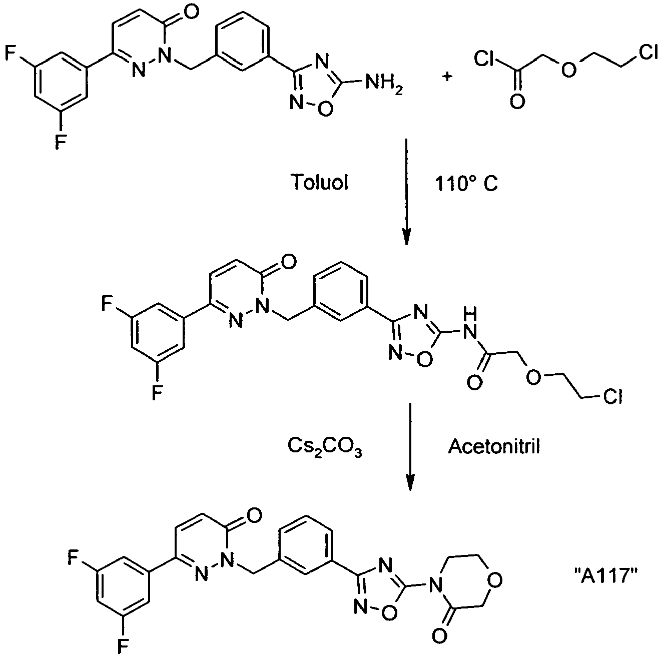
38.1 Eine Suspension von 381 mg (1.00 mmol) 2-[3-(5-Amino-[1,2,4]oxadiazol-3-yl)-benzyl]-6-(3,5-difluorphenyl)-2*H*-pyridazin-3-on in 2 ml Toluol wird mit 252 mg (1.60 mmol) (2-Chlorethoxy)-acetylchlorid versetzt und 40 Stunden bei einer Temperatur von 110° gerührt. Man lässt das Reaktionsgemisch abkühlen, verdünnt mit Petrolether und saugt ab. Der Rückstand wird aus Acetonitril umkristallisiert: 2-(2-Chlorethoxy)-N-(3-{3-[3-(3,5-difluorphenyl)-6-oxo-6*H*-pyridazin-1-ylmethyl]-phenyl}-[1,2,4]oxadiazol-5-yl)-acetamid als ockergelbe Kristalle; ESI 502.
38.2 Eine Suspension von 390 mg (0.70 mmol) 2-(2-Chlorethoxy)-N-(3-{3-[3-(3,5-difluorphenyl)-6-oxo-6*H*-pyridazin-1-ylmethyl]-phenyl}-[1,2,4]oxadiazol-5-yl)-acetamid (ca. 90%ig) und 228 mg (0.70 mmol) Caesiumcarbonat in 2 ml Acetonitril wird 18 Stunden bei Raumtemperatur gerühret. Das Reaktionsgemisch wird mit Wasser versetzt und der entstandene Niederschlag abgesaugt. Der Rückstand wird getrocknet und aus Acetonitril umkristallisiert: 4-(3-{3-[3-(3,5-Difluorphenyl)-6-oxo-6H-pyridazin-1-ylmethyl]-phenyl}-[1,2,4]oxadiazol-5-yl)-morpholin-3-on als farblose Kristalle; ESI 466.

### Beispiel 39

Die Herstellung von 6-(3-Chlorophenyl)-2-[3-(5-methyl-4,5-dihydro-thiazol-2-yl)-benzyl]-2H-pyridazin-3-on ("A118") erfolgt analog nachstehendem Schema:

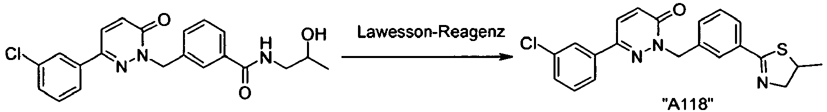

Eine Suspension von 203 mg (0.51 mmol) 3-[3-(3-Chlorphenyl)-6-oxo-6*H*-pyridazin-1-ylmethyl]-N-(2-hydroxy-propyl)-benzamid (Herstellung analog zu Beispiel 27) und 106 mg (0.261 mmol) 2,4-Bis-(4-methoxyphenyl)-2,4-dithioxo-1,3,2,4-dithiadiphosphetan (Lawesson-Reagenz) in 2 ml Toluol wird 18 Stunden zum Sieden erhitzt. Das Reaktionsgemisch wird zwischen gesättigter Natriumhydrogencarbonat-Lösung und Dichlormethan verteilt. Die organische Phase wird über Natriumsulfat getrocknet und eingedampft. Der Rückstand wird mittels präparativer HPLC gereinigt: 6-(3-Chlorophenyl)-2-[3-(5-methyl-4,5-dihydro-thiazol-2-yl)-benzyl]-2*H*-pyridazin-3-on als gelber Feststoff; ESI 396.

### Beispiel 40

Die Herstellung von 6-[4-(3-Dimethylamino-propoxy)-3,5-difluorphenyl]-2-[3-(5-methyl-[1,2,4]oxadiazol-3-yl)-benzyl]-2H-pyridazin-3-on ("A119") erfolgt analog nachstehendem Schema:

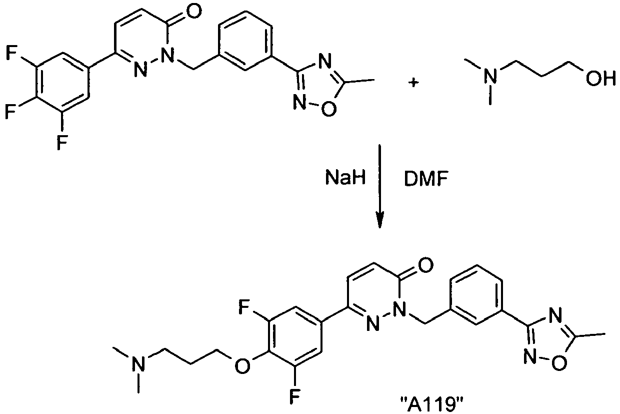

Eine Lösung von 200 mg (0.50 mmol) 2-[3-(5-Methyl-[1,2,4]oxadiazol-3-yl)-benzyl]-6-(3,4,5-trifluorphenyl)-2*H*-pyridazin-3-on in 10 ml DMF wird unter Argon mit 40.3 mg (1.01 mmol) Natriumhydrid (60%ig in Paraffinöl) versetzt und 10 Minuten bei Raumtemperatur gerührt. Dann werden 118 µl (1.01 mmol) 3-(Dimethylamino)-1-propanol zugegeben und die Mischung drei Stunden bei Raumtemperatur gerührt. Das Reaktionsgemisch wird auf Wasser gegeben und mit Ethylacetat extrahiert. Die organische Phase wird über Natriumsulfat getrocknet, eingedampft und mit präparativer HPLC gereinigt: 6-[4-(3-Dimethylamino-propoxy)-3,5-difluorphenyl]-2-[3-(5-methyl-[1,2,4]oxadiazol-3-yl)-benzyl]-2*H*-pyridazin-3-on Trifluoracetat als farbloses Glas; ESI 482.

Analog werden die nachstehenden Verbindungen erhalten

| Nr. | Name und/oder Struktur | ESI |
|---|---|---|
| "A120" | 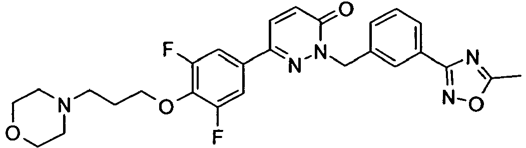 | |
| "A121" | 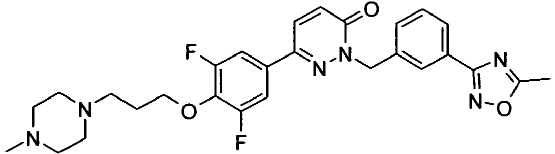 | |
| "A122" | 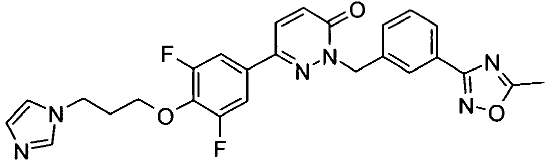 | |

### Pharmakologische Daten

Met-Kinase-Inhibierung

**Tabelle 1**

| Verbindung Nr. | IC₅₀ (Enzym Assay) | IC₅₀ (Zell Assay) |
|---|---|---|
| "A1" | A | |
| "A11" | A | |
| "A12" | A | |
| "A13" | A | |
| "A20" | A | |
| "A21 | A | |
| "A22" | A | |
| "A33" | A | |
| "A46" | A | |
| "A48" | A | |
| "A50" | A | |
| "A88" | A | |
| "A91" | A | |
| "A111" | | A |
| "A123" | | A |
| "A124" | | A |

| | |
|---|---|
| IC₅₀: | 10 nM - 1 µM = A |
| | 1 µM - 10 µM = B |
| | > 10 mM = C |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 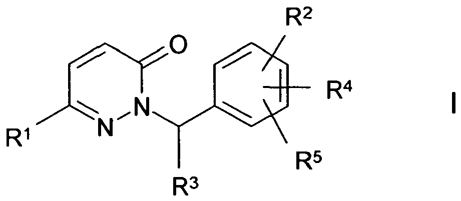 worin
R¹ Ar oder Het,
R² einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, NO₂, CN, COOR³, CON(R³)₂, NR³COA, NR³SO₂A, SO₂N(R³)_{2,} S(O)ₘA, Het¹, -[C(R³)₂]ₙN(R³)₂, -[C(R³)₂]ₙHet¹, O[C(R³)₂]ₙN(R³)₂, O[C(R³)₂]ₙHet, S[C(R³)₂]ₙN(R³)₂, S[C(R³)₂]ₙHet¹, -NR³[C(R³)₂]ₙN(R³)₂, -NR³[C(R³)₂]ₙHet¹, -NR³Het¹, NHCON(R³)₂, NHCONH[C(R³)₂]ₙN(R³)₂, NHCONH[C(R³)₂]ₙHet¹, CON(R³)₂, ONR³[C(R³)₂]ₙN(R³)₂, CONR³[C(R³)₂]ₙHet¹, COHet¹, COA und/oder =O (Carbonylsauerstoff) substituiert sein kann,
R³ H oder A,
R⁴, R⁵ jeweils unabhängig voneinander H, Hal, A, OR³, CN, COOR³, CON(R³)₂, NR³COA, NR³SO₂A, SO₂N(R³)₂ oder S(O)ₘA,
A unverzweigtes oder verzweigtes Alkyl mit 1-10 C-Atomen,
worin 1-7 H-Atome durch F, Cl und/oder Br ersetzt sein können,
und/oder worin eine oder zwei CH₂-Gruppen durch O, S, SO, SO₂ und/oder CH=CH-Gruppen ersetzt sein können,
oder
cyclisches Alkyl mit 3-7 C-Atomen,
Ar unsubstituiertes oder ein-, zwei- oder dreifach durch Hal, A, OR³, N(R³)₂, SR³, NO₂, CN, COOR³, CON(R³)₂, NR³COA, NR³SO₂A, SO₂N(R³)_{2,} S(O)ₘA, CO-Het¹, Het¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙ₋Het¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹ und/oder COA substituiertes Phenyl, Naphthyl oder Biphenyl,
Het einen ein-, zwei- oder dreikernigen gesättigten, ungesättigten oder aromatischen Heterocyclus mit 1 bis 4 N-, O- und/oder S-Atomen, der unsubstituiert oder ein-, zwei- oder dreifach durch Hal, A, OR³, N(R³)₂, SR³, NO₂ CN, COOR³, CON(R³)₂, NR³COA, NR³SO₂A, SO₂N(R³)₂, S(O)ₘA, CO-Het¹, Het¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹, CO-Het¹, CHO, COA, =S, =NH, =NA und/oder =O (Carbonylsauerstoff) substituiert sein kann,
Het¹ einen einkernigen gesättigten oder aromatischen Heterocyclus mit 1 bis 2 N und/oder O-Atomen, der ein- oder zweifach durch A, OA, OH, Hal, (CH₂)ₙN(R³)₂, (CH₂)ₙOR³, (CH₂)ₙHet² und/oder =O (Carbonylsauerstoff) substituiert sein kann,
Het² Pyrrolidino, Piperidino oder Morpholino,
Hal F, Cl, Br oder I,
m 0, 1 oder 2,
n 1, 2, 3 oder 4
bedeuten,
sowie ihre pharmazeutisch verwendbaren Solvate, Salze, Tautomere und Stereoisomere, einschließlich deren Mischungen in allen Verhältnissen,

2. Verbindungen nach Anspruch 1, worin
A unverzweigtes oder verzweigtes Alkyl mit 1-10 C-Atomen,
worin 1-7 H-Atome durch F und/oder Cl ersetzt sein können,
bedeutet,
sowie ihre pharmazeutisch verwendbaren Solvate, Salze, Tautomere und Stereoisomere, einschließlich deren Mischungen in allen Verhältnissen.

3. Verbindungen nach Anspruch 1 oder 2, worin
Ar unsubstituiertes oder ein-, zwei- oder dreifach durch Hal, CN und/oder S(O)ₘA substituiertes Phenyl,
bedeutet,
sowie ihre pharmazeutisch verwendbaren Solvate, Salze, Tautomere und Stereoisomere, einschließlich deren Mischungen in allen Verhältnissen.

4. Verbindungen nach einem oder mehreren der Ansprüche 1-3, worin
Het einen ein- oder zweikernigen aromatischen Heterocyclus mit 1 bis 3 N-, O- und/oder S-Atomen, der unsubstituiert oder ein-, zwei- oder dreifach durch Hal substituiert sein kann,
bedeutet,
sowie ihre pharmazeutisch verwendbaren Solvate, Salze, Tautomere und Stereoisomere, einschließlich deren Mischungen in allen Verhältnissen.

5. Verbindungen nach einem oder mehreren der Ansprüche 1-4, worin
Het Thiazolyl, Thienyl, Pyridyl, Benzo[1,2,5]thiadiazolyl oder Benzo[1,3]dioxolyl
bedeutet,
sowie ihre pharmazeutisch verwendbaren Solvate, Salze, Tautomere und Stereoisomere, einschließlich deren Mischungen in allen Verhältnissen.

6. Verbindungen nach einem oder mehreren der Ansprüche 1-5, worin
R² einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann,
bedeutet,
sowie ihre pharmazeutisch verwendbaren Solvate, Salze, Tautomere und Stereoisomere, einschließlich deren Mischungen in allen Verhältnissen.

7. Verbindungen nach einem oder mehreren der Ansprüche 1-6, worin
R² einen Heterocyclus ausgewählt aus der Gruppe [1,2,4]Oxadiazolyl, [1,3,4]Oxadiazolyl, Oxazolyl, Dihydro-oxazolyl, Pyrazolyl, Imidazolyl, Triazolyl, Tetrazolyl, Pyrrolyl, Furanyl, Thienyl, Thiazolyl, Dihydrothiazolyl, Thiadiazolyl, Oxazolidinyl, Pyrrolidinyl, Piperidinyl,
der ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)_{2,} -NR³[C(R³)₂]ₙHet¹, -NR³Het¹, COHet¹ und/oder =O (Carbonylsauerstoff) substituiert sein kann,
bedeutet,
sowie ihre pharmazeutisch verwendbaren Solvate, Salze, Tautomere und Stereoisomere, einschließlich deren Mischungen in allen Verhältnissen.

8. Verbindungen nach einem oder mehreren der Ansprüche 1-7, worin
R⁴, R⁵ H bedeuten,
sowie ihre pharmazeutisch verwendbaren Solvate, Salze, Tautomere und Stereoisomere, einschließlich deren Mischungen in allen Verhältnissen.

9. Verbindungen nach einem oder mehreren der Ansprüche 1-8, worin
R³ H, Methyl, Ethyl, Propyl, Isopropyl, Butyl oder tert.-Butyl,
bedeutet,
sowie ihre pharmazeutisch verwendbaren Solvate, Salze, Tautomere und Stereoisomere, einschließlich deren Mischungen in allen Verhältnissen.

10. Verbindungen nach einem oder mehreren der Ansprüche 1-9, worin
R¹ 4-Fluorphenyl, 3,5-Difluorphenyl, 3,4-Difluorphenyl, 3,4,5-Trifluorphenyl, 2-Chlorphenyl, 3-Chlorphenyl, 3-Cyanphenyl, 4-Cyanphenyl, 4-Methylsulfonylphenyl, Pyridyl, Benzo[1,3]dioxolyl oder Benzo[1,2,5]thiadiazolyl
bedeutet,
sowie ihre pharmazeutisch verwendbaren Solvate, Salze, Tautomere und Stereoisomere, einschließlich deren Mischungen in allen Verhältnissen.

11. Verbindungen nach einem oder mehreren der Ansprüche 1-10, worin
Het¹ einen einkernigen gesättigten oder aromatischen Heterocyclus mit 1 bis 2 N und/oder O-Atomen, der ein- oder zweifach durch A, (CH₂)ₙHet², (CH₂)ₙN(R³)₂ und/oder (CH₂)ₙOR³ substituiert sein kann,
bedeutet,
sowie ihre pharmazeutisch verwendbaren Solvate, Salze, Tautomere und Stereoisomere, einschließlich deren Mischungen in allen Verhältnissen.

12. Verbindungen nach einem oder mehreren der Ansprüche 1-11, worin
Het¹ Piperidin-1-yl, Pyrrolidin-1-yl, Morpholin-4-yl, Piperazin-1-yl, 1,3-Oxazolidin-3-yl, Imidazolidinyl, Oxazolyl, Thiazolyl, Thienyl, [1,4]Diazepanyl, Furanyl oder Pyridyl, wobei die Reste auch ein- oder zweifach durch A, (CH₂)ₙHet², (CH₂)ₙN(R³)₂ und/oder (CH₂)ₙOR³ substituiert sein können,
bedeutet,
sowie ihre pharmazeutisch verwendbaren Solvate, Salze, Tautomere und Stereoisomere, einschließlich deren Mischungen in allen Verhältnissen.

13. Verbindungen nach einem oder mehreren der Ansprüche 1-12, worin
R¹ Ar oder Het,
R² einen gesättigten, ungesättigten oder aromatischen 5-gliedrigen Heterocyclus mit 1 bis 4 N-, O- und/oder S-Atomen, der unsubstituiert oder ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ und/oder =O (Carbonylsauerstoff) substituiert sein kann,
R³ H oder A,
R⁴, R⁵ H,
A unverzweigtes oder verzweigtes Alkyl mit 1-10 C-Atomen,
worin 1-7 H-Atome durch F und/oder Cl ersetzt sein können,
Ar unsubstituiertes oder ein-, zwei- oder dreifach durch Hal, CN und/oder S(O)ₘA substituiertes Phenyl,
Het einen ein- oder zweikernigen aromatischen Heterocyclus mit 1 bis 3 N-, O- und/oder S-Atomen, der unsubstituiert oder ein-, zwei- oder dreifach durch Hal substituiert sein kann,
Het¹ einen einkernigen gesättigten oder aromatischen Heterocyclus mit 1 bis 2 N und/oder O-Atomen, der ein- oder zweifach durch A, (CH₂)ₙHet², (CH₂)ₙN(R³)₂ und/oder (CH₂)ₙOR³ substituiert sein kann,
Het² Pyrrolidino, Piperidino oder Morpholino,
Hal F, Cl, Br oder I,
m 0, 1 oder 2,
n 1, 2, 3 oder 4,
bedeuten,
sowie ihre pharmazeutisch verwendbaren Solvate, Salze, Tautomere und Stereoisomere, einschließlich deren Mischungen in allen Verhältnissen.

14. Verbindungen nach einem oder mehreren der Ansprüche 1-13, worin
R¹ Ar oder Het,
R² einen Heterocyclus ausgewählt aus der Gruppe [1,2,4]Oxadiazolyl, [1,3,4]Oxadiazolyl, Oxazolyl, Dihydro-oxazolyl, Pyrazolyl, Imidazolyl, Triazolyl, Thiadiazolyl, Tetrazolyl, Pyrrolyl, Furanyl, Thienyl, Thiazolyl, Dihydrothiazolyl, Oxazolidinyl, der ein- oder zweifach durch Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -COHet¹ und/oder =O (Carbonylsauerstoff) substituiert sein kann,
R³ H, Methyl, Ethyl, Propyl, Isopropyl, Butyl oder tert.-Butyl,
R⁴, R⁵ H,
A unverzweigtes oder verzweigtes Alkyl mit 1-10 C-Atomen,
worin 1-7 H-Atome durch F und/oder Cl ersetzt sein können,
Ar unsubstituiertes oder ein-, zwei- oder dreifach durch Hal, CN und/oder S(O)ₘA substituiertes Phenyl,
Het Thiazolyl, Thienyl, Pyridyl, Benzo[1,2,5]thiadiazolyl oder Benzo[1,3]dioxolyl,
Het¹ Piperidin-1-yl, Pyrrolidin-1-yl, Morpholin-4-yl, Piperazin-1-yl, 1,3-Oxazolidin-3-yl, Imidazolidinyl, Oxazolyl, Thiazolyl, Thienyl, [1,4]Diazepanyl, Furanyl oder Pyridyl, wobei die Reste auch ein- oder zweifach durch A, (CH₂)ₙHet², (CH₂)ₙN(R³)₂ und/oder (CH₂)ₙOR³ substituiert sein können,
Het² Pyrrolidino, Piperidino oder Morpholino,
Hal F, Cl, Br oder I,
m 0, 1 oder 2,
n 1, 2, 3 oder 4,
bedeuten, sowie ihre pharmazeutisch verwendbaren Solvate, Salze, Tautomere und Stereoisomere, einschließlich deren Mischungen in allen Verhältnissen.

15. Verbindungen nach Anspruch 1, ausgewählt aus der Gruppe
| Nr. | Struktur und/oder Name |
|---|---|
| "A1" | 6-(3,5-Difluorphenyl)-2-[3-(5-methyl-[1,2,4]oxadiazol-3-yl)-benzyl]-2*H*-pyridazin-3-on |
| "A2" | 2-[3-(5-Methyl-[1,2,4]oxadiazol-3-yl)-benzyl]-6-(3,4,5-trifluorphenyl)-2*H*-pyridazin-3-on |
| "A3" | 6-(3,5-Difluor-phenyl)-2-[4-(5-methyl-[1,2,4]oxadiazol-3-yl)-benzyl]-2*H*-pyridazin-3-on |
| "A4" | 6-(4-Fluor-phenyl)-2-[3-(5-methyl-[1,2,4]oxadiazol-3-yl)-benzyl]-2*H*-pyridazin-3-on |
| "A5" | 2-[3-(5-Methyl-[1,2,4]oxadiazol-3-yl)-benzyl]-6-pyridin-3-yl-2*H*-pyridazin-3-on |
| | 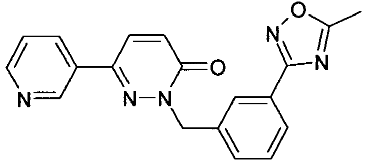 |
| "A6" | 6-(3-Chlor-phenyl)-2-[3-(5-methyl-[1,2,4]oxadiazol-3-yl)-benzyl]-2*H*-pyridazin-3-on |
| "A7" | 6-(3-Cyan-phenyl)-2-[3-(5-methyl-[1,2,4]oxadiazol-3-yl)-benzyl]-2*H*-pyridazin-3-on |
| "A8" | 6-(4-Cyan-phenyl)-2-[3-(5-methyl-[1,2,4]oxadiazol-3-yl)-benzyl]-2*H*-pyridazin-3-on |
| "A9" | 6-(4-Methylsulfonyl-phenyl)-2-[3-(5-methyl-[1,2,4]oxadiazol-3-yl)-benzyl]-2*H*-pyridazin-3-on |
| "A10" | 6-(3,5-Difluorphenyl)-2-[3-(5-dimethylaminomethyl-[1,2,4]oxadiazol-3-yl)-benzyl]-2*H*-pyridazin-3-on |
| "A11" | 6-(3,5-Difluorphenyl)-2-[3-(5-ethylamino-[1,2,4]oxadiazol-3-yl)-benzyl]-2*H*-pyridazin-3-on |
| "A12" | 6-(3,5-Difluorphenyl)-2-{3-[5-(3-dimethylamino-propylamino)-[1,2,4]oxadiazol-3-yl]-benzyl}-2*H*-pyridazin-3-on |
| "A13" | 6-(3,5-Difluor-phenyl)-2-[3-(5-methyl-oxazol-2-yl)-benzyl]-2*H*-pyridazin-3-on |
| "A14" | 6-(3,5-Difluor-phenyl)-2-[3-(3-methyl-[1,2,4]oxadiazol-5-yl)-benzyl]-2*H*-pyridazin-3-on |
| "A15" | 6-(3,5-Difluorphenyl)-2-[3-(5-oxo-4,5-dihydro-[1,3,4]oxadiazol-2-yl)-benzyl]-2*H*-pyridazin-3-on |
| "A16" | 6-(3,5-Difluorphenyl)-2-{3-[5-(2-morpholin-4-yl-ethylamino)-[1,3,4]oxadiazol-2-yl]-benzyl}-2*H*-pyridazin-3-on |
| "A 17" | 6-(3,5-Difluorphenyl)-2-[3-(5-oxo-4,5-dihydro-[1,2,4]oxadiazol-3-yl)-benzyl]-2*H*-pyridazin-3-on |
| "A18" | 6-(3,5-Difluorphenyl)-2-[3-(5-thioxo-4,5-dihydro-[1,2,4]oxadiazol-3-yl)-benzyl]-2*H*-pyridazin-3-on |
| "A19" | 6-(3,5-Difluorphenyl)-2-{3-[5-(3-dimethylamino-propylsulfanyl)-[1,2,4]oxadiazol-3-yl]-benzyl}-2*H*-pyridazin-3-on |
| "A20" | 6-(3,5-Difluorphenyl)-2-{3-[5-(4-methyl-piperazin-1-yl)-[1,2,4]oxadiazol-3-yl]-benzyl}-2*H*-pyridazin-3-on |
| "A21" | 6-(3,5-Difluorphenyl)-2-{3-[5-(4-methylpiperazin-1-carbonyl)-[1,2,4]oxadiazol-3-yl]-benzyl}-2*H-*pyridazin-3-on |
| "A22" | 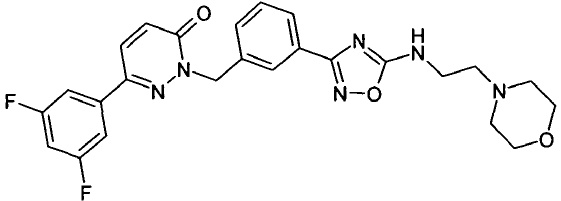 |
| "A23" | 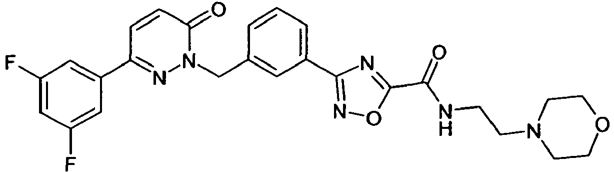 |
| "A24" | 6-(3,5-Difluor-phenyl)-2-[3-(5-piperazin-1-yl-[1,2,4]oxadiazol-3-yl)-benzyl]-2*H*-pyridazin-3-on |
| "A25" | 6-(3,5-Difluorphenyl)-2-{3-[4-(3-dimethylamino-propyl)-5-oxo-4,5-dihydro-[1,2,4]oxadiazol-3-yl]-benzyl}-2*H*-pyridazin-3-on |
| "A26" | 6-(3,5-Difluorphenyl)-2-[3-(1*H*-tetrazol-5-yl)-benzyl]-2H-pyridazin-3-on |
| "A27" | 6-(3,5-Difluor-phenyl)-2-{3-[5-(2-dimethylamino-ethylamino)-[1,2,4]oxadiazol-3-yl]-benzyl}-2*H*-pyridazin-3-on |
| "A28" | 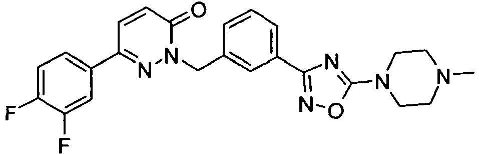 |
| "A29" | 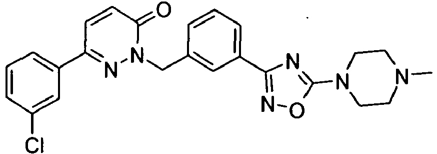 |
| "A30" | 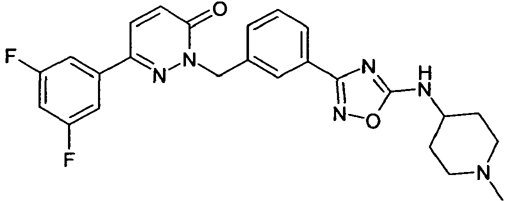 |
| "A31" | 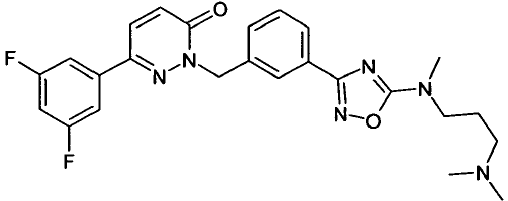 |
| "A32" | 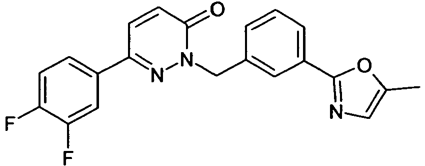 |
| "A33" | 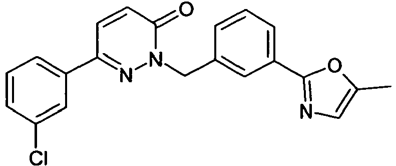 |
| "A34" | 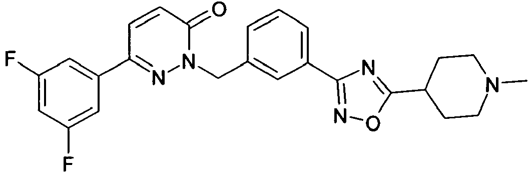 |
| "A35" | 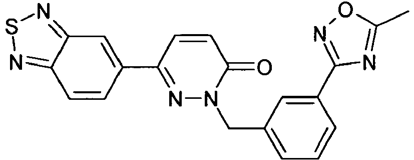 |
| "A36" | 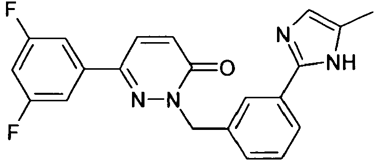 |
| "A37" | 6-(3,5-Difluorphenyl)-2-{3-[5-(4-methyl-piperazin-1-yl)-oxazol-2-yl]-benzyl}-2*H*-pyridazin-3-on |
| "A38" | 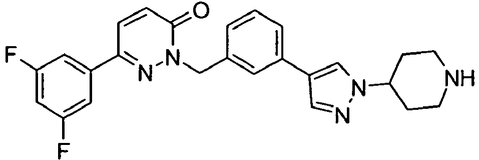 |
| "A39" | 6-(3,4-Difluorphenyl)-2-[3-(5-methyl-[1,2,4]oxadiazol-3-yl)-benzyl]-2*H*-pyridazin-3-on |
| | |
| "A41" | 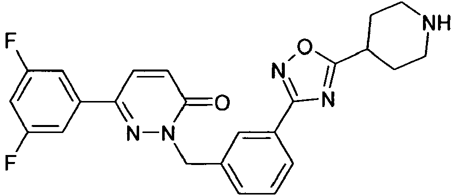 |
| "A42" | 6-(3,5-Difluor-phenyl)-2-{3-[5-(piperidin-4-ylamino)-[1,2,4]oxadiazol-3-yl]-benzyl}-2*H*-pyridazin-3-on |
| | 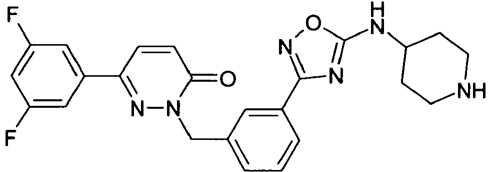 |
| "A43" | 2-{3-[5-(1-Methyl-piperidin-4-ylamino)-[1,2,4]oxadiazol-3-yl]-benzyl}-6-(3,4,5-trifluorphenyl)-2*H*-pyridazin-3-on |
| | 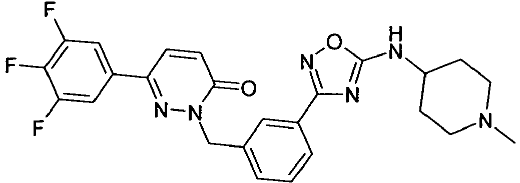 |
| "A44" | 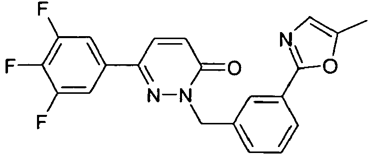 |
| "A45" | 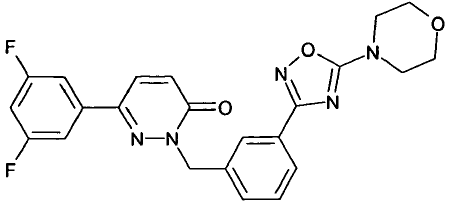 |
| "A46" | 6-(3,5-Difluor-phenyl)-2-(3-{5-[methyl-(1-methyl-piperidin-4-yl)-amino]-[1,2,4]oxadiazol-3-yl}-benzyl)-2*H*-pyridazin-3-on |
| | 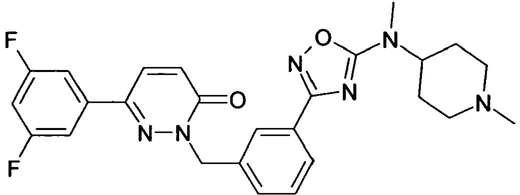 |
| "A47" | 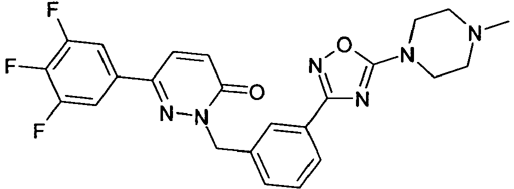 |
| "A48" | 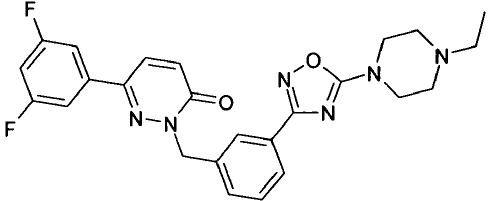 |
| "A49" | 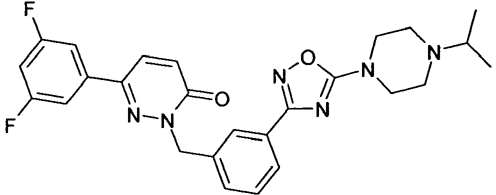 |
| "A50" | 6-(3,5-Difluor-phenyl)-2-(3-{5-[4-(2-methoxy-ethyl)-piperazin-1-yl]-[1,2,4]oxadiazol-3-yl}-benzyl)-2*H*-pyridazin-3-on |
| | 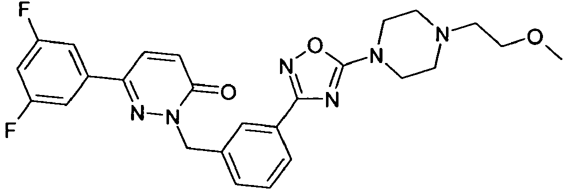 |
| "A51" | 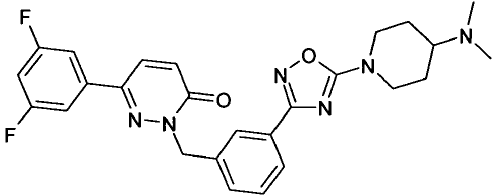 |
| "A52" | 6-(3,5-Difluor-phenyl)-2-(3-{5-[4-(2-dimethylamino-ethyl)-piperazin-1-yl]-[1,2,4]oxadiazol-3-yl}-benzyl)-2*H*-pyridazin-3-on |
| | 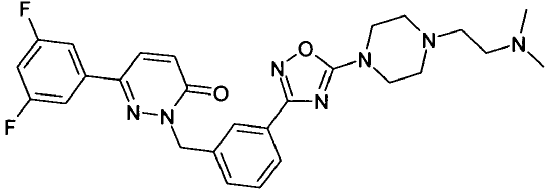 |
| "A53" | 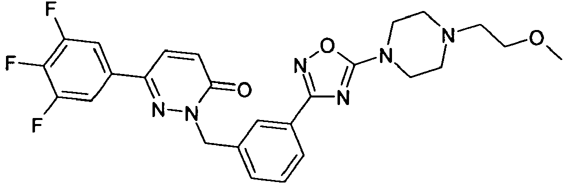 |
| "A54" | 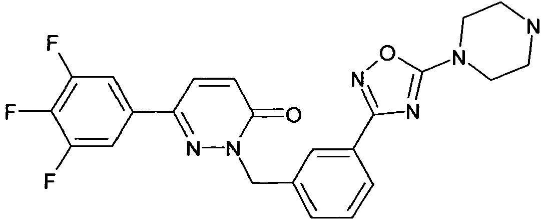 |
| "A55" | 6-(3,5-Difluor-phenyl)-2-[3-(5-oxo-4-piperidin-4-yl-4,5-dihydro-[1,2,4]oxadiazol-3-yl)-benzyl]-2*H*-pyridazin-3-on |
| | 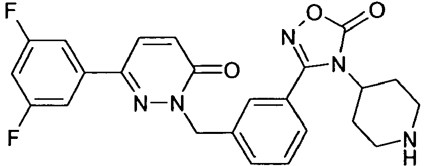 |
| "A56" | 6-(3,5-Difluor-phenyl)-2-[3-(5-hydroxymethyl-1H-imidazol-2-yl)-benzyl]-2*H*-pyridazin-3-on |
| | 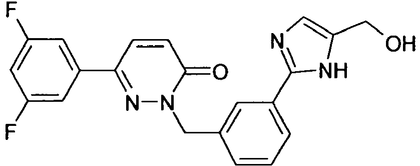 |
| "A57" | 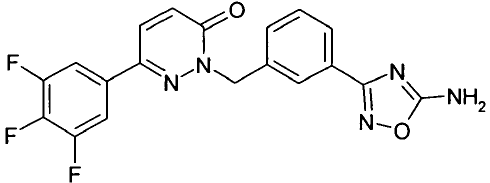 |
| "A58" | 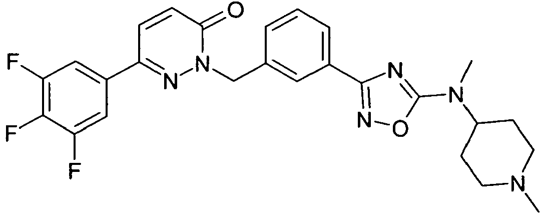 |
| "A59" | 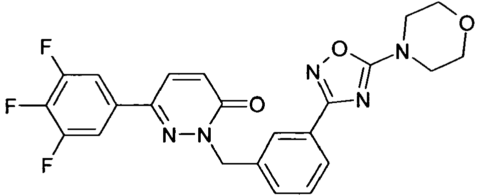 |
| "A60" | 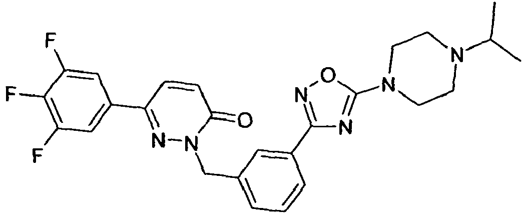 |
| "A61" | 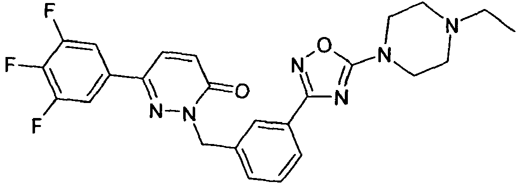 |
| "A62" | 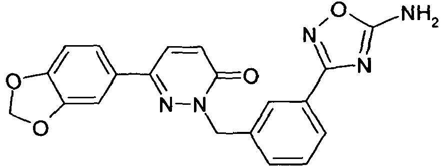 |
| "A63" | 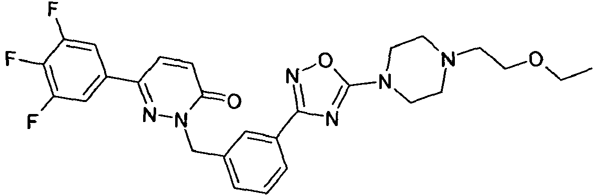 |
| "A64" | 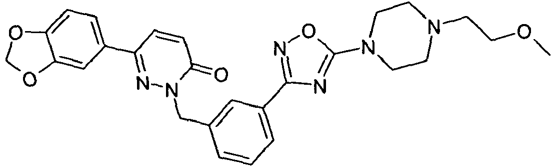 |
| "A65" | 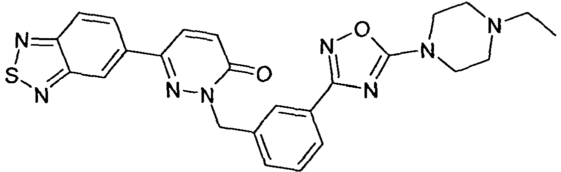 |
| "A66" | 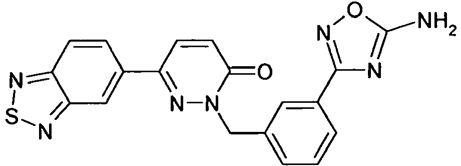 |
| "A67" | 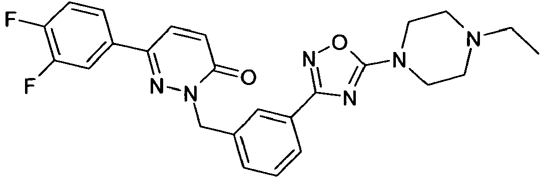 |
| "A68" | 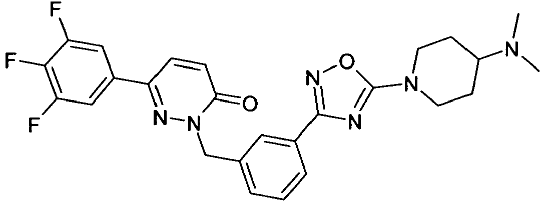 |
| "A69" | 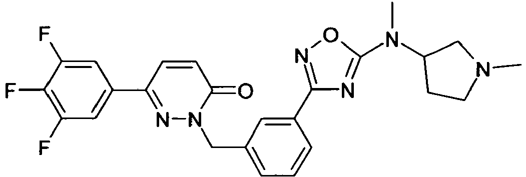 |
| "A70" | 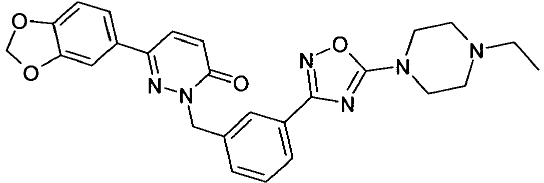 |
| "A71a" | 2-(3-{5-[(1-Ethyl-piperidin-4-yl)-methyl-amino]-[1,2,4]oxadiazol-3-yl}-benzyl)-6-(3,4,5-trifluor-phenyl)-2*H-*pyridazin-3-on |
| | 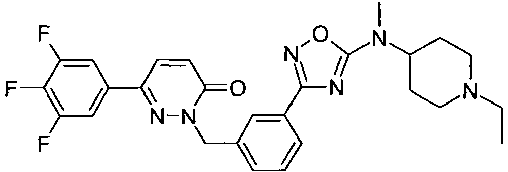 |
| "A71b" | 6-(3,5-Difluor-phenyl)-2-(3-{5-[(1-ethyl-piperidin-4-yl)-methyl-amino]-[1,2,4]oxadiazol-3-yl}-benzyl)-2*H*-pyridazin-3-on |
| | 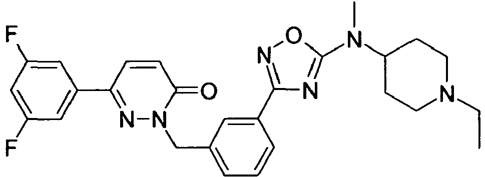 |
| "A71c" | 6-(3,5-Difluor-phenyl)-2-(3-{5-[methyl-(1-methyl-piperidin-4-ylmethyl)-amino]-[1,2,4]oxadiazol-3-yl}-benzyl)-2*H*-pyridazin-3-on |
| | 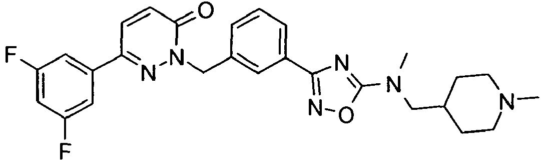 |
| "A72" | 2-[3-(5-Amino-[1,2,4]oxadiazol-3-yl)-benzyl]-6-(3,5-difluor-phenyl)-2*H*-pyridazin-3-on |
| | 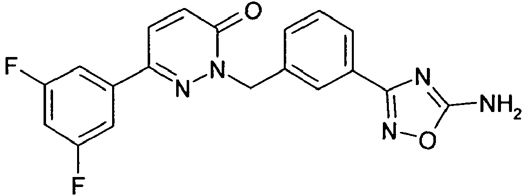 |
| "A73" | 6-(3,5-Difluor-phenyl)-2-{3-[5-(methyl-piperidin-4-yl-amino)-[1,2,4]oxadiazol-3-yl]-benzyl}-2*H*-pyridazin-3-on |
| | 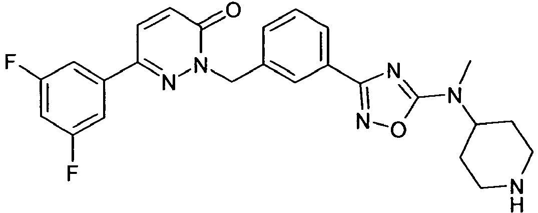 |
| "A74" | 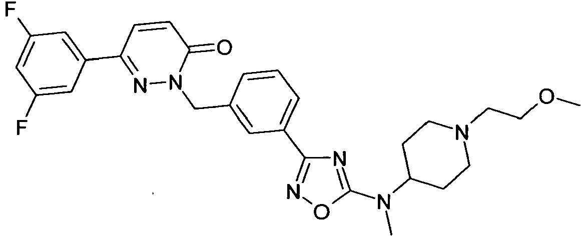 |
| "A75" | 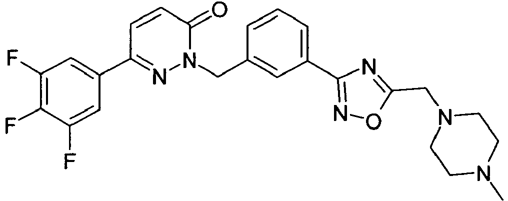 |
| "A76" | 2-[3-(5-Trifluormethyl-[1,2,4]oxadiazol-3-yl)-benzyl]-6-(3,4,5-trifluor-phenyl)-2*H*-pyridazin-3-on |
| "A77" | 2-(3-[1,2,4]Oxadiazol-3-yl-benzyl)-6-(3,4,5-trifluor-phenyl)-2*H*-pyridazin-3-on |
| "A78" | 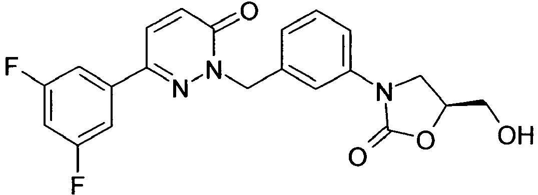 |
| "A79" | 6-(3,5-Difluor-phenyl)-2-[3-((R)-5-hydroxymethyl-2-oxo-oxazolidin-3-yl)-benzyl]-2*H*-pyridazin-3-on |
| "A80" | 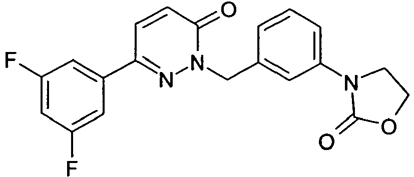 |
| "A81" | 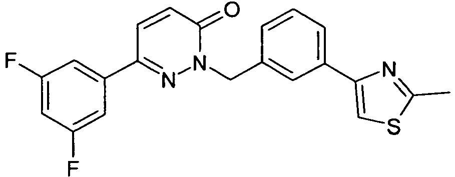 |
| "A82" | 6-(3-Cyanphenyl)-2-[3-(2-methyl-thiazol-4-yl)-benzyl]-2*H-*pyridazin-3-on |
| "A83" | 6-(3,5-Difluor-phenyl)-2-[3-(1*H*-imidazol-2-yl)-benzyl]-2*H-*pyridazin-3-on |
| "A84" | 6-(3,5-Difluor-phenyl)-2-[3-(5-methyl-thiazol-2-yl)-benzyl]-2*H*-pyridazin-3-on |
| | 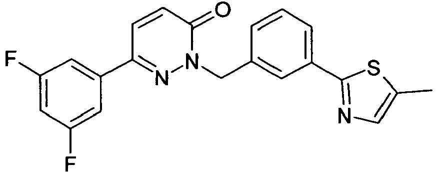 |
| "A85" | 6-(3,5-Difluor-phenyl)-2-[3-(5-methyl-4,5-dihydro-oxazol-2-yl)-benzyl]-2*H*-pyridazin-3-on |
| | 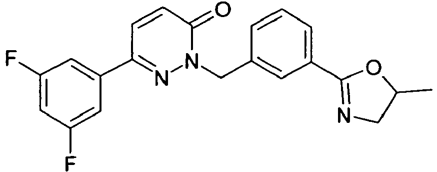 |
| "A86" | 6-(3-Chlor-phenyl)-2-[3-(5-methyl-thiazol-2-yl)-benzyl]-2*H-*pyridazin-3-on |
| "A87" | 6-(3,5-Difluor-phenyl)-2-[3-(5-piperidin-4-yl-oxazol-2-yl)-benzyl]-2*H*-pyridazin-3-on |
| "A88" | 6-(3,5-Difluor-phenyl)-2-{3-[5-(1-methyl-piperidin-4-yl)-oxazol-2-yl]-benzyl}-2*H*-pyridazin-3-on |
| "A89" | 2-[3-(5-Piperidin-4-yl-oxazol-2-yl)-benzyl]-6-(3,4,5-trifluor-phenyl)-2*H*-pyridazin-3-on |
| | 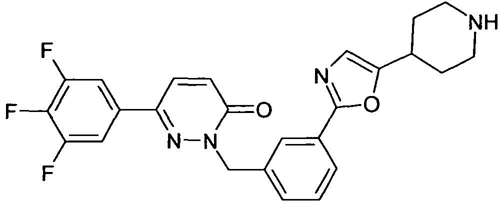 |
| "A90" | 2-[3-(5-Piperidin-4-yl-oxazol-2-yl)-benzyl]-6-(3-chlor-phenyl)-2*H*-pyridazin-3-on |
| | 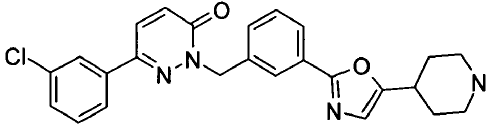 |
| "A91" | 6-(3,5-Difluor-phenyl)-2-[3-(5-piperidin-4-yl-thiazol-2-yl)-benzyl]-2*H*-pyridazin-3-on |
| | 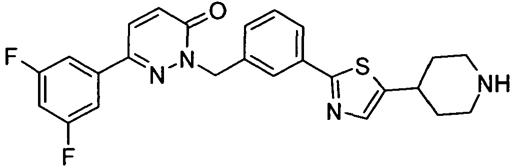 |
| "A92" | 6-(3,5-Difluor-phenyl)-2-{3-[5-(1-methyl-piperidin-4-yl)-thiazol-2-yl]-benzyl}-2*H*-pyridazin-3-on |
| | 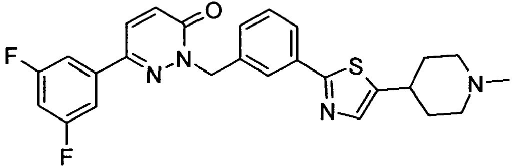 |
| "A93" | 2-[3-(5-Piperidin-4-yl-thiazol-2-yl)-benzyl]-6-(3,4,5-trifluor-phenyl)-2*H*-pyridazin-3-on |
| "A94" | 6-(3-Chlor-phenyl)-2-[3-(5-piperidin-4-yl-thiazol-2-yl)-benzyl]-2H-pyridazin-3-on |
| "A95" | 6-(3-Chlor-phenyl)-2-{3-[5-(4-methyl-piperazin-1-ylmethyl)-thiazol-2-yl]-benzyl}-2*H*-pyridazin-3-on |
| "A96" | 6-(3,5-Difluorphenyl)-2-[3-(5-piperidin-3-yl-thiazol-2-yl)-benzyl]-2*H*-pyridazin-3-on |
| "A97" | 2-[3-(5-Azetidin-3-yl-thiazol-2-yl)-benzyl]-6-(3,5-difluor-phenyl)-2H-pyridazin-3-on |
| | 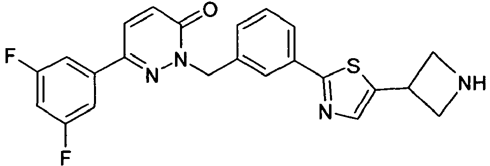 |
| "A98" | 6-(3,5-Difluorphenyl)-2-[3-(5-piperidin-4-ylmethyl-thiazol-2-yl)-benzyl]-2*H*-pyridazin-3-on |
| "A99" | 6-(3,5-Difluor-phenyl)-2-(3-{5-[1-(2-methoxy-ethyl)-piperidin-4-yl]-thiazol-2-yl}-benzyl)-2*H*-pyridazin-3-on |
| | 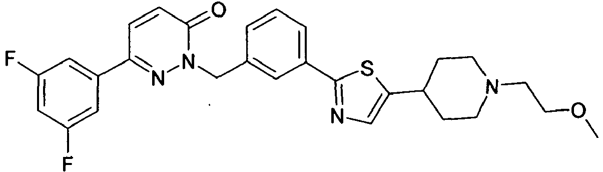 |
| "A99a" | 6-(3,5-Difluor-phenyl)-2-{3-[5-(1-methyl-piperidin-4-ylmethyl)-thiazol-2-yl]-benzyl}-2*H*-pyridazin-3-on |
| "A100" | 6-(3,5-Difluorphenyl)-2-[3-(5-pyrrolidin-2-yl-thiazol-2-yl)-benzyl]-2*H*-pyridazin-3-on |
| "A101" | 6-(3,5-Difluor-phenyl)-2-{3-[5-(4-methyl-piperazin-1-ylmethyl)-thiazol-2-yl]-benzyl}-2*H*-pyridazin-3-on |
| "A102" | 6-(3,5-Difluor-phenyl)-2-[3-(5-morpholin-4-ylmethyl-thiazol-2-yl)-benzyl]-2*H*-pyridazin-3-on |
| "A103" | 6-(3,5-Difluor-phenyl)-2-[3-(4-methyl-thiazol-2-yl)-benzyl]-2*H-*pyridazin-3-on |
| "A104" | 6-(3,5-Difluorphenyl)-2-(3-thiazol-2-yl-benzyl)-2*H*-pyridazin-3-on |
| "A105" | 6-(3,5-Difluor-phenyl)-2-{3-[1-(2-piperidin-1-yl-ethyl)-1*H-*[1,2,3]triazol-4-yl]-benzyl}-2*H*-pyridazin-3-on |
| | 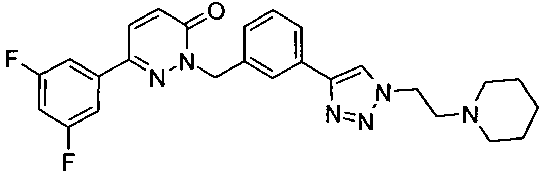 |
| "A106" | 6-(3,5-Difluor-phenyl)-2-{3-[1-(2-morpholin-4-yl-ethyl)-1*H-*[1,2,3]triazol-4-yl]-benzyl}-2*H*-pyridazin-3-on |
| "A107" | 6-(3,5-Difluor-phenyl)-2-{3-[5-(4-methyl-piperazin-1-carbonyl)-isoxazol-3-yl]-benzyl}-2*H*-pyridazin-3-on |
| | 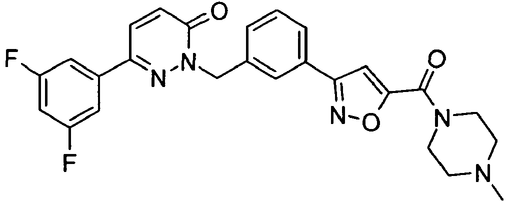 |
| "A108" | 6-(3,5-Difluor-phenyl)-2-[3-(5-oxo-4,5-dihydro-[1,2,4]thiadiazol-3-yl)-benzyl]-2*H*-pyridazin-3-on |
| | 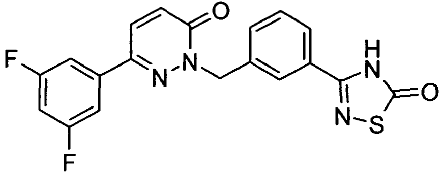 |
| "A109" | 6-(3,5-Difluor-phenyl)-2-{3-[5-(4-methyl-piperazin-1-yl)-[1,2,4]thiadiazol-3-yl]-benzyl}-2*H*-pyridazin-3-on |
| "A109a" | 6-(3,5-Difluor-phenyl)-2-(3-{5-[4-(2-methoxy-ethyl)-piperazin-1-yl]-[1,2,4]thiadiazol-3-yl}-benzyl)-2*H*-pyridazin-3-on |
| | 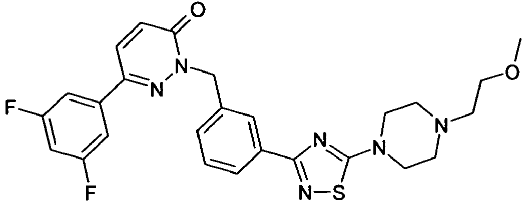 |
| "A110" | 6-(3,5-Difluor-phenyl)-2-[3-(5-morpholin-4-ylmethyl-oxazol-2-yl)-benzyl]-2*H*-pyridazin-3-on |
| "A111" | 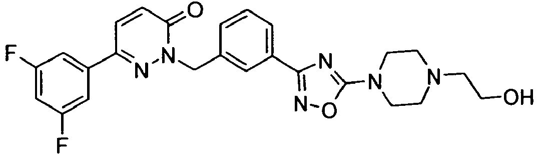 |
| "A112" | 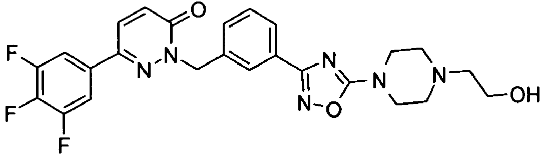 |
| "A113" | 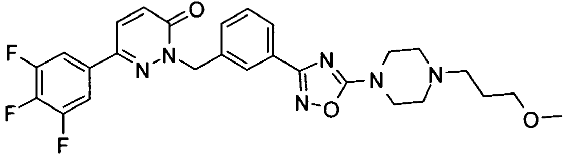 |
| "A114" | 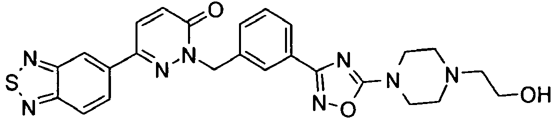 |
| "A115" | 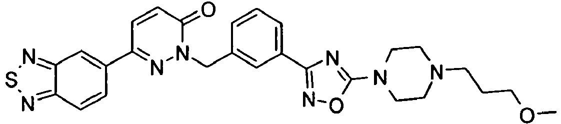 |
| "A116" | 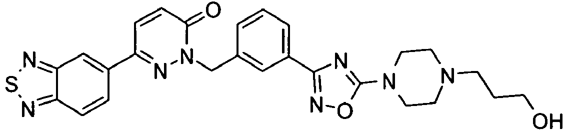 |
| "A117" | 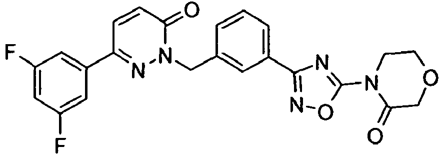 |
| "A118" | 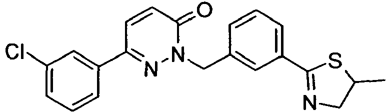 |
| "A119" | 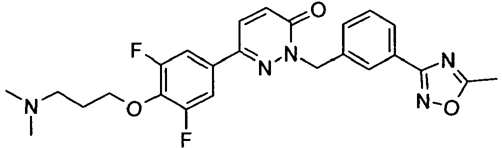 |
| "A120" | 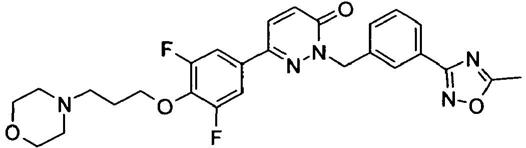 |
| "A121" | 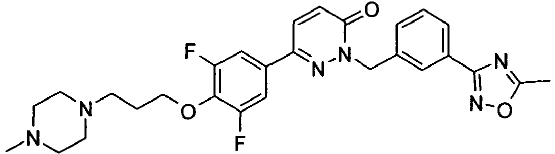 |
| "A122" | 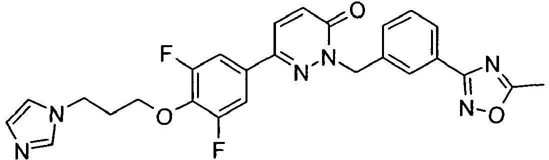 |
| "A 123" | 6-(3,5-Difluorphenyl)-2-(3-{5-[4-(2-ethoxyethyl)-piperazin-1-yl)-[1,2,4]oxadiazol-3-yl}-benzyl)-2*H*-pyridazin-3-on |
| | 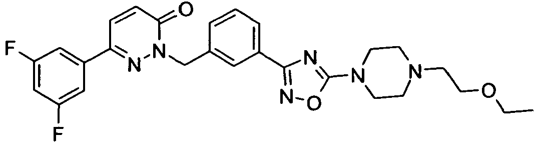 |
| "A124" | 6-(3,5-Difluorphenyl)-2-(3-{5-[4-(3-methoxypropyl)-piperazin-1-yl]-[1,2,4]oxadiazol-3-yl}-benzyl)-2H-pyridazin-3-on |
| | 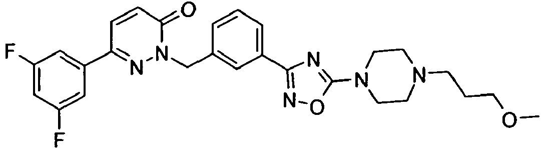 |
| "A125" | 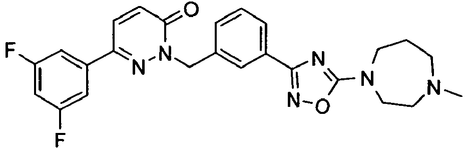 |
| "A126" | 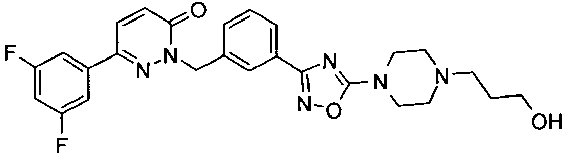 |
| "A127" | 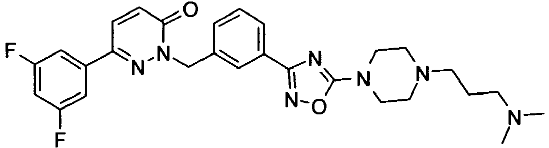 |
| "A128" | 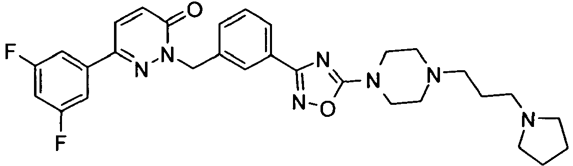 |
| "A129" | 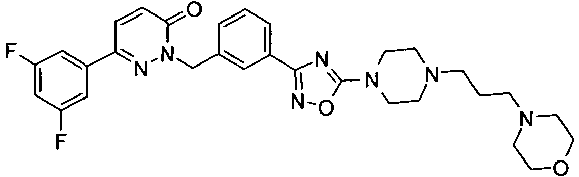 |
| "A130" | 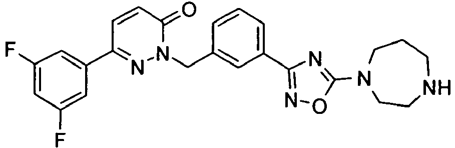 |
| "A131" | 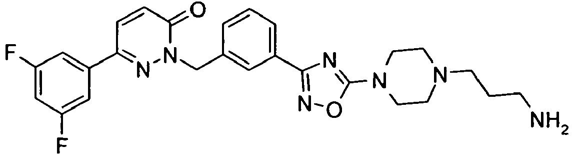 |
sowie ihre pharmazeutisch verwendbaren Solvate, Salze, Tautomere und Stereoisomere, einschließlich deren Mischungen in allen Verhältnissen.

16. Verfahren zur Herstellung von Verbindungen der Formel I nach den Ansprüchen 1-15 sowie ihrer pharmazeutisch verwendbaren Derivate, Salze, Solvate, Tautomeren und Stereoisomeren, **dadurch gekennzeichnet, daß** man
a) eine Verbindung der Formel II 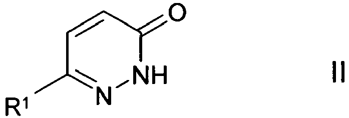 worin R¹ die in Anspruch 1 angegebene Bedeutung hat,
mit einer Verbindung der Formel III 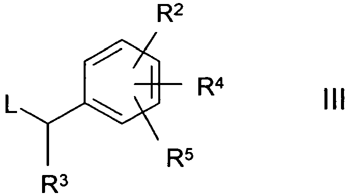 worin R², R³, R⁴ und R⁵ die in Anspruch 1 angegebenen Bedeutungen haben und
L Cl, Br, I oder eine freie oder reaktionsfähig funktionell abgewandelte OH-Gruppe bedeutet,
umsetzt,
oder
b) einen Rest R² in einen anderen Rest R² umwandelt, indem man
i) eine Aminogruppe acyliert oder alkyliert,
ii) ein Oxy-amidinderivat zu einem Oxadiazolderivat cyclisiert,
iii) ein Amidderivat zu einem Oxazolderivat cyclisiert,
iv) ein Alkylesterderivat mit einem N-Hydroxyamidinderivat zu einem Oxadiazolderivat umsetzt,
v) ein N-(Aminothiocarbonyl)-hydrazidderivat zu einem Oxadiazolderivat cyclisiert,
vi) eine SH-Gruppe alkyliert,
vii) eine Cyangruppe mit einem Azidderivat zu einem Tetrazolderivat umsetzt
oder
c) daß man sie aus einem ihrer funktionellen Derivate durch Behandeln mit einem solvolysierenden oder hydrogenolysierenden Mittel in Freiheit setzt,
und/oder
eine Base oder Säure der Formel I in eines ihrer Salze umwandelt.

17. Arzneimittel, enthaltend mindestens eine Verbindung der Formel I nach Anspruch 1-15 und/oder ihre pharmazeutisch verwendbaren Salze, Solvate, Tautomeren und Stereoisomeren, einschließlich deren Mischungen in allen Verhältnissen, sowie gegebenenfalls Träger- und/oder Hilfsstoffe.

18. Verwendung von Verbindungen nach Anspruch 1-15 sowie ihrer pharmazeutisch verwendbaren Salze, Solvate, Tautomeren und Stereoisomeren, einschließlich deren Mischungen in allen Verhältnissen,
zur Herstellung eines Arzneimittels zur Behandlung von Krankheiten, bei denen die Hemmung, Regulierung und/oder Modulation der Signaltransduktion von Kinasen eine Rolle spielt.

19. Verwendung nach Anspruch 18 von Verbindungen gemäß Anspruch 1-15, sowie ihrer pharmazeutisch verwendbaren Salze, Solvate und Stereoisomere, einschließlich deren Mischungen in allen Verhältnissen,
zur Herstellung eines Arzneimittels zur Behandlung von Krankheiten, die durch Inhibierung der Tyrosinkinasen durch die Verbindungen nach Anspruch 1-15 beeinflußt werden.

20. Verwendung nach Anspruch 18, zur Herstellung eines Arzneimittels zur Behandlung von Krankheiten, die durch Inhibierung von Met-Kinase durch die Verbindungen nach Anspruch 1-15 beeinflußt werden.

21. Verwendung nach Anspruch 19 oder 20, wobei die zu behandelnde Krankheit ein fester Tumor ist.

22. Verwendung nach Anspruch 21, wobei der feste Tumor aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopft und/oder der Lunge stammt.

23. Verwendung nach Anspruch 21, wobei der feste Tumor aus der Gruppe Monozytenleukämie, Lungenadenokarzinom, kleinzellige Lungenkarzinome, Bauchspeicheldrüsenkrebs, Glioblastome und Brustkarzinom stammt.

24. Verwendung nach Anspruch 22, wobei der feste Tumor aus der Gruppe der Lungenadenokarzinom, kleinzellige Lungenkarzinome, Bauchspeicheldrüsenkrebs, Glioblastome, Kolonkarzinom und Brustkarzinom stammt.

25. Verwendung nach Anspruch 19 oder 20, wobei die zu behandelnde Krankheit ein Tumor des Blut- und Immunsystems ist.

26. Verwendung nach Anspruch 25, wobei der Tumor aus der Gruppe der akuten myelotischen Leukämie, der chronischen myelotischen Leukämie, akuten lymphatischen Leukämie und/oder chronischen lymphatischen Leukämie stammt.

27. Arzneimittel enthaltend mindestens eine Verbindung der Formel I gemäß einem oder mehreren der Ansprüche 1 bis 15, und/oder ihre pharmazeutisch verwendbaren Salze, Solvate und Stereoisomere, einschließlich deren Mischungen in allen Verhältnissen, und mindestens einen weiteren Arzneimittelwirkstoff.

28. Set (Kit), bestehend aus getrennten Packungen von
(a) einer wirksamen Menge an einer Verbindung der Formel I gemäß einem oder mehreren der Ansprüche 1 bis 15, und/oder ihrer pharmazeutisch verwendbaren Solvate, Salze und Stereoisomere, einschließlich deren Mischungen in allen Verhältnissen,
und
(b) einer wirksamen Menge eines weiteren Arzneimittelswirkstoffs.

## Claims

1. Compounds of the formula I 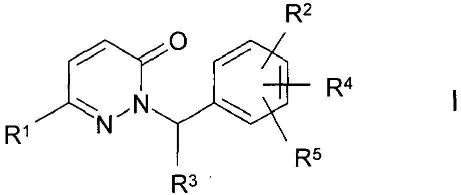 in which
R¹ denotes Ar or Het,
R² denotes a saturated, unsaturated or aromatic 5-membered heterocycle having 1 to 4 N, O and/or S atoms, which may be unsubstituted or mono- or disubstituted by Hal, A, (CH₂)ₙOR³, N(R³)₂, SR³, NO₂, CN, COOR³, CON(R³)₂, NR³COA, NR³SO₂A, SO₂N(R³)₂, S(O)ₘA, Het¹, -[C(R³)₂]ₙN(R³)₂, -[C(R³)₂]ₙHet¹, O[C(R³)₂]ₙN(R³)₂, O[C(R³)₂]ₙHet¹, S[C(R³)₂]ₙN(R³)₂, S[C(R³)₂]ₙHet¹, -NR³[C(R³)₂]ₙN(R³)₂, -NR³[C(R³)₂]ₙHet¹, -NR³Het¹, NHCON(R³)₂, NHCONH[C(R³)₂]ₙN(R³)₂, NHCONH[C(R³)₂]ₙHet¹, CON(R³)₂, ONR³[C(R³)₂]ₙN(R³)₂, CONR³[C(R³)₂]ₙHet¹, COHet¹, COA and/or =O (carbonyl oxygen),
R³ denotes H or A,
R⁴, R⁵ each, independently of one another, denote H, Hal, A, OR³, CN, COOR³, CON(R³)₂, NR³COA, NR³SO₂A, SO₂N(R³)₂ or S(O)ₘA,
A denotes unbranched or branched alkyl having 1-10 C atoms,
in which 1-7 H atoms may be replaced by F, Cl and/or Br,
and/or in which one or two CH₂ groups may be replaced by O, S, SO, SO₂ and/or CH=CH groups, or
cyclic alkyl having 3-7 C atoms,
Ar denotes phenyl, naphthyl or biphenyl, each of which is unsubstituted or mono-, di- or trisubstituted by Hal, A, OR³, N(R³)₂, SR³, NO₂, CN, COOR³, CON(R³)₂, NR³COA, NR³SO₂A, SO₂N(R³)₂, S(O)ₘA, CO-Het¹, Het¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙ₋Het¹, NHCONH[C(R³)₂]ₙN(R³)₂, NHCONH[C(R³)₂]ₙHet¹, OCONH[C(R³)₂]ₙN(R³)₂, OCONH[C(R³)₂]ₙHet¹ and/or COA,
Het denotes a mono-, bi- or tricyclic saturated, unsaturated or aromatic heterocycle having 1 to 4 N, O and/or S atoms, which may be unsubstituted or mono-, di- or trisubstituted by Hal, A, OR³, N(R³)₂, SR³, NO₂, CN, COOR³, CON(R³)₂, NR³COA, NR³SO₂A, SO₂N(R³)₂, S(O)ₘA, CO-Het¹, Het¹, O[C(R³)₂]ₙN(R³)₂, O[C(R³)₂]ₙHet¹, NHCOOA, NHCON(R³)₂, NHCOO[C(R³)₂]ₙN(R³)₂, NHCOO[C(R³)₂]ₙHet¹, NHCONH[C(R³)₂]ₙN(R³)₂ NHCONH[C(R³)₂]ₙHet¹, OCONH[C(R³)₂]ₙN(R³)₂, OCONH[C(R³)₂]ₙHet¹, CO-Het¹, CHO, COA, =S, =NH, =NA and/or =O (carbonyl oxygen),
Het¹ denotes a monocyclic saturated or aromatic heterocycle having 1 to 2 N and/or O atoms, which may be mono- or disubstituted by A, OA, OH, Hal, (CH₂)ₙN(R³)₂, (CH₂)ₙOR³, (CH₂)ₙHet² and/or =O (carbonyl oxygen),
Het² denotes pyrrolidino, piperidino or morpholino,
Hal denotes F, Cl, Br or I,
m denotes 0, 1 or 2,
n denotes 1, 2, 3 or 4,
and pharmaceutically usable solvates, salts, tautomers and stereoisomers thereof, including mixtures thereof in all ratios.

2. Compounds according to Claim 1 in which
A denotes unbranched or branched alkyl having 1-10 C atoms,
in which 1-7 H atoms may be replaced by F and/or Cl,
and pharmaceutically usable solvates, salts, tautomers and stereoisomers thereof, including mixtures thereof in all ratios.

3. Compounds according to Claim 1 or 2 in which
Ar denotes phenyl which is unsubstituted or mono-, di- or trisubstituted by Hal, CN and/or S(O)ₘA,
and pharmaceutically usable solvates, salts, tautomers and stereoisomers thereof, including mixtures thereof in all ratios.

4. Compounds according to one or more of Claims 1-3 in which
Het denotes a mono- or bicyclic aromatic heterocycle having 1 to 3 N, O and/or S atoms, which may be unsubstituted or mono-, di- or trisubstituted by Hal,
and pharmaceutically usable solvates, salts, tautomers and stereoisomers thereof, including mixtures thereof in all ratios.

5. Compounds according to one or more of Claims 1-4 in which
Het denotes thiazolyl, thienyl, pyridyl, benzo-1,2,5-thiadiazolyl or benzo-1,3-dioxolyl,
and pharmaceutically usable solvates, salts, tautomers and stereoisomers thereof, including mixtures thereof in all ratios.

6. Compounds according to one or more of Claims 1-5 in which
R² denotes a saturated, unsaturated or aromatic 5-membered heterocycle having 1 to 4 N, O and/or S atoms, which may be unsubstituted or mono- or disubstituted by Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ and/or =O (carbonyl oxygen),
and pharmaceutically usable solvates, salts, tautomers and stereoisomers thereof, including mixtures thereof in all ratios.

7. Compounds according to one or more of Claims 1-6 in which
R² denotes a heterocycle selected from the group 1,2,4-oxadiazolyl, 1,3,4-oxadiazolyl, oxazolyl, dihydrooxazolyl, pyrazolyl, imidazolyl, triazolyl, tetrazolyl, pyrrolyl, furanyl, thienyl, thiazolyl, dihydrothiazolyl, thiadiazolyl, oxazolidinyl, pyrrolidinyl, piperidinyl, which may be mono- or disubstituted by Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R³)₂, -NR³[C(R³)₂]ₙHet¹, -NR³Het¹, COHet¹ and/or =O (carbonyl oxygen),
and pharmaceutically usable solvates, salts, tautomers and stereoisomers thereof, including mixtures thereof in all ratios.

8. Compounds according to one or more of Claims 1-7 in which
R⁴, R⁵ denote H,
and pharmaceutically usable solvates, salts, tautomers and stereoisomers thereof, including mixtures thereof in all ratios.

9. Compounds according to one or more of Claims 1-8 in which
R³ denotes H, methyl, ethyl, propyl, isopropyl, butyl or tert-butyl,
and pharmaceutically usable solvates, salts, tautomers and stereoisomers thereof, including mixtures thereof in all ratios.

10. Compounds according to one or more of Claims 1-9 in which
R¹ denotes 4-fluorophenyl, 3,5-difluorophenyl, 3,4-difluorophenyl, 3,4,5-trifluorophenyl, 2-chlorophenyl, 3-chlorophenyl, 3-cyanophenyl, 4-cyanophenyl, 4-methylsulfonylphenyl, pyridyl, benzo-1,3-dioxolyl or benzo-1,2,5thiadiazolyl,
and pharmaceutically usable solvates, salts, tautomers and stereoisomers thereof, including mixtures thereof in all ratios.

11. Compounds according to one or more of Claims 1-10 in which
Het¹ denotes a monocyclic saturated or aromatic heterocycle having 1 to 2 N and/or O atoms, which may be mono- or disubstituted by A, (CH₂)ₙHet², (CH₂)ₙN(R³)₂ and/or (CH₂)ₙOR³,
and pharmaceutically usable solvates, salts, tautomers and stereoisomers thereof, including mixtures thereof in all ratios.

12. Compounds according to one or more of Claims 1-11 in which
Het¹ denotes piperidin-1-yl, pyrrolidin-1-yl, morpholin-4-yl, piperazin-1-yl, 1,3-oxazolidin-3-yl, imidazolidinyl, oxazolyl, thiazolyl, thienyl, 1,4-diazepanyl, furanyl or pyridyl, where the radicals may also be mono- or disubstituted by A, (CH₂)ₙHet², (CH₂)ₙN(R³)₂ and/or (CH₂)ₙOR³,
and pharmaceutically usable solvates, salts, tautomers and stereoisomers thereof, including mixtures thereof in all ratios.

13. Compounds according to one or more of Claims 1-12 in which
R¹ denotes Ar or Het,
R² denotes a saturated, unsaturated or aromatic 5-membered heterocycle having 1 to 4 N, O and/or S atoms, which may be unsubstituted or mono- or disubstituted by Hal, A, (CH₂)ₙOR³, N(R³)₂, SR³, Het¹, -[C(R³)₂]ₙN(R³)₂, -[C(R³)₂]ₙHet¹, S[C(R³)₂]ₙN(R³)₂, -NR³[C(R³)₂]ₙN(R₃)₂, -NR³[C(R³)₂]ₙHet¹, -NR³Het¹, COHet¹ and/or =O (carbonyl oxygen),
R³ denotes H or A,
R⁴, R⁵ denote H,
A denotes unbranched or branched alkyl having 1-10 C atoms,
in which 1-7 H atoms may be replaced by F and/or Cl,
Ar denotes phenyl which is unsubstituted or mono-, di- or trisubstituted by Hal, CN and/or S(O)ₘA,
Het denotes a mono- or bicyclic aromatic heterocycle having 1 to 3 N, O and/or S atoms, which may be unsubstituted or mono-, di- or trisubstituted by Hal,
Het¹ denotes a monocyclic saturated or aromatic heterocycle having 1 to 2 N and/or O atoms, which may be mono- or disubstituted by A, (CH₂)ₙHet², (CH₂)ₙN(R³)₂ and/or (CH₂)ₙOR³,
Het² denotes pyrrolidino, piperidino or morpholino,
Hal denotes F, Cl, Br or I,
m denotes 0, 1 or 2,
n denotes 1, 2, 3 or 4,
and pharmaceutically usable solvates, salts, tautomers and stereoisomers thereof, including mixtures thereof in all ratios.

14. Compounds according to one or more of Claims 1-13 in which
R¹ denotes Ar or Het,
R² denotes a heterocycle selected from the group 1,2,4-oxadiazolyl, 1,3,4-oxadiazolyl, oxazolyl, dihydrooxazolyl, pyrazolyl, imidazolyl, triazolyl, thiadiazolyl, tetrazolyl, pyrrolyl, furanyl, thienyl, thiazolyl, dihydrothiazolyl, oxazolidinyl,
which may be mono- or disubstituted by Hal, A, (CH₂)ₙOR³, N(R³)₂, SR³, Het¹, -[C(R³)₂]ₙN(R³)₂, -[C(R³)₂]ₙHet¹, S(C(R³)₂]ₙN(R³)₂, -NR³(C(R³)₂]ₙN(R³)₂, -NR³[C(R³)₂]ₙHet¹, -COHet¹ and/or =O (carbonyl oxygen),
R³ denotes H, methyl, ethyl, propyl, isopropyl, butyl or tert-butyl,
R⁴, R⁵ denote H,
A denotes unbranched or branched alkyl having 1-10 C atoms,
in which 1-7 H atoms may be replaced by F and/or Cl,
Ar denotes phenyl which is unsubstituted or mono-, di- or trisubstituted by Hal, CN and/or S(O)ₘA,
Het denotes thiazolyl, thienyl, pyridyl, benzo-1,2,5-thiadiazolyl or benzo-1,3-dioxolyl,
Het¹ denotes piperidin-1-yl, pyrrolidin-1-yl, morpholin-4-yl, piperazin-1-yl, 1,3-oxazolidin-3-yl, imidazolidinyl, oxazolyl, thiazolyl, thienyl, 1,4-diazepanyl, furanyl or pyridyl, where the radicals may also be mono- or disubstituted by A, (CH₂)ₙHet², (CH₂)ₙN(R₃)₂ and/or (CH₂)ₙOR³,
Het² denotes pyrrolidino, piperidino or morpholino,
Hal denotes F, Cl, Br or I,
m denotes 0, 1 or 2,
n denotes 1, 2, 3 or 4,
and pharmaceutically usable solvates, salts, tautomers and stereoisomers thereof, including mixtures thereof in all ratios.

15. Compounds according to Claim 1, selected from the group
| No. | Structure and/or name |
|---|---|
| "A1" | 6-(3,5-Difluorophenyl)-2-[3-(5-methyl-1,2,4-oxadiazol-3-yl)-benzyl]-2*H*-pyridazin-3-one |
| "A2" | 2-[3-(5-Methyl-1,2,4-oxadiazol-3-yl)benzyl]-6-(3,4,5-trifluorophenyl)-2*H*-pyridazin-3-one |
| "A3" | 6-(3,5-Difluorophenyl)-2-[4-(5-methyl-1,2,4-oxadiazol-3-yl)-benzyl]-2*H*-pyridazin-3-one |
| "A4" | 6-(4-Fluorophenyl)-2-[3-(5-methyl-1,2,4-oxadiazol-3-yl)-benzyl]-2*H*-pyridazin-3-one |
| "A5" | 2-[3-(5-Methyl-1,2,4-oxadiazol-3-yl)benzyl]-6-pyridin-3-yl-2*H-*pyridazin-3-one |
| | 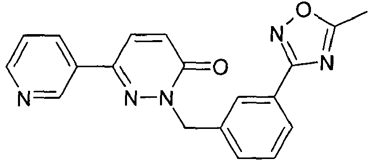 |
| "A6" | 6-(3-Chlorophenyl)-2-[3-(5-methyl-1,2,4-oxadiazol-3-yl)-benzyl]-2*H*-pyridazin-3-one |
| "A7" | 6-(3-Cyanophenyl)-2-[3-(5-methyl-1,2,4-oxadiazol-3-yl)-benzyl]-2*H*-pyridazin-3-one |
| "A8" | 6-(4-Cyanophenyl)-2-[3-(5-methyl-1,2,4-oxadiazol-3-yl)-benzyl]-2*H*-pyridazin-3-one |
| "A9" | 6-(4-Methylsulfonylphenyl)-2-[3-(5-methyl-1,2,4-oxadiazol-3-yl)benzyl]-2*H*-pyridazin-3-one |
| "A10" | 6-(3,5-Difluorophenyl)-2-[3-(5-dimethylaminomethyl-1,2,4-oxadiazol-3-yl)benzyl]-2*H*-pyridazin-3-one |
| "A11" | 6-(3,5-Difluorophenyl)-2-[3-(5-ethylamino-1,2,4-oxadiazol-3-yl)benzyl]-2*H*-pyridazin-3-one |
| "A12" | 6-(3,5-Difluorophenyl)-2-{3-[5-(3-dimethylaminopropyl-amino)-1,2,4-oxadiazol-3-yl]benzyl}-2*H*-pyridazin-3-one |
| "A13" | 6-(3,5-Difluorophenyl)-2-[3-(5-methyloxazol-2-yl)benzyl]-2*H-*pyridazin-3-one |
| "A14" | 6-(3,5-Difluorophenyl)-2-[3-(3-methyl-1,2,4-oxadiazol-5-yl)-benzyl]-2*H*-pyridazin-3-one |
| "A15" | 6-(3,5-Difluorophenyl)-2-[3-(5-oxo-4,5-dihydro-1,3,4-oxadiazol-2-yl)benzyl]-2*H*-pyridazin-3-one |
| "A16" | 6-(3,5-Difluorophenyl)-2-{3-[5-(2-morpholin-4-ylethylamino)-1,3,4-oxadiazol-2-yl]benzyl}-2*H*-pyridazin-3-one |
| "A17" | 6-(3,5-Difluorophenyl)-2-[3-(5-oxo-4,5-dihydro-1,2,4-oxadiazol-3-yl)benzyl]-2*H*-pyridazin-3-one |
| "A18" | 6-(3,5-Difluorophenyl)-2-[3-(5-thioxo-4,5-dihydro-1,2,4-oxadiazol-3-yl)benzyl]-2*H*-pyridazin-3-one |
| "A19" | 6-(3,5-Difluorophenyl)-2-{3-[5-(3-dimethylaminopropyl-sulfanyl)-1,2,4-oxadiazol-3-yl]benzyl}-2*H*-pyridazin-3-one |
| "A20" | 6-(3,5-Difluorophenyl)-2-{3-[5-(4-methylpiperazin-1-yl)-1,2,4-oxadiazol-3-yl]benzyl}-2*H*-pyridazin-3-one |
| "A21" | 6-(3,5-Difluorophenyl)-2-{3-[5-(4-methylpiperazine-1-carbonyl)-1,2,4-oxadiazol-3-yl]benzyl}-2*H*-pyridazin-3-one |
| "A22" | 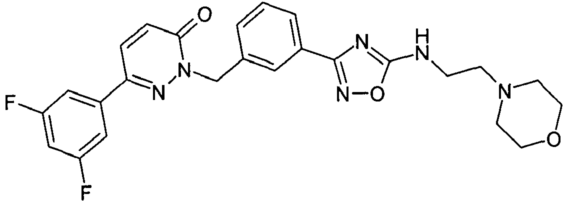 |
| "A23" | 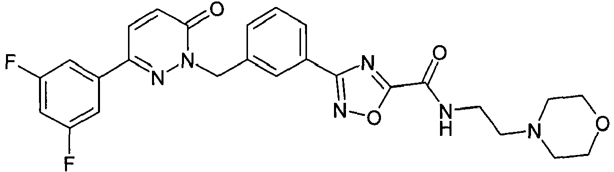 |
| "A24" | 6-(3,5-Difluorophenyl)-2-[3-(5-piperazin-1-yl-1,2,4-oxadiazol-3-yl)benzyl]-2*H*-pyridazin-3-one |
| "A25" | 6-(3,5-Difluorophenyl)-2-{3-[4-(3-dimethylaminopropyl)-5-oxo-4,5-dihydro-1,2,4-oxadiazol-3-yl]benzyl}-2*H*-pyridazin-3-one |
| "A26" | 6-(3,5-Difluorophenyl)-2-[3-(1*H*-tetrazol-5-yl)benzyl]-2*H-*pyridazin-3-one |
| "A27" | 6-(3,5-Difluorophenyl)-2-{3-[5-(2-dimethylaminoethylamino)-1,2,4-oxadiazol-3-yl]benzyl}-2*H*-pyridazin-3-one |
| "A28" | 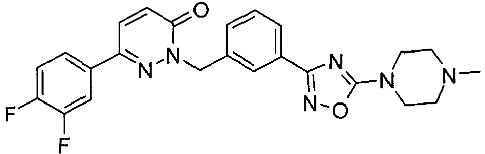 |
| "A29" | 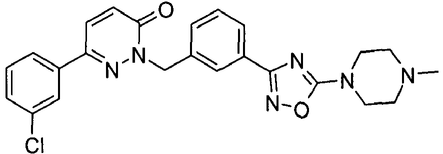 |
| "A30" | 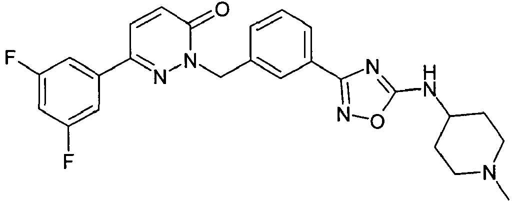 |
| "A31" | 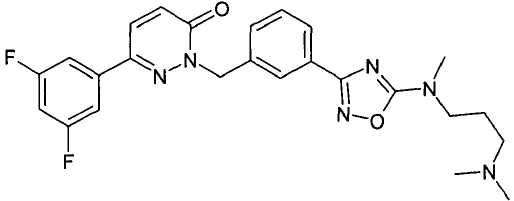 |
| "A32" | 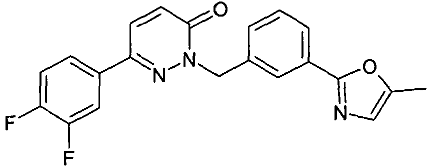 |
| "A33" | 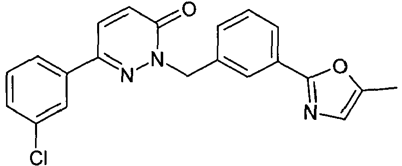 |
| "A34" | 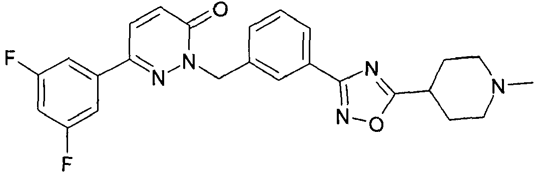 |
| "A35" | 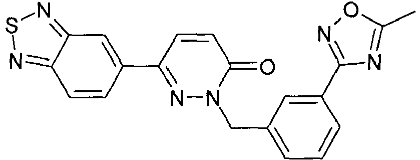 |
| "A36" | 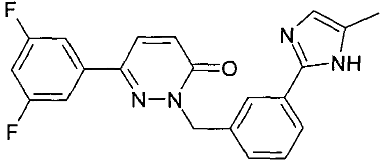 |
| "A37" | 6-(3,5-Difluorophenyl)-2-{3-[5-(4-methylpiperazin-1-yl)-oxazol-2-yl]benzyl}-2*H*-pyridazin-3-one |
| "A38" | 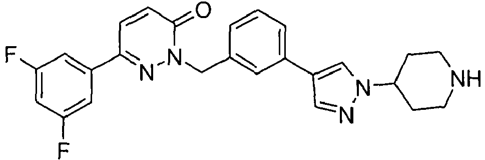 |
| "A39" | 6-(3,4-Difluorophenyl)-2-[3-(5-methyl-1,2,4-oxadiazol-3-yl)-benzyl]-2*H*-pyridazin-3-one |
| "A41" | 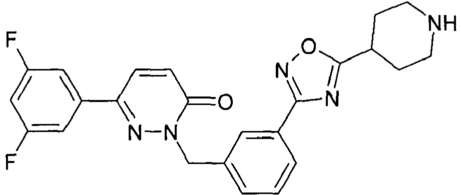 |
| "A42" | 6-(3,5-Difluorophenyl)-2-{3-[5-(piperidin-4-ylamino)-1,2,4-oxadiazol-3-yl]benzyl)-2*H*-pyridazin-3-one |
| | 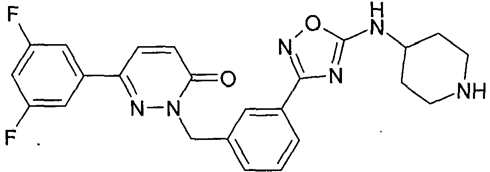 |
| "A43" | 2-{3-[5-(1-Methylpiperidin-4-ylamino)-1,2,4-oxadiazol-3-yl]benzyl}-6-(3,4,5-trifluorophenyl)-2*H-*pyridazin-3-one |
| | 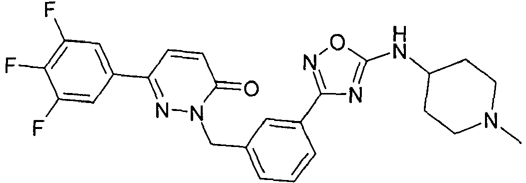 |
| "A44" | 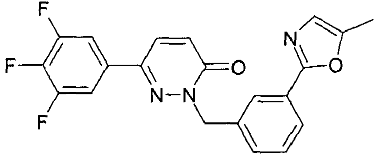 |
| "A45" | 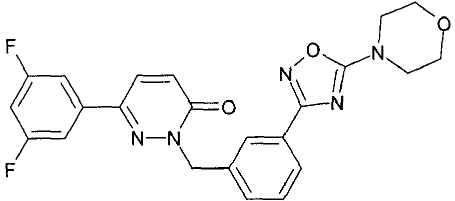 |
| "A46" | 6-(3,5-Difluorophenyl)-2-(3-{5-[methyl-(1-methylpiperidin-4-yl)amino]-1,2,4-oxadiazol-3-yl}benzyl)-2*H*-pyridazin-3-one |
| | 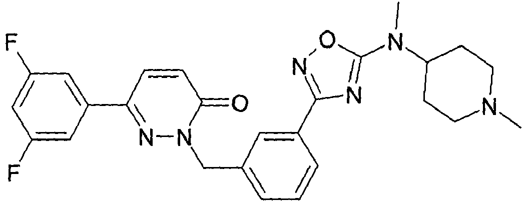 |
| "A47" | 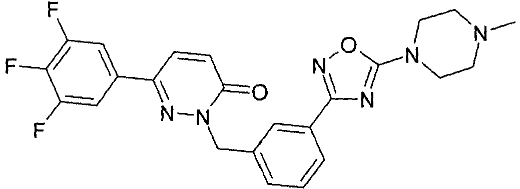 |
| "A48" | 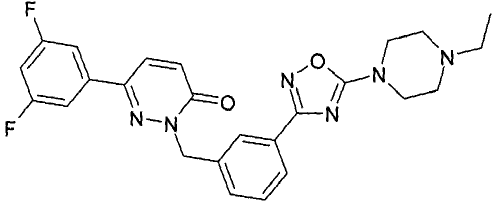 |
| "A49" | 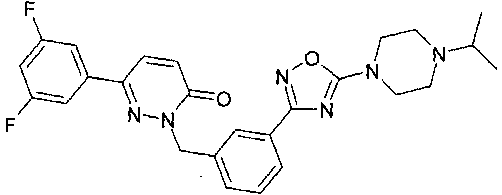 |
| "A50" | 6-(3,5-Difluorophenyl)-2-(3-{5-[4-(2-methoxyethyl)piperazin-1-yl]-1,2,4-oxadiazol-3-yl}benzyl)-2*H*-pyridazin-3-one |
| | 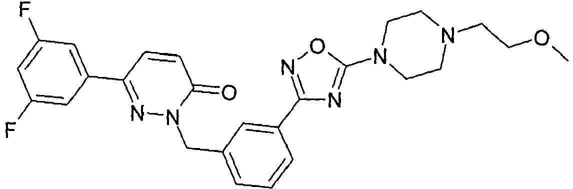 |
| "A51" | 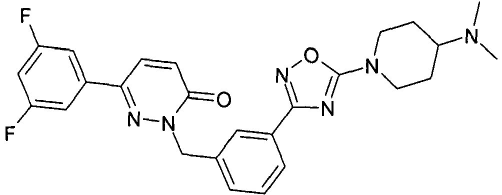 |
| "A52" | 6-(3,5-Difluorophenyl)-2-(3-{5-[4-(2-dimethylaminoethyl)-piperazin-1-yl]-1,2,4-oxadiazol-3-yl}benzyl)-2*H*-pyridazin-3-one |
| | 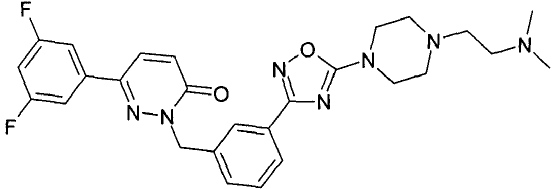 |
| "A53" | 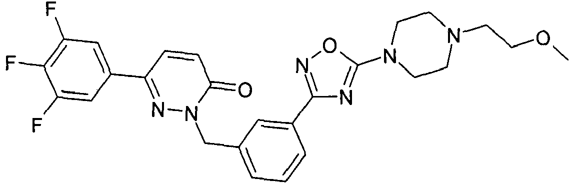 |
| "A54" | 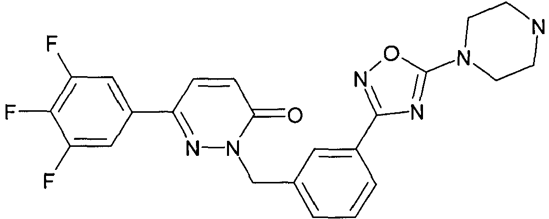 |
| "A55" | 6-(3,5-Difluorophenyl)-2-[3-(5-oxo-4-piperidin-4-yl-4,5-dihydro-1,2,4-oxadiazol-3-yl)benzyl]-2*H*-pyridazin-3-one |
| | 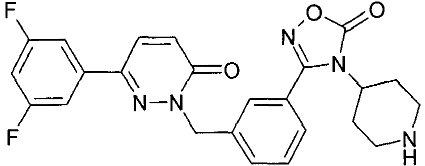 |
| "A56" | 6-(3,5-Difluorophenyl)-2-[3-(5-hydroxymethyl-1*H-*imidazol-2-yl)benzyl]-2*H*-pyridazin-3-one |
| | 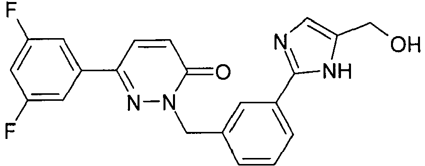 |
| "A57" | 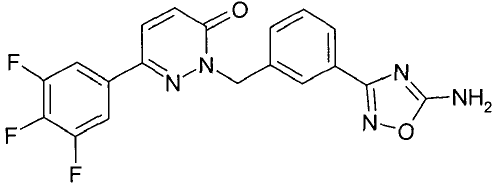 |
| "A58" | 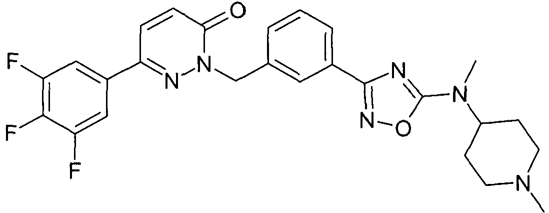 |
| "A59" | 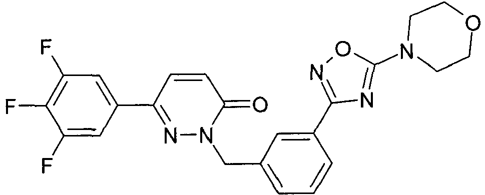 |
| "A60" | 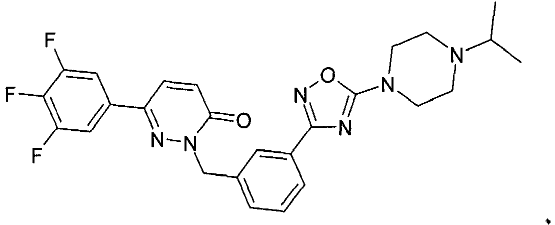 |
| "A61" | 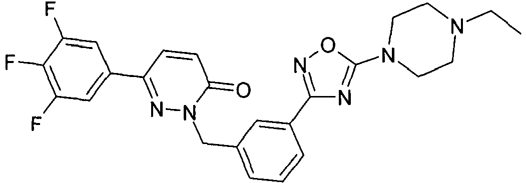 |
| "A62" | 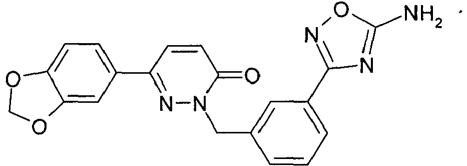 |
| "A63" | 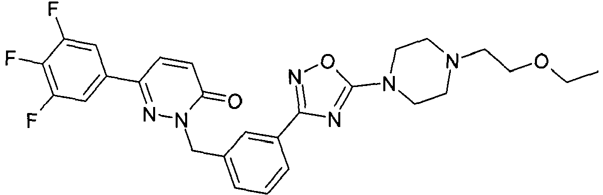 |
| "A64" | 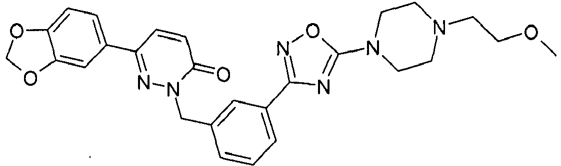 |
| "A65" | 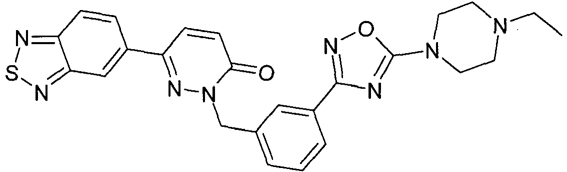 |
| "A66" | 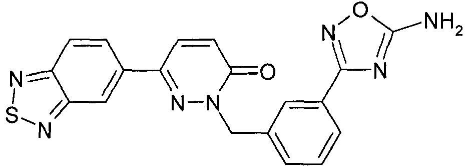 |
| "A67" | 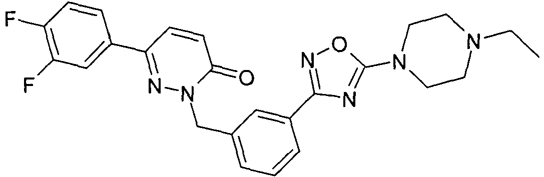 |
| "A68" | 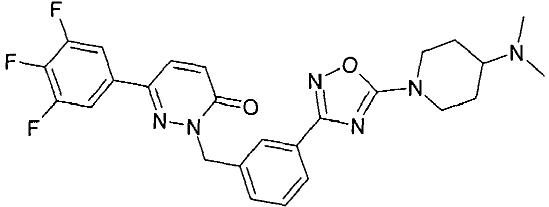 |
| "A69" | 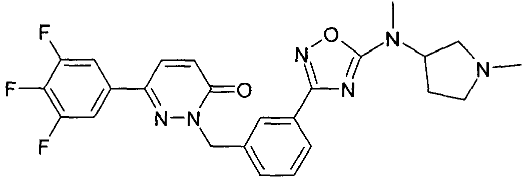 |
| "A70" | 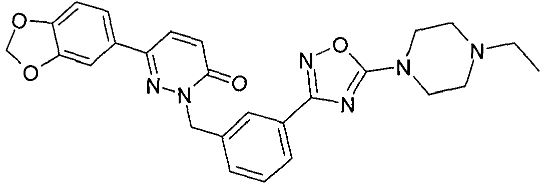 |
| "A71a" | 2-(3-{5-[(1-Ethylpiperidin-4-yl)methylamino]-1,2,4-oxadiazol-3-yl}benzyl)-6-(3,4,5-trifluorophenyl)-2*H*-pyridazin-3-one |
| | 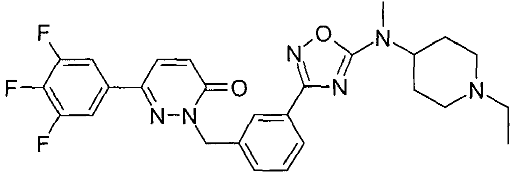 |
| "A71b" | 6-(3,5-Difluorophenyl)-2-(3-{5-[(1-ethylpiperidin-4-yl)methyl-amino]-1,2,4-oxadiazol-3-yl}benzyl)-2*H*-pyridazin-3-one |
| | 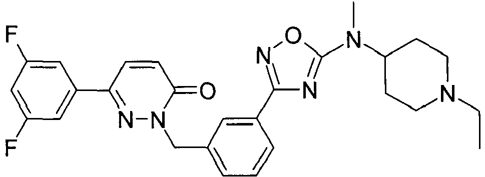 |
| "A71 c" | 6-(3,5-Difluorophenyl)-2-(3-{5-[methyl(1-methylpiperidin-4-yl-methyl)amino]-1,2,4-oxadiazol-3-yl}benzyl)-2*H*-pyridazin-3-one |
| | 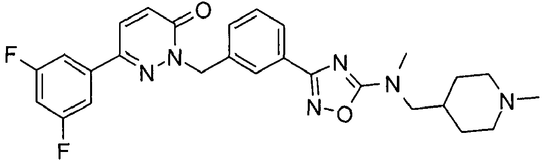 |
| "A72" | 2-[3-(5-Amino-1,2,4-oxadiazol-3-yl)benzyl]-6-(3,5-difluoro-phenyl)-2*H*-pyridazin-3-one |
| | 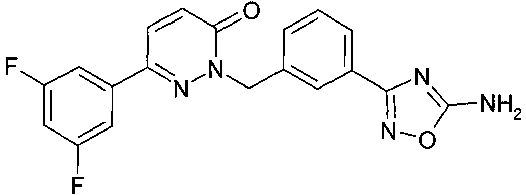 |
| "A73" | 6-(3,5-Difluorophenyl)-2-{3-[5-(methylpiperidin-4-ylamino)-1,2,4-oxadiazol-3-yl]benzyl}-2*H*-pyridazin-3-one |
| | 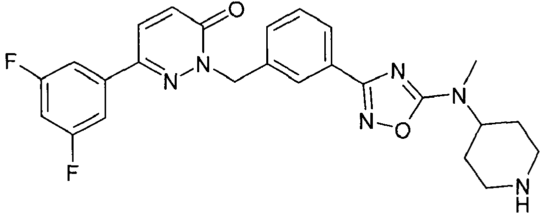 |
| "A74" | 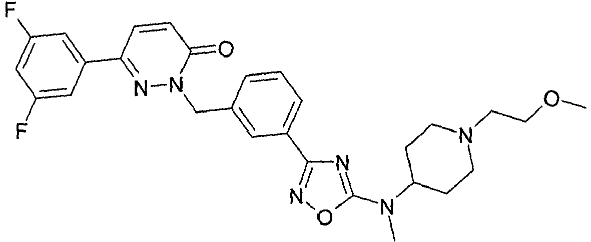 |
| "A75" | 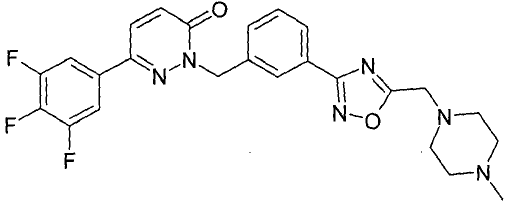 |
| "A76" | 2-[3-(5-Trifluoromethyl-1,2,4-oxadiazol-3-yl)benzyl]-6-(3,4,5-trifluorophenyl)-2*H*-pyridazin-3-one |
| "A77" | 2-(3-1,2,4-Oxadiazol-3-ylbenzyl)-6-(3,4,5-trifluorophenyl)-2*H*-pyridazin-3-one |
| "A78" | 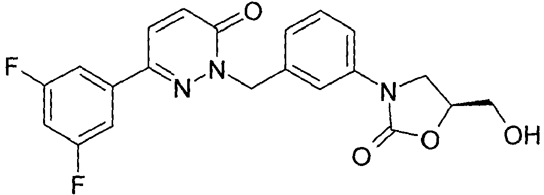 |
| "A79" | 6-(3,5-Difluorophenyl)-2-[3-((R)-5-hydroxymethyl-2-oxo-oxazolidin-3-yl)benzyl]-2*H*-pyridazin-3-one |
| "A80" | 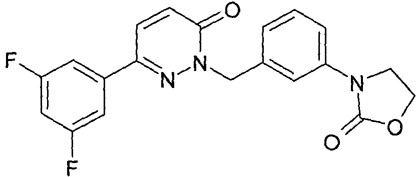 |
| "A81" | 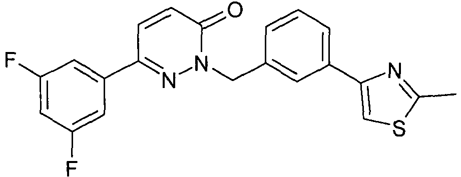 |
| "A82" | 6-(3-Cyanophenyl)-2-[3-(2-methylthiazol-4-yl)benzyl]-2*H-*pyridazin-3-one |
| "A83" | 6-(3,5-Difluorophenyl)-2-[3-(1*H*-imidazol-2-yl)benzyl]-2*H-*pyridazin-3-one |
| "A84" | 6-(3,5-Difluorophenyl)-2-[3-(5-methylthiazol-2-yl)benzyl]-2*H-*pyridazin-3-one |
| | 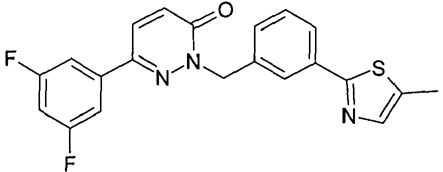 |
| "A85" | 6-(3,5-Difluorophenyl)-2-[3-(5-methyl-4,5-dihydrooxazol-2-yl)benzyl]-2*H*-pyridazin-3-one |
| | 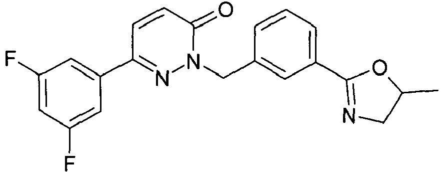 |
| "A86" | 6-(3-Chlorophenyl)-2-[3-(5-methylthiazol-2-yl)benzyl]-2*H-*pyridazin-3-one |
| "A87" | 6-(3,5-Difluorophenyl)-2-[3-(5-piperidin-4-yloxazol-2-yl)-benzyl]-2*H*-pyridazin-3-one |
| "A88" | 6-(3,5-Difluorophenyl)-2-{3-[5-(1-methylpiperidin-4-yl)-oxazol-2-yl]benzyl}-2*H*-pyridazin-3-one |
| "A89" | 2-[3-(5-Piperidin-4-yloxazol-2-yl)benzyl]-6-(3,4,5-trifluorophenyl)-2*H*-pyridazin-3-one |
| | 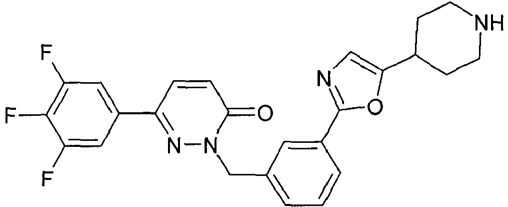 |
| "A90" | 2-[3-(5-Piperidin-4-yloxazol-2-yl)benzyl]-6-(3-chlorophenyl)-2*H*-pyridazin-3-one |
| | 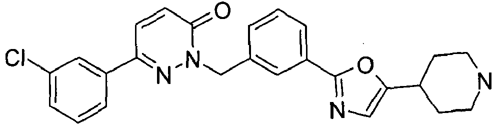 |
| "A91" | 6-(3,5-Difluorophenyl)-2-[3-(5-piperidin-4-ylthiazol-2-yl)-benzyl]-2*H*-pyridazin-3-one |
| | 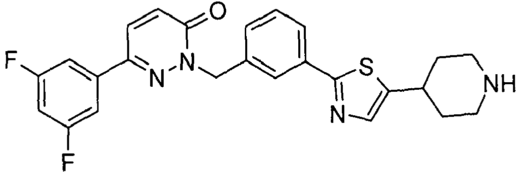 |
| "A92" | 6-(3,5-Difluorophenyl)-2-{3-[5-(1-methylpiperidin-4-yl)thiazol-2-yl]benzyl}-2*H*-pyridazin-3-one |
| | 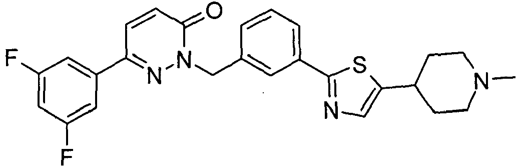 |
| "A93" | 2-[3-(5-Piperidin-4-ylthiazol-2-yl)benzyl]-6-(3,4,5-trifluorophenyl)-2*H*-pyridazin-3-one |
| "A94" | 6-(3-Chlorophenyl)-2-[3-(5-piperidin-4-ylthiazol-2-yl)benzyl]-2H-pyridazin-3-one |
| "A95" | 6-(3-Chlorophenyl)-2-{3-[5-(4-methylpiperazin-1-ylmethyl)-thiazol-2-yl]benzyl}-2*H*-pyridazin-3-one |
| "A96" | 6-(3,5-Difluorophenyl)-2-[3-(5-piperidin-3-ylthiazol-2-yl)-benzyl]-2*H*-pyridazin-3-one |
| "A97" | 2-[3-(5-Azetidin-3-ylthiazol-2-yl)benzyl]-6-(3,5-difluorophenyl)-2H-pyridazin-3-one |
| | 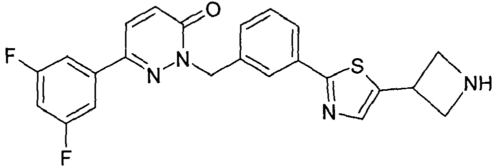 |
| "A98" | 6-(3,5-Difluorophenyl)-2-[3-(5-piperidin-4-ylmethylthiazol-2-yl)benzyl]-2*H*-pyridazin-3-one |
| "A99" | 6-(3,5-Difluorophenyl)-2-(3-{5-[1-(2-methoxyethyl)-piperidin-4-yl]thiazol-2-yl}benzyl)-2*H*-pyridazin-3-one |
| | 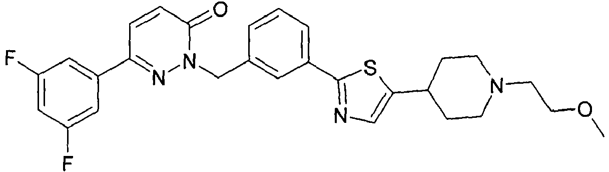 |
| "A99a" | 6-(3,5-Difluorophenyl)-2-{3-[5-(1-methylpiperidin-4-yl-methyl)thiazol-2-yl]benzyl}-2*H*-pyridazin-3-one |
| "A100" | 6-(3,5-Difluorophenyl)-2-[3-(5-pyrrolidin-2-ylthiazol-2-yl)-benzyl]-2*H*-pyridazin-3-one |
| "A101" | 6-(3,5-Difluorophenyl)-2-{3-[5-(4-methylpiperazin-1-yl-methyl)thiazol-2-yl]benzyl}-2*H*-pyridazin-3-one |
| "A102" | 6-(3,5-Difluorophenyl)-2-[3-(5-morpholin-4-ylmethylthiazol-2-yl)benzyl]-2*H*-pyridazin-3-one |
| "A103" | 6-(3,5-Difluorophenyl)-2-[3-(4-methylthiazol-2-yl)benzyl]-2*H-*pyridazin-3-one |
| "A104" | 6-(3,5-Difluorophenyl)-2-(3-thiazol-2-ylbenzyl)-2*H*-pyridazin-3-one |
| "A105" | 6-(3,5-Difluorophenyl)-2-{3-[1-(2-piperidin-1-ylethyl)-1*H-*1,2,3-triazol-4-yl]benzyl}-2*H*-pyridazin-3-one |
| | 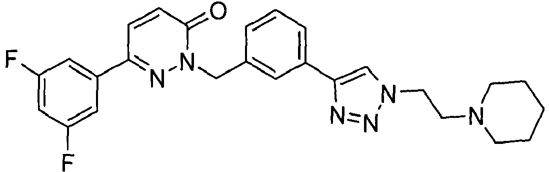 |
| "A106" | 6-(3,5-Difluorophenyl)-2-{3-[1-(2-morpholin-4-ylethyl)-1*H-*1,2,3-triazol-4-yl]benzyl}-2*H*-pyridazin-3-one |
| "A107" | 6-(3,5-Difluorophenyl)-2-{3-[5-(4-methylpiperazine-1-carbonyl)isoxazol-3-yl]benzyl}-2*H*-pyridazin-3-one |
| | 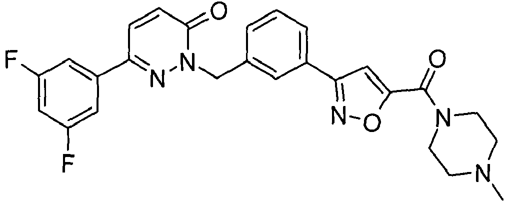 |
| "A108" | 6-(3,5-Difluorophenyl)-2-[3-(5-oxo-4,5-dihydro-1,2,4-thiadiazol-3-yl)benzyl]-2*H*-pyridazin-3-one |
| | 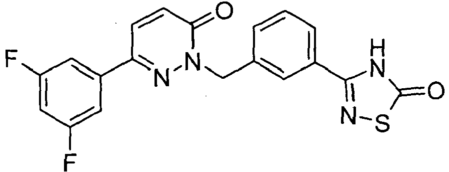 |
| "A109" | 6-(3,5-Difluorophenyl)-2-{3-[5-(4-methylpiperazin-1-yl)-1,2,4-thiadiazol-3-yl]benzyl}-2*H*-pyridazin-3-one |
| "A109a" | 6-(3,5-Difluorophenyl)-2-(3-{5-[4-(2-methoxyethyl)piperazin-1-yl]-1,2,4-thiadiazol-3-yl}benzyl)-2*H*-pyridazin-3-one |
| | 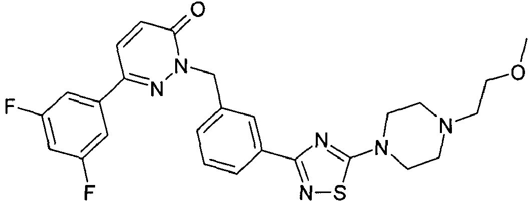 |
| "A110" | 6-(3,5-Difluorophenyl)-2-[3-(5-morpholin-4-ylmethyloxazol-2-yl)benzyl]-2*H*-pyridazin-3-one |
| "A111" | 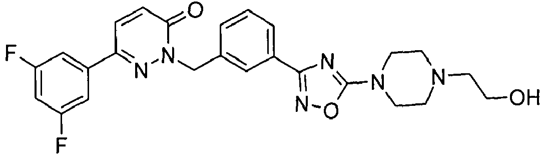 |
| "A112" | 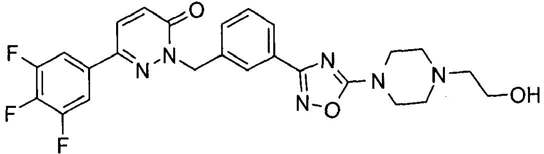 |
| "A113" | 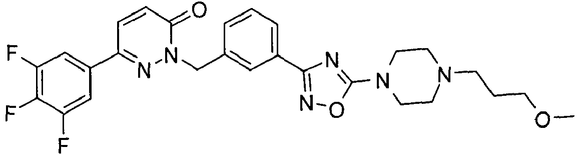 |
| "A114" | 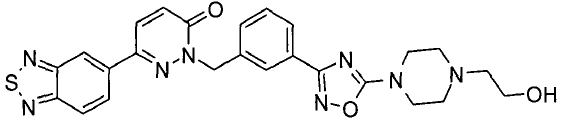 |
| "A115" | 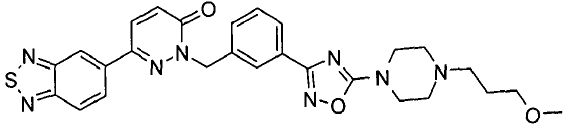 |
| "A116" | 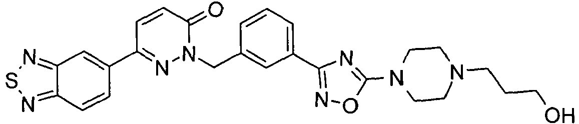 |
| "A117" | 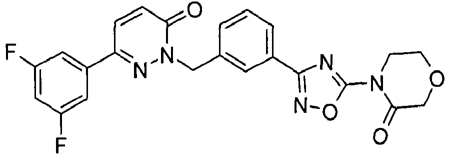 |
| "A118" | 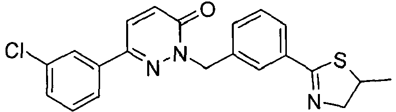 |
| "A119" | 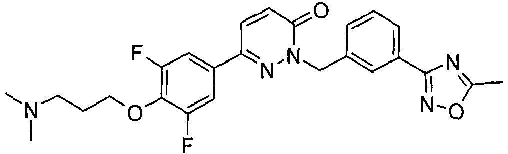 |
| "A120" | 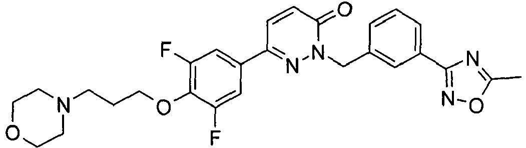 |
| "A121" | 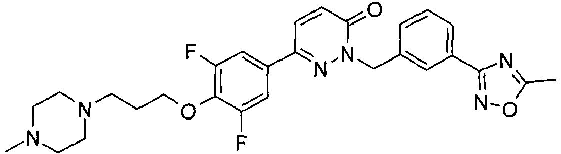 |
| "A122" | 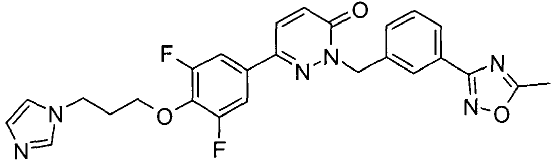 |
| "A123" | 6-(3,5-Difluorophenyl)-2-(3-{5-[4-(2-ethoxyethyl)piperazin-1-yl]-1,2,4-oxadiazol-3-yl}benzyl)-2*H*-pyridazin-3-one |
| | 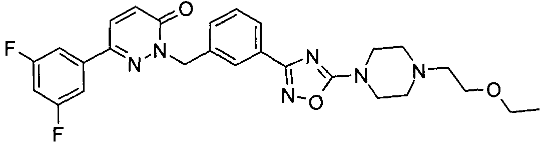 |
| "A124" | 6-(3,5-Difluorophenyl)-2-(3-{5-[4-(3-methoxypropyl)piperazin-1-yl]-1,2,4-oxadiazol-3-yl}benzyl)-2H-pyridazin-3-one |
| | 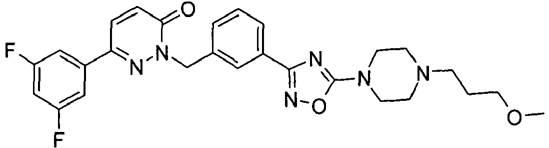 |
| "A125" | 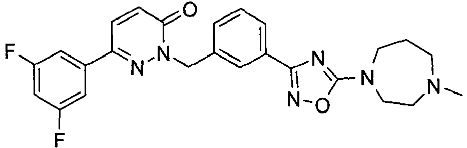 |
| "A126" | 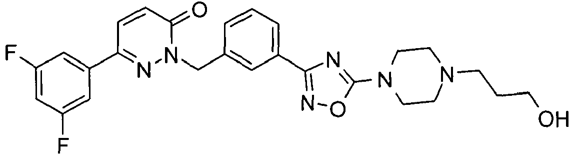 |
| "A127" | 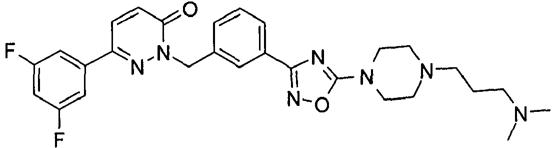 |
| "A128" | 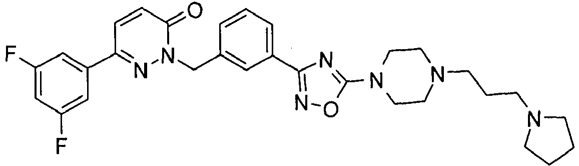 |
| "A129" | 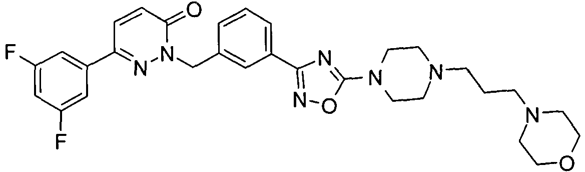 |
| "A130" | 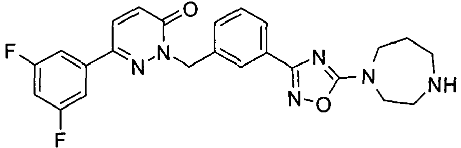 |
| "A131" | 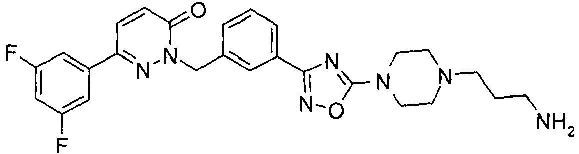 |
and pharmaceutically usable solvates, salts, tautomers and stereoisomers thereof, including mixtures thereof in all ratios.

16. Process for the preparation of compounds of the formula I according to Claims 1-15 and pharmaceutically usable derivatives, salts, solvates, tautomers and stereoisomers thereof, **characterised in that**
a) a compound of the formula II 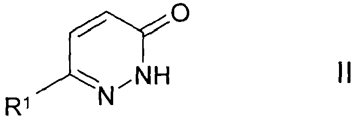 in which R¹ has the meaning indicated in Claim 1,
is reacted with a compound of the formula III 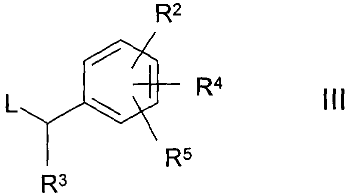 in which R², R³, R⁴ and R⁵ have the meanings indicated in Claim 1 and
L denotes Cl, Br, I or a free or reactively functionally modified OH group,
or
b) a radical R² is converted into another radical R² by
i) acylating or alkylating an amino group,
ii) cyclising an oxyamidine derivative to give an oxadiazole derivative,
iii) cyclising an amide derivative to give an oxazole derivative,
iv) reacting an alkyl ester derivative with an N-hydroxy-amidine derivative to give an oxadiazole derivative,
v) cyclising an N-(aminothiocarbonyl)hydrazide derivative to give an oxadiazole derivative,
vi) alkylating an SH group,
vii) reacting a cyano group with an azide derivative to give a tetrazole derivative
or
c) **in that** it is liberated from one of its functional derivatives by treatment with a solvolysing or hydrogenolysing agent,
and/or
a base or acid of the formula I is converted into one of its salts.

17. Medicaments comprising at least one compound of the formula I according to Claims 1-15 and/or pharmaceutically usable salts, solvates, tautomers and stereoisomers thereof, including mixtures thereof in all ratios, and optionally excipients and/or adjuvants.

18. Use of compounds according to Claims 1-15 and pharmaceutically usable salts, solvates, tautomers and stereoisomers thereof, including mixtures thereof in all ratios,
for the preparation of a medicament for the treatment of diseases in which the inhibition, regulation and/or modulation of kinase signal transduction plays a role.

19. Use according to Claim 18 of compounds according to Claims 1-15, and pharmaceutically usable salts, solvates and stereoisomers thereof, including mixtures thereof in all ratios,
for the preparation of a medicament for the treatment of diseases which are influenced by inhibition of tyrosine kinases by the compounds according to Claims 1-15.

20. Use according to Claim 18 for the preparation of a medicament for the treatment of diseases which are influenced by inhibition of Met kinase by the compounds according to Claims 1-15.

21. Use according to Claim 19 or 20, where the disease to be treated is a solid tumour.

22. Use according to Claim 21, where the solid tumour originates from the group of tumours of the squamous epithelium, of the bladder, of the stomach, of the kidneys, of head and neck, of the oesophagus, of the cervix, of the thyroid, of the intestine, of the liver, of the brain, of the prostate, of the urogenital tract, of the lymphatic system, of the stomach, of the larynx and/or of the lung.

23. Use according to Claim 21, where the solid tumour originates from the group monocytic leukaemia, lung adenocarcinoma, small-cell lung carcinomas, pancreatic cancer, glioblastomas and breast carcinoma.

24. Use according to Claim 22, where the solid tumour originates from the group of lung adenocarcinoma, small-cell lung carcinomas, pancreatic cancer, glioblastomas, colon carcinoma and breast carcinoma.

25. Use according to Claim 19 or 20, where the disease to be treated is a tumour of the blood and immune system.

26. Use according to Claim 25, where the tumour originates from the group of acute myeloid leukaemia, chronic myeloid leukaemia, acute lymphatic leukaemia and/or chronic lymphatic leukaemia.

27. Medicaments comprising at least one compound of the formula I according to one or more of Claims 1 to 15, and/or pharmaceutically usable salts, solvates and stereoisomers thereof, including mixtures thereof in all ratios, and at least one further medicament active compound.

28. Set (kit) consisting of separate packs of
(a) an effective amount of a compound of the formula I according to one or more of Claims 1 to 15, and/or pharmaceutically usable solvates, salts and stereoisomers thereof, including mixtures thereof in all ratios,
and
(b) an effective amount of a further medicament active compound.

## Revendications

1. Composés de formule I 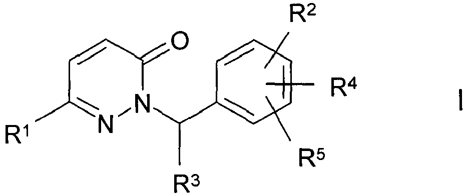 dans laquelle
R¹ désigne Ar ou Hét,
R² désigne un hétérocycle de 5 chaînons saturé, insaturé ou aromatique ayant de 1 à 4 atomes de N, O et/ou S, qui peut être non substitué ou mono- ou disubstitué par Hal, A, (CH₂)ₙOR³, N(R³)₂, SR³, NO₂, CN, COOR³, CON(R³)₂, NR³COA, NR³SO₂A, SO₂N(R³)₂, S(O)ₘA, Hét¹, -[C(R³)₂]ₙN(R³)₂, -[C(R³)₂]ₙHét¹, O[C(R³)₂]ₙN(R³)₂, O[C(R³)₂]ₙHét¹, S[C(R³)₂]ₙN(R³)₂, S[C(R³)₂]ₙHét¹, - NR³[C(R³)₂]ₙN(R³)₂, -NR³[C(R³)₂]ₙHét¹, -NR³Hét¹, NHCON(R³)₂, NHCONH[C(R³)₂]ₙN(R³)₂, NHCONH[C(R³)₂]ₙHét¹, CON(R³)₂, ONR³[C(R³)₂]ₙN(R³)₂, CONR³[C(R³)₂]ₙHét¹, COHét¹, COA et/ou =O (oxygène du carbonyle),
R³ désigne H ou A,
R⁴, R⁵ désignent chacun, indépendamment l'un de l'autre, H, Hal, A, OR³, CN, COOR³, CON(R³)₂, NR³COA, NR³SO₂A, SO₂N(R³)₂ ou S(O)ₘA,
A désigne alkyle non ramifié ou ramifié ayant 1-10 atomes de C,
où 1-7 atomes de H peuvent être remplacés par F, Cl et/ou Br,
et/ou où un ou deux groupements CH₂ peuvent être remplacés par des groupements O, S, SO, SO₂ et/ou CH=CH,
ou
alkyle cyclique ayant 3-7 atomes de C,
Ar désigne phényle, naphtyle ou biphényle, chacun d'entre eux étant non substitué ou mono-, di- ou trisubstitué par Hal, A, OR³, N(R³)₂, SR³, NO₂, CN, COOR³, CON(R³)₂, NR³COA, NR³SO₂A, SO₂N(R³)₂, S(O)ₘA, CO-Hét¹, Hét¹, O[C(R³)₂]ₙN(R³)₂, O[C(R³)₂]ₙHét¹, NHCOOA, NHCON(R³)₂, NHCOO[C(R³)₂]ₙN(R³)₂, NHCOO[C(R³)₂]ₙHét¹, NHCONH[C(R³)₂]ₙN(R³)₂, NHCONH[C(R³)₂]ₙHét¹, OCONH[C(R³)₂]ₙN(R³)₂, OCONH[C(R³)₂]ₙHét¹ et/ou COA,
Hét désigne un hétérocycle mono-, bi- ou tricyclique saturé, insaturé ou aromatique ayant de 1 à 4 atomes de N, O et/ou S, qui peut être non substitué ou mono-, di- ou trisubstitué par Hal, A, OR³, N(R³)₂, SR³, NO₂, CN, COOR³, CON(R³)₂, NR³COA, NR³SO₂A, SO₂N(R³)₂, S(O)ₘA, CO-Hét¹, Hét¹, O[C(R³)₂]ₙN(R³)₂, O(C(R³)₂]ₙHét¹, NHCOOA, NHCON(R³)₂, NHCOO[C(R³)₂]ₙN(R³)₂, NHCOO[C(R³)₂]ₙHét¹, NHCONH[C(R³)₂]ₙN(R³)₂, NHCONH[C(R³)₂]ₙHét¹, OCONH[C(R³)₂]ₙN(R³)₂, OCONH[C(R³)₂]ₙHét¹, CO-Hét¹, CHO, COA, =S, =NH, =NA et/ou =O (oxygène du carbonyle),
Hét¹ désigne un hétérocycle monocyclique saturé ou aromatique ayant de 1 à 2 atomes de N et/ou O, qui peut être mono- ou disubstitué par A, OA, OH, Hal, (CH₂)ₙN(R³)2, (CH₂)ₙOR³, (CH₂)ₙHét² et/ou =O (oxygène du carbonyle),
Hét² désigne pyrrolidino, pipéridino ou morpholino,
Hal désigne F, Cl, Br ou I,
m désigne 0, 1 ou 2,
n désigne 1, 2, 3 ou 4,
et les solvats, sels, tautomères et stéréoisomères pharmaceutiquement utilisables de ceux-ci, y compris des mélanges de ceux-ci en toutes proportions.

2. Composés selon la revendication 1, dans lesquels
A désigne alkyle non ramifié ou ramifié ayant 1-10 atomes de C,
où 1-7 atomes de H peuvent être remplacés par F et/ou Cl,
et les solvats, sels, tautomères et stéréoisomères pharmaceutiquement utilisables de ceux-ci, y compris des mélanges de ceux-ci en toutes proportions.

3. Composés selon la revendication 1 ou 2, dans lesquels
Ar désigne phényle qui est non substitué ou mono-, di- ou trisubstitué par Hal, CN et/ou S(O)ₘA,
et les solvats, sels, tautomères et stéréoisomères pharmaceutiquement utilisables de ceux-ci, y compris des mélanges de ceux-ci en toutes proportions.

4. Composés selon l'une ou plusieurs parmi les revendications 1-3, dans lesquels
Hét désigne un hétérocycle mono- ou bicyclique aromatique ayant de 1 à 3 atomes de N, O et/ou S, qui peut être non substitué ou mono-, di- ou trisubstitué par Hal,
et les solvats, sels, tautomères et stéréoisomères pharmaceutiquement utilisables de ceux-ci, y compris des mélanges de ceux-ci en toutes proportions.

5. Composés selon l'une ou plusieurs parmi les revendications 1-4, dans lesquels
Hét désigne thiazolyle, thiényle, pyridyle, benzo-1,2,5-thiadiazolyle ou benzo-1,3-dioxolyle,
et les solvats, sels, tautomères et stéréoisomères pharmaceutiquement utilisables de ceux-ci, y compris des mélanges de ceux-ci en toutes proportions.

6. Composés selon l'une ou plusieurs parmi les revendications 1-5, dans lesquels
R² désigne un hétérocycle de 5 chaînons saturé, insaturé ou aromatique ayant de 1 à 4 atomes de N, O et/ou S, qui peut être non substitué ou mono- ou disubstitué par Hal, A, (CH₂)ₙOR³, N(R³)₂, SR³, Hét¹, -[C(R³)₂]ₙN(R³)₂, -[C(R³)₂]ₙHét¹, S[C(R³)₂]ₙN(R³)₂, -NR³[C(R³)₂]ₙN(R³)₂, -NR³[C(R³)₂]ₙHét¹, -NR³Hét¹, COHét¹ et/ou =O (oxygène du carbonyle),
et les solvats, sels, tautomères et stéréoisomères pharmaceutiquement utilisables de ceux-ci, y compris des mélanges de ceux-ci en toutes proportions.

7. Composés selon l'une ou plusieurs parmi les revendications 1-6, dans lesquels
R² désigne un hétérocycle choisi dans le groupe constitué par 1,2,4-oxadiazolyle, 1,3,4-oxadiazolyle, oxazolyle, dihydro-oxazolyle, pyrazolyle, imidazolyle, triazolyle, tétrazolyle, pyrrolyle, furanyle, thiényle, thiazolyle, dihydrothiazolyle, thiadiazolyle, oxazolidinyle, pyrrolidinyle, pipéridinyle,
qui peut être mono- ou disubstitué par Hal, A, (CH₂)ₙOR³, N(R³)₂, SR³, Hét¹, -[C(R³)₂]ₙN(R³)₂, -[C(R³)₂]ₙHét¹, S[C(R³)₂]ₙN(R³)₂, -NR³[C(R³)₂]ₙN(R³)₂, -NR³[C(R³)₂]ₙHét¹, -NR³Hét¹, COHét¹ et/ou =O (oxygène du carbonyle),
et les solvats, sels, tautomères et stéréoisomères pharmaceutiquement utilisables de ceux-ci, y compris des mélanges de ceux-ci en toutes proportions.

8. Composés selon l'une ou plusieurs parmi les revendications 1-7, dans lesquels
R⁴, R⁵ désignent H,
et les solvats, sels, tautomères et stéréoisomères pharmaceutiquement utilisables de ceux-ci, y compris des mélanges de ceux-ci en toutes proportions.

9. Composés selon l'une ou plusieurs parmi les revendications 1-8, dans lesquels
R³ désigne H, méthyle, éthyle, propyle, isopropyle, butyle ou tertio-butyle,
et les solvats, sels, tautomères et stéréoisomères pharmaceutiquement utilisables de ceux-ci, y compris des mélanges de ceux-ci en toutes proportions.

10. Composés selon l'une ou plusieurs parmi les revendications 1-9, dans lesquels
R¹ désigne 4-fluorophényle, 3,5-difluorophényle, 3,4-difluorophényle, 3,4,5-trifluorophényle, 2-chlorophényle, 3-chlorophényle, 3-cyanophényle, 4-cyanophényle, 4-méthylsulfonylphényle, pyridyle, benzo-1,3-dioxolyle ou benzo-1,2,5-thiadiazolyle,
et les solvats, sels, tautomères et stéréoisomères pharmaceutiquement utilisables de ceux-ci, y compris des mélanges de ceux-ci en toutes proportions.

11. Composés selon l'une ou plusieurs parmi les revendications 1-10, dans lesquels
Hét¹ désigne un hétérocycle monocyclique saturé ou aromatique ayant de 1 à 2 atomes de N et/ou O, qui peut être mono- ou disubstitué par A, (CH₂)ₙHét², (CH₂)ₙN(R³)₂ et/ou (CH₂)ₙOR³,
et les solvats, sels, tautomères et stéréoisomères pharmaceutiquement utilisables de ceux-ci, y compris des mélanges de ceux-ci en toutes proportions.

12. Composés selon l'une ou plusieurs parmi les revendications 1-11, dans lesquels
Hét¹ désigne pipéridin-1-yle, pyrrolidin-1-yle, morpholin-4-yle, pipérazin-1-yle, 1,3-oxazolidin-3-yle, imidazolidinyle, oxazolyle, thiazolyle, thiényle, 1,4-diazépanyle, furanyle ou pyridyle, où les radicaux peuvent également être mono- ou disubstitués par A, (CH₂)ₙHét², (CH₂)ₙN(R³)₂ et/ou (CH₂)ₙOR³,
et les solvats, sels, tautomères et stéréoisomères pharmaceutiquement utilisables de ceux-ci, y compris des mélanges de ceux-ci en toutes proportions.

13. Composés selon l'une ou plusieurs parmi les revendications 1-12, dans lesquels
R¹ désigne Ar ou Hét,
R² désigne un hétérocycle de 5 chaînons saturé, insaturé ou aromatique ayant de 1 à 4 atomes de N, O et/ou S, qui peut être non substitué ou mono- ou disubstitué par Hal, A, (CH₂)ₙOR³, N(R³)₂, SR³, Hét¹, -[C(R³)₂]ₙN(R³)₂, -[C(R³)₂]ₙHét¹, S[C(R³)₂]ₙN(R³)₂, -NR³[C(R³)₂]ₙN(R³)₂, -NR³[C(R³)₂]ₙHét¹, -NR³Hét¹, COHét¹ et/ou =O (oxygène du carbonyle),
R³ désigne H ou A,
R⁴, R⁵ désignent H,
A désigne alkyle non ramifié ou ramifié ayant 1-10 atomes de C, où 1-7 atomes de H peuvent être remplacés par F et/ou Cl,
Ar désigne phényle qui est non substitué ou mono-, di- ou trisubstitué par Hal, CN et/ou S(O)ₘA,
Hét désigne un hétérocycle mono- ou bicyclique aromatique ayant de 1 à 3 atomes de N, O et/ou S, qui peut être non substitué ou mono-, di- ou trisubstitué par Hal,
Hét¹ désigne un hétérocycle monocyclique saturé ou aromatique ayant de 1 à 2 atomes de N et/ou O, qui peut être mono- ou disubstitué par A, (CH₂)ₙHét², (CH₂)ₙN(R³)₂ et/ou (CH₂)ₙOR³,
Hét² désigne pyrrolidino, pipéridino ou morpholino,
Hal désigne F, Cl, Br ou I,
m désigne 0, 1 ou 2,
n désigne 1, 2, 3 ou 4,
et les solvats, sels, tautomères et stéréoisomères pharmaceutiquement utilisables de ceux-ci, y compris des mélanges de ceux-ci en toutes proportions.

14. Composés selon l'une ou plusieurs parmi les revendications 1-13, dans lesquels
R¹ désigne Ar ou Hét,
R² désigne un hétérocycle choisi dans le groupe constitué par 1,2,4-oxadiazolyle, 1,3,4-oxadiazolyle, oxazolyle, dihydro-oxazolyle, pyrazolyle, imidazolyle, triazolyle, thiadiazolyle, tétrazolyle, pyrrolyle, furanyle, thiényle, thiazolyle, dihydrothiazolyle, oxazolidinyle, qui peut être mono- ou disubstitué par Hal, A, (CH₂)ₙOR³, N(R³)₂, SR³, Hét¹, -(C(R³)₂]ₙN(R³)₂, -[C(R³)₂]ₙHét¹, S[C(R³)₂]ₙN(R³)₂, -NR³[C(R³)₂]ₙN(R³)₂, -NR³[C(R³)₂]ₙHét¹, -COHét¹ et/ou =O (oxygène du car bonyle),
R³ désigne H, méthyle, éthyle, propyle, isopropyle, butyle ou tertio-butyle,
R⁴, R⁵ désignent H,
A désigne alkyle non ramifié ou ramifié ayant 1-10 atomes de C, où 1-7 atomes de H peuvent être remplacés par F et/ou Cl,
Ar désigne phényle qui est non substitué ou mono-, di- ou trisubstitué par Hal, CN et/ou S(O)ₘA,
Hét désigne thiazolyle, thiényle, pyridyle, benzo-1,2,5-thiadiazolyle ou benzo-1,3-dioxolyle,
Hét¹ désigne pipéridin-1-yle, pyrrolidin-1-yle, morpholin-4-yle, pipérazin-1-yle, 1,3-oxazolidin-3-yle, imidazolidinyle, oxazolyle, thiazolyle, thiényle, 1,4-diazépanyle, furanyle ou pyridyle, où les radicaux peuvent également être mono- ou disubstitués par A, (CH₂),Hét ² (CH₂)ₙN(R³)₂ et/ou (CH₂)ₙOR³,
Hét² désigne pyrrolidino, pipéridino ou morpholino,
Hal désigne F, Cl, Br ou I,
m désigne 0, 1 ou 2,
n désigne 1, 2, 3 ou 4,
et les solvats, sels, tautomères et stéréoisomères pharmaceutiquement utilisables de ceux-ci, y compris des mélanges de ceux-ci en toutes proportions.

15. Composés selon la revendication 1, choisis dans le groupe constitué par
| n° | Structure et/ou nom |
|---|---|
| "A1" | 6-(3,5-Difluorophényl)-2-[3-(5-méthyl-1,2,4-oxadiazol-3-yl)-benzyl]-2*H*-pyridazin-3-one |
| "A2" | 2-[3-(5-Méthyl-1,2,4-oxadiazol-3-yl)benzyl]-6-(3,4,5-trifluorophényl)-2*H*-pyridazin-3-one |
| "A3" | 6-(3,5-Difluorophényl)-2-[4-(5-méthyl-1,2,4-oxadiazol-3-yl)benzyl]-2*H*-pyridazin-3-one |
| "A4" | 6-(4-Fluorophényl)-2-[3-(5-méthyl-1,2,4-oxadiazol-3-yl)-benzyl]-2*H*-pyridazin-3-one |
| "A5" | 2-[3-(5-Méthyl-1,2,4-oxadiazol-3-yl)benzyl]-6-pyridin-3-yl-2*H-*pyridazin-3-one |
| | 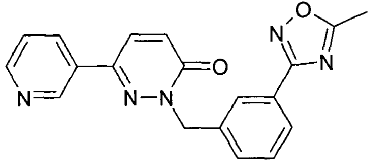 |
| "A6" | 6-(3-Chlorophényl)-2-[3-(5-méthyl-1,2,4-oxadiazol-3-yl)-benzyl]-2*H*-pyridazin-3-one |
| "A7" | 6-(3-Cyanophényl)-2-[3-(5-méthyl-1,2,4-oxadiazol-3-yl)-benzyl]-2*H*-pyridazin-3-one |
| "A8" | 6-(4-Cyanophényl)-2-[3-(5-méthyl-1,2,4-oxadiazol-3-yl)-benzyl]-2*H*-pyridazin-3-one |
| "A9" | 6-(4-Méthylsulfonylphényl)-2-[3-(5-méthyl-1,2,4-oxadiazol-3-yl)benzyl]-2*H*-pyridazin-3-one |
| "A10" | 6-(3,5-Difluorophényl)-2-[3-(5-diméthylaminométhyl-1,2,4-oxadiazol-3-yl)benzyl]-2*H*-pyridazin-3-one |
| "A11" | 6-(3,5-Difluorophényl)-2-[3-(5-éthylamino-1,2,4-oxadiazol-3-yl)benzyl]-2*H*-pyridazin-3-one |
| "A12" | 6-(3,5-Difluorophényl)-2-{3-[5-(3-diméthylaminopropyl-amino)-1,2,4-oxadiazol-3-yl]benzyl}-2*H*-pyridazin-3-one |
| "A13" | 6-(3,5-Difluorophényl)-2-[3-(5-méthyloxazol-2-yl)benzyl]-2H-pyridazin-3-one |
| "A14" | 6-(3,5-Difluorophényl)-2-[3-(3-méthyl-1,2,4-oxadiazol-5-yl)benzyl]-2*H*-pyridazin-3-one |
| "A15" | 6-(3,5-Difluorophényl)-2-[3-(5-oxo-4,5-dihydro-1,3,4-oxadiazol-2-yl)benzyl]-2*H*-pyridazin-3-one |
| "A16" | 6-(3,5-Difluorophényl)-2-{3-[5-(2-morpholin-4-yléthylamino)-1,3,4-oxadiazol-2-yl]benzyl}-2*H*-pyridazin-3-one |
| "A17" | 6-(3,5-Difluorophényl)-2-[3-(5-oxo-4,5-dihydro-1,2,4-oxadiazol-3-yl)benzyl]-2*H*-pyridazin-3-one |
| "A18" | 6-(3,5-Difluorophényl)-2-[3-(5-thioxo-4,5-dihydro-1,2,4-oxadiazoi-3-yl)benzyl]-2*H*-pyridazin-3-one |
| "A19" | 6-(3,5-Difluorophényl)-2-{3-[5-(3-diméthylaminopropyl-sulfanyl)-1,2,4-oxadiazol-3-yl]benzyl}-2*H*-pyridazin-3-one |
| "A20" | 6-(3,5-Difluorophényl)-2-{3-[5-(4-méthylpipérazin-1-yl)-1,2,4-oxadiazol-3-yl]benzyl}-2*H*-pyridazin-3-one |
| "A21" | 6-(3,5-Difluorophényl)-2-{3-[5-(4-méthylpipérazine-1-carbonyl)-1,2,4-oxadiazol-3-yl]benzyl}-2*H*-pyridazin-3-one |
| "A22" | 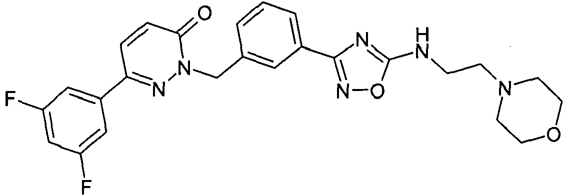 |
| "A23" | 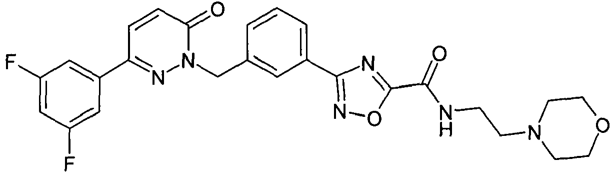 |
| "A24" | 6-(3,5-Difluorophényl)-2-[3-(5-pipérazin-1-yl-1,2,4-oxadiazol-3-yl)benzyl]-2*H*-pyridazin-3-one |
| "A25" | 6-(3,5-Difluorophényl)-2-{3-[4-(3-diméthylaminopropyl)-5-oxo-4,5-dihydro-1,2,4-oxadiazol-3-yl]benzyl}-2*H*-pyridazin-3-one |
| "A26" | 6-(3,5-Difluorophényl)-2-[3-(1*H*-tétrazol-5-yl)benzyl]-2*H-*pyridazin-3-one |
| "A27" | 6-(3,5-Difluorophényl)-2-{3-[5-(2-diméthylaminoéthylamino)-1,2,4-oxadiazol-3-yl]benzyl}-2*H*-pyridazin-3-one |
| "A28" | 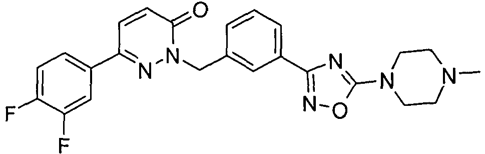 |
| "A29" | 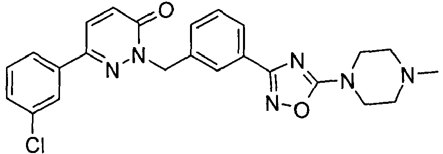 |
| "A30" | 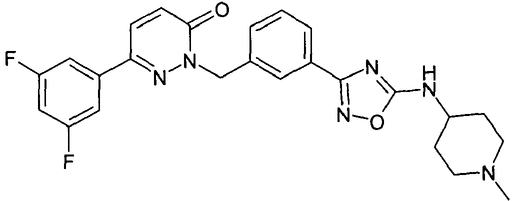 |
| "A31" | 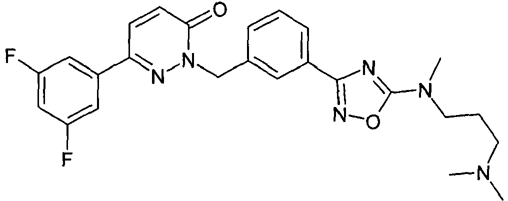 |
| "A32" | 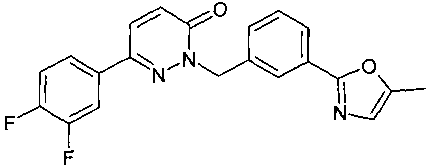 |
| "A33" | 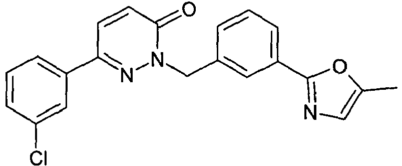 |
| "A34" | 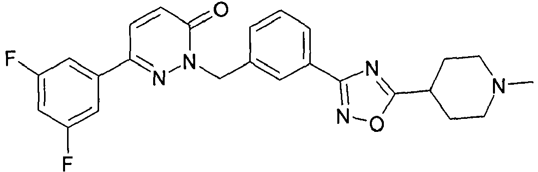 |
| "A35" | 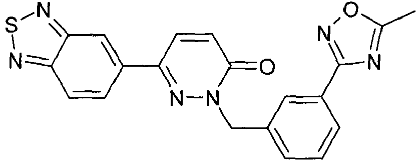 |
| "A36" | 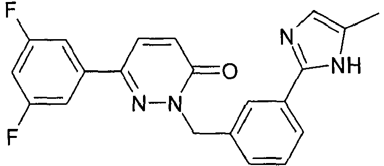 |
| "A37" | 6-(3,5-Difluorophényl)-2-{3-[5-(4-méthylpipérazin-1-yl)-oxazol-2-yl]benzyl}-2*H*-pyridazin-3-one |
| "A38" | 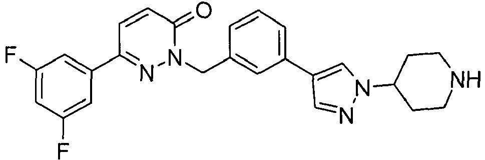 |
| "A39" | 6-(3,4-Difluorophényl)-2-[3-(5-méthyl-1,2,4-oxadiazol-3-yl)-benzyl]-2*H*-pyridazin-3-one |
| | |
| "A41" | 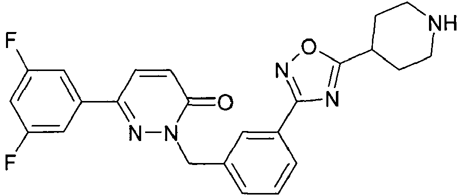 |
| "A42" | 6-(3,5-Difluorophényl)-2-{3-[5-(pipéridin-4-ylamino)-1,2,4-oxadiazol-3-yl]benzyl}-2*H*-pyridazin-3-one |
| | 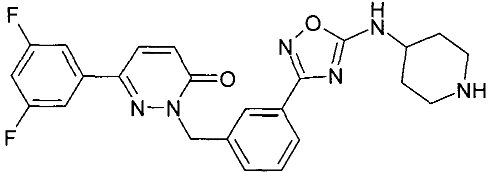 |
| "A43" | 2-{3-[5-(1-Méthylpipéridin-4-ylamino)-1,2,4-oxadiazol-3-yl]benzyl}-6-(3,4,5-trifluorophényl)-2*H-*pyridazin-3-one |
| | 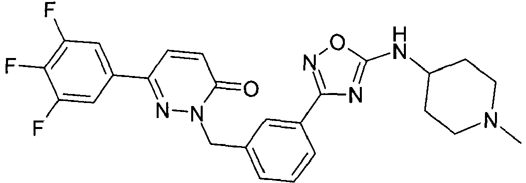 |
| "A44" | 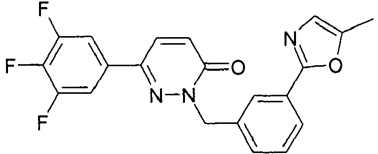 |
| "A45" | 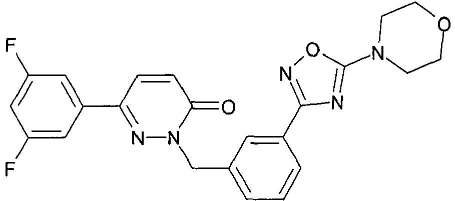 |
| "A46" | 6-(3,5-Difluorophényl)-2-(3-{5-[méthyl-(1-méthylpipéridin-4-yl)amino]-1,2,4-oxadiazol-3-yl}benzyl)-2*H*-pyridazin-3-one |
| | 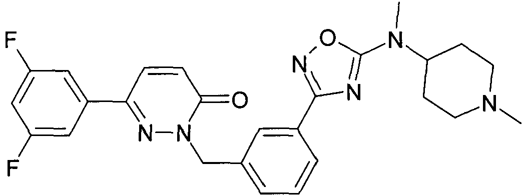 |
| "A47" | 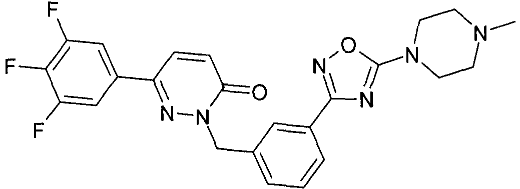 |
| "A48" | 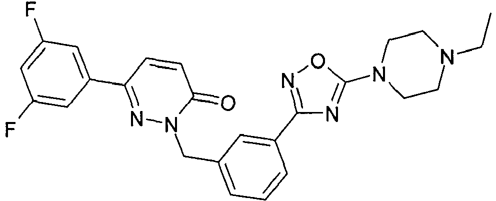 |
| "A49" | 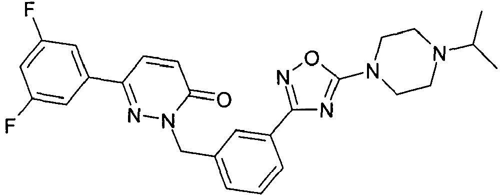 |
| "A50" | 6-(3,5-Difluorophényl)-2-(3-{5-[4-(2-méthoxyéthyl)pipérazin-1-yl]-1,2,4-oxadiazol-3-yl}benzyl)-2H-pyridazin-3-one |
| | 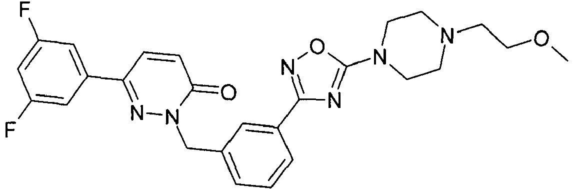 |
| "A51" | 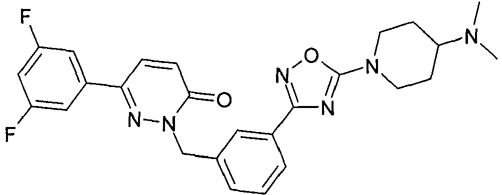 |
| "A52" | 6-(3,5-Difluorophényl)-2-(3-{5-[4-(2-diméthylaminoéthyl)-pipérazin-1-yl]-1,2,4-oxadiazol-3-yl}benzyl)-2*H*-pyridazin-3-one |
| | 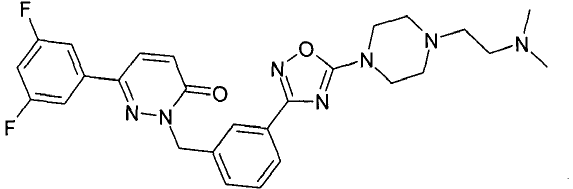 |
| "A53" | 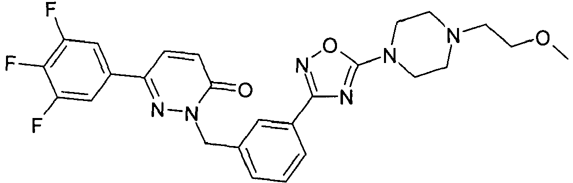 |
| "A54" | 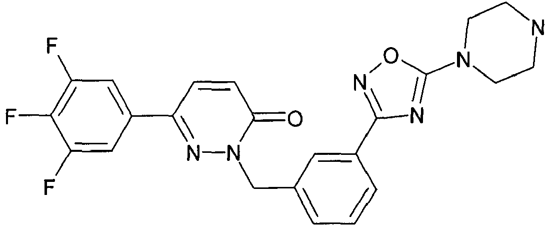 |
| "A55" | 6-(3,5-Difluorophényl)-2-[3-(5-oxo-4-pipéridin-4-yl-4,5-dihydro-1,2,4-oxadiazol-3-yl)benzyl]-2*H*-pyridazin-3-one |
| | 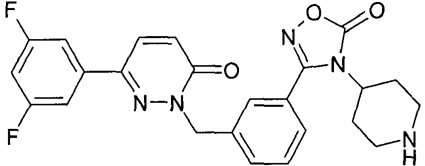 |
| "A56" | 6-(3,5-Difluorophényl)-2-[3-(5-hydroxyméthyl-1*H-*imidazol-2-yl)benzyl]-2*H*-pyridazin-3-one |
| | 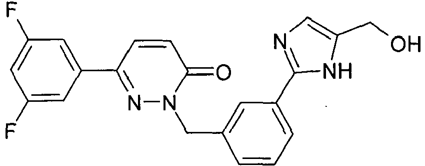 |
| "A57" | 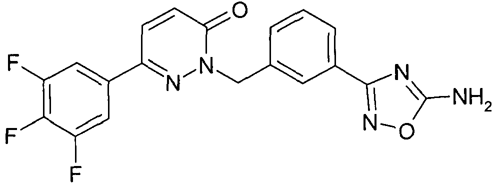 |
| "A58" | 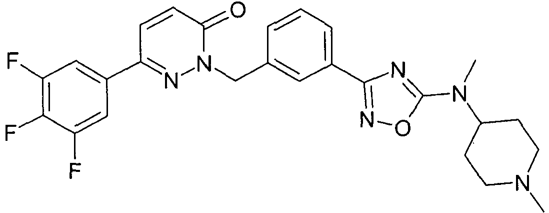 |
| "A59" | 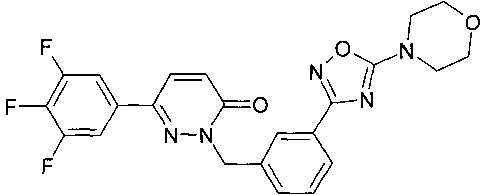 |
| "A60" | 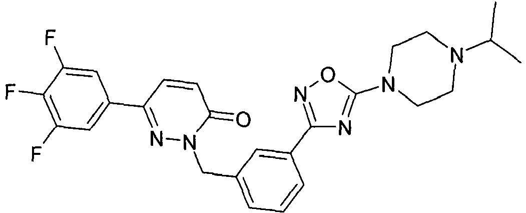 |
| "A61" | 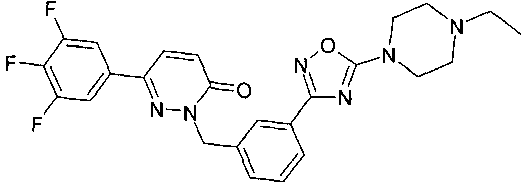 |
| "A62" | 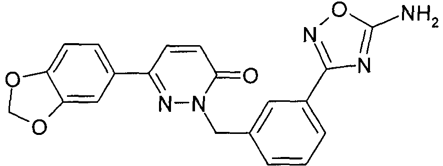 |
| "A63" | 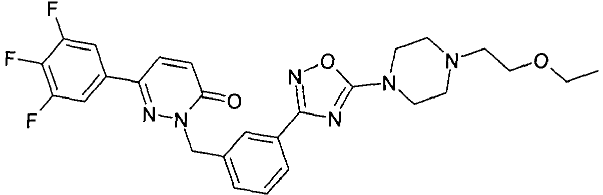 |
| "A64" | 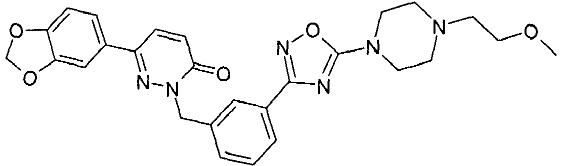 |
| "A65" | 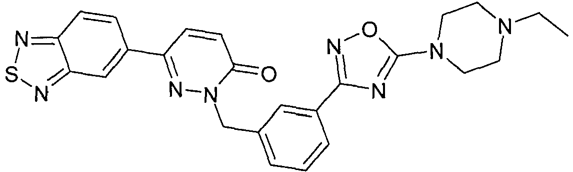 |
| "A66" | 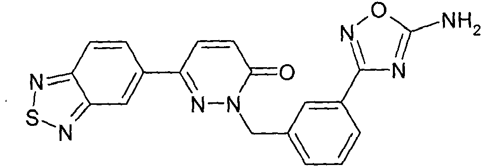 |
| "A67" | 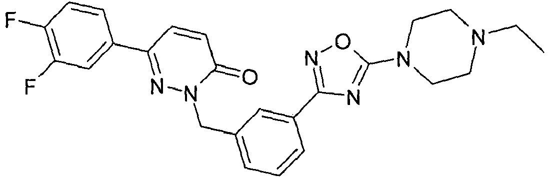 |
| "A68" | 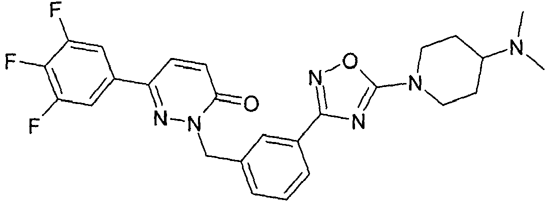 |
| "A69" | 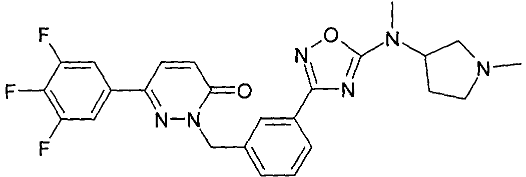 |
| "A70" | 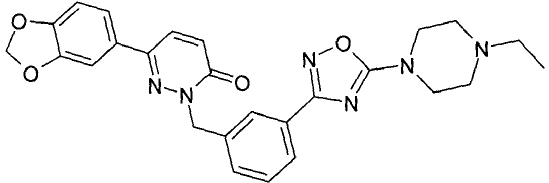 |
| "A71a" | 2-(3-{5-[(1-Éthylpipéridin-4-yl)méthylamino]-1,2,4-oxadiazol-3-yl}benzyl)-6-(3,4,5-trifluorophényl)-2*H*-pyridazin-3-one |
| | 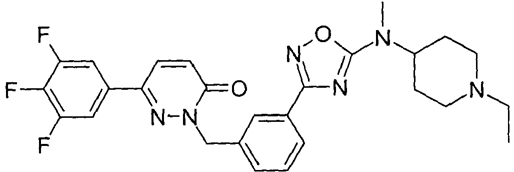 |
| "A71b" | 6-(3,5-Difluorophényl)-2-(3-{5-[(1-éthylpipéridin-4-yl)-méthylamino]-1,2,4-oxadiazol-3-yl}benzyl)-2*H*-pyridazin-3-one |
| | 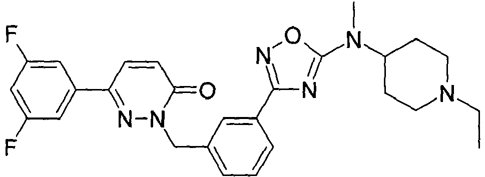 |
| "A71 c" | 6-(3,5-Difluorophényl)-2-(3-{5-[méthyl(1-méthylpipéridin-4-yl-méthyl)amino]-1,2,4-oxadiazol-3-yl}benzyl)-2*H*-pyridazin-3-one |
| | 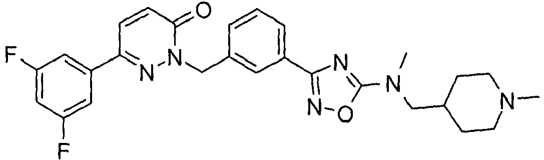 |
| "A72" | 2-[3-(5-Amino-1,2,4-oxadiazol-3-yl)benzyl]-6-(3,5-difluorophényl)-2*H*-pyridazin-3-one |
| | 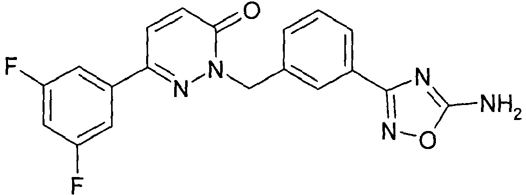 |
| "A73" | 6-(3,5-Difluorophényl)-2-{3-[5-(méthylpipéridin-4-ylamino)-1,2,4-oxadiazol-3-yl]benzyl}-2*H*-pyridazin-3-one |
| | 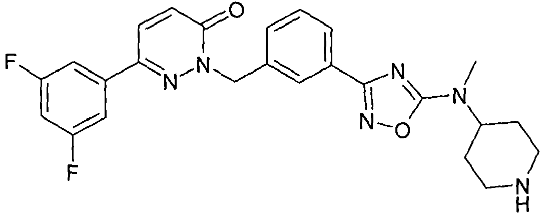 |
| "A74" | 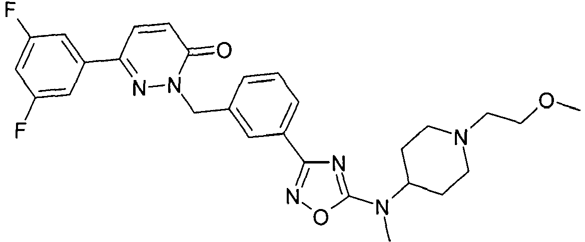 |
| "A75" | 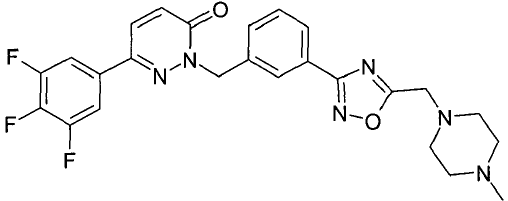 |
| "A76" | 2-[3-(5-Trifluorométhyl-1,2,4-oxadiazol-3-yl)benzyl]-6-(3,4,5-trifluorophényl)-2*H*-pyridazin-3-one |
| "A77" | 2-(3-1,2,4-Oxadiazol-3-ylbenzyl)-6-(3,4,5-trifluorophényl)-2*H*-pyridazin-3-one |
| "A78" | 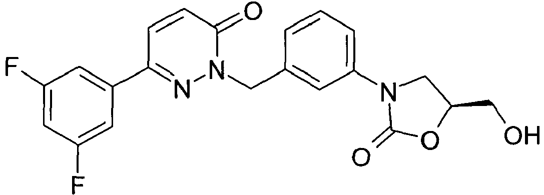 |
| "A79" | 6-(3,5-Difluorophényl)-2-[3-((R)-5-hydroxyméthyl-2-oxo-oxazolidin-3-yl)benzyl]-2*H*-pyridazin-3-one |
| "A80" | 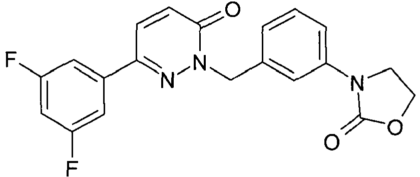 |
| "A81" | 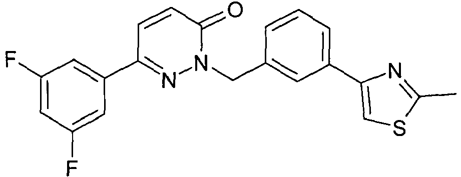 |
| "A82" | 6-(3-Cyanophényl)-2-[3-(2-méthylthiazol-4-yl)benzyl]-2*H-*pyridazin-3-one |
| "A83" | 6-(3,5-Difluorophényl)-2-[3-(1*H*-imidazol-2-yl)benzyl]-2*H-*pyridazin-3-one |
| "A84" | 6-(3,5-Difluorophényl)-2-[3-(5-méthylthiazol-2-yl)benzyl]-2*H-*pyridazin-3-one |
| | 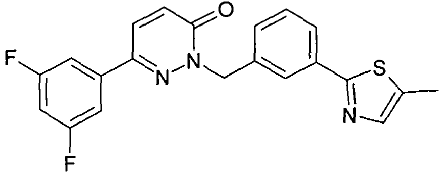 |
| "A85" | 6-(3,5-Difluorophényl)-2-[3-(5-méthyl-4,5-dihydrooxazol-2-yl)benzyl]-2*H*-pyridazin-3-one |
| | 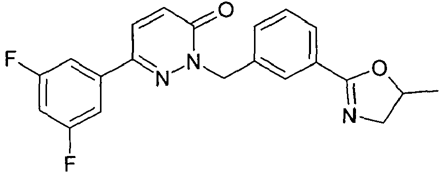 |
| "A86" | 6-(3-Chlorophényl)-2-[3-(5-méthylthiazol-2-yl)benzyl]-2*H-*pyridazin-3-one |
| "A87" | 6-(3,5-Difluorophényl)-2-[3-(5-pipéridin-4-yloxazol-2-yl)benzyl]-2*H*-pyridazin-3-one |
| "A88" | 6-(3,5-Difluorophényl)-2-{3-[5-(1-méthylpipéridin-4-yl)-oxazol-2-yl]benzyl}-2*H*-pyridazin-3-one |
| "A89" | 2-[3-(5-Pipéridin-4-yloxazol-2-yl)benzyl]-6-(3,4,5-trifluorophényl)-2*H*-pyridazin-3-one |
| | 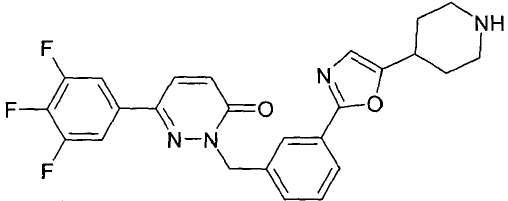 |
| "A90" | 2-[3-(5-Pipéridin-4-yloxazol-2-yl)benzyl]-6-(3-chlorophényl)-2*H*-pyridazin-3-one |
| | 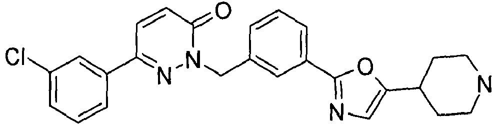 |
| "A91" | 6-(3,5-Difluorophényl)-2-[3-(5-pipéridin-4-ylthiazol-2-yl)benzyl]-2*H*-pyridazin-3-one |
| | 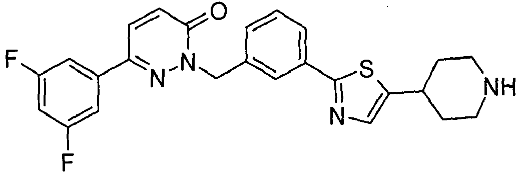 |
| "A92" | 6-(3,5-Difluorophényl)-2-{3-[5-(1-méthylpipéridin-4-yl)thiazol-2-yl]benzyl}-2*H*-pyridazin-3-one |
| | 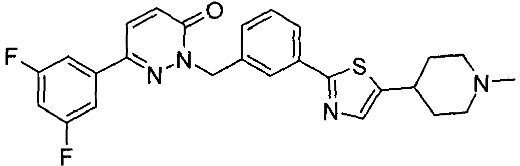 |
| "A93" | 2-[3-(5-Pipéridin-4-ylthiazol-2-yl)benzyl]-6-(3,4,5-trifluorophényl)-2*H*-pyridazin-3-one |
| "A94" | 6-(3-Chlorophényl)-2-[3-(5-pipéridin-4-ylthiazol-2-yl)benzyl]-2H-pyridazin-3-one |
| "A95" | 6-(3-Chlorophényl)-2-{3-[5-(4-méthylpipérazin-1-yl-méthyl)thiazol-2-yl]benzyl}-2*H*-pyridazin-3-one |
| "A96" | 6-(3,5-Difluorophényl)-2-[3-(5-pipéridin-3-ylthiazol-2-yl)benzyl]-2*H*-pyridazin-3-one |
| "A97" | 2-[3-(5-Azétidin-3-ylthiazol-2-yl)benzyl]-6-(3,5-difluoro-phényl)-2H-pyridazin-3-one |
| | 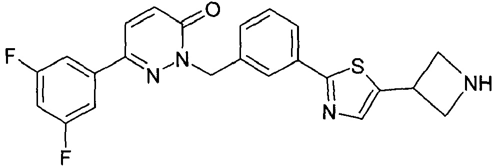 |
| "A98" | 6-(3,5-Difluorophényl)-2-[3-(5-pipéridin-4-ylméthylthiazol-2-yl)benzyl]-2*H*-pyridazin-3-one |
| "A99" | 6-(3,5-Difluorophényl)-2-(3-{5-[1-(2-méthoxyéthyl)-pipéridin-4-yl]thiazol-2-yl}benzyl)-2*H*-pyridazin-3-one |
| | 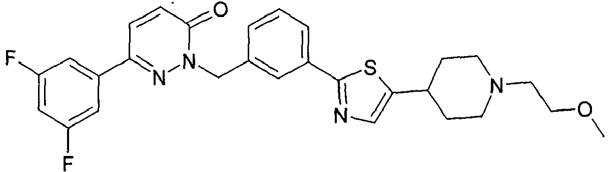 |
| "A99a" | 6-(3,5-Difluorophényl)-2-{3-[5-(1-méthylpipéridin-4-yl-méthyl)thiazol-2-yl]benzyl}-2*H*-pyridazin-3-one |
| "A100" | 6-(3,5-Difluorophényl)-2-[3-(5-pyrrolidin-2-ylthiazol-2-yl)benzyl]-2*H*-pyridazin-3-one |
| "A101" | 6-(3,5-Difluorophényl)-2-{3-[5-(4-méthylpipérazin-1-yl-méthyl)thiazol-2-yl]benzyl}-2*H*-pyridazin-3-one |
| "A102" | 6-(3,5-Difluorophényl)-2-[3-(5-morpholin-4-ylméthylthiazol-2-yl)benzyl]-2*H*-pyridazin-3-one |
| "A103" | 6-(3,5-Difluorophényl)-2-[3-(4-méthylthiazol-2-yl)benzyl]-2*H-*pyridazin-3-one |
| "A104" | 6-(3,5-Difluorophényl)-2-(3-thiazol-2-ylbenzyl)-2*H*-pyridazin-3-one |
| "A105" | 6-(3,5-Difluorophényl)-2-{3-[1-(2-pipéridin-1-yléthyl)-1*H-*1,2,3-triazol-4-yl]benzyl}-2*H*-pyridazin-3-one |
| | 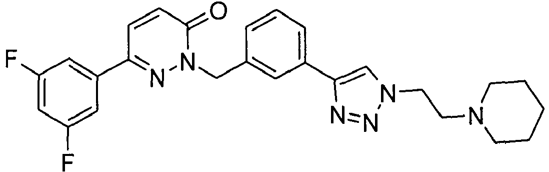 |
| "A106" | 6-(3,5-Difluorophényl)-2-{3-[1-(2-morpholin-4-yléthyl)-1*H-*1,2,3-triazol-4-yl]benzyl}-2*H*-pyridazin-3-one |
| "A107" | 6-(3,5-Difluorophényl)-2-{3-[5-(4-méthylpipérazine-1-carbonyl)isoxazol-3-yl]benzyl}-2*H*-pyridazin-3-one |
| | 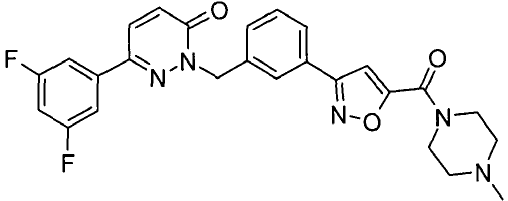 |
| "A108" | 6-(3,5-Difluorophényl)-2-[3-(5-oxo-4,5-dihydro-1,2,4-thiadiazol-3-yl)benzyl]-2*H*-pyridazin-3-one |
| | 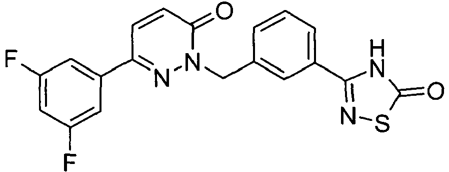 |
| "A109" | 6-(3,5-Difluorophényl)-2-{3-[5-(4-méthylpipérazin-1-yl)-1,2,4-thiadiazol-3-yl]benzyl}-2*H*-pyridazin-3-one |
| "A109a" | 6-(3,5-Difluorophényl)-2-(3-{5-[4-(2-méthoxyéthyl)pipérazin-1-yl]-1,2,4-thiadiazol-3-yl}benzyl)-2*H*-pyridazin-3-one |
| | 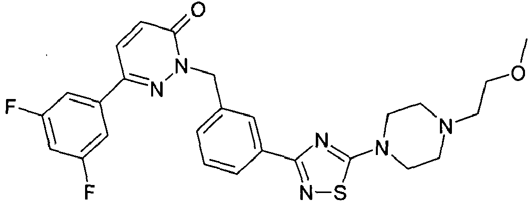 |
| "A110" | 6-(3,5-Difluorophényl)-2-[3-(5-morpholin-4-ylméthyloxazol-2-yl)benzyl]-2*H*-pyridazin-3-one |
| "A111" | 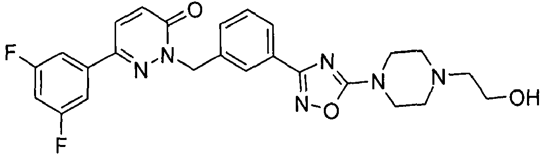 |
| "A112" | 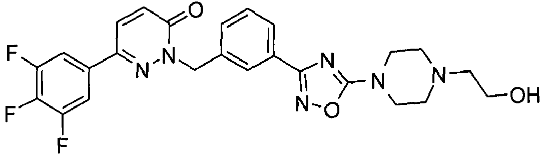 |
| "A113" | 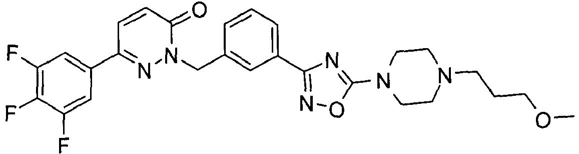 |
| "A114" | 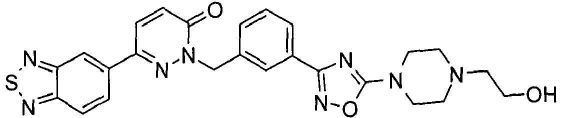 |
| "A115" | 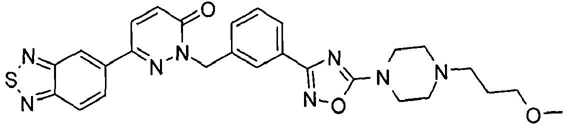 |
| "A116" | 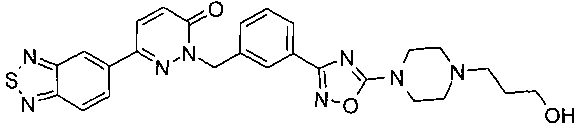 |
| "A117" | 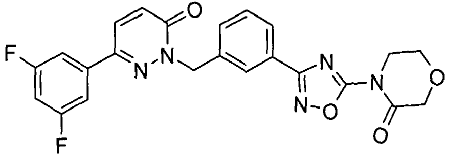 |
| "A118" | 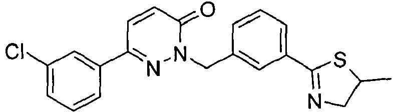 |
| "A119" | 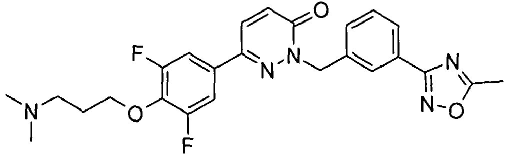 |
| "A120" | 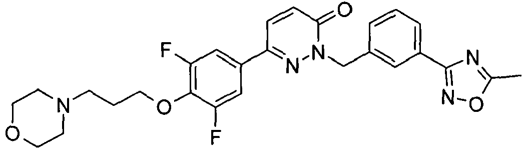 |
| "A121" | 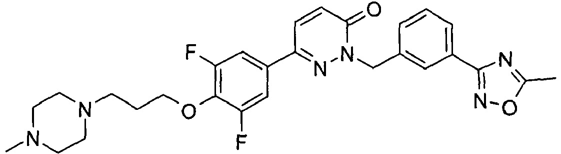 |
| "A122" | 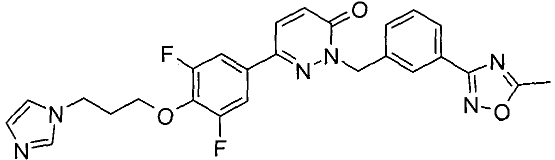 |
| "A123" | 6-(3,5-Difluorophényl)-2-(3-{5-[4-(2-éthoxyéthyl)pipérazin-1-yl]-1,2,4-oxadiazol-3-yl}benzyl)-2*H*-pyridazin-3-one |
| | 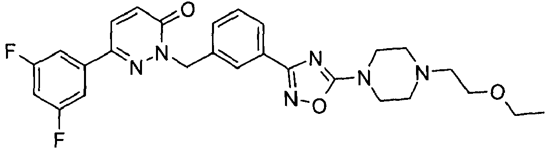 |
| "A124" | 6-(3,5-Difluorophényl)-2-(3-{5-[4-(3-méthoxypropyl)-pipérazin-1-yl]-1,2,4-oxadiazol-3-yl}benzyl)-2H-pyridazin-3-one |
| | 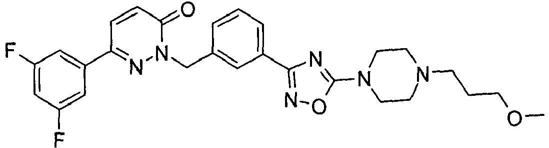 |
| "A125" | 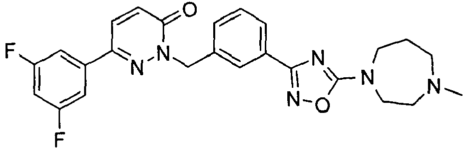 |
| "A126" | 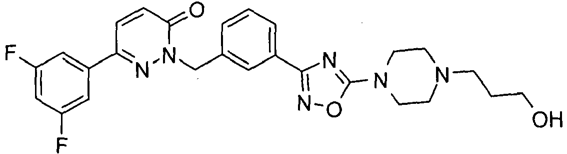 |
| "A127" | 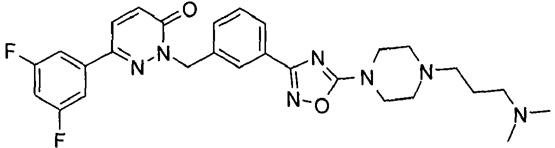 |
| "A128" | 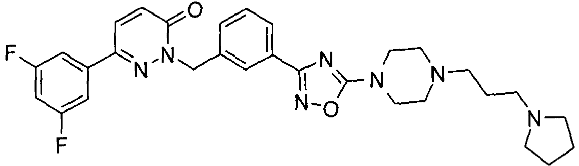 |
| "A129" | 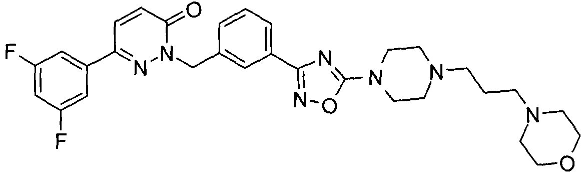 |
| "A130" | 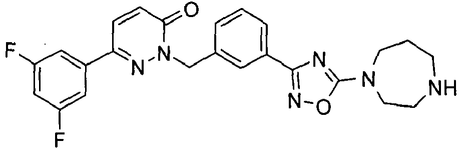 |
| "A131" | 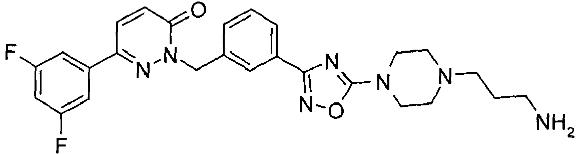 |
et les solvats, sels, tautomères et stéréoisomères pharmaceutiquement utilisables de ceux-ci, y compris des mélanges de ceux-ci en toutes proportions.

16. Procédé de préparation de composés de formule I selon les revendications 1-15 et de dérivés, sels, solvats, tautomères et stéréoisomères pharmaceutiquement utilisables de ceux-ci, **caractérisé en ce que**
a) un composé de formule II 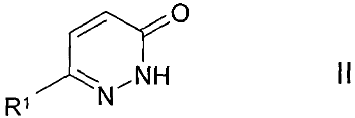 dans lequel R¹ revêt la signification indiquée selon la revendication 1,
est réagi avec un composé de formule III 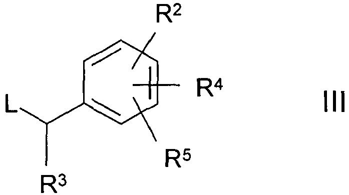 dans laquelle R², R³, R⁴ et R⁵ revêtent les significations indiquées selon la revendication 1 et
L désigne Cl, Br, I ou un groupement OH libre ou modifié de manière réactive et fonctionnelle,
ou
b) un radical R² est converti en un autre radical R² par
i) acylation ou alkylation d'un groupement amino,
ii) cyclisation d'un dérivé d'oxyamidine pour donner un dérivé d'oxadiazole,
iii) cyclisation d'un dérivé d'amide pour donner un dérivé d'oxazole,
iv) réaction d'un dérivé d'alkylester avec un dérivé de N-hydroxyamidine pour donner un dérivé d'oxadiazole,
v) cyclisation d'un dérivé de N-(aminothiocarbonyl)-hydrazide pour donner un dérivé d'oxadiazole,
vi) alkylation d'un groupement SH,
vii) réaction d'un groupement cyano avec un dérivé d'azoture pour donner un dérivé de tétrazole
ou
c) **en ce qu'**il est libéré à partir de l'un de ses dérivés fonctionnels par traitement par un agent de solvolyse ou d'hydrogénolyse,
et/ou
une base ou un acide de formule I est converti(e) en l'un de ses sels.

17. Médicaments comprenant au moins un composé de formule I selon les revendications 1-15 et/ou des sels, solvats, tautomères et stéréoisomères pharmaceutiquement utilisables de celui-ci, y compris des mélanges de ceux-ci en toutes proportions, et éventuellement des excipients et/ou des adjuvants.

18. Utilisation de composés selon les revendications 1-15, et de sels, solvats, tautomères et stéréoisomères pharmaceutiquement utilisables de ceux-ci, y compris des mélanges de ceux-ci en toutes proportions,
pour la préparation d'un médicament destiné au traitement de maladies dans lesquelles l'inhibition, la régulation et/ou la modulation de la transduction des signaux de kinases joue un rôle.

19. Utilisation selon la revendication 18 de composés selon les revendications 1-15, et de sels, solvats et stéréoisomères pharmaceutiquement utilisables de ceux-ci, y compris des mélanges de ceux-ci en toutes proportions,
pour la préparation d'un médicament destiné au traitement de maladies qui sont influencées par l'inhibition de tyrosine kinases par les composés selon les revendications 1-15.

20. Utilisation selon la revendication 18, pour la préparation d'un médicament destiné au traitement de maladies qui sont influencées par l'inhibition de Met kinase par les composés selon les revendications 1-15.

21. Utilisation selon la revendication 19 ou 20, dans laquelle la maladie à traiter est une tumeur solide.

22. Utilisation selon la revendication 21, dans laquelle la tumeur solide provient du groupe constitué par les tumeurs de l'épithélium squameux, de la vessie, de l'estomac, du rein, de la tête et du cou, de l'oesophage, du col de l'utérus, de la thyroïde, des intestins, du foie, du cerveau, de la prostate, du tractus urogénital, du système lymphatique, de l'estomac, du larynx et/ou du poumon.

23. Utilisation selon la revendication 21, dans laquelle la tumeur solide provient du groupe constitué par la leucémie monocytique, l'adénocarcinome du poumon, les carcinomes du poumon à petites cellules, le cancer du pancréas, les glioblastomes et le carcinome du sein.

24. Utilisation selon la revendication 22, dans laquelle la tumeur solide provient du groupe constitué par l'adénocarcinome du poumon, les carcinomes du poumon à petites cellules, le cancer du pancréas, les glioblastomes, le carcinome du côlon et le carcinome du sein.

25. Utilisation selon la revendication 19 ou 20, dans laquelle la maladie à traiter est une tumeur du sang et du système immunitaire.

26. Utilisation selon la revendication 25, dans laquelle la tumeur provient du groupe constitué par la leucémie myéloïde aiguë, la leucémie myéloïde chronique, la leucémie lymphatique aiguë et/ou la leucémie lymphatique chronique.

27. Médicaments comprenant au moins un composé de formule I selon l'une ou plusieurs parmi les revendications 1 à 15, et/ou des sels, solvats et stéréoisomères pharmaceutiquement utilisables de celui-ci, y compris des mélanges de ceux-ci en toutes proportions, et au moins un autre composé actif médicamenteux.

28. Ensemble (kit) constitué de conditionnements séparés
(a) d'une quantité efficace d'un composé de formule I selon l'une ou plusieurs parmi les revendications 1 à 15, et/ou de solvats, sels et stéréoisomères pharmaceutiquement utilisables de celui-ci, y compris des mélanges de ceux-ci en toutes proportions,
et
(b) d'une quantité efficace d'un autre composé actif médicamenteux.
